(19) Europäisches Patentamt
European Patent Office
Office européen des brevets

(11) EP 3 747 437 A1

(12) EUROPEAN PATENT APPLICATION

(43) Date of publication:
**09.12.2020 Bulletin 2020/50**

(21) Application number: **20183625.1**

(22) Date of filing: **24.07.2018**

(51) Int Cl.:
*A61K 31/417* (2006.01) *A61K 39/00* (2006.01)
*A61K 45/00* (2006.01) *A61P 31/00* (2006.01)
*A61P 9/10* (2006.01) *A61P 25/28* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2017 NL 2019333**
**15.05.2018 NL 2020938**

(62) Document number(s) of the earlier application(s) in accordance with Art. 76 EPC:
**18770099.2 / 3 658 141**

(71) Applicants:
- **Stichting Het Nederlands Kanker Instituut-Antoni van Leeuwenhoek Ziekenhuis**
  **1066 CX Amsterdam (NL)**
- **Academisch Ziekenhuis Leiden h.o.d.n. LUMC**
  **2333 ZA Leiden (NL)**

(72) Inventors:
- **RAABEN, Matthijs**
  **1191 PN Ouderkerk aan de Amstel (NL)**
- **SCHEEREN, Ferenc Alexander**
  **2333 ZA Leiden (NL)**
- **LOGTENBERG, Meike Emma Willemijn**
  **1066 CX Amsterdam (NL)**
- **BRUMMELKAMP, Thijn Reinout**
  **1066 CX Amsterdam (NL)**
- **SCHUMACHER, Antonius Nicolaas Maria**
  **1066 CX Amsterdam (NL)**
- **LEUSEN, Jeannette Henrica Wilhelmina**
  **3515 VV Utrecht (NL)**

(74) Representative: **Mewburn Ellis LLP**
**Aurora Building**
**Counterslip**
**Bristol BS1 6BX (GB)**

Remarks:
This application was filed on 02-07-2020 as a divisional application to the application mentioned under INID code 62.

(54) **TREATING PATHOLOGICAL CONDITIONS BY DIRECT AND INDIRECT TARGETING OF SIRPA - CD47 INTERACTION**

(57) The present invention relates to active agents or compounds as well as pharmaceutical compositions comprising said compounds, which are capable of reducing or inhibiting or blocking the enzymatic activity of the glutaminyl-peptide cyclotransferase (QPCT) protein, the glutaminyl-peptide cyclotransferase-like protein (QPCTL) protein, or combinations thereof or are capable of reducing or inhibiting the expression of QPCT gene, the QPCTL gene, or combinations thereof. Also provided are methods for screening or selecting for said compounds. The present invention further relates to a pharmaceutical composition comprising a first active agent for use in a method of treating a condition in a subject that would benefit from reducing the signaling or the binding between SIRPa and CD47 in the subject (e.g. cancer), wherein the method of treating comprises reducing expression or enzymatic activity of QPCTL, QPCT, or combinations thereof in the cell with CD47 on the surface. The compounds and pharmaceutical compositions of the invention may be particularly useful for treating a subject suffering from a disease or condition involving the CD47-SIRPa signaling axis such including e.g., various cancer types, atherosclerosis, fibrotic diseases, and infectious diseases.

EP 3 747 437 A1

## Description

### FIELD OF THE INVENTION

The present invention relates to the fields of medicine and immunity, particularly to the field of CD47-SIRPα signaling axis. Specifically, the present invention relates to active agents or compounds as well as pharmaceutical compositions comprising said compounds, which are capable of reducing or inhibiting or blocking the enzymatic activity of the glutaminyl-peptide cyclotransferase (QPCT) protein and/or glutaminyl-peptide cyclotransferase-like protein (QPCTL) protein or the expression of QPCT gene and/or QPCTL gene. Also provided are methods for screening or selecting for said compounds. The present invention further relates to a pharmaceutical composition comprising a first active agent (e.g. drug such as an anti-CD47 antibody or an anti-PD-L1 antibody) for use in a method of treating a condition in a subject that would benefit from reducing signaling or binding between SIRPα and CD47 in the subject (e.g. cancer), wherein the method of treating comprises reducing expression or enzymatic activity of QPCTL and/or QPCT in the cell with CD47 on the surface (e.g. by using the compounds as taught herein (QPCT inhibitors and/or QPCTL inhibitors)). The compounds and pharmaceutical compositions of the invention may be particularly useful for treating a subject suffering from a disease or condition involving the CD47-SIRPα signaling axis such including e.g., various cancer types, atherosclerosis, fibrotic diseases, and infectious diseases.

### BACKGROUND OF THE INVENTION

Cancer is a leading cause of death worldwide, accounting for more than 8.8 million deaths in 2015. Several therapies to treat and/or cure cancer have been developed over the years including e.g., chemotherapy, radiation, surgery, and cancer immunotherapy.

Cancer immunotherapy represents a type of cancer treatment designed to boost the body's natural immune defenses to fight cancer. Overall, the purpose of cancer immunotherapy is to promote the ability of the immune system, including the innate immune system, to specifically detect and destroy cancer cells (e.g. via phagocytosis) while leaving healthy cells unaffected.

However, cancer cells are able to evade immune surveillance in many ways, for instance by evading phagocytosis by phagocyte cells (e.g. macrophages or neutrophils) through the expression of so-called "anti-phagocytic" or "don't eat me" signals. One prominent example of such signal is the transmembrane protein "cluster of differentiation 47" (abbreviated as "CD47"). CD47 is expressed by virtually all cells in the body and is involved in a range of cellular processes, including apoptosis, proliferation, adhesion, and migration as well as angiogenic and immune responses. CD47 binds or interact with several ligands with signal-regulatory protein alpha (SIRPα) being considered as a main ligand for CD47. SIRPα is an inhibitory transmembrane receptor present on myeloid cells such as macrophages, monocytes, neutrophils, basophils, eosinophils, erythrocytes, and dendritic cells. The interaction between CD47 and SIRPα mediates or conveys "anti-phagocytic" or "don't eat me" signals between two cells, which ultimately inhibit phagocytosis.

In the case of cancer, it was found that cancer cells upregulate the expression of CD47 at their cell surface compared to the CD47 levels found in normal/healthy cells. As a result, cancer cells can evade destruction by the immune system or evade immune surveillance, e.g. by evading phagocytosis by immune cells such as phagocyte cells (e.g. macrophages, neutrophils). This phenomenon is not limited to cancer. It was also found that diseased cells in conditions other than cancer, such as e.g. atherosclerosis, fibrotic diseases as well as infectious diseases caused by pathogens (e.g. virus), also upregulate the expression of CD47 at their cell surface compared to the CD47 levels found in normal/healthy cells to evade phagocytosis by phagocytes.

In the case of cancer, current approaches to antagonize the CD47-SIRPα interactions have principally targeted CD47. For instance, several anti-CD47 antibodies aimed at interfering or blocking CD47-SIRPα interactions are currently being developed or tested in clinical trials. Although promising, such strategies are not optimal since antibodies are known to have poor tissue penetration, especially into solid tumors due to their large molecular weight. Further, such antibodies, particularly antibodies targeting CD47 lack specificity since CD47 is widely distributed throughout the body, including healthy tissue, which may cause on-target toxicity to normal cells. Other disadvantages associated with the use of anti-CD47 antibodies include the lack of oral bioavailability and undesirable side effects such as the development of anemia (which may occur as a result of a dose-dependent loss of red blood cells and platelets) as well as hemagglutination (clumping of red blood cells).

Therefore there is a need for CD47-targeting therapies that do not cause significant levels toxicity, and/or platelet depletion and/or hemagglutination (clumping of red blood cells together) and/or red blood cell depletion, and/or anemia when administered to a subject and/ or that have the potential of oral bioavailability. There is also a need for methods for screening or selecting for such compounds, as well as methods for identifying subjects susceptible from benefiting from treatment with an effective amount of said compounds.

## SUMMARY OF THE INVENTION

Described herein are methods and compositions for reducing binding between CD47 and SIRPα by reducing expression or enzymatic activity of glutaminyl-peptide cyclotransferase (QPCT) as well as its related isoenzyme, the glutaminyl-peptide cyclotransferase-like (QPCTL), or combinations thereof. In some embodiments, the reduction of binding between CD47 and SIRPα results in an inhibition or reduction of the CD47-SIRPα signaling axis.

In one aspect, compositions disclosed herein comprise an active agent for use in a method of treating a condition in a subject that would benefit from reducing binding between CD47 on the surface of a first cell and SIRPα on the surface of a second cell in the subject, wherein the active agent reduces expression or enzymatic activity of glutaminyl-peptide cyclotransferase (QPCT) as well as its related isoenzyme, the glutaminyl-peptide cyclotransferase-like (QPCTL), or combinations thereof, in said first cell with CD47 on the surface.

In some embodiments reducing expression or enzymatic activity of QPCT, QPCTL, or combinations thereof, comprises reducing the transcription, translation or combinations thereof of the genes encoding QPCT, QPCTL, or combinations thereof.

In some embodiments, the composition disclosed herein comprises a double stranded RNA molecule, a small inhibitory RNA (siRNA) molecule, an inhibitory RNA (RNAi) molecule, or combinations thereof designed to reduce expression of QPCT, QPCTL, or combinations thereof. In some embodiments, the composition disclosed herein comprises an inhibitor of QPCT, QPCTL, or combinations thereof.

Also disclosed herein, are compositions comprising a CD47 inhibitor (e.g. a CD47 antibody, or a CD47 IgA antibody) for use in a method of treating a condition in a subject, wherein the subject would benefit from reducing binding between CD47 on the surface of a first cell and SIRPα on the surface of a second cell, and wherein the CD47 inhibitor is an inhibitor that binds said CD47 on the surface of said first cell and thereby reduces the binding of said CD47 to said SIRPα on the surface of said second cell, and wherein the method of treating comprises reducing expression or enzymatic activity of QPCTL, QPCT, or combinations thereof in said first cell. In some embodiments, the CD47 inhibitor is an antibody.

Also disclosed herein, are compositions comprising a SIRPα inhibitor (e.g. a SIRPα (or "SIRPa" or "SIRP alpha") antibody) for use in a method of treating a condition in a subject, wherein the subject would benefit from reducing binding between CD47 on the surface of a first cell (e.g. a diseased cell such as a cancer cell) and SIRPα on the surface of a second cell (e.g. macrophages, monocytes, neutrophils, basophils, eosinophils, dendritic cells), and wherein the SIRPα inhibitor is an inhibitor that binds said SIRPα on the surface of said second cell and thereby reduces the binding of said SIRPα to said CD47 on the surface of said first cell, and wherein the method of treating further comprises reducing expression or enzymatic activity of QPCTL, QPCT, or combinations thereof in said first cell. In some embodiments, the SIRPα inhibitor is an antibody.

In some embodiments, the condition that would benefit from reducing binding between CD47 on the surface of a first cell and SIRPα on the surface of a second cell in the subject is selected from the group consisting of cancer, atherosclerosis, fibrotic disease, and infectious disease.

In some embodiments, reducing binding between CD47 and SIRPα targets the cell expressing CD47 for phagocytosis or targets cells expressing CD47 for antibody-dependent cellular cytotoxicity (ADCC) or targets cells expressing CD47 for antibody-dependent cellular phagocytosis (abbreviated ADCP).

Also disclosed herein are in vitro methods for selecting or screening for active agents that reduce binding between CD47 on the surface of a first cell and SIRPα on the surface of a second cell, the method comprising screening for active agents that reduce expression or enzymatic activity of QPCTL, QPCT, or combinations thereof.

Also disclosed herein is the use of an inhibitor of QPCTL, QPCT, or combinations thereof for reducing binding between CD47 on the surface of a first cell and SIRPα on the surface of a second cell in a subject.

## BRIEF DESCRIPTION OF THE DRAWINGS

**Figure 1.** Figure 1 depicts a flow-based genetic haploid screen for CD47 levels in HAP1 cells. Flow cytometry-based screen for modulators of CD47 cell surface expression in HAP1 cells. Dots represent individual genes, X axis indicates the total number of gene-trap insertion sites per gene, Y axis indicates the frequency of independent gene-trap insertion events in the CD47HIGH channel divided by the frequency of insertion events in the respective gene in the CD47LOW channel (mutation index, MI). The dots indicate those genes that are significantly enriched (PDR-corrected P-value) within the CD47HIGH (upper part of the graph) and CD47LOW (lower part of the graph) population, respectively. QPCTL (bold) and CD47 are examples of genes enriched in the CD47LOW population.

**Figure 2A.** Figure 2A depicts relative median fluorescence intensity (MFI) changes, as assessed by flow cytometry, of CD47 levels in HAP 1 cells (WT, CD47 KO cl23, QPCTLKO cl10 and QPCTLKO cl21) after immunohistological

staining with various anti-CD47 antibodies including clone CC2C6, clone B6H12, and clone 2D3. "Unstained" refers to HAP 1 cells (WT) which did not undergo immunohistological staining with various anti-CD47 antibodies including clone CC2C6, clone B6H12 and clone 2D3.

**Figure 2B.** Figure 2B depicts relative median fluorescence intensity (MFI) changes, as assessed by flow cytometry, of SIRPα-Fc binding in HAP1 cells (wild type (WT), CD47 KO cl23, QPCTLKO cl10 and QPCTLKO cl21). "Unstained" refers to HAP 1 cells (WT), which did not undergo SIRPα-Fc binding.

**Figure 3A.** Figure 3A depicts relative median fluorescence intensity (MFI) changes, as assessed by flow cytometry, of CD47 levels in A375 cells (WT, CD47 KO cl2, and QPCTLKO cl4.1) after immunohistological staining with various anti-CD47 antibodies including clone CC2C6, clone B6H12 and clone 2D3. "Unstained" refers to A375 cells (WT), which did not undergo immunohistological staining with various anti-CD47 antibodies including clone CC2C6, clone B6H12.2 and clone 2D3. A375 cells refer to a human melanoma cell line.

**Figure 3B.** Figure 3B depicts relative median fluorescence intensity (MFI) changes, as assessed by flow cytometry, of SIRPα-Fc binding in A375 cells (WT, CD47KO cl2, and QPCTLKO cl4.1). "Unstained" refers to A375 cells (WT), which did not undergo SIRPα-Fc binding. A375 cells refer to a human melanoma cell line.

**Figure 4A.** Figure 4A depicts relative median fluorescence intensity (MFI) changes, as assessed by flow cytometry, of CD47 levels in RKO cells (WT, CD47KO cl12, and QPCTLKO cl1) after immunohistological staining with various anti-CD47 antibodies including clone CC2C6and clone 2D3. "Unstained" refers to RKO cells (WT), which did not undergo immunohistological staining with various anti-CD47 antibodies including clone CC2C6 and clone 2D3. RKO cells further refer to a human rectal carcinoma cell line.

**Figure 4B.** Figure 4B depicts relative median fluorescence intensity (MFI) changes, as assessed by flow cytometry, of SIRPα-Fc binding in RKO cells (WT, CD47KO cl2, and QPCTLKO cl4.6). "Unstained" refers to RKO cells (WT), which did not undergo SIRPα-Fc binding. RKO cells further refer to a human rectal carcinoma cell line.

**Figure 5.** Figure 5 depicts relative median fluorescence intensity (MFI) changes, as assessed by flow cytometry, of SIRPα-Fc binding in HAP1 WT cells (subjected to immunohistochemically staining with anti-CD47 antibodies clone CC2C6 or clone 2D3 prior binding with SIRPα-Fc). "Unstained" refers to HAP1 WT cells, which did not undergo SIRPα-Fc binding and did not undergo immunohistochemical staining with CD47 antibodies clone CC2C6 or clone 2D3. "No ab" refers to HAP1 WT cells which did not undergo immunohistochemistry with anti-CD47 antibodies clone CC2C6 or clone 2D3 but which underwent SIRPα-Fc binding.

**Figure 6.** Figure 6 depicts relative median fluorescence intensity (MFI) changes, as assessed by flow cytometry, of CD47 levels in HAP1 QPCTL KO cells (cl10 and cl21) that were either untransduced (UT) or transduced with QPCTL transcript variant 1 ("QPCTL(1)") or QPCTL transcript variant 2 ("QPCLT(2)") after immunohistochemical staining with anti-CD47 antibodies clone CC2C6 and clone 2D3, as well as after binding to SIRPα-Fc. "WT UT" refers to HAP 1 WT cells not transduced with QPCTL transcript QPCTL(1) or QPCLT(2).

**Figure 7.** Figure 7 depicts relative median fluorescence intensity (MFI) changes, as assessed by flow cytometry, of CD47 levels in HAP1 CD47 KO cells (cl4, cl17 and cl23) that were either untransduced (UT) or transduced with CD47 wild-type ("CD47 WT") transcript or a CD47 mutant transcript ("CD47 MUT") that cannot be modified by QPCTL, after immunohistochemical staining with CD47 antibodies clone CC2C6 and clone 2D3, as well as after binding to SIRPα-Fc.

**Figure 8 A.** Figure 8A depicts median fluorescence intensity (MFI) changes, as assessed by flow cytometry, of CD47 levels in HAP1 cells treated for 48 hours with a vehicle or PBD150 1000 μM or PBD150 100 μM or isotype control, followed by immunohistochemical staining with anti-CD47 antibody clone CC2C6.

**Figure 8 B.** Figure 8B depicts median fluorescence intensity (MFI) changes, as assessed by flow cytometry, of CD47 levels in HAP1 cells treated for 120 hours with vehicle, or 72 hours with PBD150 1000 μM followed by 48 hours with PBD150 1000 μM or 72 hours with PBD150 1000 μM followed by 48 hours without PBD150 or 120 hours with isotype control, followed by immunohistochemical staining with anti-CD47 antibody clone CC2C6.

**Figure 9.** Figure 9 depicts median fluorescence intensity (MFI) changes, as assessed by flow cytometry, of CD47 levels in A375 cells, A549 cells, DLD1 cells, HAP1 cells and RKO cells treated for 72 hours with vehicle (DMSO)

or 72 hours with PBD150 1000 μM, where DMSO or PBD150 was freshly added every 24 hours, followed by staining with CD47 antibody clone CC2C6, 2D3 or SIRPα-Fc.

**Figure 10.** Identification of QPCTL as a modulator of CD47-SIRPα binding.

(A) Flow-cytometry-based haploid genetic screen for modulators of CD47, as detected by anti-CD47 antibody clone CC2C6 (αCD47-CC2C6) binding. Dots represent individual genes; x axis indicates the number of disruptive insertions per gene; *y* axis shows the frequency of independent insertions in cells with high CD47 expression ($CD47\text{-}CC2C6^{HIGH}$ channel) over the frequency of insertions in cells with low CD47 expression ($CD47\text{-}CC2C6^{LOW}$ channel) for each gene. Light-blue and orange dots indicate genes with significant enrichment of insertions (FDR-corrected $P < 0.05$) within the $CD47\text{-}CC2C6^{HIGH}$ and $CD47\text{-}CC2C6^{LOW}$ populations, respectively. Green dots represent *CD47* and *QPCTL.*
(B) Flow cytometry plot of surface binding of anti-CD47 antibody clone B6H12 (αCD47-B6H12) and αCD47-CC2C6 to HAP1 WT, CD47 KO and QPCTL KO (cl21) cells.
(C) Cell surface binding of anti-CD47 antibody clone 2D3 (αCD47-2D3), αCD47-B6H12 and αCD47-CC2C6 to HAP1 WT, CD47 KO or QPCTL KO cells, as determined by flow cytometry. Values indicate MFI relative to WT cells stained with the same reagent.
(D) Cell surface binding of human SIRPα-Fc (hSIRPα-Fc) to HAP1 WT, CD47 KO or QPCTL KO cells (cl10 and cl21), as determined by flow cytometry. Values indicate MFI relative to WT cells.
(E) Cell surface binding of αCD47-2D3, αCD47-B6H12, αCD47-CC2C6 and hSIRPα-Fc to WT, CD47 KO and QPCTL KO ("cl4.1" and "cl4.6") A375 melanoma cells and to WT, CD47 KO and QPCTL KO (cl6) A431 epidermoid carcinoma cells, as determined by flow cytometry. Values indicate MFI relative to WT cells stained with the same reagent. Data are representative of one (A), or at least two (B, C, D, E) independent experiments, and were analyzed by unpaired *t*-test (C, D, E). Data represent mean ± s.d. of triplicates. ***$P$<0.001.
MI, mutation index; MFI, mean fluorescence intensity; WT, wild-type; KO, knock-out.

**Figure 11.** Pyroglutamate formation occurs early in the CD47 protein life-cycle and is fully dependent on QPCTL. (A) SDS-PAGE analysis of αCD47-B6H12 (B) or αCD47-CC2C6 (C) immunoprecipitates from CD47-HA-overexpressing WT or QPCTL KO (cl4.1) A375 melanoma cells after a 0, 1, 2 or 4 hours (h) chase period following a 30' $^{35}S$ methionine/cysteine labelling. (B) SDS-PAGE analysis of αCD47-B6H12 (B) or αCD47-CC2C6 (C) immunoprecipitates from CD47-HA-overexpressing WT or QPCTL KO (cl4.1) A375 melanoma after a 0 or 30' chase following a 10' $^{35}S$ methionine/cysteine labelling. OE, over expression; B, αCD47-B6H12; C, αCD47-CC2C6.

**Figure 12.** Inhibition of QPCTL by small molecules affects binding of SIRPα to CD47

(A) Cell surface binding of αCD47-2D3, αCD47-CC2C6 and hSIRPα-Fc to control (DMSO)-treated (-) or SEN177-treated (+) melanoma (A375), epidermoid carcinoma (A431) and Burkitt's lymphoma (Raji) cells, as determined by flow cytometry. Values indicate MFI relative to WT cells stained with the same reagent.
(B) Flow cytometry plot of surface binding of αCD47-B6H12 and αCD47-CC2C6 to control-treated or SEN177-treated melanoma (A375) cells.
(C) Isoelectric focusing analysis of αCD47-B6H12 immunoprecipitates from CD47-HA-overexpressing WT, CD47-HA-overexpressing QPCTL KO, or CD47 KO melanoma (A375) cells left untreated (-) or treated with SEN177 (+).
(D) SDS-PAGE analysis of αCD47-B6H12 (B) or αCD47-CC2C6 (C) immunoprecipitates from CD47-HA-overexpressing WT, QPCTL KO (cl4.1), or CD47 KO melanoma (A375) cells after a 30' $^{35}S$ methionine/cysteine labelling in the presence (+) or absence (-) of SEN177.
Data are representative of at least two independent experiments. Data were analyzed by unpaired two-tailed *t*-test (A). Data represent mean ± s.d. of triplicates (A). *$P$<0.05; **$P$ <0.01; ***$P$<0.001.

**Figure 13.** Increased control of QPCTL deficient tumors by innate and adaptive immune cells.

(A) Specific lysis of WT, CD47 KO and QPCTL KO Her2-expressing murine pro-B cells (Ba/F3-Her2) by human neutrophils in the presence or absence of anti-Her2 IgA1 in a 4h $^{51}Cr$-release assay.
(B) Specific lysis of control (DMSO)-treated (-) or SEN177-treated (+) Her2-expressing murine pro-B (Ba/F3-Her2) by human neutrophils in the presence or absence of anti-Her2 IgA1 in a 4h $^{51}Cr$-release assay.
(C) In vivo killing of target cells in mice injected with a 1:1 mixture of WT and QPCTL KO Her2-expressing murine pro-B cells (Ba/F3-Her2) and treated with control (PBS) or anti-Her2 IgA1 antibody. Data represent the ratio between QPCTL KO and WT Ba/F3-Her2 in mice treated with PBS (dots) or anti-Her2 IgA1 (squares). *n*

= 6 animals per group.

(D) Representative flow analysis plots of (C) of recovered WT and QPCTL KO tumor cells in mice treated with control (PBS) or anti-Her2 IgA1.

(E) Number of peritoneal PMNs (Ly-6G$^+$CD11b$^+$), eosinophils (SSC-$^{HIGH}$Siglec-F$^+$) and macrophages (F4/80$^+$CD11b$^+$) present in recipients of a 1:1 mixture of WT and QPCTL KO Her2-expressing murine pro-B cells that were either treated with PBS (-) or with anti-Her2 (+) IgA1 antibody, 16 hours after treatment.

Data were analyzed by unpaired two-tailed *t*-test (A, B, C, E). Data represent mean ± s.d. of triplicates (A, B) of one representative donor, or mean ± s.d. of independent mice (E, F).

**P*<0.05; ***P* <0.01; ****P*<0.001.

**Figure 14.** Cell surface binding of αCD47-2D3, αCD47-B6H12, αCD47-CC2C6 and hSIRPα-Fc to WT, CD47 or QPCTL KO lung cancer (A549) (A), colorectal cancer (DLD1) (B) and rectal carcinoma (RKO) (C) cells as determined by flow cytometry. Values indicate MFI relative to WT cells stained with the same reagent.

MFI, mean fluorescence intensity; WT, wild-type; KO, knock-out.

**Figure 15.** Cell surface binding of αCD47-2D3 and αCD47-CC2C6 to melanoma (A375) (A), epidermoid carcinoma (A431) (B) and lung cancer (A549) (C) WT, QPCTL KO or QPCTL KO cells reconstituted with FLAG-tagged cDNA of QPCTL isoform 1 (OE var.1) or QPCTL isoform 2 (OE var.2), as determined by flow cytometry. (D) Western blot analysis of melanoma (A375) WT, QPCTL KO or QPCTL KO cells reconstituted with FLAG-tagged cDNA of QPCTL isoform 1 (OE var.1) or QPCTL isoform 2 (OE var.2). (E) Cell surface binding of αCD47-CC2C6 to HAP1 QPCTL KO cells reconstituted with QPCTL var.1 or a catalytically inactive QPCTL variant (QPCLT var.1 D326E), as determined by flow cytometry. (F) Cell surface binding of αCD47-CC2C6 to melanoma (A375) QPCTL KO cells reconstituted with QPCTL var.1 or QPCTL var.1 (D326E), as determined by flow cytometry. Values in A-C, E, F indicate MFI relative to WT cells stained with the same reagent. OE, over-expression.

**Figure 16.** (A) Cell surface binding of αCD47-2D3, αCD47-CC2C6 and hSIRPα-Fc to control (DMSO)-treated (-) or SEN177-treated (+) lung cancer (A549), colorectal (DLD1), HAP1, rectal carcinoma (RKO) and breast cancer (SKBR3) cells, as determined by flow cytometry. (B) Cell surface binding of αCD47-2D3, αCD47-CC2C6 and hSIRPα-Fc to control (DMSO)-treated (-), SEN177-treated, or PQ912-treated melanoma (A375) cells, as determined by flow cytometry. (C) Flow cytometry plot of surface binding of anti-CD47 antibody clone B6H12 (αCD47-B6H12) and αCD47-CC2C6 to control-treated or PQ912-treated melanoma (A375) cells. (D) Cell surface binding of αCD47-2D3, αCD47-CC2C6 and hSIRPα-Fc to control (DMSO)-treated (-) or SEN177-treated (+) wild-type and QPCTL-knockout epidermoid carcinoma (A431) and lung cancer (A549) cells, as determined by flow cytometry. Values in A, B, D indicate MFI relative to WT cells stained with the same reagent.

**Figure 17.** (A) Cell surface binding of anti-mouse CD47 antibody MIAP301 (αmCD47-MIAP301) and mouse SIRPα-Fc (mSIRPα-Fc) to WT, CD47 KO and QPCTL bulk KO populations (KO#1 and KO#2) murine melanoma (B16F10) cells, and WT, CD47 KO and QPCTL KO (cl8 and cl30) Her2-expressing mouse pro-B (Ba/F3-Her2) cells, as determined by flow cytometry. (B) Cell surface binding of αmCD47-MIAP301 and mSIRPα-Fc to murine melanoma (B16F10) WT, QPCTL KO or QPCTL KO cells reconstituted with the murine QPCTL cDNA (OE), as determined by flow cytometry. (C) Cell surface binding of αmCD47-MIAP301 and mSIRPα-Fc to control (DMSO)-treated (-) or SEN177-treated (+) murine melanoma (B16F10) or Her2-expressing murine pro-B (Ba/F3-Her2) cells, as determined by flow cytometry. (D) Specific lysis of control (DMSO)-treated (-) or SEN177-treated (+) CD47 KO or QPCTL KO murine pro-B cells (Ba/F3-Her2) by human neutrophils in the presence of anti-Her2 IgA1 in a 4h $^{51}$Cr-release assay. (E) Specific lysis of WT, CD47 KO or QPCTL KO murine pro-B cells (Ba/F3-Her2) by murine immune cells isolated from whole blood in the presence or absence of anti-Her2 IgA1 in a 4h $^{51}$Cr-release assay. (F) Specific lysis of control (DMSO)-treated (-) or SEN177-treated (+) murine pro-B cells (Ba/F3-Her2) by murine immune cells isolated from whole blood in the presence or absence of anti-Her2 IgA1 in a 4h $^{51}$Cr-release assay. Values in A-C indicate MFI relative to WT cells stained with the same reagent.

Data are representative of at least two independent experiments (D-F). Data were analyzed by unpaired *t*-test (A, C, F) or one-way paired ANOVA with repeated measures, multiple comparison (E) at 10μg/mL anti-Her2 IgA1 (E, F) and represent ± s.d. of triplicates (A - F). **P*<0.05; ***P* <0.01; ****P*<0.001.

**Figure 18.** (A) Schematic representation of in vivo set-up. (B) Absolute number (see Fig. 13C) of recovered tumor cells in mice injected with 1:1 mixtures of WT and QPCTL KO Ba/F3-Her2 cells that were treated with PBS (-) or anti-Her2 IgA1 (+). (C) Number (see Fig. 13C and 13D) of CD8 T (CD3$^+$ CD8$^+$), CD4 T (CD3$^+$ CD4$^+$) or B (B220$^+$ MHCII$^+$) cells present in mice that received a 1:1 mixture of WT and QPCTL KO Ba/F3-Her2 cells, and that were either control-treated (-) or treated with anti-Her2 IgA1 (+). (D) Ratio of in vivo killing of target cells in mice injected

with a 1:1 mixture of WT and CD47-KO cells, or a 1:1 mixture of WT and QPCTL-KO Ba/F3-Her2 cells, and that were either treated with PBS (-) or anti-Her2 IgA1 antibody (+). Dots represent mice treated with control (PBS), squares represent mice treated with anti-Her2 IgA1. *n* = 5-6 animals per group. (E) Absolute number (see Fig. 18D) of recovered tumor cells in mice injected with a 1:1 mixture of WT and CD47-KO cells, or a 1:1 mixture of WT and QPCTL-KO Ba/F3-Her2 cells, and that were either treated with PBS (-) or anti-Her2 IgA1 antibody (+). Dots represent mice treated with control (PBS), squares represent mice treated with anti-Her2 IgA1. (F) Absolute number (see Fig. 18D) of peritoneal PMNs (Ly-$6G^+$/$CD11b^+$), eosinophils ($SSC^{HIGH}$/Siglec-$F^+$), macrophages ($F4/80^+$ $CD11b^+$), CD8 T ($CD3^+$/$CD8^+$), CD4 T ($CD3^+$/$CD4^+$) or B ($B220^+$/$MHCII^+$ cells present in recipients of a 1:1 mixture of WT and QPCTL KO Ba/F3-Her2 cells that were control-treated (-) or treated with anti-Her2 IgA1 (+), 16 hours after treatment. Dots represent mice treated with control (PBS), squares represent mice treated with anti-Her2 IgA1. Data are representative of two independent (B, C) or one (D, E, F) experiment. Data were analyzed by unpaired *t*-test (B, C, D), one-way ANOVA followed by multiple comparisons testing (E, F) and represent ± s.d. of individual mice (B-F). *$P$<0.05; **$P$ <0.01; ***$P$<0.001.

**Figure 19.** Normalized mean fluorescence intensity is depicted for HAP1 (a,e), A375 (b,c) or RKO (d) cells incubated for 48 hours with PQ912 (a-d) or SEN-177 (e) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a,b,d,e) or incubated with SIRPα-Human Fc recombinant protein and subsequently stained with APC-conjugated Rabbit-anti-human secondary antibody (black bars) (c).

**Figure 20.** Normalized median fluorescence intensity is depicted for HAP1 (a-h), A375 (i-m) or RKO (n) cells incubated for 48 hours with compounds 000016 (a,h,k,n), 000035 (b,i,l), 000037 (c,j,m), 000034 (d), 000051 (e), 000054 (f) or 000055 (g) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a-j,n) or incubated with SIRPα-Human Fc recombinant protein and subsequently stained with APC-conjugated Rabbit-anti-human secondary antibody (black bars) (k-m).

**Figure 21.** Normalized median fluorescence intensity is depicted for HAP1 (a-c), A375 (d,e) or RKO (f) cells incubated for 48 hours with compounds 000024 (a,d,e,f), 000027 (b) or 000050 (c) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a-d,f) or incubated with SIRPα-Human Fc recombinant protein and subsequently stained with APC-conjugated Rabbit-anti-human secondary antibody (black bars) (e).

**Figure 22.** Normalized median fluorescence intensity is depicted for HAP1 (a-i), A375 (j-o) or RKO (p) cells incubated for 48 hours with compounds 000011 (a,j,m,p), 000010 (b,k,n), 000036 (c,l,o), 000020 (d), 000021 (e), 000022 (f), 000023 (g), 000025 (h) or 000026 (i) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a-l,p) or incubated with SIRPα-Human Fc recombinant protein and subsequently stained with APC-conjugated Rabbit-anti-human secondary antibody (black bars) (m-o).

**Figure 23.** Normalized median fluorescence intensity is depicted for HAP1 (a-k), A375 (In) or RKO (o,p) cells incubated for 48 hours with compounds 000012 (a,l,n,o), 000030 (b,m,p), 000013 (c), 000014 (d), 000029 (e), 000031 (f), 000032 (g), 000048 (h), 000049 (i), 000052 (j) or 000053 (k) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a-m,o,p) or incubated with SIRPα-Human Fc recombinant protein and subsequently stained with APC-conjugated Rabbit-anti-human secondary antibody (black bars) (n).

**Figure 24.** Normalized median fluorescence intensity is depicted for HAP1 (a-d), A375 (e,f) or RKO (g) cells incubated for 48 hours with compounds 000044 (a,e,f,g), 000060 (b), 000064 (c), or 000066 (d) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars) (a-e,g) or incubated with SIRPα-Human Fc recombinant protein and subsequently stained with APC-conjugated Rabbit-anti-human secondary antibody (black bars) (f).

**Figure 25.** Normalized median fluorescence intensity is depicted for HAP1 cells incubated for 48 hours with compounds 000015 (a), 000033 (b), 000046 (c) or 000040 (d) and stained with FITC-conjugated anti-human CD47 clone 2D3, recognizing total CD47 (gray bars) and Alexa647-conjugated anti-human CD47 clone CC2C6, recognizing pyroglutamylated CD47 (black bars).

**Figure 26.** Normalized isoQC activity compared to control in the presence of indicated compounds, tested at the maximum concentration indicated between brackets (white bars) and ten- and hundredfold lower concentrations (gray and black bars, respectively).

**Figure 27.** Normalized pGAPase activity compared to control in the presence of indicated compounds, tested at the maximum concentration indicated between brackets (white bars) and ten- and hundredfold lower concentrations (gray and black bars, respectively).

**Figure 28.** Cell surface binding of hSIRPα-Fc and αCD47-CC2C6 both recognizing pyroglutamylated CD47 and αCD47-2D3 (recognizing pan-CD47 specific) for six short-term cultures of melanoma xenografts treated with SEN177.

## DETAILED DESCRIPTION

### Definitions

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Any methods and materials similar or equivalent to those described herein can be used in the practice for testing of the present invention. For purposes of the present invention, the following terms are defined below.

As used herein, the singular forms "a," "an" and "the" include plural referents unless the context clearly dictates otherwise. For example, a method for administrating a drug includes the administrating of a plurality of molecules (e.g. 10’s, 100’s, 1000’s, 10’s of thousands, 100’s of thousands, millions, or more molecules).

As used herein, the term "and/or" indicates that one or more of the stated cases may occur, alone or in combination with at least one of the stated cases, up to with all of the stated cases.

As used herein, "to comprise" and its conjugations is used in its non-limiting sense to mean that items following the word are included, but items not specifically mentioned are not excluded. It also encompasses the more limiting "to consist of."

The term "about" and "approximately" as used herein refer to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The term "conventional techniques" or "methods known to the skilled person" as used herein refers to a situation wherein the methods of carrying out the conventional techniques used in methods of the invention will be evident to the skilled worker. The practice of conventional techniques in molecular biology, biochemistry, cell culture, genomics, sequencing, drug screening, and related fields are well-known to those skilled in the art.

The term "diseased cells" as used herein refers to a cell which is found in a diseased individual (suffering from a disease or pathological condition, e.g. cancer) and which is abnormal in terms of its structure and/or functioning and/or metabolism and/or genome compared to a cell having a structure, function, metabolism, and genome that are characteristic of a cell found in a healthy individual (not suffering from a disease or condition, e.g. cancer). In the context of the present invention, non-limiting examples of diseased cells include cancer or tumor cells (e.g. in the case of cancer), diseased vascular smooth muscle cells and diseased endothelial cells (e.g. in the case of atherosclerosis), diseased cells infected by a pathogen such as a virus (e.g. in the case of infectious diseases), diseased cells undergoing fibrosis (e.g. in the case of fibrotic diseases), and others. It is further understood that the phenotype, physical aspects or characteristics of the diseased cells will vary depending on the disease or condition (e.g. cancer, atherosclerosis, fibrotic disease and infectious disease, etc.). For instance, in the case of cancer, diseased cells divide relentlessly, forming solid tumors or flooding the blood with abnormal cells (e.g. expressing specific markers at their cell surface, having an altered morphology, altered cell cycle, altered genome, etc., which are distinct from (healthy) cells derived from a non-diseased or healthy individuals (e.g. not suffering from cancer)). The skilled person knows how to distinguish, using standard techniques and knowledge (e.g. using disease-specific markers), a diseased cell from a non-diseased or healthy cell depending on the diseases or conditions, e.g. cancer, atherosclerosis, fibrotic disease and infectious disease, etc., including various cancer types, fibrosis disease type as well as infectious disease types. In addition to the presence of disease-specific markers, the diseased cells also express or overexpress CD47 (although overexpression is not necessary for detecting a diseased cell according to the present invention) at its surface. /pct

The term "don’t eat me signal" or "anti-phagocytic signal" as used herein is a term commonly used in immunology to refer to a signal (e.g. molecular or chemical signal(s)) that impedes or interferes or prevents or reduces the action of

phagocytes (e.g. macrophages, neutrophils) towards a given cell or substances or material, e.g. reducing or preventing or blocking or inhibiting phagocytosis.

The term "small molecule" as used herein refers to a term commonly used in molecular biology and pharmacology for referring to an organic compound having a low molecular weight (< 900 daltons) with a size on the order of 1 nm. Small molecules are actively sought after for their ability to regulate biological processes, which explains why most drugs are small molecules. Because of their upper molecular-weight limit of approximately 900 daltons, small molecules can rapidly diffuse across cell membranes to reach intracellular sites of action (e.g. Golgi). Although not essential, a lower molecular-weight limit of approximately 500 daltons is often recommended for small molecule drug development candidates based on the observation that clinical attrition rates are significantly reduced if the molecular weight is kept below this 500 dalton limit. Small molecules are selected or categorized based on ability to bind to a specific biological target, such as a specific protein (e.g. QPCTL or QPCT protein) or nucleic acid (e.g. QPCTL or QPCT gene), and for their ability to act as an effector (e.g. activating or inhibiting) for altering the activity or function of the target (e.g. blocking or reducing enzymatic activity, prevent binding to a target, prevent posttranslational modification of a target, etc.). Small molecules may be natural (such as secondary metabolites) or artificial (e.g. drugs); they may have a beneficial effect against a disease (e.g. drugs for treatment of cancer) or may be detrimental (e.g. teratogens and carcinogens). Very small oligomers may also be considered small molecules, such as dinucleotides, peptides such as the antioxidant glutathione, and disaccharides such as sucrose. Small molecules may also be used as research tools to probe biological function as well as leads in the development of new therapeutic agents. Some can inhibit a specific function of a multifunctional protein or disrupt protein-protein interactions (e.g. block the interaction or binding between CD47 and SIRPα), etc. In the present invention, the small molecule may be an enzyme inhibitor, i.e. a molecule that binds to an enzyme and decreases its activity.

The term "biological sample" or "sample from a subject" or "biopsy" as used herein encompasses a variety of sample types (for instance biological cancer sample) obtained from an organism or a subject and which can be used in a diagnostic or monitoring assay or screening assays as taught herein. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

The terms "treatment", "treating", "treat" and the like as used herein, generally refer to obtaining a desired pharmacologic and/or physiologic effect (e.g. reduction of tumor size or cancer remission). The effect may be prophylactic in terms of completely or partially preventing a disease (e.g. a certain cancer) or symptom thereof and/or may be therapeutic in terms of a partial or complete stabilization or cure for a disease (e.g. cancer comprising cells positive for CD47 or involving the CD47-SIRPα signaling axis) and/or adverse effect attributable to the disease. "Treatment" as used herein also covers any treatment of a disease (e.g. cancer such as a cancer comprising cells positive for CD47 or involving the CD47-SIRPα signaling axis) in a mammal, particularly a human, and includes: (a) preventing the disease or symptom from occurring in a subject which may be predisposed to the disease or symptom but has not yet been diagnosed as having it; (b) inhibiting or alleviating or reducing the disease symptom or consequences, i.e., arresting its development (e.g. reducing tumor size); or (c) relieving the disease symptom, i.e., causing regression of the disease or symptom.

With respect to the pharmaceutical compositions used in the treatments disclosed herein, it will be understood these may be presented in unit dose forms containing a predetermined amount of active ingredient per unit dose. As is known to those skilled in the art, the amount of active ingredient per dose will depend on the condition being treated, the route of administration and the age, weight and condition of the patient. Such pharmaceutical compositions may be prepared by any of the methods well known in the art.

The compounds used in the treatments as disclosed herein may be administered by any appropriate route. Suitable routes include oral, rectal, nasal, topical, buccal, sublingual, vaginal, parenteral, subcutaneous, intramuscular, intravenous, intradermal, intrathecal, by inhalation, and epidural. A preferred route of administration may depend, for example, on the condition of the patient and the disease to be treated. It will also be understood that, in case of combination treatments, each of the active compounds may be administered by the same or different routes.

Pharmaceutical compositions may be presented as capsules, tablets, powders, granules, solutions, suspensions in aqueous or non-aqueous liquids, edible, oil-in-water liquid emulsions, water-in-oil liquid emulsions, solution, syrups and elixirs, in microencapsulated form, liposome delivery systems, such as small unilamellar vesicles, large unilamellar vesicles and multilamellar vesicles, transdermal patches, ointments, creams, suspensions, lotions, powders, solutions, pastes, gels, drops, sprays, aerosols, oils, lozenges, pastilles, mouth washes, suppositories, enemas, aqueous and non-aqueous sterile injection solutions, and so on.

It will be appreciated that the compositions may include other agents conventional in the art having regard to the type of formulation.

Depending on the agent to be administered, the pharmaceutical compositions and compounds as disclosed herein may suitably be provided several times per day, once every day, once every other day, once per one, two or three week, once per one, two, three or four months, and so on. In some embodiments treatment with the compound is performed for a certain period of time, for example, for one, two, the, four, five weeks or months and then discontinued for a certain period of time, for example, for one, two, the, four, five weeks or months.

With respect to any of the combination treatments as described herein, the compounds in such combination treatment may be employed in combination in accordance with the invention by administration simultaneously in a pharmaceutical composition including both compounds. Alternatively, the combination may be administered separately in separate pharmaceutical compositions, each including different compound(s) and in a sequential manner wherein a first compound is administered first and the other second. Sequential administration may be close in time (e.g. simultaneously) or remote in time. Furthermore, it does not matter if the compounds are administered in the same dosage form or the same route of administration.

Thus in one embodiment, one or more doses of a first compound is administered simultaneously or separately with one or more doses of a second (or third, fourth,...) compound.

Suitably the combinations of this invention are administered within a "specified period" (the interval of time between the administration of the first compound of the combination and last compound of the combination). For example, within 1, 2, 6, 8, 12, 24 hours, two, three, four, five, six, seven days, one, two, three, four weeks, one, two, three, four, five, six or more months. For example, a first compound may be provided daily whereas the second compound is provided weekly; in such example the specified period wherein the combination of the invention is provided is one week.

Alternatively, the compounds in the combination are administered sequentially. For example, the first compound is provided to the patient for a certain period, e.g. for two or more consecutive days or weeks, then followed by administration of a next compound of the combination of the invention as disclosed herein, for example for a period of two, three or four days or weeks. As mentioned, also, contemplated herein is a drug holiday utilized among the administration of the compounds (either single or in the combination of the inventions).

An example of a dosage regimen may be that a first compound is administered for from 1 to 30 consecutive days, followed by an optional drug holiday, followed by administration of second compound for from 1 to 30 consecutive days, followed by an optional drug holiday. Another example may be that a first compound is administered once every two weeks for from 2 to 10 weeks and, optionally a second compound is administered daily for from 1 to 30 consecutive days or longer.

It will be understood that a "specified period" administration and a "sequential" administration can be followed by repeat dosing or can be followed by an alternate dosing protocol, and a drug holiday may precede the repeat dosing or alternate dosing protocol.

The terms "recipient", "individual", "subject", "host", and "patient" are used herein interchangeably and refer to any mammalian subject (e.g. human, rat, mouse, cat, dogs, horses, etc.) for whom diagnosis, treatment, or therapy is desired, particularly humans.

The term "condition or disease involving the CD47-SIRPα signaling axis" as used herein refers to any conditions or diseases, wherein cells (e.g. diseased cells such as cancer cells, diseased vascular smooth muscle cells, diseased endothelial cells, diseased cells infected by a pathogen (e.g. virus), diseased cells undergoing fibrosis, etc.)) make use of the CD47-SIRPα signaling axis, for example, so as to convey a "anti-phagocytic signals" or "don't eat me signals" for the purpose of evading or escaping or avoiding phagocytosis by phagocytes (e.g. macrophages, neutrophils). Non-limiting examples of conditions or diseases involving the CD47-SIRPα signaling axis include various cancer types, atherosclerosis, various fibrotic diseases as well as various infectious diseases, specific examples of which are as taught herein. The term "condition or disease involving the CD47-SIRPα signaling axis" also encompasses conditions wherein it is beneficial to perform cell depletion or cell replacement from the body, and wherein CD47 expression on said the depleted cells (e.g. hematopoietic stem cells) or said replaced cells (e.g. hematopoietic stem cells) impede or reduce the efficiency or benefit of said depletion or replacement. Non-limiting examples of such conditions include hematopoietic stem cell transplantation, blood transfusion or other administration of other blood products to treat blood cell deficiencies (such as, e.g., thrombocytopenia).

The term "a condition in a subject that would benefit from reducing signaling or binding between CD47 and SIRPα" as used herein refers to any conditions or diseases wherein the diseased cells make use or take advantage of the CD47-SIRPα signaling axis, for example to evade elimination, by e.g. phagocytosis by phagocytes (e.g. macrophages) by expressing anti-phagocytic signals such as CD47 (e.g. by expressing or overexpressing CD47 at their cell surface) to convey a "don't eat me signal". In the context of the present invention, non-limiting examples of a conditions or diseases in a subject that would benefit from reducing signaling or binding between CD47 and SIRPα include various types of cancer (e.g. leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, Gastric cancer, esophageal cancer, T-ALL, glioma, mesothelioma, glioblastoma, melanoma and NSCLC, and others), various type of

fibrotic diseases (e.g. idiopathic pulmonary fibrosis (IPF), scleroderma, myelofibrosis, kidney fibrosis, liver fibrosis, lung fibrosis, pancreas fibrosis, heart fibrosis, and bladder fibrosis, and others), various type of infectious diseases caused by a pathogens such as a virus (e.g. infectious diseases caused by lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania, and others), atherosclerosis, and others.

The term "cell with CD47 on the surface" or "cell expressing or overexpressing CD47 on its surface" as used herein refers to the phenotype of said cell, such as a diseased cell as taught herein, wherein the phenotype is defined by the presence of the CD47 protein or polypeptide, preferably at the cell surface of said cell. Non-limiting examples of cells expressing or overexpressing CD47 on their surface include diseased cells such as cancer cells, diseased vascular smooth muscle cells, diseased endothelial cells, diseased cells infected by a pathogen (e.g. virus), and diseased cells undergoing fibrosis. Cells expressing CD47 can be identified by flow cytometry using a suitable anti-CD47 antibody as the affinity ligand or by immunohistochemistry using a suitable anti-CD47 antibody or by *in situ* hybridization techniques using suitable CD47 mRNA probes, or by any other suitable methods leading to the detection of CD47 protein or fragments thereof and/or CD47 gene (DNA or mRNA) or variants thereof. The cells examined for CD47 phenotype may be cells derived from standard biopsy samples including tissue or cell samples and/or blood samples taken from a subject.

The term "active agent" as used herein refers to a compound such as a pharmaceutical compound or an effective drug or therapeutic, which has biological or pharmacological activity in a living system. To be an effective drug (with biological activity), a compound not only must be active against a specific target, but also possess the appropriate ADME (Absorption, Distribution, Metabolism, and Excretion) properties necessary to make it suitable for use as a drug in a living system (e.g. in humans). It is further understood that the biological activity of a given active agent or compound is generally dosage-dependent.

Further, the term "active agent capable of reducing the expression or the enzymatic activity of the QPCTL protein and/or QPCT protein or the expression of the QPCTL gene and/or QPCT gene in a cell expressing CD47 at its surface", and related terms, as used herein also refers to any compound, such as those described herein, capable of down-regulating or reducing or blocking the enzymatic activity of the QPCTL protein and/or QPCT protein or down-regulating or reducing or blocking the expression of the QPCTL gene and/or QPCT gene in a cell (e.g. cancer cell) contacted or treated with said compound, by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of enzymatic activity of the QPCTL protein and/or QPCT protein or the level of expression of QPCTL gene and/or QPCT gene in a cell (e.g. cancer cell) not contacted or not treated with said compound.

The term "active agent (compounds as taught herein) capable of reducing the binding between CD47 and SIRPα", and related terms, as used herein also refers to any compound, such as those described herein, capable of down-regulating or reducing or blocking the binding between CD47 on the surface of a first cell (e.g. cancer cell) and SIRPα on the surface of a second cell (e.g. immune cell such as a macrophage) when said first cell is contacted or treated with said compound, and wherein the binding between CD47 and SIRPα is down-regulated or reduced or blocked by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of binding between CD47 on the surface of a first cell (e.g. cancer cell) and SIRPα on the surface of a second cell (e.g. macrophage, neutrophils) when said first cell is not contacted or treated with said compound.

The term "active agent (compounds as taught herein) capable of "modulating (e.g. boosting or up-regulating or increasing) phagocytosis of a diseased cell" and related terms, as used herein also refers to any compound, such as those described herein, capable of modulating or boosting or increasing phagocytosis of a diseased cell, when said diseased cell is contacted or treated with said compound, and wherein the modulating (e.g. up-regulating or boosting or increasing) phagocytosis of a diseased cell is boosted or up-regulated or increased or modulated by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of phagocytosis of a diseased cell when said diseased cell is not contacted or treated with said compound. Phagocytosis or levels of phagocytosis of diseased cells can be measured using standard techniques (e.g. Ring et al (2017), Proceedings of the National Academy of Sciences of the United States of America, Vol. 114(49), E10578-E10585, http://doi.org/10.1073/pnas.1710877114; Ho et al (2015), The Journal of Biological Chemistry, Vol. 290(20), pages 12650-12663, http://doi.org/10.1074/jbc.M115.648220; Sockolosky et al 2016), Proceedings of the National Academy of Sciences of the United States of America, Vol. 113(19), E2646-54, http://doi.org/10.1073/pnas.1604268113).

The term "phagocytosis of a diseased cell" as used herein encompasses all means by which a cell (e.g. a

diseased cell) can be eliminated from the body or system as a result of phagocytosis by a phagocyte cell (e.g. macrophage, monocyte, neutrophil, basophil, eosinophil, or dendritic cell). For instance, phagocytosis of a diseased cell can be achieved by a process wherein a phagocyte cell engulfs a solid particle or a cell (e.g. diseased cell) to form an internal compartment known as a phagosome. The phagosome of ingested material (e.g. cell) is then fused with a lysosome to form a phagolysosome. Within the phagolysosome, enzymes and toxic peroxides digest the ingested material (e.g. diseased cell), resulting in its elimination from the body. Another example by which phagocytosis of a diseased cell may be achieved is through "antibody-dependent cellular phagocytosis" (abbreviated (ADCP)). Briefly, ADCP involves Fc receptors, which are proteins found on the surface of certain cells including, among others, B lymphocytes, follicular dendritic cells, natural killer cells, macrophages, neutrophils, eosinophils, basophils, human platelets, and mast cells. The Fc receptor binds specifically to a part of an antibody known as the Fc (Fragment, crystallizable) region. Antibody-dependent cellular phagocytosis occurs when Fc receptors on cells (e.g. B lymphocytes, follicular dendritic cells, natural killer cells, macrophages, neutrophils, eosinophils, basophils, and others) bind to antibodies (e.g. CD47 antibody such as a CD47 IgA antibody) that are attached to diseased cells (e.g. cancer cells) or infected cells or invading pathogens. This in turn stimulates phagocytic cells (e.g. macrophages, neutrophils) or cytotoxic cells to destroy diseased cells (e.g. cancer cells) or microbes, or infected cells by antibody-mediated phagocytosis or antibody-dependent cell-mediated cytotoxicity. In the present invention, it was found that the killing of diseased cells (e.g. cancer cells) via ADCP (using compounds as taught herein) is greater or enhanced when the Fc receptors on cells (e.g. macrophages, neutrophils) bind to IgA antibodies (e.g. any type of IgA antibodies, e.g. a CD47 IgA antibody) compared to IgG antibodies.

The term "active agent (compounds as taught herein) capable of "modulating (e.g. boosting or up-regulating or increasing) the killing or the death of a diseased cell via ADCP and related terms, as used herein also refers to any compound, such as those described herein, capable of modulating or boosting or increasing the killing or the death of a diseased cell via ADCP, when said diseased cell is contacted or treated with said compound, and wherein the modulating (e.g. up-regulating or boosting or increasing) the killing or death of a diseased cell via ADCP is boosted or up-regulated or increased or modulated by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of killing or death of a diseased cell via ADCP when said diseased cell is not contacted or treated with said compound. Killing or death or levels of killing or death of diseased cells via ADCP can be measured using standard techniques (e.g. Treffers et al (2017), European Journal of Immunology., Vol. 48. 10.1002/eji.201747215, e.g. using macrophages as effector cells to assay ADCP). In some embodiments, the "modulating (e.g. boosting or up-regulating or increasing) of the killing of a diseased cell (e.g. cancer cells) via ADCP (using compounds as taught herein) involves or uses IgA antibodies (e.g. anti- Her2-IgA1 antibody or anti-CD47-IgA antibody, and others).

The term "active agent (compounds as taught herein) capable of "modulating (e.g. boosting or up-regulating or increasing) immune-cell mediated killing of a diseased cell" and related terms, as used herein also refers to any compound, such as those described herein, capable of modulating or boosting or increasing immune-cell mediated killing of a diseased cell, when said diseased cell is contacted or treated with said compound, and wherein the modulating (e.g. up-regulating or boosting or increasing) of immune-cell mediated killing of a diseased cell is boosted or up-regulated or increased or modulated by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of immune-cell mediated killing of a diseased cell when said diseased cell is not contacted or treated with said compound. Immune-cell mediated killing or levels of immune-cell mediated killing of diseased cells can be measured using standard techniques (Treffers et al (2017), European Journal of Immunology., Vol. 48. 10.1002/eji.201747215, using neutrophils as effector cells to assay ADCC or macrophages as effector cells to assay ADCP; Ring et al (2017), Proceedings of the National Academy of Sciences of the United States of America, Vol. 114(49), E10578-E10585, http://doi.org/10.1073/pnas.1710877114 (to assay phagocytosis); Ho et al (2015), The Journal of Biological Chemistry, Vol. 290(20), pages 12650-12663, http://doi.org/10.1074/jbc.M115.648220 (to assay phagocytosis); Sockolosky et al (2016), Proceedings of the National Academy of Sciences of the United States of America, Vol. 113(19), E2646-54, http://doi.org/10.1073/pnas.1604268113 (to assay phagocytosis). In some embodiments, the "modulating (e.g. boosting or up-regulating or increasing) of the killing of a diseased cell (e.g. cancer cells) via ADCP (using compounds as taught herein) involves or uses IgA antibodies (e.g. anti- Her2-IgA1 antibody or anti-CD47-IgA antibody, and others). In some embodiments, the "modulating (e.g. boosting or up-regulating or increasing) of the killing of a diseased cell (e.g. cancer cells) via ADCC (using compounds as taught herein) involves or uses IgA antibodies (e.g. anti- Her2-IgA1 antibody or anti-CD47-IgA antibody, and others).

The term "immune-cell mediated killing of a diseased cell" as used herein refers to ways by which a diseased cell (e.g. cancer cell) may be killed or eliminated by the immune system (or immune cells, e.g. macrophages, myeloid cells) and include killing cell s or inducing cell death via phagocytosis or via antibody-dependent cellular cytotoxicity (ADCC) or via antibody-dependent cellular phagocytosis" (abbreviated (ADCP) of diseased cells.

The term "antibody-dependent cellular cytotoxicity (ADCC). ADCC refers to a mechanism of cell-mediated immune defense whereby an effector cell of the immune system (e.g. neutrophil such as neutrophil Fcγ) actively lyses

a target cell (e.g. diseased cells such as cancer cell) whose membrane-surface antigens have been bound by specific antibodies (e.g. a CD47 antibody). It was shown that ADCC can be promoted by interference with CD47-SIRPα interactions, e.g. blocking or reducing the interaction or binding between CD47 and SIRPα results in enhanced or increased phagocyte ADCC (Treffers et al (2017), Eur. J. Immunol., Vol 48(2), pages 1-11). Contrary to ADCP, killing or death of a diseased cell (e.g. cancer cell) via ADCC occurs as a result of direct toxicity and not via phagocytosis. In the present invention, it was found that the killing of diseased cells (e.g. cancer cells) via ADCC (using compounds as taught herein) is greater or enhanced when the Fc receptors on cells (e.g. neutrophils) bind to IgA antibodies (e.g. any type of IgA antibodies, e.g. a CD47 IgA antibody) compared to IgG antibodies.

The term "active agent (compounds as taught herein) capable of "modulating (e.g. boosting or up-regulating or increasing) the killing or the death of a diseased cell via ADCC and related terms, as used herein also refers to any compound, such as those described herein, capable of modulating or boosting or increasing the killing or the death of a diseased cell via ADCC, when said diseased cell is contacted or treated with said compound, and wherein the modulating (e.g. up-regulating or boosting or increasing) the killing or death of a diseased cell via ADCC is boosted or up-regulated or increased or modulated by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of killing or death of a diseased cell via ADCP when said diseased cell is not contacted or treated with said compound. Killing or death or levels of killing or death of diseased cells via ADCC can be measured using standard techniques (e.g. Treffers et al (2017), Eur. J. Immunol., Vol 48(2), pages 1-11, using neutrophils as effector cells). In some embodiments, the "modulating (e.g. boosting or up-regulating or increasing) of the killing of a diseased cell (e.g. cancer cells) via ADCC (using compounds as taught herein) involves or uses IgA antibodies (e.g. anti- Her2-IgA1 antibody or anti-CD47-IgA antibody, and others).

In the context of the present invention, in some embodiments, the compounds as taught herein (e.g. compounds selected from compounds of Formula (I), (II), (III), (IV), (V), (VI), (VII), or (VIII), or a compound disclosed in Table A, B, C, D, or E, e.g. PBD150, PQ912, PQ1565, 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, and 000066, may be advantageously used to promote or increased or enhance or boost phagocyte ADCC of diseased cells (e.g. cancer cells) as taught herein.

In the context of the present invention, the terms "phagocyte cells", "phagocytic cells" and "phagocytes" are used interchangeably herein to refer to cells that are capable of phagocytosis. Non-limiting examples of phagocytes include macrophages, mononuclear cells (e.g. histiocytes and monocytes), polymorphonuclear leukocytes (e.g. neutrophils), and dendritic cells, basophil, eosinophil, and others.

The term "active agent (compounds as taught herein) capable of "modulating or preventing or inhibiting or reducing the formation of a pyroglutamyl residue (pGlu) at the N-terminus of the CD47 protein expressed at the surface of a diseased cell", and related terms, as used herein also refers to any compound, such as those described herein, capable of down-regulating or reducing or blocking or modulating the formation of a pGlu residue at the N-terminus of the CD47 protein expressed at the surface of a diseased cell, when said diseased cell is contacted or treated with said compound, and wherein the formation of a pGlu residue at the N-terminus of the CD47 protein expressed at the surface of a diseased cell is down-regulated or reduced or blocked by at least about 5% or up to about 10%, 15%, 20%, 25%, 30%, 35%, 40%, 45%, 50%, 55%, 60%, 65%, 70%, 75%, 80%, 85%, 90%, or about 95% or more or up (preferably at least 50%, 60%, 70%, 80%, 90% and more) compared to the level of the formation of a pGlu residue at the N-terminus of the CD47 protein expressed at the surface of a diseased cell, when said diseased cell is not contacted or treated with said compound. The formation of a pyroglutamyl residue (pGlu) at the N-terminus of the CD47 protein expressed at the surface of a diseased cell can be measured using standard methods, e.g. flow cytometry using an CD47 antibody capable of binding the pGlu residue on CD47 (e.g. antibody clone CC2C6, as taught herein).

The term "diseased cells" as used herein refers to e.g. cancer cells or other diseased cells such as diseased vascular smooth muscle cells, diseased endothelial cells, diseased cells infected by a pathogen (e.g. virus), diseased cells undergoing fibrosis, expressing or overexpressing CD47 at their cell surface, and which are derived from- or are like diseased cells (e.g. cancer cell lines) derived from subjects suffering from a disease or condition involving the CD47-SIRPα signaling axis, such as e.g. cancer, atherosclerosis, fibrotic diseases as well as infectious diseases.

The term "glutaminyl-peptide cyclotransferase" (abbreviated "QPCT protein" or "QC protein", also known as glutaminyl cyclase) as used herein refers to an enzyme (EC 2.3.2.5) that is encoded by the QPCT gene (NM_012413, in human), and which is found in a secreted form due to the absence of a membrane anchor in its sequence (i.e. not membrane bound). QPCT protein is abundantly expressed in neuroendocrine tissues (e.g. pituitary) and has been implicated in disease conditions such as rheumatoid arthritis, osteoporosis and Alzheimer's disease. QPCT has also been found to be expressed, although to a lesser extent, in peripheral blood lymphocytes and other blood cells. QPCT has also been shown to be expressed by thyroid cancer cells (Kehlen et al (2013), Endocrine-Related Cancer, Vol. 20, pages 79-90) and melanoma cells (Gillis J. S. (2006) Journal of Translational Medicine, Vol. 4:27, page 1-7).

The term "glutaminyl-peptide cyclotransferase-like" (abbreviated "QPCTL protein" or "QCL protein", also known as "isoglutaminyl cyclase") as used herein refers to the isoenzyme (i.e. enzyme that differs in amino acid sequence but catalyzes the same chemical reaction) form of QPCT (E.C. 2.3.2.5). QPCTL protein is encoded by the QPCTL gene (NM_017659, in human). QPCTL protein is ubiquitously expressed throughout the body but is particularly abundant in peripheral blood lymphocytes and other blood cells. QPCTL protein has also been shown to be expressed by cancer cells (Kehlen et al (2013), Endocrine-Related Cancer, Vol. 20, pages 79-90). In contrast to QPCT protein (which is secreted), QPCTL protein is exclusively localized within the Golgi complex (e.g. is Golgi bound and is not secreted within or outside the cell) due to the presence of a membrane anchor in its sequence. QPCTL (a protein of 382 amino acid) shares 46% (DNA) sequence identity with QPCT protein and exhibits nearly identical enzymatic activity *in vitro,* i.e. both proteins are responsible for posttranslational modifications consisting of catalyzing the formation of pyroglutamyl (or pyroglutamate (pE or pGlu)) residues at the N-terminus portion of several peptides/ proteins (Cynis et al (2008), J, Mol Biol, Vol. 379, pages 966-89; Stephan et al (2009), FEBS Journal, Vol. 276, pages 6522-36).

The term "reducing the expression or enzymatic activity of QPCTL, QPCT, or combinations thereof in the cell with CD47 expressed on its surface" is understood to include reducing transcription and/or translation of the gene(s) encoding QPCTL, QPCT, or combinations thereof, as taught herein.

The term "CD47 inhibitor" as used herein refers to any active agents or compounds capable of binding to CD47 expressed on the surface of a cell (e.g. a diseased cell such as a cancer cell) so as to hinder or prohibit the binding of CD47 to SIRP$\alpha$, thereby reducing or preventing the binding of CD47 to SIRP$\alpha$ expressed on the surface of another cell (e.g. a macrophage). In the context of the present invention, non-limiting examples of CD47 inhibitors include anti-CD47 antibodies and SIRP$\alpha$-based fusion proteins (e.g. Hu5F9-G4 (Forty Seven, Inc.); CC-90002 (Celgene); TTI-621 (Trillium Therapeutics Inc.); as well as others currently under development including Novimmune, NI-1701 (CD47-CD19 bispecific), NI-1801 (CD47-meso bispecific), Tioma Therapeutics anti-CD47, Surface oncology SRF231 anti-CD47. In some embodiments, the CD47 inhibitor is a CD47 IgA antibody.

The term "SIRP alpha inhibitor" (abbreviated as SIRP$\alpha$ or SIRPalpha) as used herein refers to any active agents or compounds capable of binding to SIRP$\alpha$ expressed on the surface of a cell (e.g. macrophages, monocytes, neutrophils, basophils, eosinophils, dendritic cells) so as to hinder or prohibit the binding of SIRP$\alpha$ to CD47, thereby reducing or preventing the binding of SIRP$\alpha$ to CD47 expressed on the surface of another cell (e.g. a diseased cell such as a cancer cell). In the context of the present invention, non-limiting examples of SIRP$\alpha$ inhibitors include anti-SIRPa antibodies (e.g. OSE-172 from Ose Immunotherapeutics, Nantes, France); other non-limiting examples include recombinant human CD47Fc chimera (fusion)protein, which consists of an engineered CD47 protein coupled to a Fc domain (e.g. Trillium Therapeutics).

The "Programmed Death-1 (PD-1)" receptor as used herein refers to an immune-inhibitory receptor belonging to the CD28 family. PD-1 is expressed on previously activated T cells in vivo but also on myeloid cells, and binds to two ligands, PD-L1 and PD-L2. The term "PD-1" as used herein includes human PD-1 (hPD-1), variants, isoforms, and species homologs of hPD-1, and analogs having at least one common epitope with hPD-1. The complete hPD-1 sequence can be found under GENBANK Accession No. U64863. PD-1 is expressed on immune cells such as activated T cells (including effector T cells), B cells, myeloid cells, thymocytes, and natural killer (NK) cells (Suya Dai et al (2014) Cellular Immunology, Vol:290, pages 72-79; Gianchecchi et al (2013), Autoimmun. Rev. 12 (2013) 1091-1100).

"Programmed Death Ligand-1 (PD-L1)" as used herein refers to one of two cell surface glycoprotein ligands for PD-1 (the other being PD-L2) that down-regulates immune cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The complete hPD-L1 sequence can be found under GENBANK Accession No. Q9NZQ7. PD-L1 is expressed on a variety of cells including cells of hematopoietic lineage such as activated T cells, B cells, monocytes, dendritic cells (DCs), mast cells, and macrophages. PD-L1 is also expressed on peripheral non-hematopoietic tissue such as heart cells, skeletal muscle cells, pancreatic islet cells, placenta cells, lung cells, hepatocytes, epithelium cells, kidney cells, mesenchymal stem cells, liver cells, and others (Suya Dai et al (2014) Cellular Immunology, Vol:290, pages 72-79).

The term "PD-1/PD-L1 axis" as used herein consists of the PD-1 receptor and its ligand PD-L1. The term "PD-1/PD-L1 axis signaling" is a way of communication between cells (cell signaling), for instance between a first cell expressing PD-1 and a second cell expressing PD-L1, and which involves the release of a biochemical signal (e.g. release of proteins, lipids, ions, neurotransmitters, enzymes, gases, etc.), which in turn causes an effect (e.g. inhibition, activation, blockade, etc.) on one or both cells. An example of "PD-1/PD-L1 axis signaling" is when PD-L1 expressed at the cell surface of a first cell (e.g. cancer cells or a cancer-infiltrating immune cells) binds to its receptor PD-1 expressed at the cell surface of a second cell (e.g. a T cell, such as an effector T cell). The binding of PD-L1 to its receptor PD-1 transmits an inhibitory signal to the T-cell which results in a decrease in T cell proliferation (e.g. effector T cells) as well as T cell activity (e.g. secretion of cytokines and chemokines as discussed herein; Wei F et al (2013) PNAS; Vol: 110, E2480-2489). Thus, one possible end result of PD-1/PD-L1 axis signaling is the dampening or inhibition of immune activity or function mediated by T cells (e.g. effector T cells). Such situation may be detrimental in the context of cancer. Further, it has

been hypothesized that PD-1 may also mediate an anti-phagocytic signal on macrophages (Gordon et al (2017), Nature, Vol. 545, pages 495-499) and inhibition of the CD47-SIRPα signaling axis has been shown to enhance the anti-tumor effect of blockade of the PD-1 - PD-L1 axis (Manguso et al. (2017), Nature, Vol. 547, pages 413-418.

The term "providing to the subject an active agent that reduces expression or enzymatic activity of QPCTL, QPCT, or combinations thereof in a cell with CD47 on the surface" as used herein refers to providing said subject with an effective amount of said active agent.

The term "effective amount" or "therapeutically effective amount" as used herein refers to an amount of a given compound (e.g. an active agent and pharmaceutical composition thereof as taught herein) which is effective, at dosages and for a particular period of time necessary, to achieve the desired therapeutic result (e.g. treat cancer, e.g. reduction of tumor size or promoting or increasing phagocytosis of cancer cells expressing the CD47 protein, or treating atherosclerosis or fibrotic diseases or an infectious diseases caused by pathogens (e.g. virus)). A therapeutically effective amount of the pharmacological agent or compound (e.g. an active agent as taught herein) may vary according to factors such as the disease state, disease type, age, sex, and weight of the individual, and the ability of the pharmacological agentto elicit a desired response in the individual. A therapeutically effective amount of a given compound is also one in which any toxic or detrimental effects (if any) of the pharmacological agent or compound (e.g. a compound as taught herein) are outweighed by the therapeutically beneficial effects.

The term "test compound" as used herein refers to a chemically defined molecule whose ability to: 1) reduce or inhibit or block the enzymatic activity of the QPCTL protein and/or QPCT protein or the expression of the QPCTL gene and/or QPCT gene in a cell, and/or 2) reduce or inhibiting or block or prevent the formation of a pyroglutamyl residue at the N-terminus of the CD47 protein expressed in a cell is assessed in an assay or method according to the invention. Test compounds include, but are not limited to drugs, ligands (natural or synthetic), polypeptides, peptides, peptide mimics, polysaccharides, saccharides, glycoproteins, nucleic acids, polynucleotides, antibodies, enzymatic inhibitors, and small organic molecules. The test compound may also be candidate drug or lead compound, a chemical intermediate, environmental pollutant, or a mixture of compounds. In one embodiment, the test compound may be comprised within an existing library of compounds. In another embodiment, test compounds may be comprised within a novel library of compounds. In other words, the test compound(s) may be a known compound(s) or an unknown (novel) compound(s). In an embodiment, the test compound may be any of the active agents (capable of reducing the expression or the enzymatic activity of QPCT and/or QPCTL) and pharmaceutical compositions thereof as taught herein, e.g. compounds selected from Tables A, B, C, D and/or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066, as taught herein. Such compounds may also be referred to as "reference compound".

The term "reference compound" as used herein refers to a compound which is known (a priori) to: 1) reduce or inhibit or block the enzymatic activity of the QPCTL and/or QPCT protein or the expression of the QPCTL and/or QPCT, and/or 2) reduce or inhibit or prevent or block the formation of a pyroglutamyl residue at the N-terminus of the CD47 protein. Such reference compounds may be useful to validate and/or optimize the method as taught herein for the purpose of finding or detecting or screening for new (not a priori known for) compound(s) capable of: 1) reducing or inhibiting the enzymatic activity of the QPCTL and/or QPCT protein or the expression of the QPCTL and/or QPCT gene in a cell, and/or 2) reducing or inhibiting the formation of a pyroglutamyl residue at the N-terminus of the CD47 protein expressed in a cell.

## CD47

The term "Cluster of Differentiation 47" (abbreviated as "CD47") as used herein refers to a 50 kDa transmembrane protein (receptor) encoded by the CD47 gene (Ensembl reference: ENSG00000196776 in human). CD47 is also known as integrin associated protein (IAP). CD47 belongs to the immunoglobulin (Ig) superfamily and is characterized by the presence of an extracellular N-terminal IgV domain, five transmembrane domains, and a short C-terminal intracellular tail.

CD47 is expressed by all normal/healthy mammalian (e.g. human, mouse, rat, etc.) tissues and cells (e.g. red blood cells such as erythrocyte cells), as revealed by CD47 mRNA expression and CD47 immunohistochemical staining studies (Wiersma et al (2015), Atlas of Genetics and Cytogenetics in Oncology and Haematology, Vol. 19, pages 417-431; Lindberg et al (1993), Journal of Cell Biology, vol. 123, pages 485-496).

CD47 has also been found to be expressed in several cancer types, such as e.g. leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, bladder cancer, HNSCC, gastric cancer, esophageal cancer, T-ALL, glioma, mesothelioma, glioblastoma, melanoma, NSCLC, and others (Chao et al (2012), Current Opinion Immunol., Vol.24, pages 225-3;

Matlung et al. (2017), Immunol Rev. Vol.276, pages145-164).

It was reported that cancer cells upregulate the expression of (or overexpress) CD47 at their cell surface, which results in CD47 levels which are higher compared to CD47 levels found in normal cells (which are relatively low) (Majeti et al (2009), Cell, Vol.138, pages 286-99; Chao et al (2012), Curr Opin Immunol, Vol.24, pages 225-32). Overexpression of CD47 in cancer was first found in ovarian cancer in the 1980s (Poels et al (1986), J. Natl. Cancer Inst. Vol.76, pages 781-91). In the context of the present invention, the term "overexpression of CD47" in a diseased cell (e.g. cancer cells but also diseased vascular smooth muscle cells, diseased endothelial cells, diseased cells infected by a pathogen (e.g. virus), diseased cells in tissues undergoing fibrosis, can also express anti-phagocytic signals such as CD47) refers to CD47 levels in said cell, which are higher than the CD47 levels found in a normal cells (e.g. non-diseased or healthy cell of the same cellular type) such as 1.5-fold higher, 2.0-fold higher, 2.5-fold higher, 3-fold higher or more.

**SIRPα**

The term "signal-regulatory protein alpha (abbreviated "SIRPα" or "SIRP a", also termed CD172a or SHPS-1) as used herein refers to a regulatory transmembrane glycoprotein from the SIRP family, which is encoded by the SIRPα gene (Ensembl reference: ENSG00000198053 in human). SIRPα is characterized by the presence of three extracellular Ig-like domains, a transmembrane domain and an intracellular tail containing four immunoreceptor tyrosine-based inhibitory motifs (ITIMs) (Barclay and Van den Berg (2014), Annu Rev Immunol, Vol. 32, pages 25-50). The SIRP family comprises 3 members, namely SIRPα, SIRPβ and SIRPγ, which are closely related in terms of sequence and overall structure but have different activity. X-ray crystallography studies have shown that despite sequence and domain similarities, SIRPα, SIRPβ and SIRPγ differ in their abilities to bind to CD47. While SIRPα binds to CD47 with reasonably high affinity, binding of SIRPβ and SIRPγ to CD47 is negligible or not possible because of differences in the three dimensional structure (e.g., loops) of the protein (Hatherley et al (2008), Molecular cell, Vol. 31, pages 266-77).

SIRPα is mainly expressed by myeloid cells (e.g. macrophages, monocytes, neutrophils, basophils, eosinophils, dendritic cells), neurons, and (*in vitro*) cardiomyocytes derived from induced pluripotent stem cells (Matozaki et al (2009), Trends Cell Biol., Vol.19 (2), pages 72-80; and Dubois et al (2011), Nature Biotechnology, Vol. 29, pages 1011-1018). SIRPα acts as inhibitory receptor by interacting with or binding to CD47, i.e. as part of the CD47-SIRPα signaling axis, as described herein. This interaction leads to inhibition of cell killing by immune cells, such as inhibition of cell killing through phagocytosis of cells expressing CD47 at their cell surface (e.g. cancer cells positive for CD47) by immune cells such as phagocytes (e.g. macrophages, neutrophils), and also inhibition of killing through antibody-dependent cellular cytotoxicity (ADCC) of cells expressing CD47 at their cell surface, as explained herein.

**CD47-SIRPα Signaling Axis**

The term "CD47-SIRPα signaling system or axis or pathway" as used herein refers to a signaling axis or system characterized by the interaction or binding between CD47 expressed on the cell surface of one cell (e.g. expressed at the cell surface of a diseased cell such as a cancer cell) and SIRPα expressed on the cell surface of another/different cell (e.g. expressed at the cell surface of an immune cell such as a phagocyte (e.g. macrophage, neutrophil) and includes the molecular (e.g. phosphorylation events, gene and protein expression, recruitment, transport, etc.) and physiological responses (e.g. generation of a *"don't eat me signal"* resulting in the inhibition of phagocytosis, ADCC and ADCP, e.g. CD47 positive cells engaged into CD47-SIRPα signaling will evade phagocytosis by an immune cell such as a macrophage and/or cell death via ADCC derived from or triggered by this interaction.

CD47 has several binding ligands including the signal-regulatory protein alpha (SIRPα). Depending on its binding ligand as well as its expression pattern (e.g. level of expression, location), CD47 plays various biological roles including in apoptosis, proliferation, adhesion, migration as well as angiogenic and immune responses.

One prominent role of CD47 is to control phagocytic activity through its interaction or binding with SIRPα. When CD47 interacts or binds with SIRPα (CD47-SIRPα interaction), it initiates a cascade of signaling events in the cells (i.e. the cell expressing CD47 and the cell expressing SIRPα). Specifically, CD47-SIRPα interaction causes tyrosine phosphorylation of SIRPα cytoplasmic immunoreceptor tyrosine-based inhibitory motifs (ITIM) motifs, which in turn leads to concomitant activation or recruitment of Src homology 2 domain tyrosine phosphatase 1 (SHP-1) and Src homology 2 domain tyrosine phosphatase 2 (SHP-2). SHP-1 and SHP-2 are cytoplasmic protein tyrosine phosphatases, which mediate signaling events causing inhibition of phagocytosis by for instance dephosphorylating myosin-IIA (Wiersma et al (2015), Atlas of Genetics and Cytogenetics in Oncology and Haematology, Vol. 19, pages 417-431). Myosin-IIA is an important feature of the actin-myosin contractile system, which mediates the engulfment of material (e.g. cell to be eliminated) by phagocytes (e.g. macrophage, neutrophil) during phagocytosis.

Therefore, for these reasons (i.e. because it triggers a cascade of signaling events leading to inhibition of phagocytosis by binding or interacting with SIRPα), CD47 is often referred to as a "don't eat me signal" or "anti-phagocytic signal". In addition, the binding of CD47 to SIRPα can also inhibit death of CD47 expressing cells by other mechanisms,

such as ADCC. In all CD47-SIRPα interaction-dependent mechanisms of cell death, inhibition of this interaction may be exploited to enhance death of the CD47 expressing cells.

Under normal conditions, the CD47-SIRPα signaling axis serves an important role in preventing removal of healthy/normal cells expressing CD47 (e.g. healthy red blood cells or erythrocytes). On the other hand, (naturally-occurring) down-regulation of CD47 on damaged, aged and superfluous cells (e.g. old red blood cells) ensures their timely removal from the body.

Under pathological situations, such as in the context of cancer, the CD47-SIRPα signaling system or axis may be used by cancer cells (e.g. cancer cells positive for CD47 or expressing CD47 at their cell surface) to evade immune surveillance, e.g. to escape phagocytosis by immune cells such as macrophages. As discussed earlier, it was shown that as a result of expressing or overexpressing CD47, cancer cells can evade destruction by the immune system or evade immune surveillance (e.g. evading phagocytosis) by activating the CD47-SIRPα signaling system or axis (i.e. through interaction or binding between CD47 and SIRPα) (Oldenborg et al (2000) Science, Vol. 288, pages 2051-2054; Jaiswal et al (2009) Cell, Vol.138, pages 271-285). Overall, it was found that expression (increased expression or overexpression) of CD47 in several cancers, e.g. leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, bladder cancer, HNSCC, gastric cancer, esophageal cancer, T-ALL, glioma, mesothelioma, glioblastoma, melanoma, NSCLC, and others, was associated with worse clinical prognosis and greater chances of refractoriness (no response) to chemotherapies (Majeti et al (2009), Cell, Vol.138, pages 286-99).

**Role of the CD47-SIRPα Signaling Axis in Cancer and other Conditions**

Cancer cells are able to evade immune surveillance in many ways, for instance by evading phagocytosis by phagocyte cells (e.g. macrophages, neutrophils) through the expression of so-called "anti-phagocytic" or "don't eat me" signals. One prominent signal is the transmembrane protein "cluster of differentiation 47" (abbreviated as "CD47"). CD47 is also known as integrin associated protein (IAP). CD47 is expressed by virtually all cells in the body, e.g. blood cells such as erythrocyte cells, and is involved in a range of cellular processes, including apoptosis, proliferation, adhesion, and migration as well as angiogenic and immune responses. CD47 binds or interact with several ligands including the signal-regulatory protein alpha (SIRPα), thrombospondin-1 (TSP-1) and membrane integrins (e.g. αvβ3 integrin, α2βi integrin), with SIRPα being considered as a main ligand for CD47. SIRPα is an inhibitory transmembrane receptor present on myeloid cells such as macrophages, monocytes, neutrophils, basophils, eosinophils, erythrocytes, and dendritic cells.

The interaction or binding between CD47 and SIRPα has been widely studied because it mediates or conveys "anti-phagocytic" or "don't eat me" signals between two cells, e.g. a cancer cell and a phagocyte cell (e.g. macrophage), which ultimately inhibit phagocytosis (i.e. the cells positive or expressing CD47 at their cell surface (e.g. red blood cell) will not undergo phagocytosis orwill be less prone to phagocytosis by phagocyte cells expressing SIRPα (e.g. macrophages). For this reason, CD47 is often referred to as a "don't eat me signal" and a marker of self, as loss of CD47 leads to homeostatic phagocytosis of aged or damaged cells. Expression of CD47 in normal/healthy cells serves to maintain tissue homeostasis (e.g. to prevent immune attacks against tissues or cells that are constituents of the "self" (e.g. prevent auto-immunity) and to rid the body of old or defective cells or foreign cells.

However, CD47 expression is not limited to normal/healthy cells. Specifically, diseased cells (such as cancer cells, diseased vascular smooth muscle cells, diseased endothelial cells, diseased cells infected by a pathogen (e.g. virus), or diseased cells undergoing fibrosis) can also express anti-phagocytic signals such as CD47, and thus can convey a "do not eat me signal" or "anti-phagocytic signal".

In the case of cancer, cancer cells upregulate the expression of CD47 at their cell surface compared to the CD47 levels found in normal/healthy cells (which are relatively low) (Majeti et al (2009), Cell, Vol.138, pages 286-99; Chao et al (2012), Curr Opin Immunol, Vol.24, pages 225-32). As a result of having their CD47 expression, cancer cells can evade destruction by the immune system or evade immune surveillance, e.g. by evading phagocytosis by immune cells such as phagocyte cells (e.g. macrophages, neutrophils) (Oldenborg et al (2000) Science, Vol. 288, pages 2051-2054; Jaiswal et al (2009) Cell, Vol.138, pages 271-285). Overall, increased expression (or overexpression) of CD47 in several cancers (e.g. hematologic cancers or blood cancers such as leukemia) is associated with worse clinical prognosis and greater chances of refractoriness (no response) to chemotherapies (Majeti et al (2009), Cell, Vol.138, pages 286-99).

Expression of CD47 in cancer was first found in ovarian cancer in the 1980s (Poels et al (1986), J. Natl. Cancer Inst. Vol. 76, pages 781-91). Since then, a large body of evidence has been gathered documenting the expression of CD47 as well as the involvement of the CD47-SIRPα signaling axis in many cancers including e.g. leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic

neuroendocrine tumors, small cell lung cancer, bladder cancer, HNSCC, gastric cancer, esophageal cancer, T-ALL, glioma, mesothelioma, glioblastoma, melanoma, NSCLC, and others (Matlung et al. (2017), Immunol Rev. Vol.276, pages145-164.)

Diseased cells in conditions other than cancer, such as e.g. atherosclerosis, fibrotic diseases as well as infectious diseases caused by pathogens (e.g. virus), also upregulate the expression of CD47 at their cell surface compared to the CD47 levels found in normal/healthy cells to evade phagocytosis by phagocytes (Kojima et al (2016) Nature, Vol. 536, pages 86-90; Wernig et al (2017) PNAS, Vol.114, pages 4757-4762; and WO2014124028).

These results have prompted increasing interest in using the CD47-SIRPα signaling axis as a clinical target not only for cancer immunotherapy but also other conditions such as atherosclerosis, fibrotic diseases as well as infectious diseases caused by pathogens (e.g. virus).

In the case of cancer, current approaches to antagonize the CD47-SIRPα interactions in cancer have principally targeted CD47 (Chao et al (2011), Cancer Res., Vol. 71, pages 1374-84). For instance, several anti-CD47 antibodies aimed at interfering or blocking CD47-SIRPα interactions are currently being developed or tested in clinical trials. The anti-CD47 monoclonal antibody (mAb) B6H12 has shown pre-clinical efficacy in several hematologic malignancies and solid tumor models through its ability to block SIRPα binding to CD47 (Chao et al (2011) Cancer Res. Vol. 71, pages 1374-1384; Edris et al (2012) PNAS, Vol. 109, pages 6656-6661; Willingham et al (2012) PNAS, Vol. 109, pages 6662-6667). Other non-limiting examples of anti-CD47 antibodies and SIRPα-based protein therapeutics being developed or being considered for clinical applications include Hu5F9-G4 (Forty Seven, Inc.) for the treatment of solid tumors and advanced colorectal cancer, CC-90002 (Celgene) for the treatment of AML as well as advanced solid and hematologic cancers, and the SIRPα-FC fusion protein TTI-621 (Trillium Therapeutics Inc.) for the treatment of hematologic malignancies. Other non-limiting examples being developed (in preclinical stage) include Novimmune, NI-1701 (CD47-CD19 bispecific), NI-1801 (CD47-meso bispecific), Tioma Therapeutics anti-CD47, Surface oncology SRF231 anti-CD47, OSE immunotherapeutics Effi-DEM anti-SIRPα. Another non-limiting example of anti-CD47 compound is ALX148 (Alexo Therapeutics, Inc., an engineered protein coupled to a Fc domain) for the treatment of solid tumors and lymphoma. Other approaches consist of the use of agents such as anti-SIRPα antibodies (Sarfati et al (2008), Curr Drug Targets, Vol.9, pages 842-50, Zao et al (2011) PNAS, Vol. 108, pages 18342-18347).

Although promising, such strategies are not optimal since antibodies are known to have poor tissue penetration, especially into solid tumors due to their large molecular weight. Further, such antibodies, particularly antibodies targeting CD47 lack specificity since CD47 is widely distributed throughout the body, including healthy tissue, which may cause on-target toxicity to normal cells (Ho et al (2015), J. Biol. Chem, Vol. 290, pages 12650-12663).

Other disadvantages associated with the use of anti-CD47 antibodies include the lack of oral bioavailability and undesirable side effects such as the development of anemia (which may occur as a result of a dose-dependent loss of red blood cells and platelets) as well as hemagglutination (clumping of red blood cells). For instance, such undesirable side effects were observed during clinical trials led by Forty Seven, Inc. Specifically, a humanized monoclonal anti-CD47 antibody (Hu5F9-G4) was administered to patients with diverse (advanced) solid tumors. It was observed that patients who received the highest dose of the anti-CD47 antibody (Hu5F9-G4, 3 mg/kg) experienced toxicity including abdominal pain, red blood cell hemagglutination and headache (Sikic et al (2016), J Clin. Oncol., Vol. 34).

The disadvantages associated with the use of anti-CD47 antibodies in the context of cancer therapy, as discussed above, will also manifest in other therapies where the use of anti-CD47 antibodies may be indicated such as for instance in the treatment of atherosclerosis, fibrotic diseases as well as infectious diseases caused by pathogens (e.g. virus) (Kojima et al (2016) Nature, Vol. 536, pages 86-90; Wernig et al (2017) PNAS, Vol.114, pages 4757-4762; and WO2014124028).

Therefore there is a need for CD47-targeting therapies that do not cause significant levels toxicity, and/or platelet depletion and/or hemagglutination (clumping of red blood cells together) and/or red blood cell depletion, and/or anemia when administered to a subject and/ or that have the potential of oral bioavailability. Further, there is also a need for additional, adjuvant, alternative, or improved strategies including compounds and pharmaceutical compositions, use of such compounds and pharmacological compositions, and/or methods, which are devoid of at least some of the limitations and which confer the following advantages or uses:

1) Blocking or reducing or inhibiting the activity of the CD47-SIRPα signaling axis, particularly in conditions or diseases involving CD47-SIRPα signaling axis (i.e. where diseased cells use the CD47-SIRPα signaling axis to evade or escape killing by immune cells, such as phagocytosis by phagocytes); and/or

2) Blocking or reducing or inhibiting the interaction or binding between CD47 and SIRPα, particularly in conditions or diseases involving CD47-SIRPα signaling axis; and/or

3) Treating subjects suffering from a disease or condition involving the CD47-SIRPα signaling axis, such as e.g. cancer, atherosclerosis, fibrotic diseases as well as infectious diseases; and/or

4) Modulating (e.g. boosting or increasing) immune cell-mediated killing (e.g. via phagocytosis or via antibody-dependent cellular cytotoxicity (ADCC) or via antibody-dependent cellular phagocytosis" (abbreviated ADCP) of diseased cells (e.g. cancer cells or other diseased cells such as diseased vascular smooth muscle cells, diseased endothelial cells, diseased cells infected by a pathogen (e.g. virus), diseased cells undergoing fibrosis) expressing or overexpressing CD47 at their cell surface by phagocytes (e.g. macrophages, neutrophils) in subjects suffering from a disease or condition involving the CD47-SIRPα signaling axis, such as e.g. cancer, atherosclerosis, fibrotic diseases as well as infectious diseases. In some embodiments, the "modulating (e.g. boosting or up-regulating or increasing) of the killing of a diseased cell (e.g. cancer cells) via ADCP or ADCC (using compounds as taught herein) involves or uses IgA antibodies (e.g. anti-Her2-IgA1 antibody or anti-CD47-IgA antibody, and others); and/or

5) Complementing or enhancing the effects of a therapeutic treatment (monotherapy) with a first active agent (e.g. drug), e.g. anti-CD47 antibody (e.g. an anti-CD47 IgA antibody) or an anti-SIRPa antibody or other active agents including for instance anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the first active agent is an IgA antibody; and/or

6) Complementing or enhancing the effects of a therapeutic treatment consisting of a combination of two active agents (i.e. combination therapy), where the first active agent (e.g. drug) is an anti-CD47 antibody (e.g. an anti-CD47 IgA antibody) or an anti-SIRPa antibody and the second active agent is selected from the groups consisting of anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the first and second active agents are IgA antibodies; and/or

7) Substituting for the use of an anti-CD47 antibody or an anti-SIRPα antibody in the context of a therapeutic treatment (monotherapy with an anti-CD47 antibody or an anti SIRPα antibody) or in the context of a therapeutic (combination therapy) where an anti-CD47 antibody or an anti-SIRPα antibody is administered in combination with a second active agent (e.g. drug), e.g. anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the second active agent is an IgA antibody;

**Role of QPCT and/or QPCTL in the CD47-SIRPα Signaling Axis**

Disclosed herein is a new mechanism for modulating the CD47-SIRPα signaling axis. In some embodiments as disclosed herein, reducing or blocking or inhibiting activity or expression of enzymes referred to as glutaminyl-peptide cyclotransferase (QPCT) as well as its related isoenzyme, the glutaminyl-peptide cyclotransferase-like (QPCTL), or combinations thereof, is associated with a reduction or inhibition or blockade of the interaction or binding between CD47 and SIRPα In some embodiments, this reduction of binding between CD47 and SIRPα results in a reduction or inhibition or blockade of the CD47-SIRPα signaling axis.

In some embodiments as disclosed herein, generation of an "anti-phagocytic signal" or "do not eat me signal" by a cell (for instance a diseased cell in a disease or condition involving the CD47-SIRPα signaling axis, such as e.g. a cancer cell) could not only be prevented or attenuated by using antibodies interfering with the CD47-SIRPα signaling axis or interfering with the binding or interaction between CD47 and SIRPα, but also by interfering with enzymes found to be involved in or to be capable of performing post-translational modifications of the CD47 protein, such as QPCTL and/or QPCT enzymes. In other embodiments as disclosed herein, reducing or blocking or inhibiting the expression of QPCT and/or QPCTL gene and/or QPCT and/or QPCTL protein in a cell (e.g. using, gene inactivation methods such as knockout technology, interference RNA technology, or using inhibitor compounds/enzyme inhibitors as taught herein, etc.) reduces, prevents or blocks the interaction or binding between CD47 (e.g. expressed at the cell surface of one diseased cell, such as cancer) and SIRPα (e.g. expressed at the cell surface of another cell, e.g. macrophage, neutrophil). In other embodiments as disclosed herein, reducing, preventing or blocking the activity of the CD47-SIRPα signaling axis through reducing or blocking or inhibiting the expression of QPCT and/or QPCTL gene and/or QPCT and/or QPCTL protein in a cell, leads to increased phagocytosis of cells expressing CD47 (e.g. cancer cells) by phagocytes (e.g. macrophages, neutrophils) expressing SIRPα at their cell surface or leads to increased killing or death of cells expressing CD47 (e.g. diseased cells such as cancer cells) via ADCC or leads to increased killing or death of cells expressing CD47 (e.g. diseased cells such as cancer cells) via ADCP. In some embodiments, the killing of a diseased cell (e.g. cancer cells) via ADCP or ADCC (using compounds as taught herein) involves or uses IgA antibodies (e.g. anti- Her2-IgA1 antibody or anti-CD47-IgA antibody, and others).

As disclosed herein, reducing or inhibiting QPCT and/or QPCTL activity or expression, for example by using inhibitors of the activity of the QPCT and/or QPCTL enzyme or using compounds inhibiting the expression of the QPCT

and/or QPCTL enzyme, represents an effective way of increasing phagocytosis of diseased cells (e.g. cancer cells) or way of increasing killing or death of diseased (e.g. cancer cells) via ADCC or ADCP, particularly in cancer cells, which otherwise would escape phagocytosis or death via ADCC or ADCP through activation of the CD47-SIRPα signaling axis. In some embodiments, the killing of a diseased cell (e.g. cancer cells) via ADCP or ADCC (using compounds as taught herein) involves or uses IgA antibodies (e.g. anti- Her2-IgA1 antibody or anti-CD47-IgA antibody, and others). As disclosed herein, QPCT gene and protein and/or QPCTL gene and protein represent biological targets or "druggable" targets in relation to disease or conditions involving the CD47-SIRPα axis, such as e.g. cancer, atherosclerosis, fibrotic diseases (e.g. idiopathic pulmonary fibrosis, scleroderma, myelofibrosis, kidney fibrosis, liver fibrosis, lung fibrosis, pancreas fibrosis, heart fibrosis, and bladder fibrosis) as well as infectious diseases caused by pathogens (e.g. virus).

In some embodiments as disclosed herein, reducing or inhibiting or blocking the enzymatic activity of the QPCT protein and/or QPCTL protein or the expression of QPCT gene and/or QPCTL gene is used for important clinical or medical applications such as for:

1) Reducing or blocking or inhibiting the interaction or binding between CD47 and SIRPα; and/or

2) Reducing or blocking or inhibiting the activity of the CD47-SIRPα signaling axis in a subject suffering from a disease or condition involving the CD47-SIRPα signaling axis, such as e.g. cancer, atherosclerosis, fibrotic diseases as well as infectious diseases caused by pathogens (e.g. virus); and/or

3) Treating a subject suffering from a disease or condition involving the CD47-SIRPα signaling axis, such as e.g. cancer; and/or

4) Modulating (e.g. boosting or increasing) immune cell-mediated killing (e.g. via phagocytosis or via antibody-dependent cellular cytotoxicity (ADCC) or via antibody-dependent cellular phagocytosis" (abbreviated ADCP) of diseased cells (e.g. cancer cells or other diseased cells such as diseased vascular smooth muscle cells, diseased endothelial cells, diseased cells infected by a pathogen (e.g. virus), diseased cells undergoing fibrosis) expressing or overexpressing CD47 at their cell surface by phagocytes (e.g. macrophages, neutrophils) in subjects suffering from a disease or condition involving the CD47-SIRPα signaling axis, such as e.g. cancer, atherosclerosis, fibrotic diseases as well as infectious diseases. In some embodiments, the "modulating (e.g. boosting or up-regulating or increasing) of the killing of a diseased cell (e.g. cancer cells) via ADCP or ADCC (using compounds as taught herein) involves or uses IgA antibodies (e.g. anti- Her2-IgA1 antibody or anti-CD47-IgA antibody, and others); and/or

5) Modulating (e.g. preventing or inhibiting or reducing) the formation of a pyroglutamyl residue at the N-terminus of the CD47 protein expressed at the surface of a diseased cells such as a cancer cells, diseased vascular smooth muscle cells, diseased endothelial cells, diseased cells infected by a pathogen (e.g. virus), diseased cells undergoing fibrosis, etc., in a subject suffering from a disease or condition involving the CD47-SIRPα signaling axis, such as e.g. cancer, atherosclerosis, fibrotic diseases as well as infectious diseases caused by pathogens (e.g. virus); and/or

6) Complementing or enhancing the effects of treatment (monotherapy) with a first active agent or drug, e.g. anti-CD47 antibody (e.g. an anti-CD47 IgA antibody) or other agents including for instance anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the first active agent is a IgA antibody; and/or

7). Complementing or enhancing the effects of treatment (monotherapy) with a first active agent or drug, e.g. anti-SIRPα antibody or other agents including for instance anti-SIRP, an antibody OSE-172 from Ose Immunotherapeutics, Nantes, France; or recombinant human CD47Fc chimera protein, which consists of an engineered CD47 protein coupled to a Fc domain. In some embodiments, the first active agent is a IgA antibody; and/or

8) Complementing or enhancing the effects of a therapeutic treatment consisting of a combination of two active agents (i.e. combination therapy), where the first active agent (e.g. drug) is an anti-CD47 antibody (e.g. an anti-Cd47 IgA antibody) or an anti-SIRPα antibody and the second active agent is selected from the groups consisting of anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the first and second active agents are IgA antibodies; and/or

9) Substituting for the use of an anti-CD47 antibody or an anti-SIRPα antibody in the context of a therapeutic treatment (monotherapy with an anti-CD47 antibody or an anti-SIRPα antibody) or in the context of a therapeutic

(combination therapy) where an anti-CD47 antibody or an anti-SIRPα antibody is administered in combination with a second active agent (e.g. drug), e.g. anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. In some embodiments, the second active agent is a IgA antibody;

In some embodiments as disclosed herein, further advantages of targeting the CD47-SIRPα axis through inhibition of QPCTL (using the compounds as taught herein) are observed in the context of conditions such as anemia or thrombocytopenia that have been observed upon targeting of the CD47 - SIRPα pathway with inhibitors. Specifically, unlike with agents that directly bind to CD47 such as antibodies or recombinant SIRPα or SIRPα variants, mature CD47 molecules that have already been modified with pyro-glutamate are not affected. This represents an advantage in context wherein infused cells are preferentially spared (e.g. not cleared, not targeted by macrophages, phagocytes or other myeloid cells). For instance, in the context where anemia or thrombocytopenia is treated by infusion of blood cell products, the mature CD47 molecules already present on the infused cells will not be targeted, thereby avoiding increased susceptibility of these cells to phagocytosisis or other clearance mechanisms. In another further example, blockade of the CD47 axis in combination with antibodies against hematopoietic stem cells (HSC) has been proposed as a strategy to achieve hematopoietic stem cell transplant with little or no requirement for chemotherapy, (Chhabra et al (2016), Science Translational Medicine, Vol: 8(351), pp. 351ra105) and similar approaches may be considered for other cell systems. In such a setting, patients normally undergo chemotherapy to eliminate the host HSC and enable engraftment of the infused HSC. However, the combination of antibodies against the host HSC and CD47 blockade through QPCTL inhibition would improve host HSC elimination without affecting the infused HSC, reducing or removing the requirement for toxic chemotherapy regimens. A preferred setting in such an approach is that the cell targeting components (e.g. a stem cell targeting antibody plus a modulator of the CD47-SIRPα axis) do not induce substantial clearance of the incoming cells. Therefore, because mature CD47 molecules on incoming cells are not affected by QPCTL inhibition, and because small molecule inhibitors of QPCTL can be designed such that QPCTL inhibition rapidly wanes at the time of cell infusion, the use of QPCTL inhibitors, as taught herein, represents a particular attractive approach to achieve this goal, relative to therapeutics that directly bind to either CD47 or SIRPα, which would also increase elimination of infused cells.

In some embodiments as disclosed herein, further advantages of targeting the CD47-SIRPα axis through inhibition of QPCTL (using the compounds as taught herein) are observed in the context of the treatment of tumors (any tumor types as taught herein, e.g. micro-satellite instable (MSI) tumors, melanoma, and others) having low major histocompatibility complex (MHC) I expression levels or for the treatment of tumors that have downregulated their MHC I expression levels as a result of T-cell based immunotherapies, such as TIL therapy, CAR T cell therapy, and T cell checkpoint blockade..

It is known that drugs targeting the CD47-SIRPα pathway (e.g. CD47 antibodies) have broad efficacy in inducing the phagocytosis of cancer cells. However, not all cancer cells or tumor respond to such therapy, i.e. some cancer cells or tumors exhibit intrinsic resistance to drugs targeting the CD47-SIRPα pathway (e.g. CD47 antibodies), which in turn protects them from phagocytosis. It was recently found that resistance to drugs targeting the CD47-SIRPα pathway was related to the levels of MHC class I in resistant cancer cells. Further, it was also shown that cancer cells lacking expression of both CD47 and MHC class I or having low expression levels of both CD47 and MHC class 1, were the most sensitive to phagocytosis (Barkal et al (2018), Nat Immunol., Vol. 19(1), pages 76-84). Therefore, in some embodiments, the compounds as taught herein, may be advantageously used to decrease or reduce or block the activity of the CD47-SIRPα pathway or decrease or reduce or block the binding between CD47 and SIRPα to treat tumors (any tumor types as taught herein, e.g. MSI tumors, melanoma) having low major histocompatibility complex (MHC) I expression levels, or for the treatment of tumors that have downregulated their MHC I expression levels as a result of T-cell based immunotherapies, so as to increase or promote or boost phagocytosis of the cancer cells in this context.

Further embodiments on the applications, uses, advantages and methods exploiting the present invention are disclosed in the following sections.

**Use of Active Agents to Alter the Post-Translational Modification of CD47**

In one aspect disclosed herein, using active agents and pharmaceutical compositions thereof that are capable of reducing the expression or enzymatic activity of QPCTL, QPCT, or combinations thereof in a cell expressing or overexpressing CD47 at its surface allows for reducing or preventing or blocking the addition of or the formation of pyroglutamate (pE) residues at the N-terminus portion of CD47. In some embodiments the compounds as disclosed herein are thereofor compounds that can be used to reduce, inhibit or prevent pyroglutamylation of CD47, for example in vitro or in vivo, for example to treat patients that would benefit from such reduced, inhibited or prevented pyroglutamylation of CD47.

In some embodiments, this further reduces the interaction or binding between CD47 and SIRPα. In some embodiments, this further reduces the activity of the CD47-SIRPα signaling axis. In some embodiments, this modulates or boosts or

increases immune cell-mediated killing of diseased cells (e.g. cancer cells) via e.g. phagocytosis of cells expressing CD47 by immune cells such as phagocytes (e.g. macrophages, neutrophils) or via antibody-dependent cellular cytotoxicity (ADCC) of cells expressing CD47 or via antibody-dependent cellular phagocytosis" (abbreviated (ADCP) of cells expressing CD47. In some embodiments, the "modulating (e.g. boosting or up-regulating or increasing) of the killing of a diseased cell (e.g. cancer cells) via ADCP or ADCC (using compounds as taught herein) involves or uses IgA antibodies (e.g. anti- Her2-IgA1 antibody or anti-CD47-IgA antibody, and others).

In some embodiments disclosed herein, reducing or preventing or blocking the addition of or the formation of pyroglutamate (pE) residues at the N-terminus portion of CD47 by using a compound or method as taught herein results in the prevention or inhibition or reduction of the "don't eat me signal" or "anti-phagocytic signal" conveyed by activation of the CD47-SIRPα signaling axis. In some embodiments, this causes an increased in immune cell-mediated killing (e.g. via phagocytosis or ADCC or ADCP) or increased killing activity (e.g. phagocytic activity or cytotoxic activity) of immune cells (e.g. macrophage, myeloid cells) toward cells expressing CD47, e.g. cancer cells. In some embodiments, the killing of a diseased cell (e.g. cancer cells) via ADCP or ADCC (using compounds as taught herein) involves or uses IgA antibodies (e.g. anti- Her2-IgA1 antibody or anti-CD47-IgA antibody, and others).

## Compounds and Compositions

In another aspect, disclosed herein is a pharmaceutical composition comprising an active agent for use in a method of treating a condition in a subject that would benefit from reducing signaling or binding between CD47 and SIRPα in the subject, wherein the active agent reduces expression or enzymatic activity of QPCTL, QPCT, or combinations thereof, in a cell with CD47 on the surface.

In some embodiments, the active agents are compounds, such as small molecules identified by screening methods such as those as taught herein, which have biological activity in a living system and which are able to reduce the expression or enzymatic activity of QPCTL, QPCT, or combinations thereof in a cell expressing CD47 on its surface.

In some embodiments, the active agents are inhibitors of QPCTL, QPCT, or combinations thereof such as PBD150, PQ912, PQ1565 (Buchholz M et al (2009), J. Med. Chem., Vol 52, pages 7069-7080; Buchholz M et al (2006), J. of Medicinal Chemistry, Vol. 49, pages 664-677; Schilling et al (2008), Nature Medicine, Vol. 14, pages 1106-1111; Lues et al (2015), Alzheimer's & Dementia: Translational Research & Clinical Interventions, Vol.1, pages 182-195), and other inhibitors of QPCTL, QPCT, or combinations thereof such as those disclosed in WO2004/098625 (EP1620082), WO2004/098591 (EP1620091), WO2005/039548 (EP1675578), WO2005/075436 (EP1713780), WO2011029920 (EP2475428), WO2014140279 (EP2970235), US8889709B2, and Hoang et al (2017), J. Med. Chem, Vol. 60, pages 2573-2590, which are incorporated herein in their entirety.

In some embodiments, the active agents (QPCT and/or QPCTL inhibitors) are compounds selected from compounds of Formula (I), (II), (III), (IV), (V), (VI), (VII), or (VIII), or from compounds disclosed in Tables A, B, C, D and/or E, as taught herein, e.g. e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066.

In some embodiments, non-limiting examples of active agents that are inhibitors of QPCTL, QPCT, or combinations thereof include the following compounds:

### 1. PBD150

In some embodiments, the active agent is PBD150. PBD150 is an inhibitor of QPCTL and/or QPCT, which was developed by Buchholz M et al (2006), J. of Medicinal Chemistry, Vol. 49, pages 664-677). PBD150 was shown to inhibit human QPCT and QPCTL with a $K_i$ value in the low nanomolar range (i.e. 60 nM). The chemical structure of compound PBD150 is below:

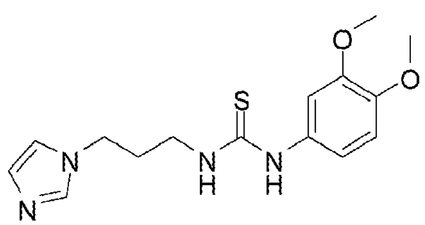

**PBD150**

**2. PQ912**

In some embodiments, the active agent is PQ912. PQ912 (also referred to as (S)-1-(1H-benzo[d]imidazole-5-yl)-5-(4-propoxyphenyl)imidazolidin-2-one) is a QPCT and/or QPCTL inhibitor currently being developed in clinical stage program by Probiodrug AG (Germany) for Alzheimer's Disease. PQ912 was developed according to a comprehensive drug discovery program (Buchholz et al (2006), J. Med. Chem, Vol. 49, pages 664-677; Buchholz et al (2009), J. Med. Chem, Vol. 52, pages 7069-7080; Ramsbeck et al (2013), J. Med. Chem, Vol. 56, pages 6613-6625; Hoffmann et al (2017), The Journal of Pharmacology and Experimental Therapeutics, Vol. 362, pages 119-130). Specifically, the compound PQ912 was shown to inhibit human, rat and mouse QPCT and QPCTL activity, with $K_i$ values ranging between 20 nM and 65 nM (Hoffmann et al (2017), The Journal of Pharmacology and Experimental Therapeutics, Vol. 362, pages 119-130). Compound PQ912 has been shown to be safe and well-tolerated by human subjects and revealed a high level of QPCT and QPCTL inhibition in a Phase 1 study with 200 healthy young and elderly volunteers (Lues et al (2015), Alzheimer's & Dementia: Translational Research & Clinical Interventions, Vol.1, pages 182-195). The chemical structure of compound PQ912 is below:

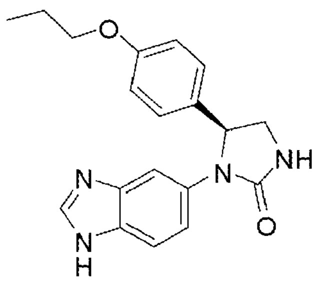

**PQ912**

**3. PQ1565**

In some embodiments, the active agent is PQ1565. PQ1565 is a QPCT and/or QPCTL inhibitor currently being developed in preclinical stage program by Probiodrug AG (Germany) for Alzheimer's disease.

**4. QPCT and QPCTL inhibitor compounds as disclosed in WO2008128983 and EP2160380**

In some embodiments, the active agent is a QPCT and/or QPCTL inhibitor as described in WO2008128983 and EP2160380. In some embodiments, the active agent is a compound having Formula (I) below, or a pharmaceutically acceptable salt, solvate, polymorph, tautomer, or stereoisomer thereof:

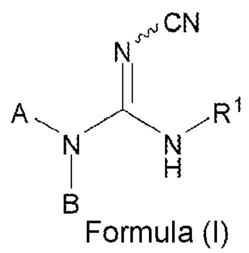

Formula (I)

wherein:

$R^1$ represents $C_{1-12}$alkyl; $C_{2-12}$alkenyl, wherein the double bond is not adjacent to the nitrogen; $C_{3-12}$carbocyclyl; -$C_{1-6}$alkyl- $C_{3-12}$carbocyclyl; $C_{3-12}$heterocyclyl; -$C_{1-6}$alkyl-$C_{312}$heterocyclyl; $C_{6-12}$aryl; $C_{5-12}$heteroaryl; -$C_{1-6}$alkylaryl; -$C_{1-6}$alkyl-$C_{5-12}$heteroaryl; -phenyl fused to $C_{3-12}$carbocyclyl or -phenyl fused to $C_{3-12}$heterocyclyl;
in which any of the aforesaid $C_{3-12}$carbocyclyl and $C_{3-12}$heterocyclyl groups may optionally be substituted by one or more groups selected from methyl and oxo;
and in which any of the aforesaid phenyl, $C_{6-12}$aryl and $C_{5-12}$heteroaryl groups may optionally be substituted by one or more substituents selected from $C_{1-6}$alkyl, $C_{2-6}$alkenyl, $C_{2-6}$alkynyl, $C_{1-6}$haloalkyl, -$C_{1-6}$thioalkyl, -$SO_2C_{1-4}$alkyl, $C_{1-6}$alkoxy-, -O-$C_{3-8}$cycloalkyl, $C_{3-8}$cycloalkyl, -$SO_2C_{3-8}$cycloalkyl, $C_{3-6}$alkenyloxy-, $C_{3-6}$alkynyloxy-, -C(O)$C_{1-6}$alkyl, $C_{1-6}$alkoxy-$C_{1-6}$alkyl-, nitro, halogen, cyano, hydroxyl, -C(O)OH, -$NH_2$, -NH$C_{1-4}$alkyl, -N($C_{1-4}$alkyl)($C_{1-4}$alkyl),

-C(O)N($C_{1-4}$alkyl)($C_{1-4}$alkyl), -C(O)$NH_2$,-C(O)NH($C_{1-4}$alkyl), -C(O)O$C_{1-6}$alkyl, -SO$C_{1-4}$alkyl and -SO$C_{3-6}$cycloalkyl; or $R^1$ represents phenyl substituted by phenyl, or phenyl substituted by an optionally substituted monocyclic $C_{5-12}$heteroaryl group; in which any of the aforesaid phenyl and monocyclic $C_{5-12}$heteroaryl groups may optionally be substituted by one or more groups selected from $C_{1-4}$alkyl, halogen and $C_{1-4}$alkoxy;
or $R^1$ represents phenyl substituted by benzyloxy- in which any of the aforesaid phenyl or benzyloxy groups may optionally be substituted on the ring by one or more groups selected from $C_{1-4}$alkyl, halogen and $C_{1-4}$alkoxy;

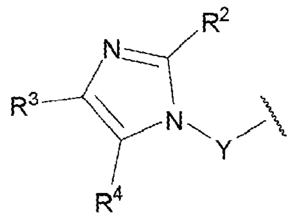

A represents

wherein Y represents a $C_{2-5}$ alkylene chain, which may optionally be substituted by one or two methyl groups or may optionally be substituted by two alkylene substituents at the same position wherein the two alkylene substituents are joined to each other to form a $C_{3-5}$spiro-cycloalkyl group;
and $R^2$, $R^3$ and $R^4$ independently represent H or $C_{1-2}$alkyl, provided that $R^2$ and $R^3$ and $R^4$ do not all represent H; and B represents H or methyl.

In some embodiments, the active agent is a QPCT and/or QPCTL inhibitor as described in WO2008128983 and EP2160380 and is selected from the compounds in Table A below:

**Table A.**

| Name | Structure |
|---|---|
| 2-cyano(4-ethylphenyl)-3-(3-(5-methyl-1 H-imidazol-1-yl)propyl) guanidine | 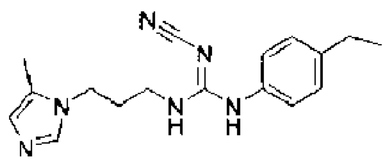 |
| (2-cyano(4-isopropylphenyl)-3-(3-(5-methyl-1H-imidazol-1-yl)propyl) guanidine | 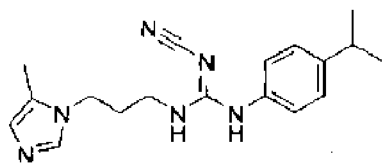 |
| 2-cyano(2,3-dihydrobenzo[b][1,4]di oxin-7-yl)-3-(3-(5-methyl-1 H-imidazol-1-yl)propyl)guanidine | 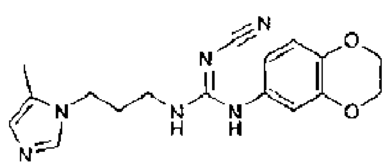 |
| 2-cyano(4-cyanophenyl)-3-(3-(5-methyl-1 H-imidazol-1-yl)propyl) guanidine | 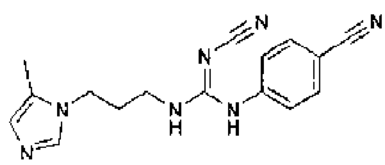 |

(continued)

| Name | Structure |
|---|---|
| 2-cyano(3,4,5-trimethoxyphenyl)-3-(3-(5-methyl-1 H-imidazol-1-yl)propyl) guanidine | 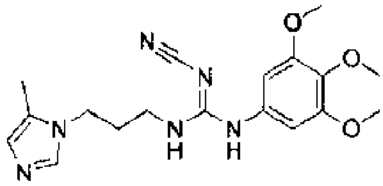 |
| 2-cyano(4-ethoxyphenyl)-3-(3-(5-methyl-1 H-imidazol-1-yl)propyl) guanidine | 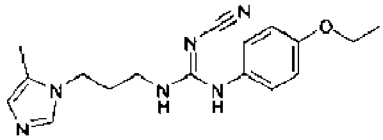 |
| 2-cyano(3-(5-methyl-1 H-imidazol-1-yl)propyl)-3-(3,4-dimethylphenyl) guanid ine | 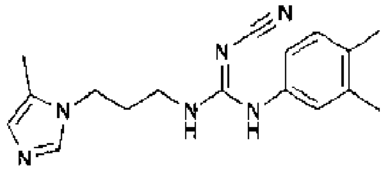 |
| (3-(5-methyl-1 H-imidazol-1-yl) propyl)-2-cyano-3-mesitylguanidine | 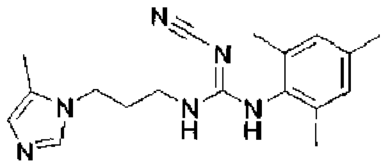 |
| (3-(5-methyl-1 H-imidazol-1-yl) propyl)-2-cyano-3-(biphenyl-4-yl) guanidine | 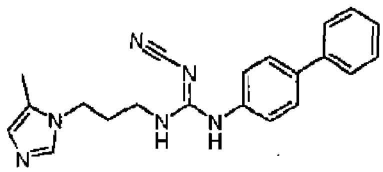 |
| (3-(5-methyl-1 H-imidazol-1-yl) propyl)-2-cyano-3-(naphthalen-2-yl) guanidine | 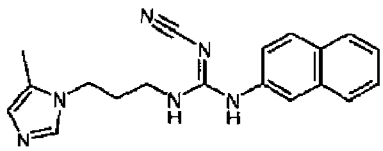 |
| (3-(5-methyl-1 H-imidazol-1-yl) propyl)-2-cyano-3-(naphthalen-1-yl) guanidine | 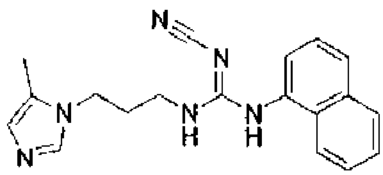 |
| (3-(5-methyl-1 H-imidazol-1-yl)propyl)-3-(benzo[c][1,2,5]thiadiazol-6-yl)-2-cyanoguanidine | 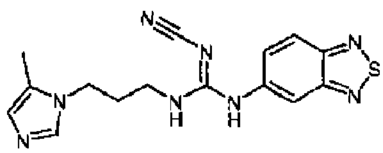 |
| (3-(5-methyl-1 H-imidazol-1-yl)propyl)-3-(3,4-dichlorophenyl)-2-cyanoguanidine | 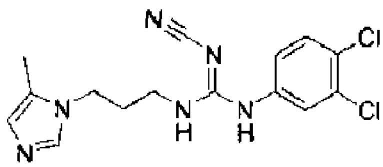 |

(continued)

| Name | Structure |
|---|---|
| (benzo[d][1,3]dioxol-6-yl)-2-cyano-3-(3-(5-methyl-1 H-imidazol-1-yl) propyl)guanidine | 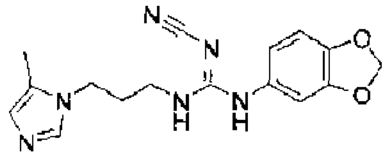 |
| 2-cyano(4-methoxyphenyl)-3-(3-(5-methyl-1 H-imidazol-1-yl)propyl) guanidine | 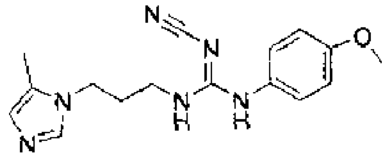 |
| 2-cyano(3,5-dimethoxyphenyl)-3-(3-(5-methyl-1 H-imidazol-1-yl)propyl) guanidine | 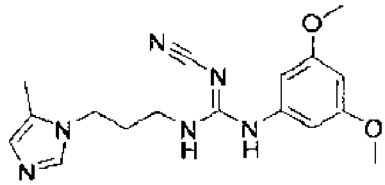 |
| 2-cyano(4-ethoxyphenyl)-3-(3-(4-methyl-1 H-imidazol-1-yl)propyl) guanidine | 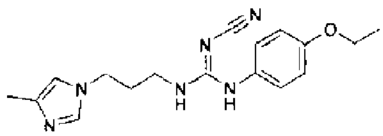 |
| 2-cyano(3,5-dimethoxyphenyl)-3-(3-(4-methyl-1 H-imidazol-1-yl)propyl) guanidine | 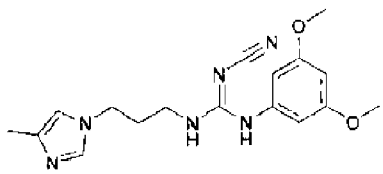 |
| 2-cyano(2,3-dihydrobenzo[b][1,4]di oxin-7-yl)-3-(3-(4-methyl-1 H-imidazol-1-yl)propyl)guanidine | 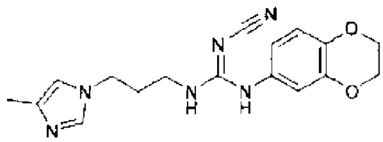 |
| 2-cyano(mesityl)-3-(3-(4-methyl-1 H-imidazol-1-yl)propyl)guanidine | 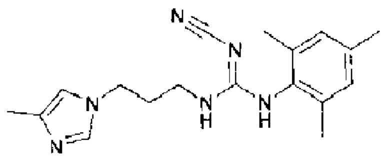 |
| 2-cyano(4-isopropylphenyl)-3-(3-(4-methyl-1 H-imidazol-1-yl)propyl) guanidine | 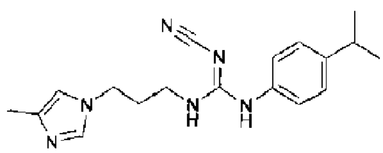 |
| 2-cyano(4-ethylphenyl)-3-(3-(4-methyl-1 H-imidazol-1-yl)propyl) guanidine | 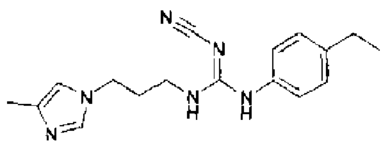 |

(continued)

| Name | Structure |
|---|---|
| 2-cyano(3-(4-methyl-1 H-imidazol-1-yl)propyl)-3-(naphthalen-1-yl) guanidine | 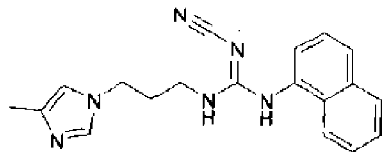 |
| (benzo[c][1,2,5]thiadia zol-6-yl)-2-cyano-3-(3-(4-methyl-1 H-imidazol-1-yl) propyl)guanidine | 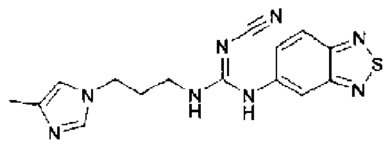 |
| 2-cyano(3,4,5-trimethoxyphenyl)-3-(3-(4-methyl-1 H-imidazol-1-yl)propyl) guanidine | 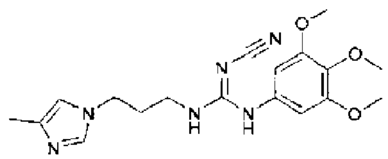 |
| 2-cyano(4-cyanophenyl)-3-(3-(4-methyl-1 H-imidazol-1-yl)propyl) guanidine | 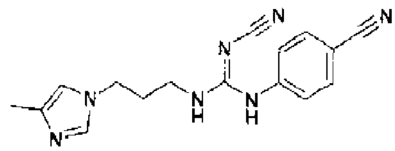 |
| (3,4-dichlorophenyl)-2-cyano-3-(3-(4-methyl-1 H-imidazol-1-yl)propyl) guanidine | 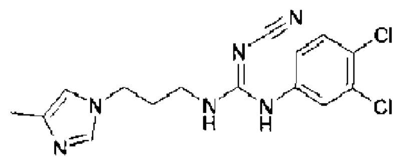 |
| 2-cyano(4-methoxyphenyl)-3-(3-(4-methyl-1 H-imidazol-1-yl)propyl) guanidine | 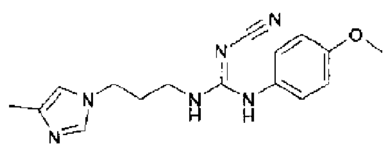 |
| 2-cyano-1-[3-(4-methyl-1 H-imidazol-1-yl)propyl]-4-phenylbenzene-1-guanidine | 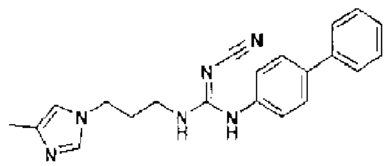 |
| 2-cyano(3-(4-methyl-1 H-imidazol-1-yl)propyl)-3-(naphthalen-2-yl) guanidine | 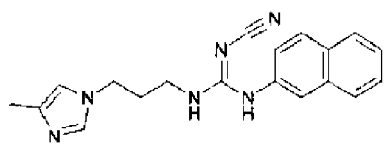 |

## 5. QPCT and QPCTL inhibitor compounds as disclosed in WO2004098591 and EP1620091

In some embodiments, the active agent is a QPCT and/or QPCTL inhibitor as described in WO2004098591 and EP1620091. In some embodiments, the active agent is a compound having formula (II) below, or a pharmaceutically acceptable salt, solvate, polymorph, tautomer, or stereoisomer thereof:

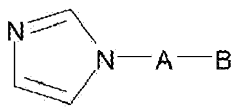

formula (II)

wherein:

A is a moiety selected from the group consisting of (II-a), (II-b), or (II-c):

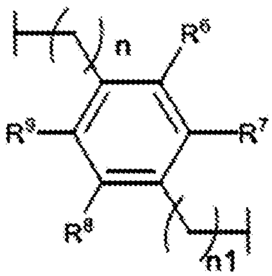

(II-a)

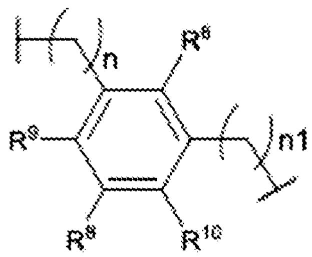

(II-b)

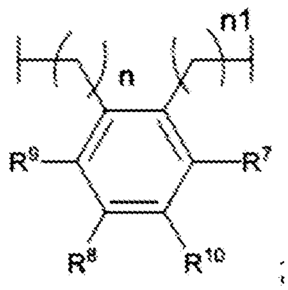

(II-c)

wherein:

$R^6$-$R^{10}$ are H or methyl;
n and n1 are independently 1-5; and

B is ;

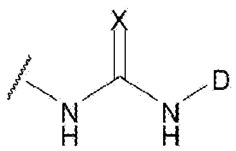

wherein:

D represents substituted phenyl, wherein substitution means -oxyalkyl, - thioalkyl, halogenyl;
or D represents dihydrobenzodioxine, benzodioxole, benzodithiole dihydrobenzodithiine, benzooxathiole or dihydrobenzooxathiine; and
X represents O, S, N-CN.

In some embodiments, the active agent is a QPCT and/or QPCTL inhibitor as described in Tables 2 and 3 of WO2004098591 and EP1620091, as well as the specific compounds disclosed in the examples in WO2004098591 and EP1620091.

**6. QPCT and QPCTL inhibitors compounds as disclosed in WO2005039548 and EP1675578.**

In some embodiments, the active agent is a QPCT and/or QPCTL inhibitor as described in WO2005039548 and EP1675578. In some embodiments, the active agent is a compound having formula (III) below, or a pharmaceutically acceptable salt, solvate, polymorph, tautomer, or stereoisomer thereof:

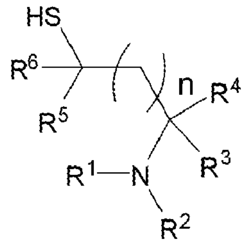

formula (III)

wherein:

$R^1$-$R^6$ are independently H or a branched or unbranched alkyl chain, a branched or unbranched alkenyl chain, a branched or unbranched alkynyl chain, carbocyclic, aryl, heteroaryl, heterocyclic, aza-amino acid, amino acid or a mimetic thereof, peptide or a mimetic thereof; all of the above residues optionally being substituted; and
n can be 0, or 2.

In some embodiments, the active agent is a QPCT and/or QPCTL inhibitor as described in Tables 2, 3 and 5 of WO2005039548 and EP1675578, as well as the specific compounds as described in the examples in WO2005039548 and EP1675578.

**7. QPCT and QPCTL inhibitors compounds as disclosed in WO2005075436 and EP1713780.**

In some embodiments, the active agent is a QPCT and/or QPCTL inhibitor as described in WO2005075436 and EP1713780. In some embodiments, the active agent is a compound having formula (IV) or (V) below, or a pharmaceutically acceptable salt, solvate, polymorph, tautomer, or stereoisomer thereof:

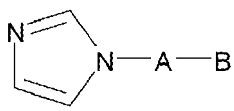

formula (IV)

wherein:

A is an unbranched $C_3$ alkyl chain; and
B is a group selected from (IV-a) or (IV-b):

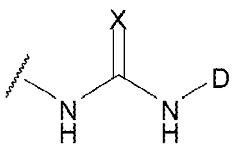

(IV-a)

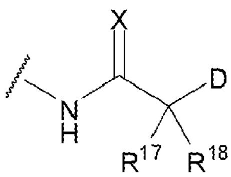

(IV-b)

wherein:

when B is a group (IV-a),
D represents dihydrobenzodioxine, benzodioxole, benzodithiole dihydrobenzodithiine, benzooxathiole or dihydrobenzooxathiine;
or D represents tert-butyl, benzyl, phenyl, 4-fluoro-phenyl, 4-chloro-phenyl, 4-ethyl-phenyl, 4-(trifluoromethyl)-phenyl, 4-(methoxycarbonyl)-phenyl, 4-(acetyl)-phenyl, 4-(methoxy)-phenyl, bicyclo[2.2.1]hept-5-en-2-yl, 3,4-(dimethoxy)-phenyl, 2,4-(dimethoxy)-phenyl, 3,5-(dimethoxy)-phenyl, 2-(methoxycarbonyl)-phenyl, 4-(oxazol-5-yl)-phenyl, 4-(pyrazol-1-yl)-phenyl, 4-isopropylphenyl, 4-(piperidine-1-sulfonyl)-phenyl, 4-(morpholin-4-yl)-phenyl, 4-cyano-phenyl 2,3-dihydro-benzo[1,4]-dioxin-6-yl, benzo[1,3]dioxol-5-yl, 3,4,5-(trimethoxy)-phenyl, 3-(methoxy)-phenyl, 4-(ethoxy)-phenyl, 4-(benzyloxy)-phenyl, 4-iodophenyl, 4-bromo-phenyl, 4-methyl-phenyl, naphthalen-1-yl, 4-nitro-phenyl, cyclooctyl, furan-2-yl-methyl, tetrahydrofuran-2-yl-methyl, benzo[1,3]dioxol-5-ylmethyl, 2-(morpholin-4-yl)-ethyl, 4-(methylsulfanyl)-phenyl, 4-(dimethylamino)-phenyl, 4-(trifluoromethoxy)-phenyl, benzoyl or pyridin-4-yl;
or when B is a group (IV-b),
D represents substituted phenyl, wherein substitution means alkoxy-, -thioalkyl, halogen, or a carboxylic acid alkyl or aryl ester;
or D represents dihydrobenzodioxine, benzodioxole, benzodithiole dihydrobenzodithiine, benzooxathiole or dihydrobenzooxathiine;
or when B is a group (IV-b) and $R^{17}$ and $R^{18}$ are both hydrogen, D is additionally phenyl; X represents S;
Y represents S;
one of $R^{17}$ and $R^{18}$ is H and the other is methyl;
or $R^{17}$ and $R^{18}$ can be connected to form a carbocycle with up to 6 ring atoms;
or when D represents phenyl or 3,4-(dimethoxy)-phenyl, the groups $R^{17}$ and $R^{18}$ are both H;
and wherein the term "alkyl" denotes a $C_{1-6}$ alkyl group.

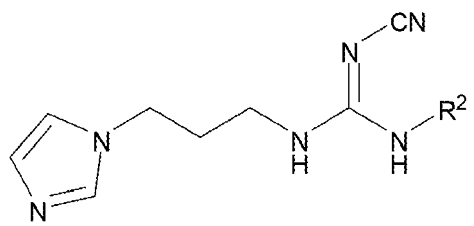

(V)

wherein:

$R^2$ represents phenyl optionally substituted at the 4-position with a substituent selected from ethoxy, benzyloxy, methoxy, acetyl, nitro, halo, methyl, ethyl, methylthio, dimethylamino or trifluoromethyl; or 3-methoxyphenyl, 3,4-dimethoxyphenyl, 2,4-dimethoxyphenyl, 3,5-dimethoxyphenyl or 3,4,5-trimethoxyphenyl;
or methyl, 2,3-dihydrobenzo[b][1,4]dioxin-7-yl, benzo[d][1,3]dioxol-6-yl, benzyl, naphthalenyl, cyclooctyl, tert-butyl, butyl, trityl, benzo[d][1,3]dioxol-6-ylmethyl, (tetrahydrofuran-2-yl)methyl, (furan-2-yl)methyl or 2-(morpholin-4-yl)ethyl.

**8. QPCT and QPCTL inhibitor compounds as disclosed in WO2014140279 and EP2970235**

In some embodiments, the active agent is a QPCT and/or QPCTL inhibitor as described in WO2014140279 and EP2970235. In some embodiments, the active agent is a compound having formula (VI) below, or a pharmaceutically acceptable salt, solvate, polymorph, tautomer, or stereoisomer thereof:

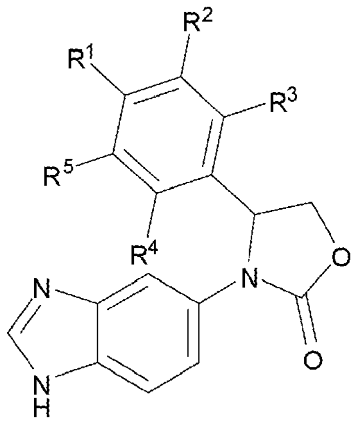

Formula (VI)

wherein:

$R^1$ represents alkyl, -O-alkyl, heterocyclyl or cycloalkyl;
$R^2$ and $R^3$ independently represent hydrogen, halogen or CN;
$R^4$ and $R^5$ independently represent hydrogen or halogen;
wherein at least one of $R^2$, $R^3$, $R^4$, and $R^5$ is halogen or CN;
and wherein the above alkyl, -O-alkyl, heterocyclyl or cycloalkyl groups are substituted by one or more halogen.

In some embodiments, the active agent is a QPCT and/or QPCTL inhibitor as described in WO2014140279 and EP2970235 and is selected from the compounds in Table B below. In some embodiments, the active agent is selected from examples 1-3, 5-15, 17-21, 23-26 and 28-30 in Table B below.

**Table B**

| Cpd. No. | Structure | Name |
|---|---|---|
| 1 | 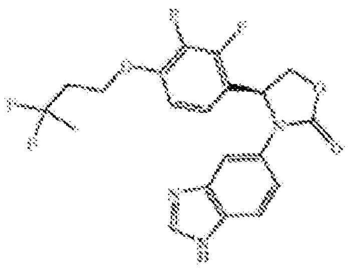 | (S)-3-(1 H-benzo[d]imidazol-5-yl)-4-(4-(3,3-difluorobutoxy)-2,3-difluorophenyl)-oxazolidin-2-one |
| 2 | 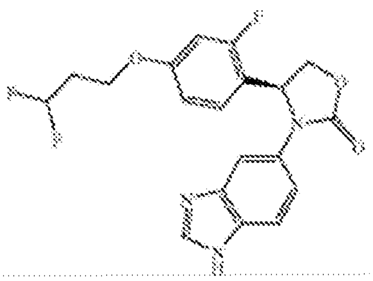 | (S)-3-(1H-benzo[d]imidazol-5-yl)-4-(4-(3,3-difluoropropoxy)-2-fluorophenyl)oxazolidin-2-one |

(continued)

| Cpd. No. | Structure | Name |
|---|---|---|
| 3 | 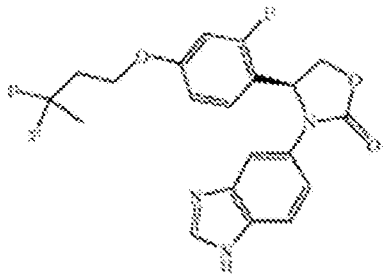 | (S)-3-(1 H-benzo[d]imidazol-5-yl)-4-(4-(3,3-difluorobutoxy)-2-fluorophenyl)oxazolidi n-2-one |
| 4 | 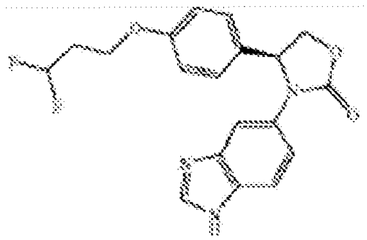 | (S)-3-(1 H-benzo[d]imidazol-5-yl)-4-(4-(3,3-difluoropropoxy) phenyl)oxa zolidin-2-one |
| 5 | 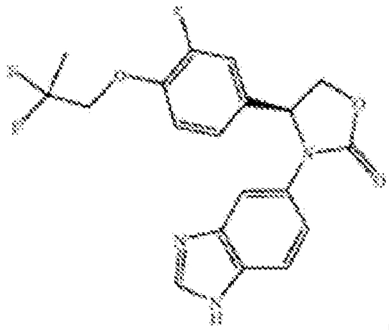 | (S)-3-(1 H-benzo[d]imidazol-5-yl)-4-(4-(2,2-difluoropropoxy)-3-fluorophenyl)oxazolidi n-2-one |
| 6 | 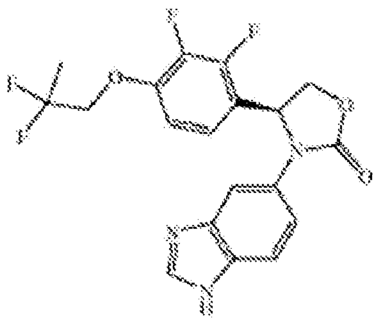 | (S)-3-(1H-benzo[d]imidazol-5-yl)-4-(4-(2,2-difluoropropoxy)-2,6-difluorophenyl)oxazolidin-2-one |
| 7 | 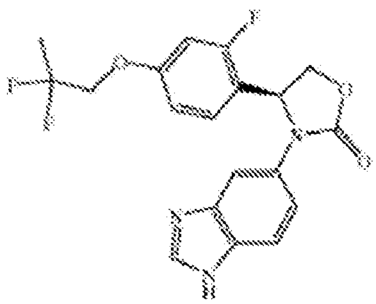 | (S)-3-(1H-benzo[d]imidazol-5-yl)-4-(4-(2,2-difluoropropoxy)-2-fluorophenyl)oxazolidin-2-one |

(continued)

| Cpd. No. | Structure | Name |
|---|---|---|
| 8 | 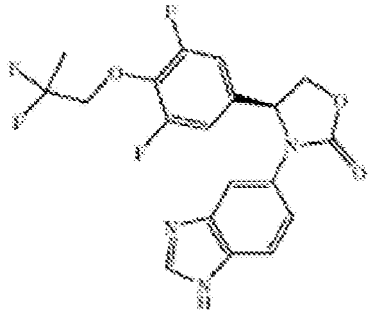 | (S)-3-(1H-benzo[d]imidazol-5-yl)-4-(4-(2,2-difluoropropoxy)-3,5-difluorophenyl)oxazolidi n-2-one |
| 10 | 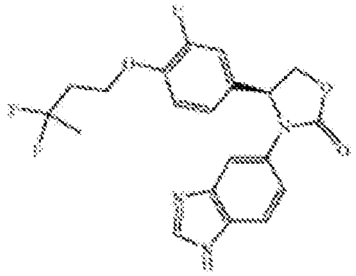 | (S)-3-(1H-benzo[d]imidazol-5-yl)-4-(4-(3,3-difluorobutoxy)-3-fluorophenyl)oxazolidin-2-one |
| 11 | 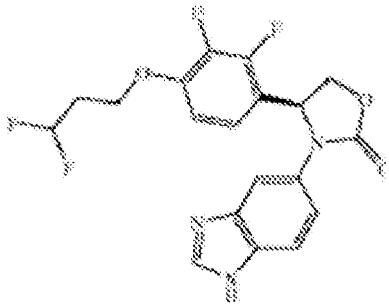 | (S)-3-(1H-benzo[d]imidazol-5-yl)-4-(4-(3,3-difluoropropoxy)-2,3-difluorophenyl)oxazolidi n-2-one |
| 12 | 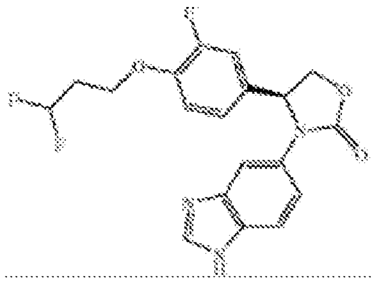 | (S)-3-(1 H-benzo[d]imidazol-5-yl)-4-(4-(3,3-difluoropropoxy)-3-fluorophenyl)oxazolidin-2-one |
| 13 | 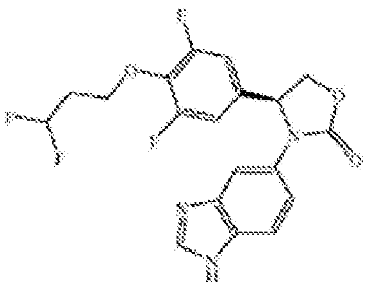 | S)-3-(1 H-benzo[d]imidazol-6yl)-4-(4-(3,3-difluoropropoxy)-3,5-difluorophenyl)oxazolidin-2-one |

(continued)

| Cpd. No. | Structure | Name |
|---|---|---|
| 14 | 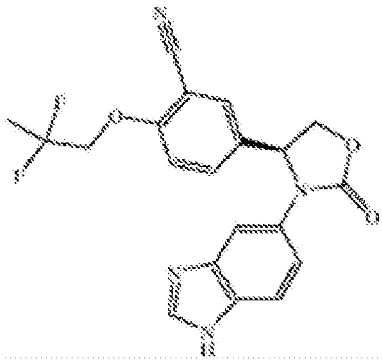 | (S)-5-(3-(1 H-benzo[d]imidazol-5-yl)-2-oxooxazolidin-4-yl)-2-(2,2-difluoropropoxy)benzonitril e |
| 15 | 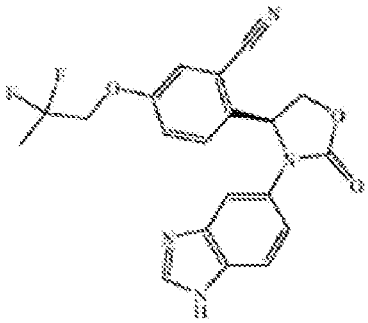 | (S)-2-(3-(1 H-benzo[d]imidazol-5-yl)-2-oxooxazolidin-4-yl)-5-(2,2-difluoropropoxy)benzonitrili e |
| 16 | 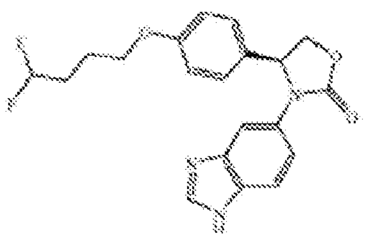 | (S)-3-(1 H-benzo[d]imidazol-5-yl)-4-(4-(4,4-difluorobutoxy) phenyl)oxaz olidin-2-one |
| 17 | 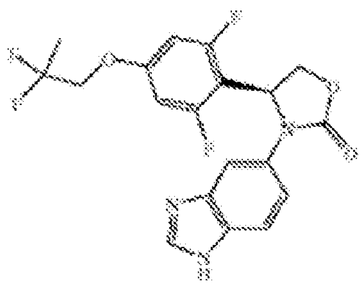 | (S)-3-(1H-benzo[d]imidazol-5-yl)-4-(4-(2,2-difluoropropoxy)-2,6-difluorophenyl)oxazolidin-2-one |
| 18 | 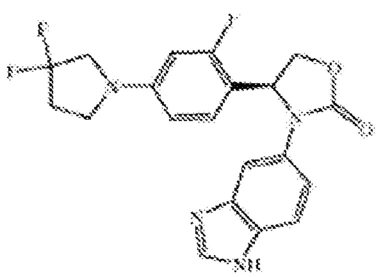 | (S)-3-(1H-benzo[d]imidazol-5-yl)-4-(4-(3,3-difluoropyrrolidin-1-yl)-2-fluorophenyl)-oxazolidin-2-one |

(continued)

| Cpd. No. | Structure | Name |
|---|---|---|
| 19 | 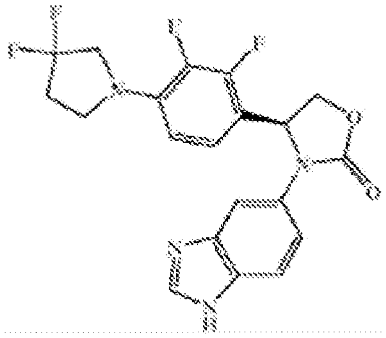 | (S)-3-(1 H-benzo[d]imidazol-5-yl)-4-(4-(3,3-difluoropyrrolidin-1-yl)-2,3-difluorophenyl)-oxazolidin-2-one |
| 20 | 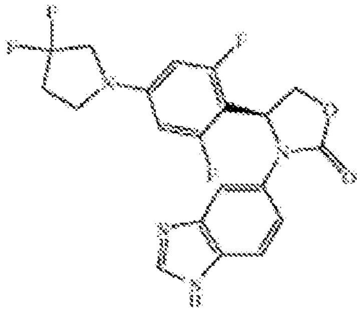 | (S)-3-(1 H-benzo[d]imidazol-5-yl)-4-(4-(3,3-difluoropyrrolidiri-1-yl)-2,6-difluorophenyl)oxazolidin-2-one |
| 21 | 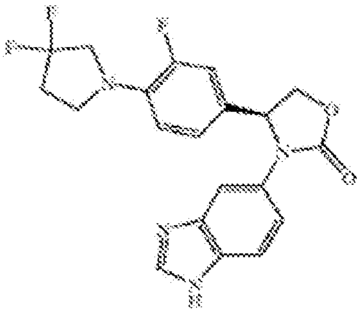 | (S)-3-(1H-benzo[d]imidazol-5-yl)-4-(4-(3,3-difluoropyrrolidin-1-yl)-3-fluorophenyl)oxazolidin-2-one |
| 22 | 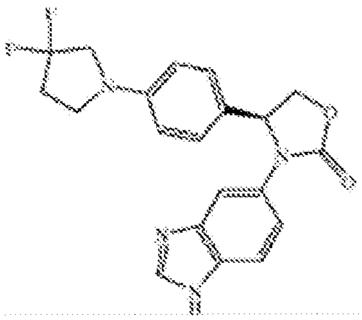 | 3-(1 H-benzo[d]imidazol-5-yl)-4-(4-(3,3-difluoropyrrolidin-1-yl)phenyl)oxazolidin-2-one |

(continued)

| Cpd. No. | Structure | Name |
|---|---|---|
| 23 | 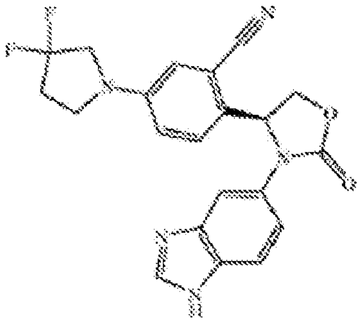 | (S)-2-(3-(1 H-benzo[d]imidazol-5-yl)-2-oxooxazolidin-4-yl)-5-(3,3-difluoropyrrolidin-1-yl)benzonitrile |
| 24 | 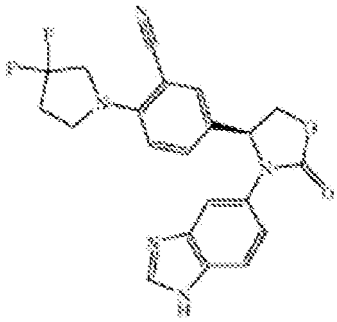 | (S)-5-(3-(1 H-benzo[d]imidazol-5-yl)-2-oxooxazolidin-4-yl)-2-(3,3-difluoropyrrolidin-1-yl)benzonitrile |
| 25 | 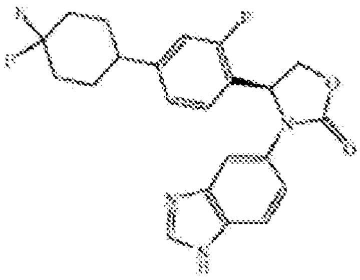 | (S)-3-(1H-benzo[d]imidazol-5-yl)-4-(4-(4,4-difluorocyclohexyl)-2-fluorophenyl)oxazolidi n-2-one |
| 26 | 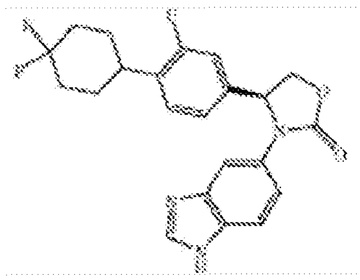 | (S)-3-(1 H-benzo[d]imidazol-5-yl)-4-(4-(4,4-difluorocyclohexyl)-3-fluorophenyl)oxazolidi n-2-one |
| 27 | 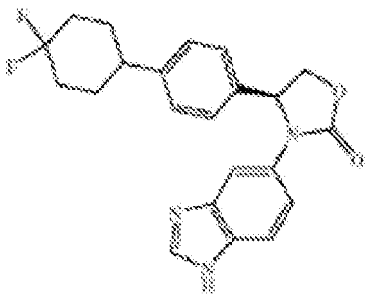 | (S)-3-(1 H-benzo[d]imidazol-5-yl)-4-(4-(4,4-difluorocyclohexyl)phenyl)o xazolidin-2-one |

(continued)

| Cpd. No. | Structure | Name |
|---|---|---|
| 28 | 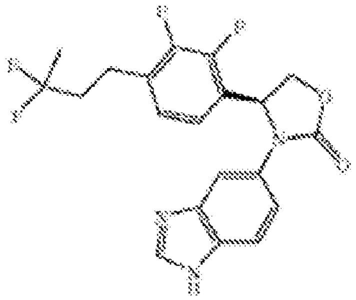 | (S)-3-(1H-benzo[d]imidazol-5-yl)-4-(4-(3,3-difluorobutyl)-2,3-difluorophenyl)oxazolidin-2-one |
| 29 | 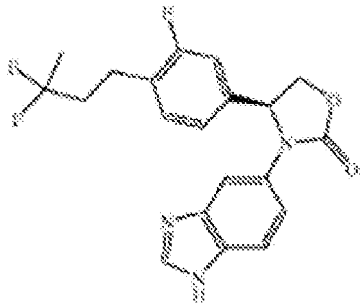 | (S)-3-(1 H-benzo[d]imidazol-5-yl)-4-(4-(3,3-difluorobutyl)-3-fluorophenyl)oxazolidi n-2-one |
| 30 | 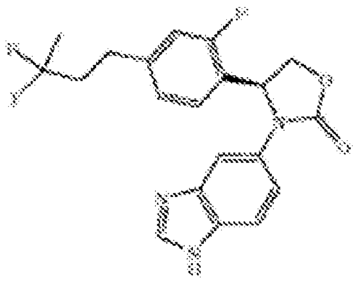 | (S)-3-(1 H-benzo[d]imidazol-5-yl-4-(4-(3,3-difluorobutyl)-2-fluorophenyl)oxazolidi n-2-one |

In some embodiments, the active agent is a QPCT and/or QPCTL inhibitor as described in WO2014140279 and EP2970235 and is selected from the compounds in Table C below.

**Table C**

| Cpd. No. | Structure | Name |
|---|---|---|
| 7a | 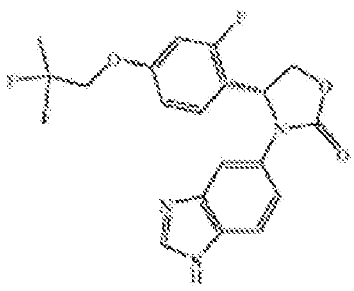 | (R)-3-(1 H-benzo[d]imidazole-5-yl)-4-(4-(2,2-difluoropropoxy)-2-fluorophenyl)oxazolidin-2-one |

(continued)

| Cpd. No. | Structure | Name |
|---|---|---|
| 7b | 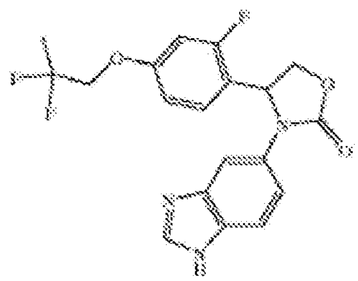 | 3-(1 H-benzo[d]imidazole-5-yl)-4-(4-(2,2-difluoropropoxy)-2-fluorophenyl)oxazolidin-2-one |
| 11a | 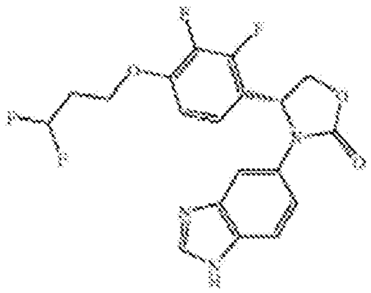 | ®-3-(1 H-benzo[d]imidazole-5-yl)-4-(4-(3,3-difluoropropoxy)-2,3-difluorophenyl)oxazolidin-2-one |

**9. QPCT and QPCTL inhibitor compounds as disclosed in Hoang et al (2017), J. Med. Chem, Vol. 60, pages 2573-2590.**

In some embodiments, the active agent is a QPCT and/or QPCTL inhibitor as described in Hoang et al (2017), J. Med. Chem, Vol. 60, pages 2573-2590. In some embodiments, the active agent is the compound referred to as "compound 212" below:

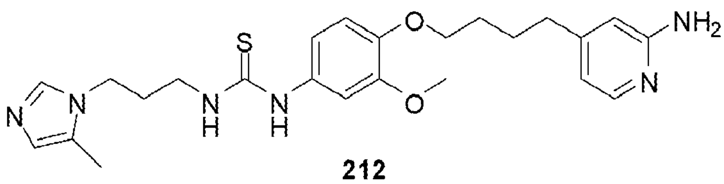

**212**

**10. QPCT and QPCTL inhibitor compounds as disclosed in WO2011029920 and EP2475428.**

In some embodiments, the active agent is a QPCT and/or QPCTL inhibitor as described in WO2011029920 and EP2475428. In some embodiments, the active agent is a compound having formula (VII) below, or a pharmaceutically acceptable salt, solvate, polymorph, tautomer, or stereoisomer thereof:

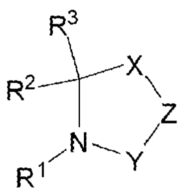

(VII)

wherein:

$R^1$ represents

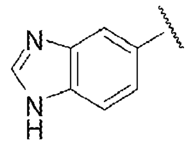 or 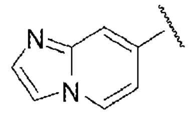 ;

$R^2$ represents $C_{1-8}$alkyl, aryl, heteroaryl, carbocyclyl, heterocyclyl, $-C_{1-4}$alkylaryl, $-C_{1-4}$alkylheteroaryl, $-C_{1-4}$alkylcarbocyclyl or $-C_{1-4}$alkylheterocyclyl; in which any of aforesaid aryl and heteroaryl groups may optionally be substituted by one or more groups selected from $C_{1-6}$alkyl, $C_{2-6}$alkenyl, $C_{2-6}$alkynyl, $C_{1-6}$haloalkyl, $-C_{1-6}$thioalkyl, $-SOC_{1-4}$alkyl, $-SO_2C_{1-4}$alkyl, $C_{1-6}$alkoxy-, $-O-C_{3-8}$cycloalkyl, $C_{3-8}$cycloalkyl, $-SO_2C_{3-8}$cycloalkyl, $-SOC_{3-6}$cycloalkyl, $C_{3-6}$alkenyloxy-, $C_{3-6}$alkynyloxy-, $-C(O)C_{1-6}$alkyl, $-C(O)OC_{1-6}$alkyl, $C_{1-6}$alkoxy-$C_{1-6}$alkyl-, $C_{1-6}$alkoxy-$C_{1-6}$alkoxy-, nitro, halogen, halo$C_{1-6}$alkyl, halo$C_{1-6}$alkoxy, cyano, hydroxyl, -C(O)OH, $-NH_2$, $-NHC_{1-4}$alkyl, $-N(C_{1-4}$alkyl)($C_{1-4}$alkyl), $-N(C_{1-4}$alkyl)($C_{1-4}$alkyl)-N($C_{1-4}$alkyl)($C_{1-4}$alkyl), $-C_{1-4}$alkyl-N($C_{1-4}$alkyl)($C_{1-4}$alkyl), $-C_{1-4}$alkoxy-N($C_{1-4}$alkyl)($C_{1-4}$alkyl), -N($C_{3-8}$cycloalkyll)($C_{3-8}$cycloalkyl), -N($-C_{1-6}$alkyl-$C_{1-6}$alkoxy)($-C_{1-6}$alkyl-$C_{1-6}$alkoxy), -C(O)N($C_{1-4}$alkyl)($C_{1-4}$alkyl), $-C(O)NH_2$, -C(O)NH($C_{1-4}$alkyl) and -C(O)NH($C_{3-10}$cycloalkyl); and in which any of aforesaid carbocyclyl and heterocyclyl groups may optionally be substituted by one or more groups selected from $C_{1-4}$alkyl, oxo, halogen, $-C(O)C_{1-6}$alkyl and $C_{1-4}$alkoxy;

or $R^2$ represents phenyl substituted by phenyl, phenyl substituted by a monocyclic heteroaryl group, phenyl substituted by phenoxy, phenyl substituted by heterocyclyl, phenyl substituted by heterocyclyl wherein said heterocyclyl is substituted by phenyl, phenyl substituted byy $-O-C_{1-4}$alkyl-heterocyclyl, phenyl substituted by benzyloxy, phenyl substituted by carbocyclyl, phenyl substituted by carbocyclyl wherein said carbocyclyl is substituted by heterocyclyl, phenyl substituted by -O-carbocyclyl, heterocyclyl substituted by phenyl, carbocyclyl substituted by phenyl, phenyl fused to carbocyclyl, phenyl fused to heterocyclyl, $-C_{1-4}$alkyl(phenyl substituted by phenyl), $-C_{1-4}$alkyl(phenyl substituted by a monocyclic heteroaryl group), $-C_{1-4}$alkyl(phenyl substituted by a monocyclic heterocyclyl group), $-C_{1-4}$alkyl(phenyl substituted by an -O-carbocyclyl group), $-C_{1-4}$alkyl(phenyl substituted by benzyloxy), $-C_{1-4}$alkyl(optionally substituted phenyl fused to optionally substituted carbocyclyl or $-C_{1-4}$alkyl(optionally substituted phenyl fused to optionally substituted heterocyclyl); in which any of aforesaid phenyl, benzyloxy and heteroaryl groups may optionally be substituted by one or more groups selected from $C_{1-4}$alkyl, halogen and $C_{1-4}$alkoxy, and in which any of aforesaid carbocyclyl and heterocyclyl groups may optionally be substituted by one or more groups selected from methyl, phenyl, oxo, halogen, hydroxyl and $C_{1-4}$alkoxy; $R^3$ represents H, $-C_{1-4}$alkyl or aryl; in which aforesaid aryl may optionally be substituted by one or more groups selected from $C_{1-6}$alkyl, $C_2$-salkenyl, $C_{2-6}$alkynyl, $C_{1-6}$haloalkyl, $-C_{1-3}$thioalkyl, $-SOC_{1-4}$alkyl, $-SO_2C_{1-4}$alkyl, $C_{1-6}$alkoxy-, $-O-C_{3-8}$cycloalkyl, $C_{3-8}$cycloalkyl, $-SO_2C_{3-8}$cycloalkyl, $-SOC_{3-6}$cycloalkyl, $C_{3-6}$alkenyloxy-, $C_{3-6}$alkynyloxy-, $-C(O)C_{1-6}$alkyl, $-C(O)OC_{1-6}$alkyl, $C_{1-6}$alkoxy-$C_{1-6}$alkyl-, nitro, halogen, cyano, hydroxyl, -C(O)OH, $-NH_2$, $-NHC_{1-6}$alkyl, -N($C_{1-4}$alkyl)($C_{1-4}$alkyl), -C(O)N($C_{1-4}$alkyl)($C_{1-4}$alkyl), $-C(O)NH_2$, -C(O)NH($C_{1-4}$alkyl) and, - C(O)NH($C_{3-10}$cycloalkyl);

or $R^2$ and $R^3$ are joined to form a carbocyclyl ring which is optionally substituted by one or more $C_{1-2}$alkyl groups;

or $R^2$ and $R^3$ are joined to form a carbocyclyl ring which is fused to phenyl, wherein aforesaid carbocyclyl and/or phenyl may optionally be substituted by one or more groups selected from $C_{1-4}$alkyl, halogen and $C_{1-4}$alkoxy;

or $R^2$ and $R^3$ are joined to form a carbocyclyl ring which is fused to monocyclic heteroaryl, wherein aforesaid carbocyclyl and/or heteroaryl may optionally be substituted by one or more groups selected from $C_{1-4}$alkyl, halogen and $C_{1-4}$alkoxy;

X represents C=O, O, S, $CR^7R^8$, $-O-CH_2-$ or $-CH_2-CH_2-$;

Y represents $CHR^9$, C=O or C=S;

Z represents $-N-R^4$, O or $CHR^{10}$, such that when X represents O or S, Z must represent $CHR^{10}$;

or X and Z represent two adjacent carbon atoms of a phenyl ring which is fused in that position and which is optionally substituted by one or more halogen or $C_{1-2}$alkyl groups;

$R^4$ represents H, $-C_{1-8}$alkyl, $-C(O)C_{1-6}$alkyl or $-NH_2$;

$R^7$ and $R^8$ independently represent H or $-C_{1-4}$ alkyl;

$R^9$ and $R^{10}$ independently represent H or methyl;

provided that the moiety -Y-Z-X- represents a moiety other than $-C(=O)-N(-R^4)-C(=O)-$ or $-C(=S)-N(-R^4)-C(=O)-$.

In some embodiments, the active agent is a QPCT and/or QPCTL inhibitor as described in WO2011029920 and EP2475428 and is selected from the compounds in Table D below.

**Table D**

| Chemical Name | Structure |
|---|---|
| 5-tert-butyl-1-(1 H-benzo[d]imidazol-5-yl)imidazolidin-2-one | 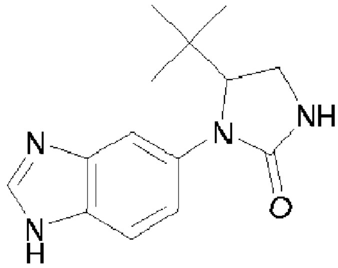 |
| 1-(1 H-benzo[d]imidazol-5-yl)-5-cyclohexylimidazolidin-2-one | 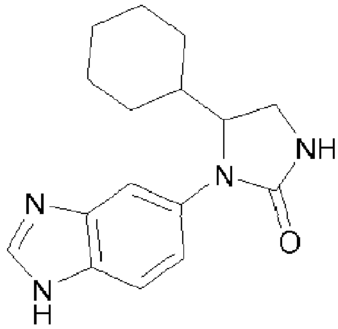 |
| 1-(1H-benzo[d]imidazol-5-yl)-5-phenylimidazolidin-2-one | 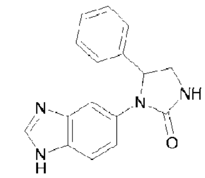 |
| 1-(1 H-benzo[d]imidazol-5-yl)-5-m-tolylimidazolidin-2-one | 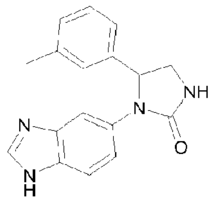 |
| 1-(1 H-benzo[d]imidazol-5-yl)-5-(4-methoxyphenyl)imidazolid in-2-one | 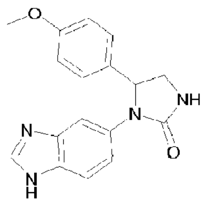 |
| 1-(1 H-benzo[d]imidazol-5-yl)-5-(4-methoxyphenyl)imidazolidin-2-one | 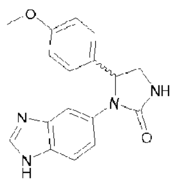 enantiomer 1 |

(continued)

| Chemical Name | Structure |
|---|---|
| 1-(1 H-benzo[d]imidazol-5-yl)-5-(4-methoxyphenyl)imidazolid in-2-one | 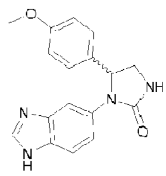 enantiomer 2 |
| (4R,5S)-1-(1 H-benzo[d]imidazol-6-yl)-5-(4-methoxyphenyl)-4-methylimidazolidin-2-one | 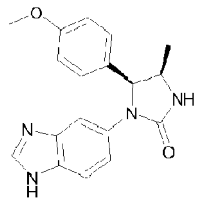 |
| 1-(1 H-benzo[d]imidazol-5-yl)-5-(3-methoxyphenyl)imidazolidin-2-one | 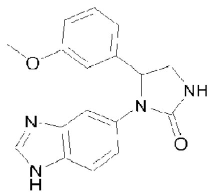 |
| 1-(1 H-benzo[d]imidazol-5-yl)-5-(2-methoxyphenyl) imidazolidin-2-one | 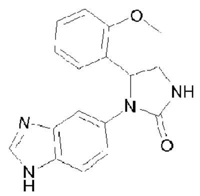 |
| 1-(1 H-benzo[d]imidazol-5-yl)-5-(4-ethoxyphenyl)imidazolidin-2-one | 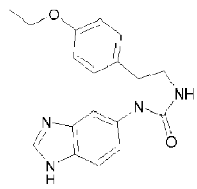 |
| 1-(1 H-benzo[d]imidazol-5-yl)-5-(4-propoxyphenyl) imidazolidin-2-one | 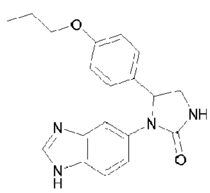 |
| (R)-1-(1 H-benzo[d]imidazol-5-yl)-5-(4-propoxyphenyl)imidazolidin-2-one | 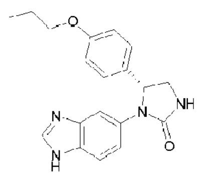 |
| (S)-1-(1 H-benzo[d]imidazol-5-yl)-5-(4-propoxyphenyl)imidazolidin-2-one | 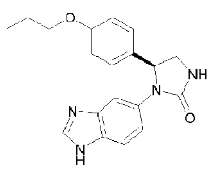 |

(continued)

| Chemical Name | Structure |
|---|---|
| 1-(1 H-benzo[d]imidazol-5-yl)-5-(4-butoxyphenyl) imidazolidin-2-one | 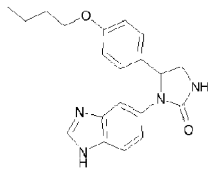 |
| 1-(1 H-benzo[d]imidazol-5-yl)-5-(4-(pentyloxy)phenyl)imidazolidin-2-one | 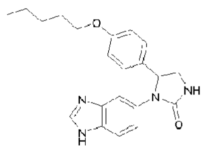 |
| 1-(1 H-benzo[d]imidazol-5-yl)-5-(4-isopropoxyphenyl) imidazolidin-2-one | 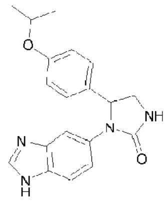 |
| 1-(1 H-benzo[d]imidazol-5-yl)-5-(4-methoxybenzo[d][1,3]dioxol-6-yl) imidazolidin-2-one | 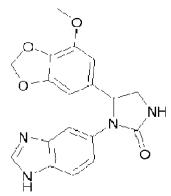 |
| 1-(1 H-benzo[d]imidazol-5-yl)-5-(2,3-dihydrobenzo[b][1,4]dioxin-6-yl) imidazolidin-2-one | 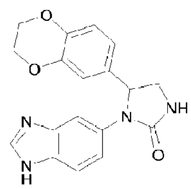 |
| 5-(4-(1,1,2,2-tetrafluoroethoxy)phenyl)-1-(1 H-benzo[d]imidazol-5-yl) imidazolidin-2-one | 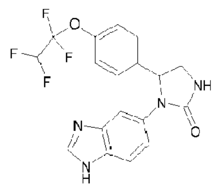 |
| 1-(1 H-benzo[d]imidazol-5-yl)-5-(2,2-difluorobenzo[d][1,3]dioxol-5-yl) imidazolidin-2-one | 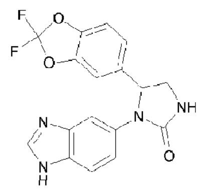 |
| 1-(1 H-benzo[d]imidazol-5-yl)-5-(3-fluoro-4-methoxyphenyl)imidazolidin-2-one | 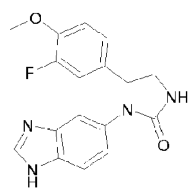 |

(continued)

| Chemical Name | Structure |
|---|---|
| 1-(1 H-benzo[d]imidazol-5-yl)-5-(2,6-difluoro-4-methoxyphenyl)imidazolidin-2-one | 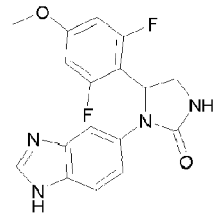 |
| 5-(4-(2-morpholinoethoxy)phenyl)-1-(1 H-benzo[d]imidazol-6-yl)imidazolidin-2-one | 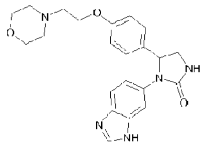 |
| 5-(4-(3-morpholinopropoxy)phenyl)-1-(1H-benzo[d]imidazol-5-yl)imidazolidin-2-one | 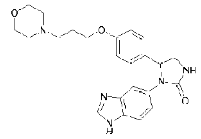 |
| 5-(2-(2-morpholinoethoxy)phenyl)-1-(1 H-benzo[d]imidazol-5-yl)imidazolidin-2-one | 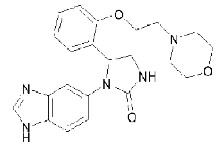 |
| 1-(1 H-benzo[d]imidazol-5-yl)-5-(4-fluorophenyl)imidazolidin-2-one | 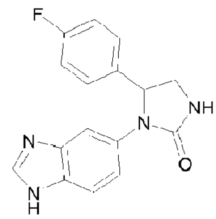 |
| 1-(1 H-benzo[d]imidazol-5-yl)-5-(2-fluorophenyl)imidazolidin-2-one | 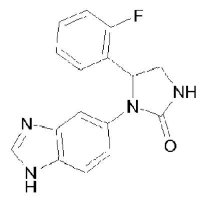 |
| 1-(1 H-benzo[d]imidazol-5-yl)-5-(3-fluorophenyl)imidazolidin-2-one | 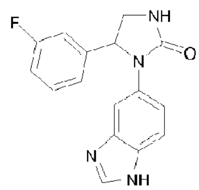 |
| 1-(1 H-benzo[d]imidazol-5-yl)-5-(2,6-difluorophenyl) imidazolidin-2-one | 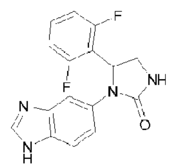 |

(continued)

| Chemical Name | Structure |
|---|---|
| 1-(1 H-benzo[d]imidazol-5-yl)-5-(3,4-difluorophenyl) imidazolidin-2-one | 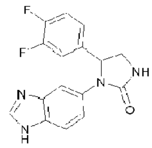 |
| 1-(1H-benzo[d]imidazol-5-yl)-5-(2-fluoro-5-(trifluoromethyl)phenyl) imidazolidin-2-one | 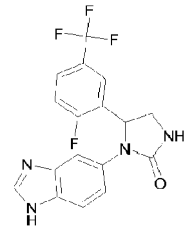 |
| 1-(1 H-benzo[d]imidazol-5-yl)-5-(3-fluoro-5-(trifluoromethyl)phenyl) imidazolidin-2-one | 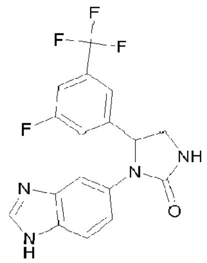 |
| 1-(1H-benzo[d]imidazol-5-yl)-5-(2-fluoro-4-(trifluoromethyl)phenyl) imidazolidin-2-one | 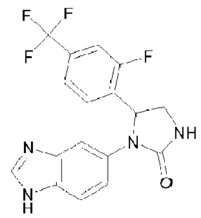 |
| 1-(1H-benzo[d]imidazol-5-yl)-5-(3-fluoro-4-(trifluoromethyl)phenyl) imidazolidin-2-one | 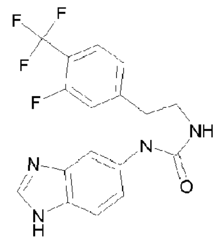 |
| 1-(1 H-benzo[d]imidazol-5-yl)-5-(2-chlorophenyl)imidazolidin-2-one | 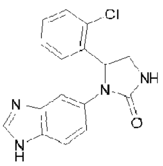 |
| 1-(1 H-benzo[d]imidazol-5-yl)-5-(3-chlorophenyl)imidazolidin-2-one | 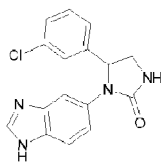 |

(continued)

| Chemical Name | Structure |
|---|---|
| 1-(1 H-benzo[d]imidazol-5-yl)-5-(2,6-dichlorophenyl) imidazolidin-2-one | 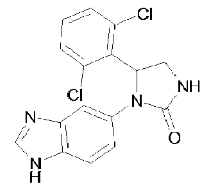 |
| 1-(1 H-benzo[d]imidazol-5-yl)-5-(2,3-dichlorophenyl) imidazolidin-2-one | 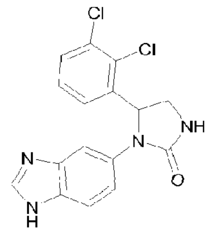 |
| 1-(1 H-benzo[d]imidazol-5-yl)-5-(3,4-dichlorophenyl) imidazolidin-2-one | 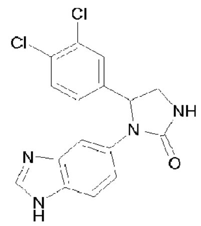 |
| (S)-1-(1 H-benzo[d]imidazol-5-yl)-5-(3,4-dichlorophenyl) imidazolidin-2-one | 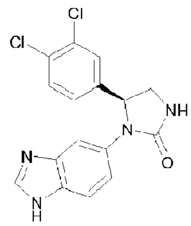 |
| 1-(1 H-1 ,3-benzodiazol-5-yl)-5-(4-biphenyl)imidazolidin-2-one | 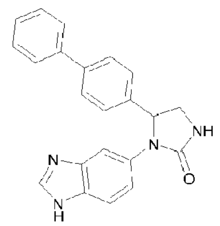 |
| (S)-1-(1 H-1,3-benzodiazol-5-yl)-5-(4-biphenyl)imidazolidin-2-one | 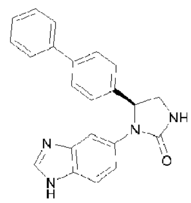 |
| (R)-1-(1H-1,3-benzodiazol-5-yl)-5-(4-biphenyl)imidazolidin-2-one | 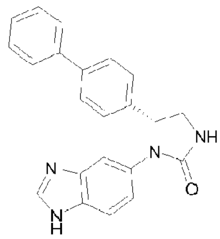 |

(continued)

| Chemical Name | Structure |
|---|---|
| 1-(1 H-1 ,3-benzodiazol-5-yl)-5-(3-fluoro-4-biphenyl)imidazolidin-2-one | 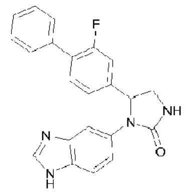 |
| 1-(1 H-benzo[d]imidazol-5-yl)-5-[4-(3-chlorophenyl)phenyl]imidazolidin-2-one | 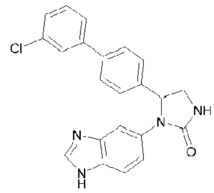 |
| 1-(1 H-benzo[d]imidazol-5-yl)-5-(3',4'-dichloro-4-biphenyl)imidazolidin-2-one | 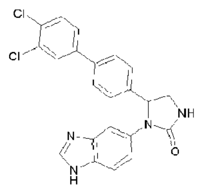 |
| 1-(1 H-benzo[d]imidazol-5-yl)-5-(3-phenylphenyl)imidazolidin -2-one | 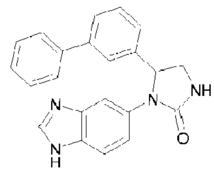 |
| 1-(1 H-benzo[d]imidazol-5-yl)-5-[3-(3-chlorophenyl)phenyl]imidazolidin-2-one | 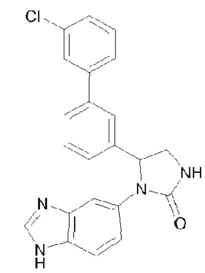 |
| 1-(1 H-benzo[d]imidazol-5-yl)-5-(3-chloro-4-morpholinophenyl)imidazolidin-2-one | 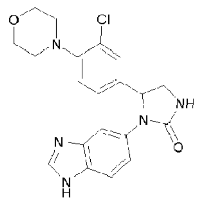 |
| 1-(1 H-benzo[d]imidazol-5-yl)-5-(4-(4-phenylpiperazin-1-yl)phenyl) imidazolidin-2-one | 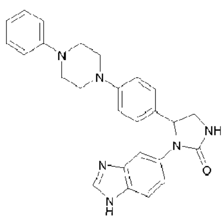 |

(continued)

| Chemical Name | Structure |
|---|---|
| 1-(1 H-benzo[d]imidazol-5-yl)-5-(2-chloro-6-(4-ethylpiperazin-1-yl)phenyl) imidazolidin-2-one | 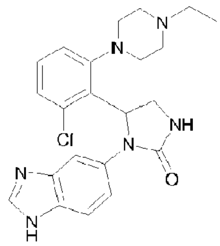 |
| 1-(H-imidazo[1,2-a]pyridin-7-yl)-5-phenylimidazol idin-2-one | 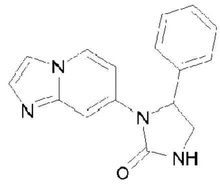 |
| 1-(H-imidazo[1,2-a]pyridin-7-yl)-5-(4-propoxyphenyl)imidazolidin-2-one | 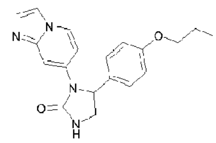 |
| 5-(4-butoxyphenyl)-1-(H-imidazo[1,2-a]pyridin-7-yl)imidazolidin-2-one | 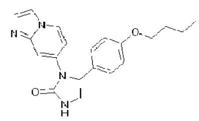 |
| 5-(2,6-difluoro-4-methoxyphenyl)-1-(H-imidazo[1,2-a]pyridin-7-yl)imidazolidin-2-one | 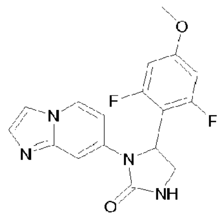 |
| 1-(H-imidazo[1,2-a]pyridin-7-yl)-5-(4-methoxybenzo[d][1,3]dioxol-6-yl) imidazolidin-2-one | 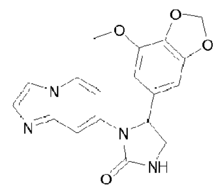 |
| 5-(4-(2-morpholinoethoxy)phenyl)-1-(H-imidazo[1,2-a]pyridin-7-yl) imidazolidin-2-one | 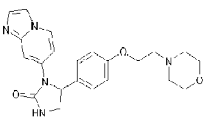 |
| 5-(2,6-difluorophenyl)-1-(H-imidazo[1,2-a]pyridin-7-yl)imidazolidin-2-one | 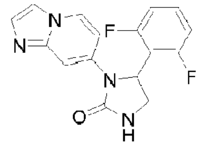 |

(continued)

| Chemical Name | Structure |
|---|---|
| 5-(biphenyl)-1-(H-imidazo[1,2-a]pyridin-7-yl)imidazolidin-2-one | 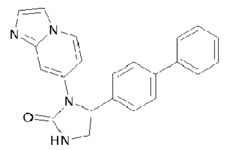 |
| 5-(3-fluorobiphenyl)-1-(H-imidazo[1,2-a]pyridin-7-yl)imidazolidin-2-one | 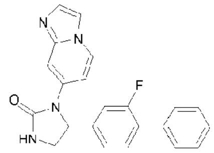 |
| 1-(H-imidazo[1,2-a]pyridin-7-yl)-5-(4-(4-phenylpiperazin-1-yl)phenyl) imidazolidin-2-one | 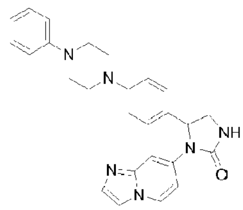 |
| 1-(1H-benzo[d]imidazol-5-yl)-5-phenylimidazolidin-4-one | 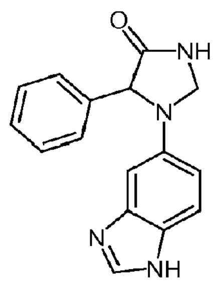 |
| 1-(1H-benzo[d]imidazol-5-yl)-5-(2,3,5-trifluorophenyl)imidazolidin-4-one | 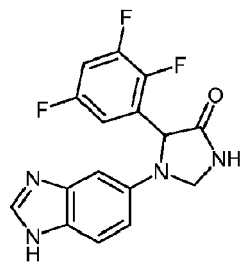 |
| 1-Amino-3-(1 H-benzo[d]imidazol-5-yl)-4-(4-methoxyphenyl)imidazolidin-2-one | 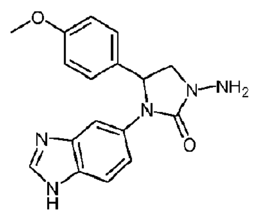 |
| (S)-3-(1 H-benzo[d]imidazol-6-yl)-4-phenyloxazolidin-2-one | 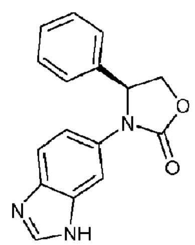 |

(continued)

| Chemical Name | Structure |
|---|---|
| (R)-3-(1 H-benzo[d]imidazol-6-yl)-4-phenyloxazolidin-2-one | 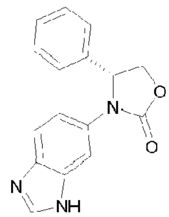 |
| (S)-3-(1 H-benzo[d]imidazol-5-yl)-4-isopropyloxazolidin-2-one | 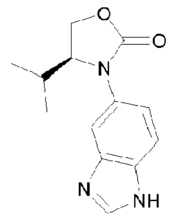 |
| (S)-3-(1 H-benzo[d]imidazol-5-yl)-4-benzyloxazolidin-2-one | 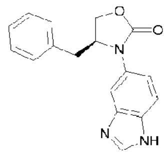 |
| (4S,5R)-3-(1H-benzo[d]imidazol-6-yl)-4,5-diphenyloxazolidin-2-one | 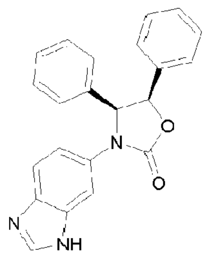 |
| (4S,5S)-3-(1 H-benzo[d]imidazol-6-yl)-5-methyl-4-phenyloxazolidin-2-one | 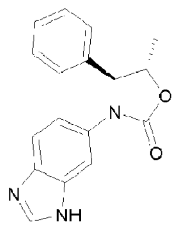 |
| (S)-3-(1 H-benzo[d]imidazol-6-yl)-5,5-dimethyl-4-phenyloxazolidin-2-one | 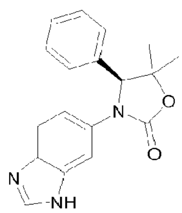 |
| (S)-3-(1 H-benzo[d]imidazol-6-yl)-4-(4-propoxyphenyl)oxazolidin -2-one | 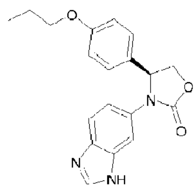 |

(continued)

| Chemical Name | Structure |
|---|---|
| (S)-3-(1 H-benzo[d]imidazol-6-yl)-4-(2,3-dihydrobenzo[b][1,4]dioxin-7-yl) oxazolidin-2-one | 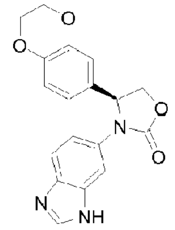 |
| (S)-4-(benzo[d][1,3]dioxol-6-yl)-3-(1 H-benzo[d]imidazol-6-yl)oxazolidin-2-one | 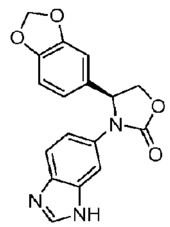 |
| (4S,5R)-3-(1H-benzo[d]imidazol-6-yl)-4,5-bis(4-propoxyphenyl)oxazolidin-2-one | 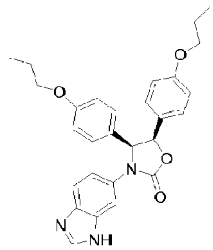 diastereomer 1 |
| (4S,5R)-3-(1 H-benzo[d]imidazol-6-yl)-4,5-bis(4-propoxyphenyl)oxazolidin-2-one | 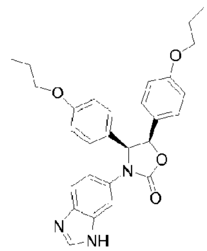 diastereomer 2 |
| 3-(1 H-benzo[d]imidazol-6-yl)-5-phenyl-4-(4-propoxyphenyl)oxazolidin-2-one | 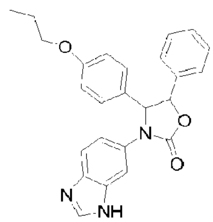 diastereomer 1 |
| (1 H-benzo[d]imidazol-6-yl)-5-phenyl-4-(4-propoxyphenyl)oxazolidin-2-one | 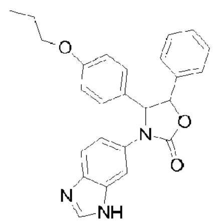 diastereomer 2 |

(continued)

| Chemical Name | Structure |
|---|---|
| (S)-4-(4-(2-(piperazin-1-yl)ethoxy)phenyl)-3-(1 H-benzo[d]imidazol-6-yl) oxazolidin-2-one | 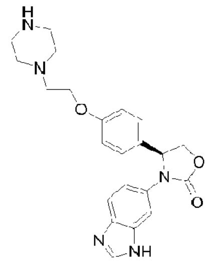 |
| (S)-4-(4-(2-morpholinoethoxy)phenyl)-3-(1 H-benzo[d]imidazol-6-yl) oxazolidin-2-one | 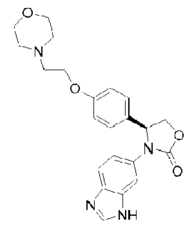 |
| (S)-3-(1 H-benzo[d]imidazol-6-yl)-4-(2,3-difluorophenyl)oxazolidin-2-one | 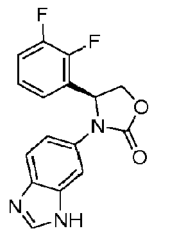 |
| (S)-3-(1 H-benzo[d]imidazol-6-yl)-4-(3-fluorophenyl)oxazolidin-2-one | 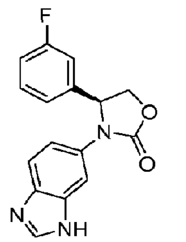 |
| (S)-3-(1 H-benzo[d]imidazol-6-yl)-4-(3-fluoro-5-(trifluoromethyl)phenyl) oxazolidin-2-one | 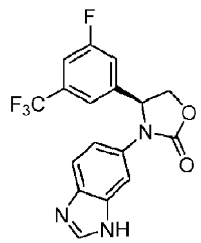 |
| (S)-3-(1 H-benzo[d]imidazol-6-yl)-4-(3-chlorophenyl)oxazolidin-2-one | 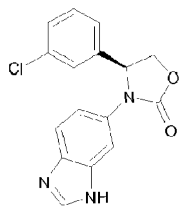 |

(continued)

| Chemical Name | Structure |
|---|---|
| (S)-3-(1 H-benzo[d]imidazol-6-yl)-4-(4-chlorophenyl)oxazolidin-2-one | 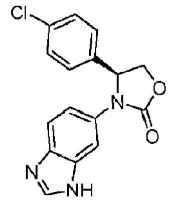 |
| (S)-3-(1 H-benzo[d]imidazol-6-yl)-4-[4-(3-chlorophenyl)phenyl]oxazolidin-2-one | 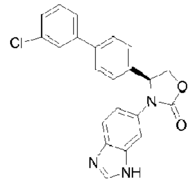 |
| (S)-3-(1 H-benzo[d]imidazol-6-yl)-4-[3-(3-chlorophenyl)phenyl]oxazolidin-2-one | 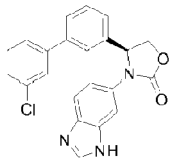 |
| (S)-3-(1 H-benzo[d]imidazol-6-yl)-4-(4-(4-phenylpiperazin-1-yl)phenyl) oxazolidin-2-one | 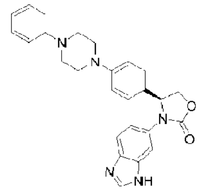 |
| (S)-3-(1H-benzo[d]imidazol-6-yl)-4-(4-(4-methylpiperazin-1-yl)phenyl) oxazolidin-2-one | 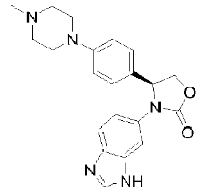 |
| (S)-3-(1 H-benzo[d]imidazol-6-yl)-4-(3-(4-phenylpiperazin-1-yl)phenyl) oxazolidin-2-one | 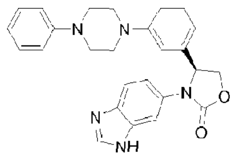 |
| (S)-3-(2-methyl-1 H-benzo[d]imidazol-6-yl)-4-phenyloxazolidin-2-one | 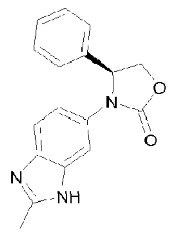 |
| (S)-4-(1 H-benzo[d]imidazol-6-yl)-5-(4-propoxyphenyl)morpholin-3-one | 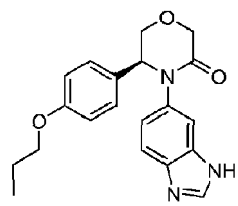 |

(continued)

| Chemical Name | Structure |
|---|---|
| 3-(1 H-benzo[d]imidazol-6-yl)-4-(4-propoxyphenyl)-1,3-oxazinan-2-one | 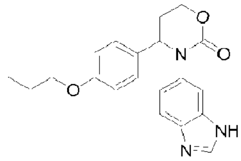 |
| (S)-3-(H-imidazo[1,2-a]pyridin-7-yl)-4-phenyloxazolidin-2-one | 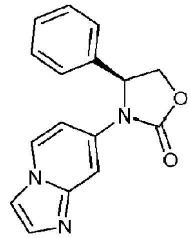 |
| (4S,5R)-3-(H-imidazo[1,2-a]pyridin-7-yl)-4,5-diphenyloxazolidin-2-one | 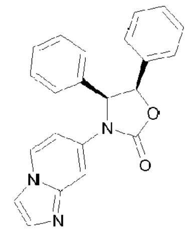 |
| (4S,5R)-3-(imidazo[1,2-a]pyridin-6-yl)-4,5-diphenyloxazolidin-2-one | 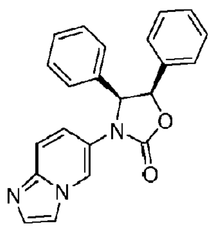 |
| (S)-3-(H-imidazo[1,2-a]pyridin-7-yl)-4-(4-propoxyphenyl)oxazolidin-2-one | 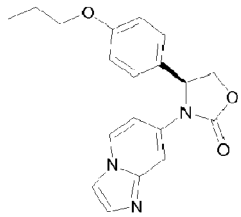 |
| (S)-4-(4-chlorophenyl)-3-(H-imidazo[1,2-a]pyridin-7-yl)oxazolidin-2-one | 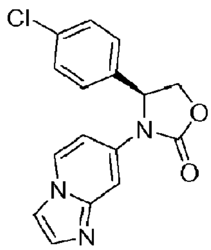 |
| 3-(imidazo[1,2-a]pyridin-7-yl)-4-(4-propoxyphenyl)-1,3-oxazinan-2-one | 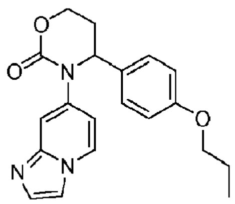 |

(continued)

| Chemical Name | Structure |
|---|---|
| 5-(2-phenylpyrrolidin-1-yl)-1 H-benzo[d]imidazole | 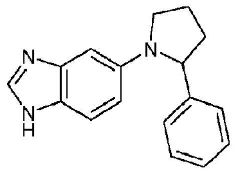 |
| 5-(2-(4-methoxyphenyl)pyrrolidin-1-yl)-1 H-benzo[d]imidazole | 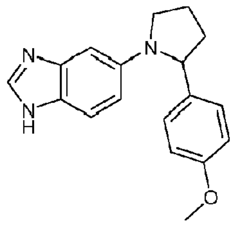 |
| 5-(2-(4-fluorophenyl)pyrrolidin-1-yl)-1 H-benzo[d]imidazole | 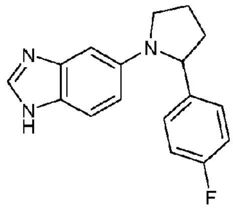 |
| 5-(2-(4-chlorophenyl)pyrrolidin-1-yl)-1 H-benzo[d]imidazole | 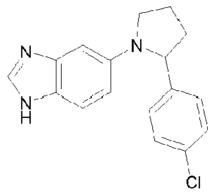 |
| 5-(2-benzylpyrrolidin-1-yl)-1 H-benzo[d]imidazole | 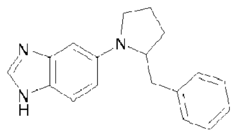 |
| 5-(2-(4-chlorobenzyl)pyrrolidin-1-yl)-1H-benzo[d]imidazole | 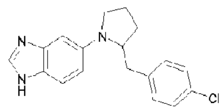 |
| 5-(2-(4-fluorobenzyl)pyrrolidin-1-yl)-1 H-benzo[d]imidazole | 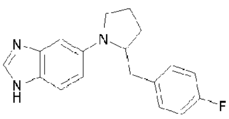 |
| 5-(pyrrolidin-1-yl)-1 H-benzo[d]imidazole | 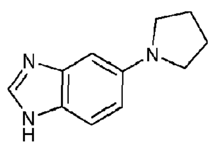 |
| 5-(2-(4-methoxybenzyl)pyrrolidin-1-yl)-1 H-benzo[d]imidazole | 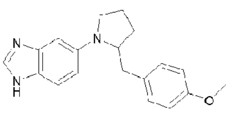 |

(continued)

| Chemical Name | Structure |
|---|---|
| 3-(1 H-benzo[d]imidazol-6-yl)-2-(4-chlorophenyl)thiazolidin-4-one | 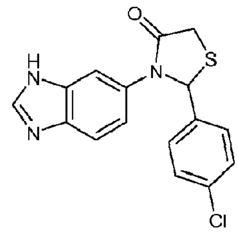 |
| 3-(1 H-benzo[d]imidazol-5-yl)-2-phenylthiazolidin-4-one | 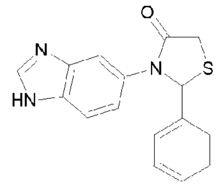 |
| 3-(1 H-benzo[d]imidazol-6-yl)-2-(4-fluorophenyl)thiazolidin-4-one | 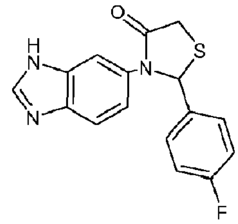 |
| 3-(1 H-benzo[d]imidazol-6-yl)-2-(naphthalen-1-yl)thiazolidin-4-one | 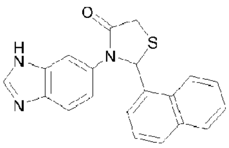 |
| 3-(1 H-benzo[d]imidazol-6-yl)-2-(4-phenoxyphenyl)thiazolidin-4-one | 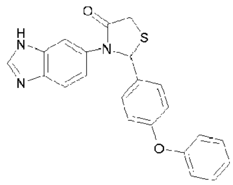 |
| 3-(1 H-benzo[d]imidazol-6-yl)-2-(2,6-difluorophenyl)thiazolidin-4-one | 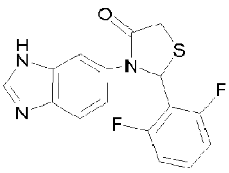 |
| 3-(1 H-benzo[d]imidazol-6-yl)-2-(thiophen-3-yl)thiazolidin-4-one | 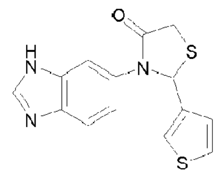 |
| 3-(1 H-benzo[d]imidazol-6-yl)-5-methyl-2-phenylthiazolidin-4-one | 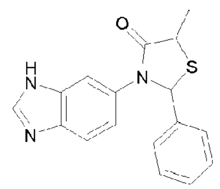 |

(continued)

| Chemical Name | Structure |
|---|---|
| 3-(1 H-benzo[d]imidazol-5-yl)-2-phenylthiazolidine-4-thione | 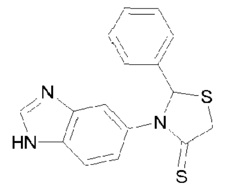 |
| 3-(1 H-benzo[d]imidazol-6-yl)-2-(4-phenoxyphenyl) thiazolidine-4-thione | 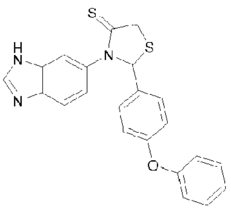 |
| 1-(1 H-benzo[d]imidazol-5-yl)-5-(4-fluorophenyl)pyrrolidin-2-one | 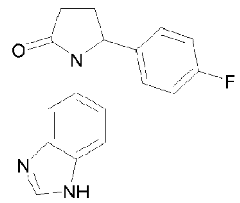 |
| 1-(1H-benzo[d]imidazol-5-yl)-5-(4-methoxyphenyl)pyrrolidin-2-one | 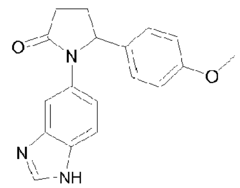 |
| 1-(1H-benzo[d]imidazol-5-yl)-5-(4-propoxyphenyl)pyrrolidin-2-one | 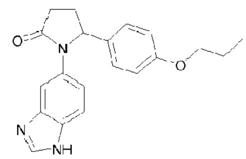 |
| 1-(1 H-benzo[d]imidazol-5-yl)-5-(2,3-dihydrobenzo[b][1,4]dioxin-6-yl) pyrrolidin-2-one | 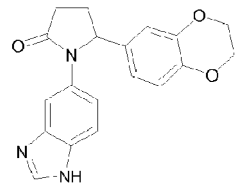 |
| 1-(1H-benzo[d]imidazol-5-yl)-5-phenylpyrrolidin-2-one | 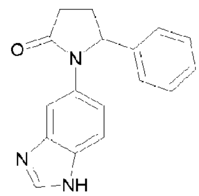 |

(continued)

| Chemical Name | Structure |
|---|---|
| 2-(1 H-benzo[d]imidazol-5-yl)-3-phenylisoindolin-1-one | 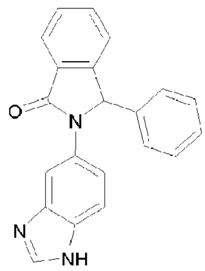 |
| 2-(1 H-benzo[d]imidazol-5-yl)-3-(4-biphenyl)isoindolin-1-one | 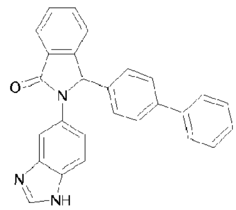 |
| 2-(1 H-benzo[d]imidazol-5-yl)-3-(4-fluorophenyl)isoindolin-1-one | 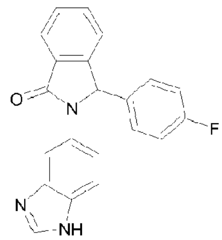 |
| 2-(1 H-benzo[d]imidazol-5-yl)-3-(3-fluorophenyl)isoindolin-1-one | 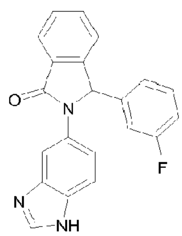 |
| 2-(1 H-benzo[d]imidazol-5-yl)-3-(3,5-difluorophenyl)isoindolin-1-one | 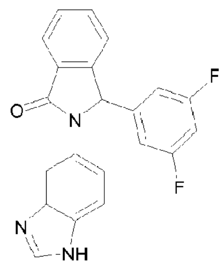 |
| 2-(1 H-benzo[d]imidazol-5-yl)-3-(4-chlorophenyl)isoindolin-1-one | 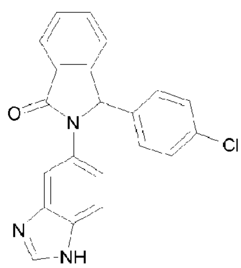 |

(continued)

| Chemical Name | Structure |
|---|---|
| 2-(1 H-benzo[d]imidazol-5-yl)-3-(3,4-dichlorophenyl)isoindolin-1-one | 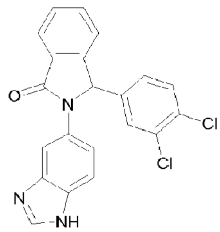 |
| 2-(1 H-benzo[d]imidazol-5-yl)-3-(3-chloro-5-fluorophenyl)isoindolin-1-one | 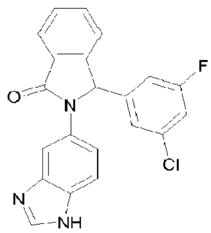 |
| 2-(1 H-benzo[d]imidazol-5-yl)-3-(4-methoxyphenyl)isoindolin-1-one | 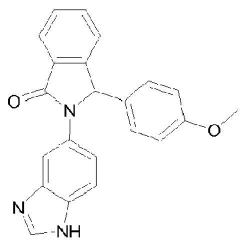 |
| 2-(1 H-benzo[d]imidazol-5-yl)-3-(4-propoxyphenyl)isoindolin-1-one | 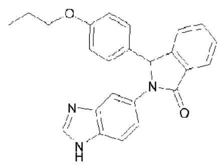 |
| 2-(1 H-benzo[d]imidazol-5-yl)-3-(3-fluoro-4-methoxyphenyl)isoindolin-1-one | 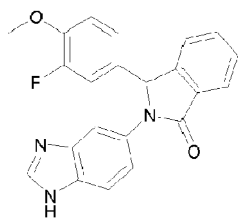 |
| 2-(1 H-benzo[d]imidazol-5-yl)-3-(3,4-dimethoxyphenyl)isoindolin-1-one | 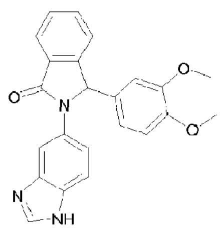 |
| 3-(benzo[d][1,3]dioxol-6-yl)-2-(1 H-benzo[d]imidazol-5-yl)isoindolin-1-one | 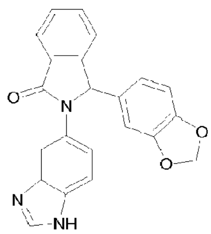 |

(continued)

| Chemical Name | Structure |
|---|---|
| 2-(1 H-benzo[d]imidazol-5-yl)-3-(4-phenoxyphenyl)isoindolin-1-one | 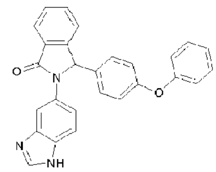 |
| 2-(1 H-benzo[d]imidazol-5-yl)-4,7-dichloro-3-(4-methoxyphenyl)isoindolin-1-one | 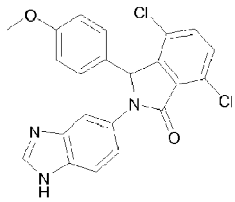 |
| 2-(1 H-benzo[d]imidazol-5-yl)-5,6-dichloro-3-(4-methoxyphenyl)isoindolin-1-one | 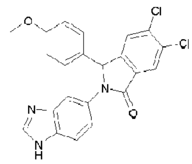 |
| 2-(1 H-benzo[d]imidazol-5-yl)-5,6-dichloro-3-(4-propoxyphenyl)isoindolin-1-one | 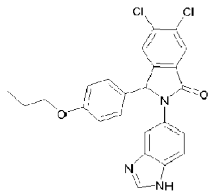 |
| (S)-2-(1 H-benzo[d]imidazol-5-yl)-3-(3,4-dimethoxyphenyl)isoindolin-1-one | 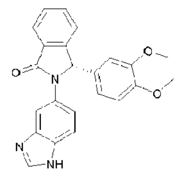 |
| (R)-2-(1 H-benzo[d]imidazol-5-yl)-3-(3,4-dimethoxyphenyl)isoindolin-1-one | 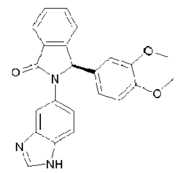 |
| (R)-2-(1 H-benzo[d]imidazol-5-yl)-3-(4-propoxyphenyl)isoindolin-1-one | 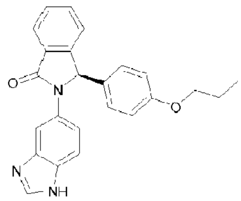 |
| (S)-2-(1 H-benzo[d]imidazol-5-yl)-3-(4-propoxyphenyl)isoindolin-1-one | 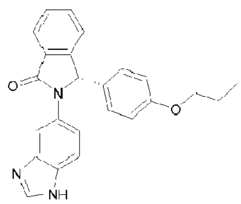 |

(continued)

| Chemical Name | Structure |
|---|---|
| (R)-2-(1 H-benzo[d]imidazol-5-yl)-3-(4-chlorophenyl)isoindolin-1-one | 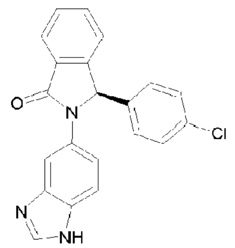 |
| (S)-2-(1 H-benzo[d]imidazol-5-yl)-3-(4-chlorophenyl)isoindolin-1-one | 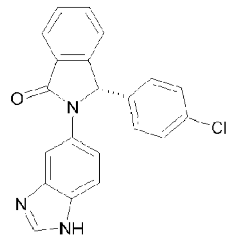 |
| 1-(1 H-benzo[d]imidazol-5-yl)-5-(4-phenylcyclohexyl) imidazolidin-2-one | 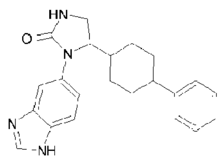 |
| 1-(1 H-benzo[d]imidazol-6-yl)-5-(1-phenylpiperidin-4-yl)imidazolidin-2-one | 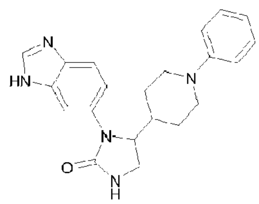 |
| 1-(1 H-benzo[d]imidazol-5-yl)-5-(4-(3-methoxypropyl)phenyl)imidazolidin-2-one | 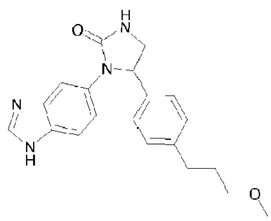 |
| 1-(1 H-benzo[d]imidazol-5-yl)-5-(4-hydroxyphenyl) imidazolidin-2-one | 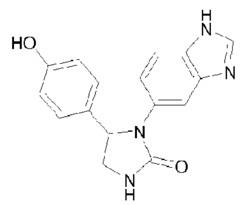 |
| 1-(1 H-benzo[d]imidazol-5-yl)-5-(2-hydroxyphenyl) imidazolidin-2-one | 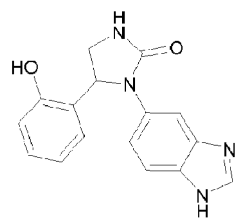 |

(continued)

| Chemical Name | Structure |
|---|---|
| 1-(1H-benzo[d]imidazol-5-yl)-5-(2,4-dihydroxyphenyl)imidazolidin-2-one | 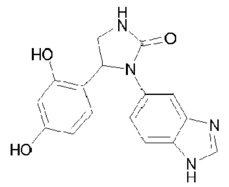 |
| 1-(1H-benzo[d]imidazol-5-yl)-5-(3,4-dihydroxyphenyl)imidazolidin-2-one | 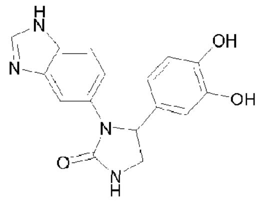 |
| 1-(1 H-benzo[d]imidazol-5-yl)-5-(3-hydroxyphenyl) imidazolidin-2-one | 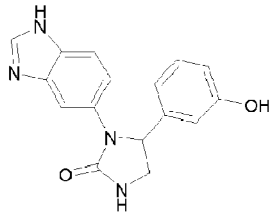 |
| 1-(1 H-benzo[d]imidazol-5-yl)-5-(4-(cyclohexyloxy)phenyl)imidazolidin-2-one | 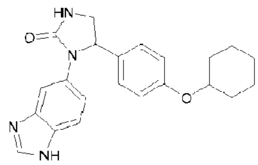 |
| 5-(4-(2-methoxyethoxy)phenyl)-1-(1H-benzo[d]imidazol-5-yl)imidazolidin-2-one | 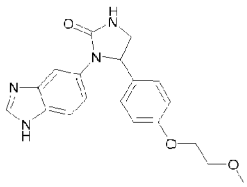 |
| (S)-5-(4-(2-(dimethylamino)ethoxy)ph enyl)-1-(1 H-benzo[d]imidazol-5-yl) imidazolidin-2-one | 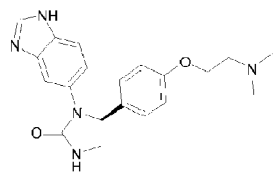 |
| 3-(1 H-benzo[d]imidazol-5-yl)-1-phenethyl-4-(4-propoxyphenyl)imidazolidin-2-one | 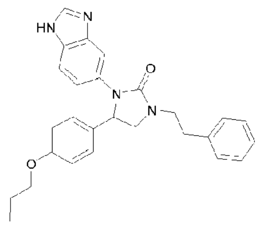 |

(continued)

| Chemical Name | Structure |
|---|---|
| 3-(1 H-benzo[d]imidazol-5-yl)-1-((naphthalen-2-yl)methyl)-4-(4-propoxyphenyl)imidazolidin-2-one | 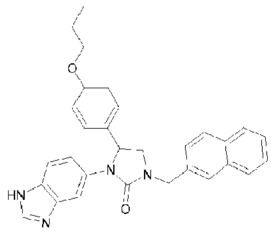 |
| 3-(1 H-benzo[d]imidazol-5-yl)-1-(3-phenylpropyl)-4-(4-propoxyphenyl) imidazolidin-2-one | 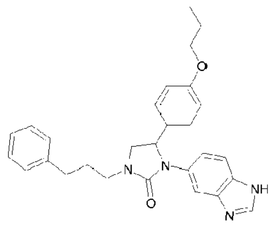 |
| 3-(1 H-benzo[d]imidazol-5-yl)-1-benzyl-4-(4-propoxyphenyl)imidazolidin-2-one | 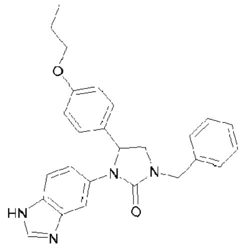 |
| 1-(1 H-benzo[d]imidazol-5-yl)-5-(4-fluoro-3-methoxyphenyl)imidazolidin-2-one | 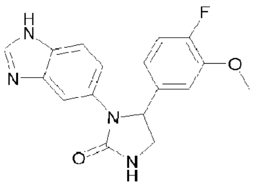 |
| 1-(1 H-benzo[d]imidazol-5-yl)-5-(3-fluoro-4-propoxyphenyl)imidazolidin-2-one | 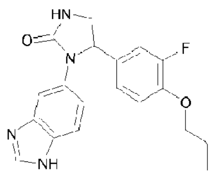 |
| 1-(1 H-benzo[d]imidazol-5-yl)-5-(2-fluoro-4-propoxyphenyl)imidazolidin-2-one | 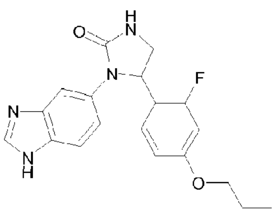 |
| (S)-1-(1 H-benzo[d]imidazol-5-yl)-5-(4-(diethylamino)phenyl)imidazolidin-2-one | 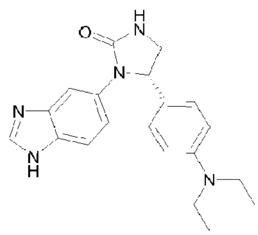 |

(continued)

| Chemical Name | Structure |
|---|---|
| 1-(1H-benzo[d]imidazol-5-yl)-5-(4-chlorophenyl)imidazolidin-2-one | 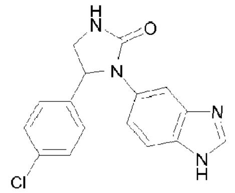 |
| 1-(1 H-benzo[d]imidazol-5-yl)-5-(4-cyclohexylphenyl) imidazolidin-2-one | 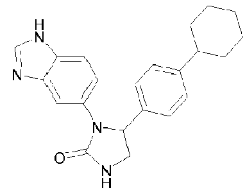 |
| 1-(1 H-benzo[d]imidazol-5-yl)-5-(4-(4-morpholinocyclohexyl)phenyl) imidazolidin-2-one | 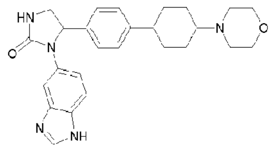 |
| (S)-1-(1H-benzo[d]imidazol-5-yl)-5-(4-(1-methylpiperidin-4-yl)phenyl) imidazolidin-2-one | 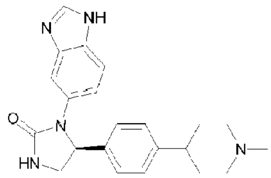 |
| 1-(1 H-benzo[d]imidazol-5-yl)-5-(4-(tetrahydro-2H-pyran-4-yl)phenyl) imidazolidin-2-one | 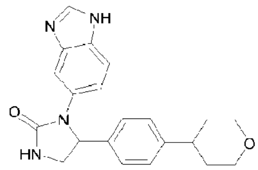 |
| 1-(1 H-benzo[d]imidazol-5-yl)-5-(4-(4-oxocyclohexyl)phenyl) imidazolidin-2-one | 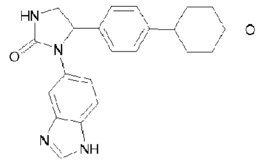 |
| (S)-1-(1 H-benzo[d]imidazol-5-yl)-5-(4-(4,4-difluorocyclohexyl)phenyl) imidazolidin-2-one | 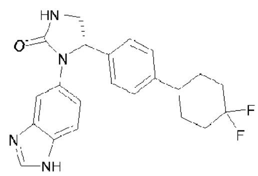 |
| 1-(1 H-benzo[d]imidazol-5-yl)-5-(3-(pyrrolidin-1-yl)phenyl)imidazolidin-2-one | 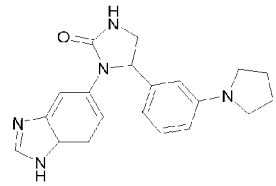 |

(continued)

| Chemical Name | Structure |
|---|---|
| 1-(1H-benzo[d]imidazol-5-yl)-5-(4-(piperidin-1-yl)phenyl)imidazolidin-2-one | 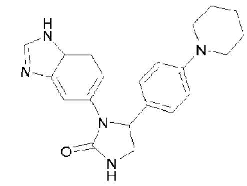 |
| 1-(1H-benzo[d]imidazol-5-yl)-5-(3-(piperidin-1-yl)phenyl)imidazolidin-2-one | 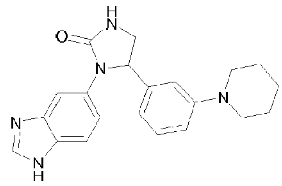 |
| 1-(1 H-benzo[d]imidazol-5-yl)-5-(4-morpholinophenyl)imidazolidin-2-one | 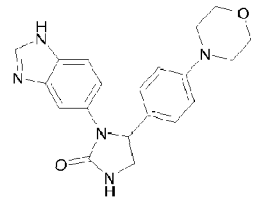 |
| 5-(4-cyclohexylphenyl)-1-(H-imidazo[1,2-a]pyridin-7-yl)imidazolidin-2-one | 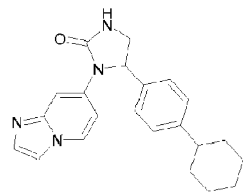 |
| 1-(H-imidazo[1,2-a]pyridin-7-yl)-5-(4-(pyrrolidin-1-yl)phenyl)imidazolidin-2-one | 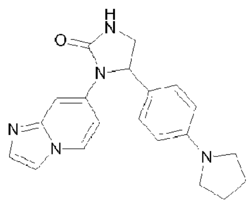 |
| 1-(H-imidazo[1,2-a]pyridin-7-yl)-5-(3-(pyrrolidin-1-yl)phenyl)imidazolidin-2-one | 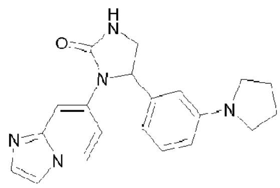 |
| 1-(H-imidazo[1,2-a]pyridin-7-yl)-5-(4-(piperidin-1-yl)phenyl)imidazolidin-2-one | 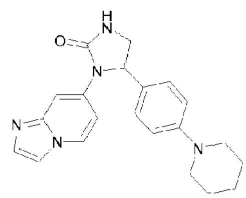 |
| 1-(H-imidazo[1,2-a]pyridin-7-yl)-5-(3-(piperidin-1-yl)phenyl)imidazolidin-2-one | 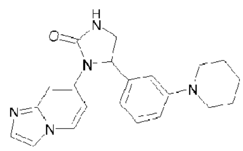 |

(continued)

| Chemical Name | Structure |
|---|---|
| 1-(H-imidazo[1,2-a]pyridin-7-yl)-5-(1-phenylpiperidin-4-yl)imidazolidin-2-one | 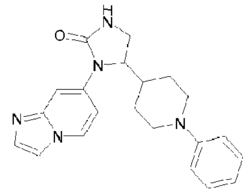 |
| (S)-3-(1 H-benzo[d]imidazol-5-yl)-4-(4-(3-methoxypropyl)phenyl)oxazolidin-2-one | 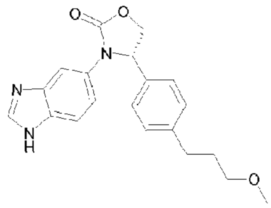 |
| 3-(1 H-benzo[d]imidazol-5-yl)-4-(4-(3-(dimethylamino)propyl)phenyl) oxazolidin-2-one | 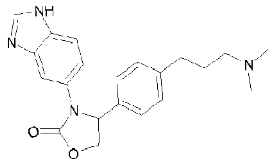 |
| (S)-3-(7-methyl-1 H-benzo[d]imidazol-5-yl)-4-phenyloxazolidin-2-one | 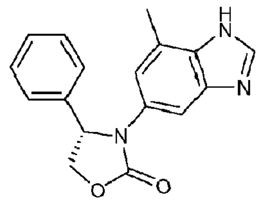 |
| (S)-3-(6-fluoro-1 H-benzo[d]imidazol-5-yl)-4-phenyloxazolidin-2-one | 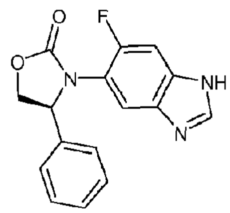 |
| (S)-3-(7-fluoro-1H-benzo[d]imidazol-5-yl)-4-phenyloxazolidin-2-one | 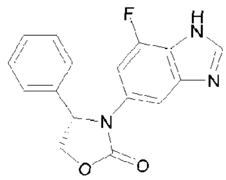 |
| (S)-3-(1 H-benzo[d]imidazol-5-yl)-4-(cyclohexylmethyl)oxazolidin-2-one | 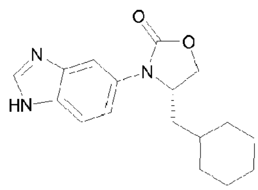 |

(continued)

| Chemical Name | Structure |
|---|---|
| (S)-3-(1 H-benzo[d]imidazol-5-yl)-4-cyclohexyloxazolidin-2-one | 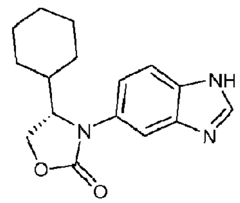 |
| (S)-3-(1 H-benzo[d]imidazol-5-yl)-4-(4-phenylcyclohexyl) oxazolidin-2-one | 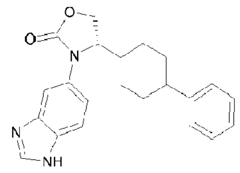 |
| (S)-3-(1 H-benzo[d]imidazol-5-yl)-4-(1-phenylpiperidin-4-yl)oxazolidin-2-one | 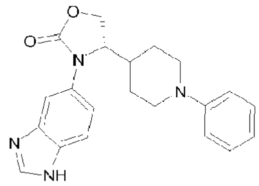 |
| (S)-4-(1-acetylpiperidin-4-yl)-3-(1H-benzo[d]imidazol-5-yl)oxazolidin-2-one | 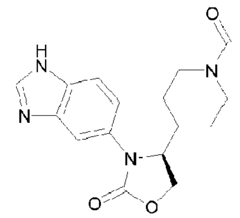 |
| 3-(1H-benzo[d]imidazol-5-yl)-4-(1-phenylethyl)oxazolidin-2-one | 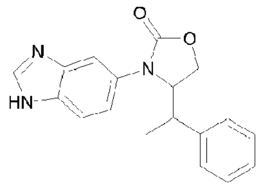 |
| (S)-4-(4-propoxybenzyl)-3-(1 H-benzo[d]imidazol-5-yl)oxazolidin-2-one | 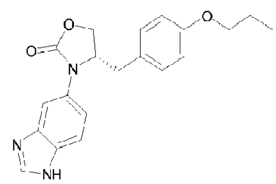 |
| (S)-4-(4-isopropoxybenzyl)-3-(1 H-benzo[d]imidazol-5-yl)oxazolidin-2-one | 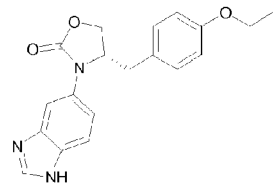 |
| (S)-4-(4-(cyclohexyloxy)benzyl)-3-(1 H-benzo[d]imidazol-5-yl)oxazolidin-2-one | 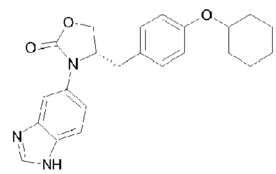 |

(continued)

| Chemical Name | Structure |
|---|---|
| 4-(4-morpholinobenzyl)-3-(1 H-benzo[d]imidazol-5-yl)oxazolidin-2-one | 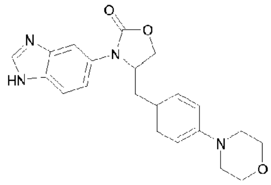 |
| (S)-3-(1 H-benzo[d]imidazol-5-yl)-4-phenethyloxazolidin-2-one | 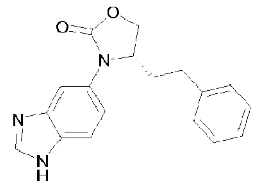 |
| 3-(1 H-benzo[d]imidazol-5-yl)-4-(4-(cyclohexyloxy)phenyl)oxazolidin-2-one | 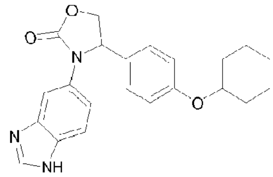 |
| (S)-3-(7-methyl-1 H-benzo[d]imidazol-5-yl)-4-(4-propoxyphenyl)oxazolidin-2-one | 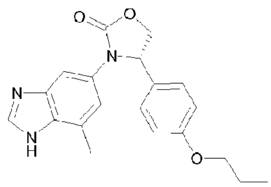 |
| (S)-3-(6,7-dimethyl-1H-benzo[d]imidazol-5-yl)-4-(4-propoxyphenyl)oxazolidin-2-one | 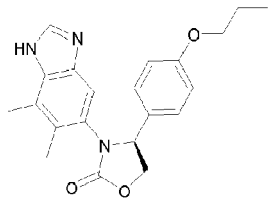 |
| (S)-4-(4-(2-methoxyethoxy)phenyl)-3-(1 H-benzo[d]imidazol-5-yl)oxazolidin-2-one | 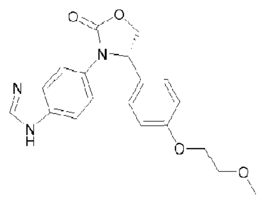 |
| (S)-4-(4-(2-(dimethylamino)ethoxy)phenyl)-3-(1 H-benzo[d]imidazol-5-yl) oxazolidin-2-one | 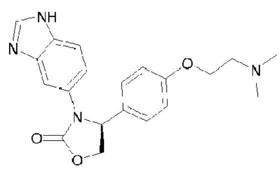 |
| 3-(1 H-benzo[d]imidazol-5-yl)-4-(2,6-difluoro-4-methoxyphenyl)oxazolidin-2-one | 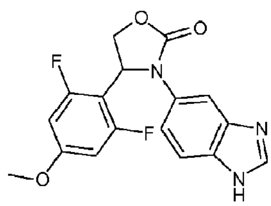 |

(continued)

| Chemical Name | Structure |
|---|---|
| (S)-3-(1 H-benzo[d]imidazol-5-yl)-4-(4-(diethylamino)phenyl)oxazolidin-2-one | 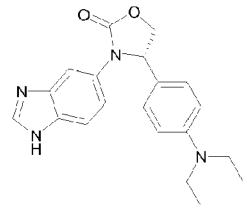 |
| (S)-3-(1 H-benzo[d]imidazol-5-yl)-4-(4-(bis(2-methoxyethyl)amino)phenyl) oxazolidin-2-one | 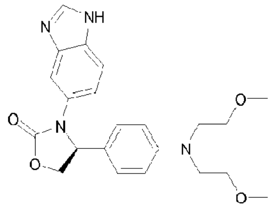 |
| (S)-3-(1 H-benzo[d]imidazol-5-yl)-4-(4-(dicyclopropylamino)phenyl)oxazolidin-2-one | 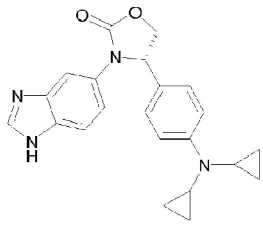 |
| (S)-3-(1 H-benzo[d]imidazol-6-yl)-4-(biphenyl-4-yl)oxazolidin-2-one | 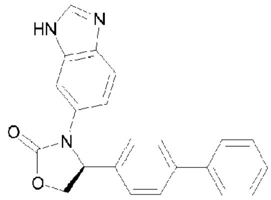 |
| 3-(1H-benzo[d]imidazol-5-yl)-4-(4-(4-oxocyclohexyl)phenyl)oxazolidin-2-one | 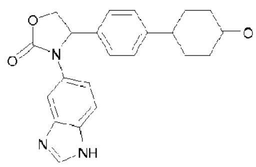 |
| 3-(1H-benzo[d]imidazol-5-yl)-4-(4-(4-methoxycyclohexyl)phenyl)oxazolidin-2-one | 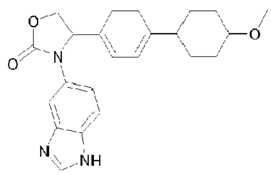 |
| 3-(1 H-benzo[d]imidazol-5-yl)-4-(4-(4-hydroxycyclohexyl)phenyl)oxazolidin-2-one | 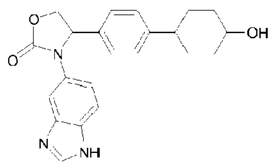 |
| 3-(1H-benzo[d]imidazol-5-yl)-4-(4-(4-morpholinocyclohexyl)phenyl)oxazolidin-2-one | 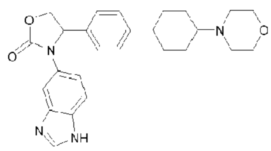 |

(continued)

| Chemical Name | Structure |
|---|---|
| 3-(1 H-benzo[d]imidazol-5-yl)-4-(4-(pyrrolidin-1-yl)phenyl)oxazolidin-2-one | 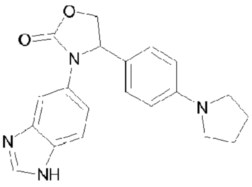 |
| (S)-3-(1 H-benzo[d]imidazol-5-yl)-4-(4-(piperidin-1-yl)phenyl)oxazolidin-2-one | 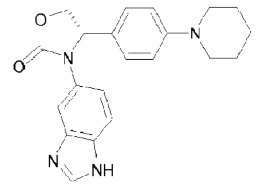 |
| (S)-3-(1 H-benzo[d]imidazol-5-yl)-4-(3-(piperidin-1-yl)phenyl)oxazolidin-2-one | 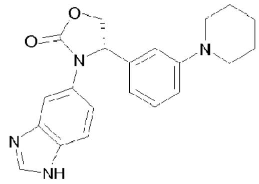 |
| (S)-3-(1 H-benzo[d]imidazol-5-yl)-4-(4-morpholinophenyl)oxazolidin-2-one | 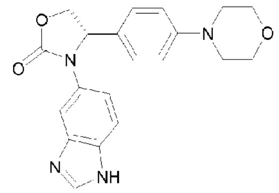 |
| (S)-3-(1 H-benzo[d]imidazol-5-yl)-4-(3-morpholinophenyl)oxazolidin-2-one | 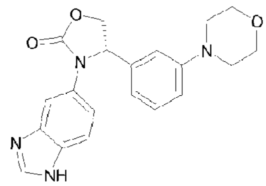 |
| 3-(1 H-benzo[d]imidazol-5-yl)-4-(4-(tetrahydro-2H-pyran-4-yl)phenyl) oxazolidin-2-one | 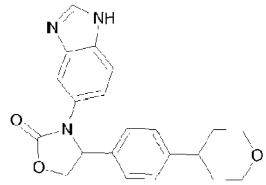 |
| 3-(1 H-benzo[d]imidazol-5-yl)-4-(4-(1-methylpiperidin-4-yl)phenyl)oxazolidin-2-one | 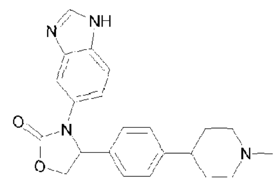 |
| (S)-3-(1 H-benzo[d]imidazol-6-yl)-4-(3-(4-methylpiperazin-1-yl)phenyl) oxazolidin-2-one | 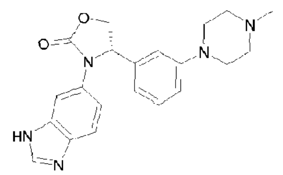 |

(continued)

| Chemical Name | Structure |
|---|---|
| (S)-3-(3-methylH-imidazo[1,2-a]pyridin-7-yl)-4-phenyloxazolidin-2-one | 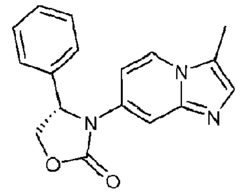 |
| (S)-3-(3-(trifluoromethyl)H-imidazo[1,2-a]pyridin-7-yl)-4-phenyloxazolidin-2-one | 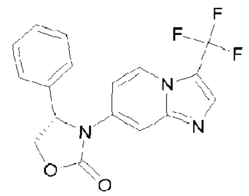 |
| (S)-4-(2,3-dihydrobenzo[b][1,4] dioxin-6-yl)-3-(H-imidazo[1,2-a]pyridin-7-yl) oxazolidin-2-one | 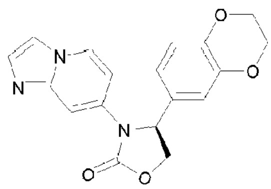 |
| (S)-4-(4-cyclohexylphenyl)-3-(H-imidazo[1,2-a]pyridin-7-yl)oxazolidin-2-one | 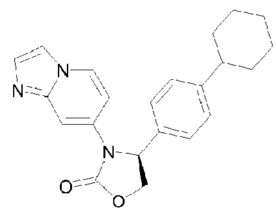 |
| (S)-3-(H-imidazo[1,2-a]pyridin-7-yl)-4-(4-(piperidin-1-yl)phenyl)oxazolidin-2-one | 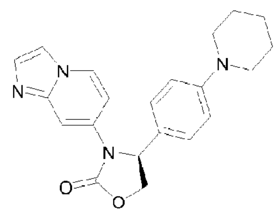 |
| (S)-3-(H-imidazo[1,2-a]pyridin-7-yl)-4-(4-morpholinophenyl) oxazolidin-2-one | 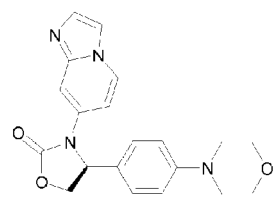 |
| (S)-3-(H-imidazo[1,2-a]pyridin-7-yl)-4-(4-(4-phenylpiperazin-1-yl)phenyl) oxazolidin-2-one | 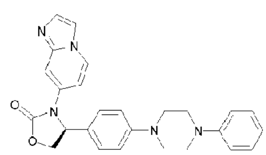 |

(continued)

| Chemical Name | Structure |
|---|---|
| (S)-1-(1 H-benzo[d]imidazol-5-yl)-5-(4-(bis(2-methoxyethyl)amino)phenyl) imidazolidin-2-one | 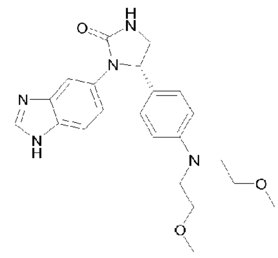 |
| 5-(4-(N-(2-(dimethylamino)ethyl)-N-methylamino)phenyl)-1-(1 H-benzo[d] imidazol-5-yl)imidazolidin-2-one | 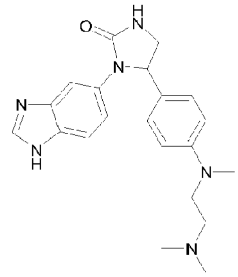 |
| 3-(1 H-benzo[d]imidazol-5-yl)-4-(4-(4,4-difluorocyclohexyl)phenyl) oxazolidin-2-one | 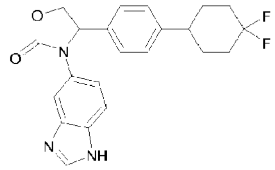 |
| 2-(1 H-benzo[d]imidazol-5-yl)-4,7-difluoro-3-(4-propoxyphenyl)isoindolin-1-one | 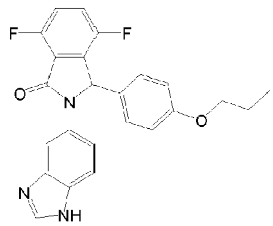 |
| 2-(H-imidazo[1,2-a]pyridin-7-yl)-3-(3,4-dimethoxyphenyl)isoindolin-1-one | 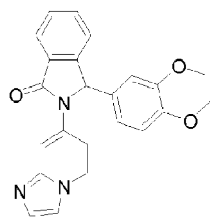 |
| (S)-2-(H-imidazo[1,2-a]pyridin-7-yl)-3-(3,4-dimethoxyphenyl)isoindolin-1-one | 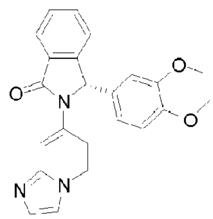 |

(continued)

| Chemical Name | Structure |
|---|---|
| (S)-3-(3,4-dimethoxyphenyl)-2-(3-methylH-imidazo[1,2-a]pyridin-7-yl) isoindolin-1-one | 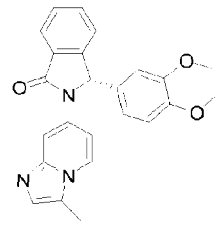 |

**11. QPCT and QPCTL inhibitor compounds as disclosed in WO2008128983 and EP2160380**

In some embodiments, the active agent is a QPCT and/or QPCTL inhibitor as described in US8889709B2. In some embodiments, the active agent is a compound having formula (VIII) below, or a pharmaceutically acceptable salt, solvate, polymorph, tautomer, or stereoisomer thereof:

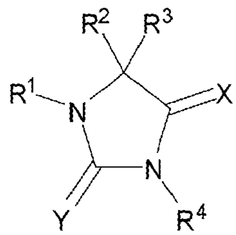

Formula (VIII)

wherein:

$R^1$ represents $-C_{3-8}$carbocyclyl-heteroaryl, $-C_{2-6}$alkenylheteroaryl, $-C_{1-6}$alkylheteroaryl, or $(CH_2)_aCR^5R^6(CH_2)_b$ heteroaryl;
wherein a and b independently representing integers 0-5 provided that a+b=0-5; and
$R^5$ and $R^6$ being alkylene which, together with the carbon to which they are attached, form a $C_3$-$C_5$ cycloalkyl group, or a bicyclic heteroaryl group;
wherein any of said heteroaryl groups being optionally substituted by one or more groups selected from $C_{1-6}$alkyl, $C_{2-6}$alkenyl, $C_{2-6}$alkynyl, $C_{1-6}$haloalkyl, $-C_{1-6}$ thioalkyl, $-SOC_{1-4}$alkyl, $-SO_2C_{1-4}$alkyl, $C_{1-6}$alkoxy-, $-O-C_{3-8}$cycloalkyl, $C_{3-8}$cycloalkyl, $-SO_2C_{3-8}$cycloalkyl, $-SOC_{3-6}$cycloalkyl, $C_{3-6}$alkenyloxy-, $C_{3-6}$alkynyloxy-, $-C(O)C_{1-6}$alkyl, $-C(O)OC_{1-6}$alkyl, $C_{1-6}$alkoxy-$C_{1-6}$alkyl-, nitro, halogen, cyano, hydroxyl, -C(O)OH, $-NH_2$, $-NHC_{1-4}$alkyl, $-N(C_{1-4}$alkyl)($C_{1-4}$alkyl), $-C(O)N(C_{1-4}$alkyl)($C_{1-4}$alkyl), $-C(O)NH_2$, $-C(O)NH(C_{1-4}$alkyl) and-$C(O)NH(C_{3-10}$cycloalkyl) and any of said carbocyclyl groups being optionally substituted by one or more groups selected from $C_{1-4}$alkyl, oxo, halogen and $C_{1-4}$ alkoxy;
$R^2$ and $R^3$ are one of (i), (ii), (iii), (iv), or (v) defined as follows:

(i) $R^2$ represents $C_{1-8}$ alkyl, aryl, heteroaryl, carbocyclyl, heterocyclyl, $-C_{1-4}$alkylaryl, $-C_{1-4}$alkylheteroaryl, $-C_{1-4}$alkylcarbocyclyl or $-C_{1-4}$alkylheterocyclyl; wherein any of said aryl and heteroaryl groups optionally substituted by one or more groups selected from $C_{1-6}$alkyl, $C_{2-6}$alkenyl, $C_{2-6}$alkynyl, $C_{1-6}$haloalkyl, $-C_{1-6}$thioalkyl, $-SOC_{1-4}$alkyl, $-SO_2C_{1-4}$alkyl, $C_{1-6}$alkoxy, $-O-C_{3-8}$cycloalkyl, $C_{3-8}$cycloalkyl, $-SO_2C_{3-8}$cycloalkyl, $-SOC_{3-6}$cycloalkyl, $C_{3-6}$alkenyloxy-, $C_{3-6}$alkynyloxy-, $-C(O)C_{1-6}$alkyl, $-C(O)OC_{1-6}$alkyl, $C_{1-6}$alkoxy-$C_{1-6}$alkyl-, nitro, halogen, cyano, hydroxyl, -C(O)OH, $-NH_2$, $-NHC_{1-4}$alkyl, $-N(C_{1-4}$alkyl)($C_{1-4}$alkyl), $-C(O)N(C_{1-4}$alkyl) ($C_{1-4}$alkyl), $-C(O)NH_2$, $-C(O)NH(C_{1-4}$alkyl) and $-C(O)NH(C_{3-10}$cycloalkyl); and
any of aforesaid carbocyclyl and heterocyclyl groups optionally substituted by one or more groups selected from $C_{1-4}$alkyl, oxo, halogen and $C_{1-4}$ alkoxy; and
$R^3$ represents H, $-C_{1-4}$ alkyl or aryl with said aryl optionally substituted by one or more groups selected from $C_{1-6}$alkyl, $C_{2-6}$alkenyl, $C_{2-6}$alkynyl, $C_{1-6}$haloalkyl, $-C_{1-6}$thioalkyl, $-SOC_{1-4}$alkyl, $-SO_2C_{1-4}$alkyl, $C_{1-6}$alkoxy-, $-O-C_{3-8}$cycloalkyl, $C_{3-8}$cycloalkyl, $-SO_2C_{3-8}$cycloalkyl, $-SOC_{3-6}$cycloalkyl, $C_{3-6}$alkenyloxy-, $C_{3-6}$alkynyloxy-,

$-C(O)C_{1-6}alkyl$, $-(O)OC_{1-6}alkyl$, $C_{1-6}alkoxy-C_{1-6}alkyl-$, nitro, halogen, cyano, hydroxyl, -C(O)OH, $-NH_2$, $-NHC_{1-4}alkyl$, $-N(C_{1-4}alkyl)(C_{1-4}alkyl)$, $-C(O)N(C_{1-4}alkyl)(C_{1-4}alkyl)$, $-C(O)NH_2$, $-C(O)NH(C_{1-4}alkyl)$ and $-C(O)NH(C_{3-10}cycloalkyl)$;

(ii) $R^2$ represents phenyl substituted by phenyl, phenyl substituted by a monocyclic heteroaryl group, phenyl substituted by benzyloxy, phenyl fused to carbocyclyl, phenyl fused to heterocyclyl, $-C_{1-4}alkyl$(phenyl substituted by phenyl), $-C_{1-4}alkyl$(phenyl substituted by a monocyclic heteroaryl group), $-C_{1-4}alkyl$(phenyl substituted by benzyloxy), $-C_{1-4}alkyl$(optionally substituted phenyl fused to optionally substituted carbocyclyl or $-C_{1-4}alkyl$(optionally substituted phenyl fused to optionally substituted heterocyclyl);

wherein any of said phenyl, benzyloxy and heteroaryl groups optionally substituted by one or more groups selected from $C_{1-4}alkyl$, halogen and $C_{1-4}alkoxy$; and

any of said carbocyclyl and heterocyclyl groups optionally substituted by one or more groups selected from $C_{1-4}alkyl$, oxo, halogen and $C_{1-4}alkoxy$; and

$R^3$ represents H, $-C_{1-4}alkyl$ or aryl with said aryl optionally substituted by one or more groups selected from $C_{1-6}alkyl$, $C_{2-6}alkenyl$, $C_{2-6}alkynyl$, $C_{1-6}haloalkyl$, $-C_{1-6}thioalkyl$, $-SOC_{1-4}alkyl$, $-SO_2C_{1-4}alkyl$, $C_{1-6}alkoxy-$, $-O-C_{3-8}cycloalkyl$, $C_{3-8}cycloalkyl$, $-SO_2C_{3-8}cycloalkyl$, $-SOC_{3-8}cycloalkyl$, $C_{3-6}alkenyloxy-$, $C_{3-6}alkynyloxy-$, $-C(O)C_{1-6}alkyl$, $-C(O)OC_{1-6}alkyl$, $C_{1-6}alkoxy-C_{1-6}alkyl-$, nitro, halogen, cyano, hydroxyl, -C(O)OH, $-NH_2$, $-NHC_{1-4}alkyl$, $-N(C_{1-4}alkyl)(C_{1-4}alkyl)$, $-C(O)N(C_{1-4}alkyl)(C_{1-4}alkyl)$, $-C(O)NH_2$, $-C(O)NH(C_{1-4}alkyl)$ and $-C(O)NH(C_{3-10}cycloalkyl)$;

(iii) $R^2$ and $R^3$ are joined to form a carbocyclyl ring which is optionally substituted by one or more $C_{1-2}alkyl$ groups;

(iv) $R^2$ and $R^3$ are joined to form a carbocyclyl ring which is fused to phenyl, with said carbocyclyl or phenyl optionally substituted by one or more groups selected from $C_{1-4}alkyl$, halogen and $C_{1-4}alkoxy$;

(v) $R^2$ and $R^3$ are joined to form a carbocyclyl ring which is fused to monocyclic heteroaryl with said carbocyclyl or heteroaryl optionally substituted by one or more groups selected from $C_{1-4}alkyl$, halogen and $C_{1-4}alkoxy$;

$R^4$ represents H, $-C_{1-8}alkyl$, $-C(O)C_{1-6}alkyl$, or $-NH_2$;

X represents O or S; and

Y represents O or S.

In some embodiments, the active agent is a QPCT and/or QPCTL inhibitor as described in Table 3 of US8889709B2, for instance compound 6 as shown below.

12. QPCT and QPCTL inhibitor compounds tested herein

In some embodiments, the active agent is a QPCT and/or QPCTL inhibitor as tested herein, includinging a pharmaceutically acceptable salt, solvate, polymorph, tautomer, or stereoisomer thereof, said QPCT and/or QPCTL inhibitor being selected from Table E below:

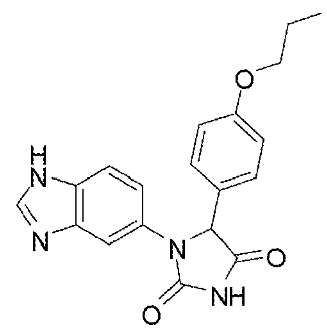

**6**

**Table E. Compounds tested (see Figures 19-14)**

| Compound ID | Cluster | Name | Structure |
|---|---|---|---|
| 000051 | Cluster 1 | 1-(1 H-Benzoimidazol-5-yl)-5-benzo[c][1,2,5] thiadiazol-5-yl-4-cyclopropanecarbonyl-3-hydroxy-1,5-dihydro-pyrrol-2-one | 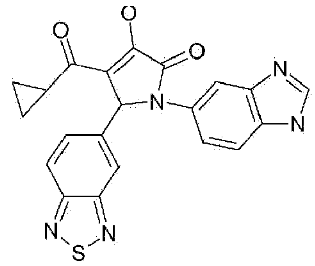 |
| 000054 | Cluster 1 | 1-(1H-Benzoimidazol-5-yl)-3-hydroxy-4-phenyl-5-quinolin-3-yl-1,5-dihydro-pyrrol-2-one | 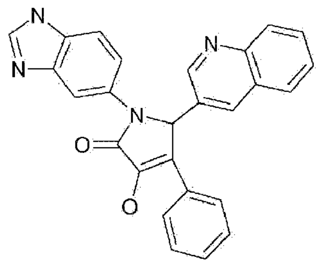 |
| 000016 | Cluster 1 | 1-(1H-benzo[d]imidazol-6-yl)-5-(2,3-difluorophenyl)-3-hydroxy-4-methyl-1H-pyrrol-2(5H)-one | 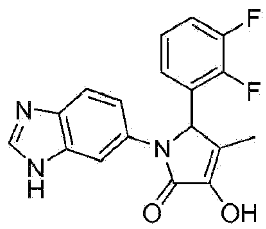 |
| 000034 | Cluster 1 | 1-(1H-benzo[d]imidazol-5-yl)-5-(2,3-dichlorophenyl)-3-hydroxy-4-methyl-1H-pyrrol-2(5H)-one | 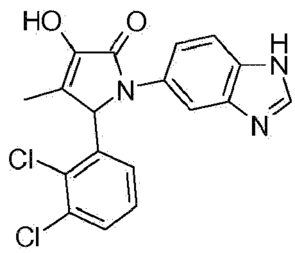 |
| 000035 | Cluster 1 | 1-(1H-Benzoimidazol-5-yl)-3-hydroxy-5-(8-hydroxy-quinolin-2-yl)-4-(3-methyl-butyryl)-1,5-dihydro-pyrrol-2-one | 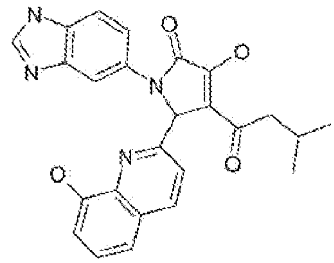 |
| 000037 | Cluster 1 | 1-(1H-benzo[d]imidazol-5-yl)-4-(cyclopropanecarbonyl)-3-hydroxy-5-(8-hydroxyquinolin-2-yl)-1,5-dihydro-2H-pyrrol-2-one | 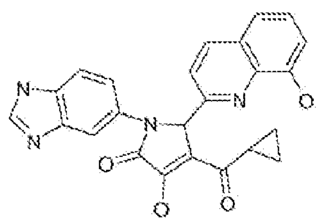 |

(continued)

| Compound ID | Cluster | Name | Structure |
|---|---|---|---|
| 000055 | Cluster 1 | 1-(1H-Benzoimidazol-5-yl)-3-hydroxy-4-phenyl-5-(2,3,5-trifluoro-phenyl)-1,5-dihydro-pyrrol-2-one | 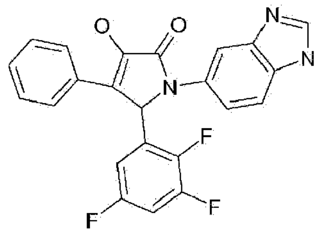 |
| 000024 | Cluster 2 | 1-(1H-Benzo[d]imidazol-6-yl)-5-(2,3-difluorophenyl)-3-methoxy-4-methyl-1H-pyrrol-2(5H)-one | 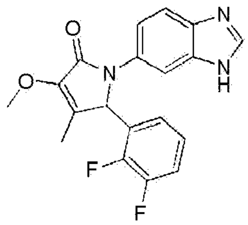 |
| 000027 | Cluster 2 | 1-(1H-Benzo[d]imidazol-6-yl)-5-(2,3-dichlorophenyl)-3-methoxy-4-methyl-1H-pyrrol-2(5H)-one | 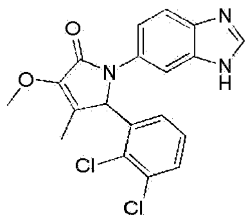 |
| 000050 | Cluster 2 | 1-(1H-Benzo[d]imidazol-6-yl)-5-(4-cyclohexylphenyl)-3-methoxy-4-methyl-1H-pyrrol-2(5H)-one | 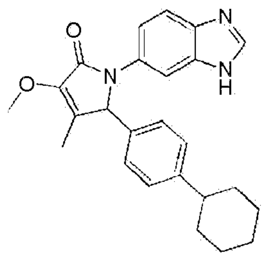 |
| 000020 | Cluster 4 | 5-(benzo[c] [1,2,5]thiadiazol-6-yl)-1-(1 H-benzo[d] imidazol-5-yl)imidazolidine-2,4-dione | 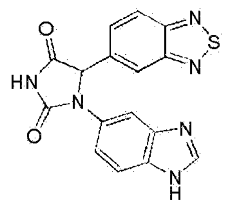 |
| 000021 | Cluster 4 | 5-phenyl-1-(1 H-benzo[d]imidazol-5-yl)imidazolidine-2,4-dione | 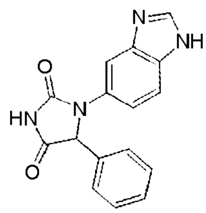 |

(continued)

| Compound ID | Cluster | Name | Structure |
|---|---|---|---|
| 000022 | Cluster 4 | 1-(1 H-benzo[d]imidazol-5-yl)-5-(2-bromo-5-fluorophenyl)imidazolidine-2,4-dione | 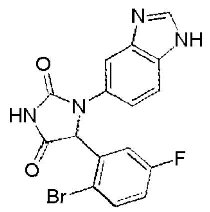 |
| 000023 | Cluster 4 | 1-(1 H-benzo[d]imidazol-5-yl)-5-(4-propoxyphenyl) imidazolidine-2,4-dione | 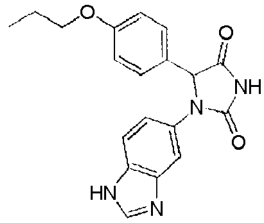 |
| 000025 | Cluster 4 | 1-(1 H-benzo[d]imidazol-5-yl)-5-(3-hydroxy-4-methoxyphenyl)imidazolidine-2,4-dione | 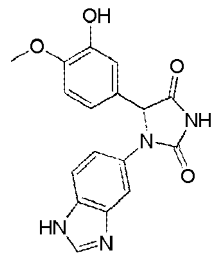 |
| 000010 | Cluster 4 | 1-(1H-benzimidazol-5-yl)-5-(1,1'-biphenyl-4-yl) imidazolidine-2,4-dione | 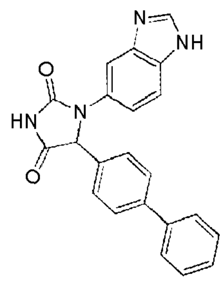 |
| 000026 | Cluster 4 | 1-(1 H-benzo[d]imidazol-5-yl)-5-(3-chlorophenyl) imidazolidine-2,4-dione | 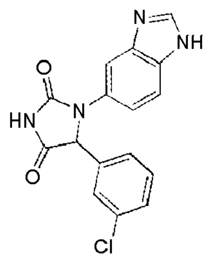 |
| 000011 | Cluster 4 | 1-(1 H-benzo[d]imidazol-5-yl)-5-(2,3-dihydrobenzo[b][1,4]dioxin-7-yl)imidazolidine-2,4-dione | |
| 000036 | Cluster 4 | 1-(1 H-benzo[d]imidazol-5-yl)-5-(2-chlorophenyl) imidazolidine-2,4-dione | 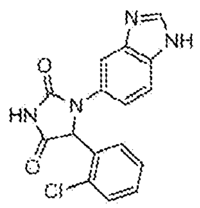 |

(continued)

| Compound ID | Cluster | Name | Structure |
|---|---|---|---|
| 000029 | Cluster 5 | 1-(1H-Benzoimidazol-5-yl)-4-benzylimino-5-(4-chlorophenyl)-imidazolidin-2-one | 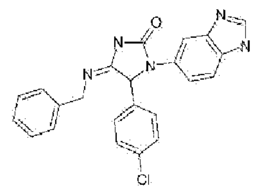 |
| 000048 | Cluster 5 | 5-Benzo[1,3]dioxol-5-yl-1-(1H-benzoimidazol-5-yl)-4-(3-(2-oxopyrrolidin-1-yl)-propylimino)-imidazolidin-2-one | 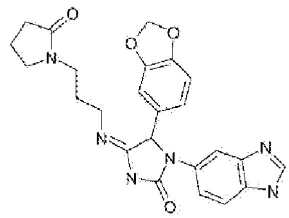 |
| 000049 | Cluster 5 | 1-(1H-Benzoimidazol-5-yl)-5-(3-chloro-2,6-difluoro-phenyl)-4-(1,2,3,4-tetrahydro-naphthalen-1-ylimino)-imidazolidin-2-one | 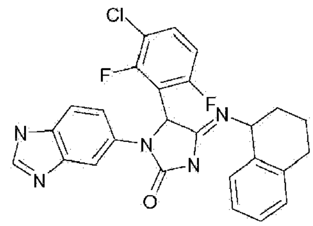 |
| 000012 | Cluster 5 | 1-(1 H-Benzoimidazol-5-yl)-5-(4-bromo-phenyl)-4-(cyclohexylimino)-imidazolidin-2-one | 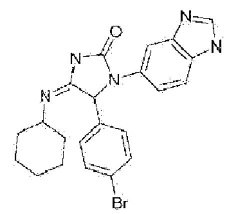 |
| 000030 | Cluster 5 | 1-(1 H-Benzoimidazol-5-yl)-5-(4-chloro-phenyl)-4-(cyclopentylimino)-imidazolidin-2-one | 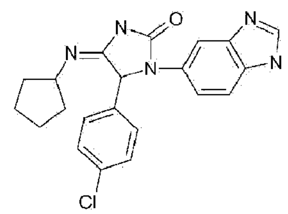 |
| 000031 | Cluster 5 | 1-(1H-Benzoimidazol-5-yl)-4-benzylimino-5-(4-bromo-phenyl)-imidazolidin-2-one | 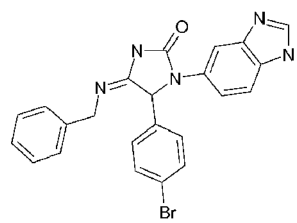 |
| 000013 | Cluster 5 | 1-(1H-Benzoimidazol-5-yl)-5-(3-chloro-2,6-difluoro-phenyl)-4-(cyclohexylimino)-imidazolidin-2-one | 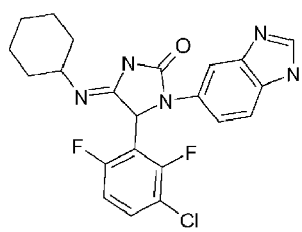 |

(continued)

| Compound ID | Cluster | Name | Structure |
|---|---|---|---|
| 000014 | Cluster 5 | 1-(1H-Benzoimidazol-5-yl)-4-(cyclopentylimino)-5-(1H-indol-5-yl)-imidazolidin-2-one | 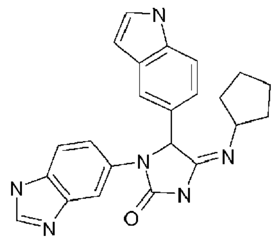 |
| 000032 | Cluster 5 | 1-(1H-Benzoimidazol-5-yl)-5-(3-chloro-2,6-difluoro-phenyl)-4-(1,2,2-trimethyl-propylimino)-imidazolidin-2-one | 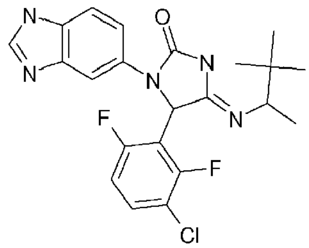 |
| 000052 | Cluster 5 | 1-(1H-Benzoimidazol-5-yl)-5-(4-bromo-phenyl)-4-[2,3-dihydro-benzo[1,4]dioxin-6-ylimino]-imidazolidin-2-one | 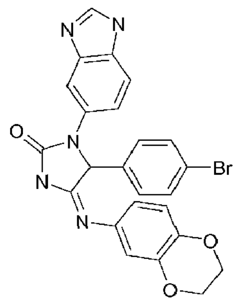 |
| 000053 | Cluster 5 | (R,S)-1-(1H-Benzoimidazol-5-yl)-5-(4-bromo-phenyl)-4-((S)-indan-1-ylimino)-imidazolidin-2-one | 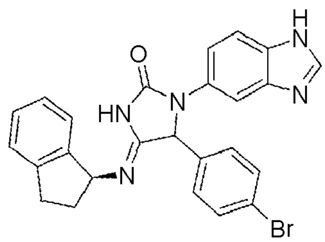 |
| 000060 | Cluster 7 | 5-(4-propoxyphenyl)-1-(H-imidazo[1,2-a]-pyridin-7-yl) imidazolidin-2-one, single enantiomer of unknown configuration | |

(continued)

| Compound ID | Cluster | Name | Structure |
|---|---|---|---|
| 000064 | Cluster 7 | (S)-3-(1 H-benzo[d]imidazol-6-yl)-4-(4-propoxy phenyl)oxazolidin-2-one | 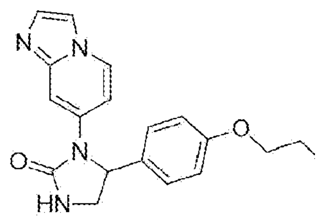 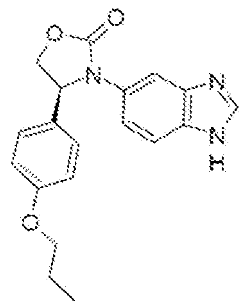 |
| 000044 | Cluster 7 | 2-(1 H-benzo[d]imidazol-5-yl)-3-(3,4-dimethoxyphenyl)isoindolin-1-one, single enantiomer of unknown configuration | |
| 000066 | Cluster 7 | 2-(1 H-benzo[d]imidazol-5-yl)-3-(4-propoxy phenyl) isoindolin-1-one, single enantiomer of unknown configuration | |

**Cotreatment**

In some embodiments, the active agents described herein can be used in combination with a second active agent for treating the conditions disclosed herein. In some embodiments, the second active agent is used in the treatment of cancer. In some embodiments, the second active agent is a therapeutic antibody (e.g. a IgA antibody). In some embodiments, the second therapeutic antibody is an anti-CD47 antibody (e.g. Hu5F9-G4, CC-90002), anti-CD20 antibody (e.g. Rituximab), anti-PD-L1 antibody (Atezolizumab), anti-Her2 antibody (e.g. Trastuzumab), anti-EGFR antibody (e.g. Cetuximab), anti-CD20-CD47 bispecific antibody (Piccione et al (2015), mAbs, Vol. 7, pages 946-956), anti-CD56 antibody (Weiskoft et al (2016), Journal of Clinical Investigation, Vol, 126, pages 2610-2620), anti-CD271-sporin antibody (Ngo et al (2016) Cell Reports, Vol. 16, pages 1701-1716), and the like or the second therapeutic antibody is an anti-SIRPa antibody (e.g. OSE-172 from Ose Immunotherapeutics, Nantes, France). In some embodiments, the second active agent is an IgA antibody.

In some embodiments, the methods as taught herein further comprise providing to the subject with a CD47 inhibitor (e.g. an anti-CD47 IgA antibody), or a SIRPα inhibitor (e.g. an anti-SIRPa IgA antibody), or an active agent selected from the group of anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody. Non-limiting examples of said active agents are as described herein.

In some embodiments, the CD47 inhibitor (e.g. anti-CD47 IgA antibody) is an inhibitor that binds CD47 on the surface of a cell and thereby reduces the binding of CD47 to SIRPα on the surface of another cell.

In some embodiments, the SIRPα inhibitor (e.g. anti-SIRPa antibody) is an inhibitor that binds SIRPα on the surface of a cell and thereby reduces the binding of SIRPα to CD47 on the surface of another cell.

In some embodiment, the active agent is an anti-PD-L1 antibody (e.g. Atezolizumab, Genentech, Durvalumab, MedImmune).

In some embodiments, the CD47 inhibitor is any CD47 inhibitor (e.g. anti-CD47 IgA antibody) that is capable of binding to CD47 expressed at the surface of a cell (e.g. cancer cell) to reduce the binding of CD47 to SIRPα expressed on the surface of another cell (e.g. macrophage), such as the CD47 inhibitor as taught herein, preferably an anti-CD47

antibody (e.g. CC-90002 Celgene; Hu5F9-G4, Forty Seven Inc, and the SIRPα-FC fusion protein TTI-621, Trillium Therapeutics Inc, etc.).

In some embodiments, the SIRPα inhibitor is any SIRPα inhibitor that is capable of binding to SiRPα expressed at the surface of a cell (e.g. myeloid cells such as macrophages, monocytes, neutrophils, basophils, eosinophils, dendritic cells) to reduce the binding of SIRPα to CD47 expressed on the surface of another cell (e.g. diseased cells such as cancer cells), such as the SIRPα inhibitor as taught herein, preferably an anti-SIRPa antibody (e.g. OSE-172 from Ose Immunotherapeutics, Nantes, France).

In some embodiments, it is understood that the CD47 inhibitor or the SIRPα inhibitor may be provided together (i.e. within the same composition) with any one of the compounds of the invention, i.e. a compound capable of reducing or inhibiting or blocking the enzymatic activity of the glutaminyl-peptide cyclotransferase (QPCT) protein and/or glutaminyl-peptide cyclotransferase-like protein (QPCTL) protein or the expression of QPCT gene and/or QPCTL gene (e.g. PBD150, PQ912, PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066) or in a separate composition. It is further understood that in cases where the CD47 inhibitor or the SIRPα inhibitor and the compound of the invention as taught herein are provided to a subject as separate compositions, said separate compositions may be administered to said subject in need thereof simultaneously (e.g. at the same time, although not necessarily via the same administration route) or sequentially (e.g. one after the other, in any order, and not necessarily via the same administration route).

**Use of Compounds and Compositions**

As disclosed herein, one aspect of the invention is a pharmaceutical composition comprising an active agent for use in a method of treating a condition in a subject that would benefit from reducing signaling or binding between CD47 and SIRPα in the subject, wherein the active agent reduces expression or enzymatic activity of QPCTL, QPCT, or combinations thereof, in a cell with CD47 on the surface.

In some embodiments provided herein, the active agent which reduces expression or enzymatic activity of QPCTL, QPCT, or combinations thereof is also capable of 1) reducing or inhibiting the formation of a pyroglutamyl residue at the N-terminus of a CD47 protein, 2) blocking or reducing or inhibiting the activity of the CD47-SIRPa signaling axis, 3) blocking or reducing or inhibiting the interaction or binding between CD47 and SIRPα, or combinations thereof.

In some embodiments, the present invention also relates to the use of any one of the active agents and pharmaceutical compositions thereof that are capable of reducing the expression or enzymatic activity of QPCTL, QPCT, or combinations thereof for the purpose of:

1) treating a subject (e.g. human) suffering from a disease or condition involving the CD47-SIRPα signaling axis, such as e.g. cancer, atherosclerosis, fibrotic diseases as well as infectious diseases (as taught herein); and/or

2) modulating (e.g. boosting or increasing) immune cell-mediated killing of diseased cells (e.g. cancer cells or other diseased cells such as diseased vascular smooth muscle cells, diseased endothelial cells, diseased cells infected by a pathogen (e.g. virus), diseased cells undergoing fibrosis) expressing or overexpressing CD47 at their cell surface via e.g. phagocytosis (by e.g. phagocytes such as macrophages) or via ADCC or via ADCP in a subject suffering from a disease or condition involving the CD47-SIRPa signaling axis, such as e.g. cancer, atherosclerosis, fibrotic diseases as well as infectious diseases (e.g. as caused by a virus). In some embodiments, the "modulating (e.g. boosting or up-regulating or increasing) of the killing of a diseased cell (e.g. cancer cells) via ADCP or ADCC (using compounds as taught herein) involves or uses IgA antibodies (e.g. anti-Her2-IgA1 antibody or anti-CD47-IgA antibody, and others); and/or

3) complementing or enhancing the effects of a therapeutic treatment (monotherapy) with a first active agent (e.g. drug), e.g. anti-CD47 antibody (anti-CD47 IgA antibody) or other active agents including for instance anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody, and others. In some embodiments, the first active agent is an IgA antibody; and/or

4) complementing or enhancing the effects of a therapeutic treatment (monotherapy) with a first active agent (e.g. drug), e.g. anti-SIRPa antibody or other active agents including for instance the anti-SIRPa antibody OSE-172 from Ose Immunotherapeutics, Nantes, France; or a recombinant human CD47Fc chimera protein, which consists of an engineered CD47 protein coupled to a Fc domain, and others. In some embodiments, the first active agent is an IgA antibody; and/or

5) substituting for the use of an anti-CD47 antibody or an anti-SIRPa antibody in the context of a therapeutic treatment (monotherapy with an anti-CD47 antibody or SIRPα antibody) or in the context of a therapeutic (combination therapy) where an anti-CD47 antibody or an anti-SIRPa antibody is administered in combination with a second active agent (e.g. drug), e.g. anti-CD20 antibody, anti-PD-L1 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody, and others. In some embodiments, the second active agent is an IgA antibody.

**Diseases and Conditions**

In some embodiments, the compositions disclosed herein are used for treating a subject suffering from a disease or condition involving the CD47-SIRPa signaling axis. In some embodiments, the compounds or compositions herein, reduce binding between CD47 on the surface of a first cell and SIRPα on the surface of second cell in the subject. In some embodiments, the condition is characterized by overexpression of CD47 on a diseased cell. In some embodiments, overexpression refers to when expression of CD47 is 1.5-fold higher, 2.0-fold higher, 2.5-fold higher, 3.0-fold higher or more in diseased cells than in non-diseased cells. In some embodiments, expression is 1.5-fold higher in diseased cells than in non-diseased cells. In some embodiments, expression is 2.0-fold higher in diseased cells than in non-diseased cells. In some embodiments, expression is 2.5-fold higher in diseased cells than in non-diseased cells. In some embodiments, expression is 3.0-fold higher in diseased cells than in non-diseased cells. In some embodiments, expression is more than 3.0-fold higher in diseased cells than in non-diseased cells.

In some embodiments, the condition is selected from the group consisting of cancer, atherosclerosis, fibrotic disease, and infectious disease, as disclosed in the following sections.

***Cancer***

The terms "cancer", "neoplasm", "tumor", and "carcinoma", are used interchangeably herein to refer to diseased cells, which exhibit relatively autonomous growth, so that they exhibit an aberrant growth phenotype characterized by a significant loss of control of cell proliferation. In general, cells of interest for detection or treatment in the present application include precancerous (e.g., benign), malignant, pre-metastatic, metastatic, and non-metastatic cells, particularly precancerous (e.g., benign), malignant, pre-metastatic, metastatic, and non-metastatic cells which express or overexpress CD47 gene and/or CD47 protein (preferably the CD47 protein is expressed at the cell surface, i.e. plasma membrane, and can convey or is associated with a "do not eat me signal" or "anti-phagocytic signal"). The skilled person knows how to detect or identify diseased cells such as cancer cells having a precancerous (e.g., benign), malignant, pre-metastatic, metastatic, and non-metastatic phenotype, particularly precancerous (e.g., benign), malignant, pre-metastatic, metastatic phenotypes using cancer-specific markers (e.g. alpha-fetoprotein (AFP) for liver cancer and germ cell tumors; Beta-2-microglobulin (B2M) for multiple myeloma, chronic lymphocytic leukemia and some lymphomas; CD20 for Non-Hodgkin lymphoma; EGFR gene mutation analysis in non-small cell lung cancer; HER2/neu gene amplification in breast cancer, gastric cancer, and gastro-esophageal junction adenocarcinoma; Prostate-specific antigen in prostate cancer, and the like.

In some embodiments, the condition is cancer. In some embodiments, the condition is cancer and the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, Gastric cancer, esophageal cancer, T-ALL, glioma, mesothelioma, glioblastoma, melanoma and NSCLC, and others). In some embodiments, the cancer is leukemia or acute myeloid leukemia (AML).

***Atherosclerosis***

The term "atherosclerosis" as used herein refers to condition recognized as the main disease process underlying heart attack and stroke. More specifically, atherosclerosis is characterized as a systemic, progressive disease process in which the arterial wall thickens through a pathological process involving inflammation, oxidative stress, and dyslipidemia (Yoko Kojima et al (2016), Nature. Vol. 536(7614), pages 86-90; Ross et al (1999), Am Heart J., Vol. 138: S419-420; Wang et al (2012), Circ Res, Vol. 11 1, pages 245-259; Quinn et al (1987), PNAS, Vol. 84, pages2995- 2998). This pathological process leads to plaque formation and flow limitation in the vessel lumen of subjects afflicted with the condition. The mechanisms underlying atherosclerosis are being actively studied. For instance, it was reported that the accumulation of diseased vascular cells (e.g. diseased vascular smooth muscle cells), diseased endothelial cells, and apoptotic cellular debris in the vessel lumen debris contributes to worsen the pathological process leading to plaque formation. A recent study has revealed that diseased cells such as diseased vascular smooth muscle cells, diseased endothelial cells upregulate the expression of CD47 at their cell surface thereby conveying a 'don't eat me signal', which

allows said diseased cells to evade phagocytosis by phagocyte cells, e.g. macrophages (i.e. diseased cells are not cleared by the immune system) (Yoko Kojima et al (2016), Nature. Vol. 536(7614), pages 86-90). This is consistent with the observation that CD47 is consistently upregulated in human atherosclerotic plaque compared to non-atherosclerotic vascular tissue, and in subjects with symptomatic cerebrovascular disease (stroke or transient ischemic attack) compared to those with stable asymptomatic lesions (Yoko Kojima et al (2016), Nature. Vol. 536(7614), pages 86-90). It was further reported that administration of an anti-CD47 antibody improved clearance of diseased cells by phagocyte cells and ameliorated atherosclerosis (Yoko Kojima et al (2016), Nature. Vol. 536(7614), pages 86-90). In the context of the present invention, the term "atherosclerosis" refers to diseased cells such as diseased vascular smooth muscle cells and diseased endothelial cells which express or overexpress CD47 gene and/or CD47 protein (preferably the CD47 protein is expressed at the cell surface, i.e. plasma membrane, and can convey or is associated with a "do not eat me signal" or "anti-phagocytic signal"). The skilled person knows how to detect or identify said diseased cells using known methods in the art such as detecting the presence or absence of disease-specific, cell type-specific (molecular) makers (e.g. smooth muscle $\alpha$-actin, Casp3, etc.), morphological characteristics, and the like.

In some embodiments, the condition is atherosclerosis.

***Fibrotic Diseases***

The term "fibrotic diseases" as used herein refers to a condition that is characterized by the accumulation of excess extracellular matrix components (e.g., collagen, fibronectin) that forms fibrous connective tissue in and around an inflamed or damaged tissue. Fibrosis may cause overgrowth, hardening, and/or scarring that disrupts the architecture of the underlying organ or tissue. While controlled tissue remodeling and scarring is part of the normal wound healing process promoted by transdifferentiation of fibroblasts into myofibroblasts, excessive and persistent scarring due to severe or repetitive injury or dysregulated wound healing (e.g., persistence of myofibroblasts) can eventually result in permanent scarring, organ dysfunction and failure, and even death.

Fibrotic changes can occur in vascular disorders (e.g., peripheral vascular disease, cardiac disease, cerebral disease and other) and in all main tissue and organ systems (e.g., lung, liver, kidney, heart, skin, pancreas). Fibrotic disorders include a wide range of clinical presentations, including multisystemic disorders, such as systemic sclerosis, multifocal fibrosclerosis, scleroderma, myelofibrosis, and organ-specific disorders, such as pulmonary (e.g. idiopathic pulmonary fibrosis (IPF)), liver fibrosis, kidney fibrosis, pancreas fibrosis, heart fibrosis, and bladder fibrosis (Rosenbloom et al(2010), Ann. Intern. Med., Vol.152, page 159; Wynn et al (2004), Nat. Rev. Immunol., Vol 4, pages 583; Wernig et al (2017), PNAS, Vol. 114, pages 4757-4762). The mechanisms underlying fibrotic diseases are being actively studied. For instance, it was reported that diseased cells such as diseased fibroblasts upregulate the expression of CD47 at their cell surface thereby conveying a ’don’t eat me signal’, which allows said diseased cells to evade phagocytosis by phagocyte cells, e.g. macrophages and/or neutrophils (i.e. diseased cells are not cleared by the immune system) (Wernig et al (2017), PNAS, Vol. 114, pages 4757-4762). It was further found that treatment with an anti-CD47 antibody lead to an increased phagocytosed diseased fibroblast, which in turn reduced fibrosis in the tissue (Wernig et al (2017), PNAS, Vol. 114, pages 4757-4762). In the context of the present invention, the term "fibrotic diseases" refers to diseased cells such as diseased fibroblasts, which are found in tissues undergoing fibrosis. Diseased fibroblasts express or overexpress CD47 gene and/or CD47 protein (preferably the CD47 protein is expressed at the cell surface, i.e. plasma membrane, which can convey or is associated with a "do not eat me signal" or "anti-phagocytic signal"). The skilled person knows how to detect or identify said diseased cells using known methods in the art such as detecting the presence or absence of disease-specific, cell type-specific (molecular) makers such as described for instance in WO2015/120350 (e.g., $\alpha$-smooth muscle actin, c-Jun, EIF2AK1, EIF2AK2, EIF2AK3, EIF2AK4, EIF5A, mTOR, DOHH, DHPS, HDAC6, SIRT2, RSK, AHCY, etc.), morphological characteristics, and the like.

In some embodiments, the condition is fibrotic disease. In some embodiments, the condition is fibrotic disease and the fibrotic disease is selected from the group consisting of idiopathic pulmonary fibrosis (IPF), scleroderma, myelofibrosis, kidney fibrosis, liver fibrosis, lung fibrosis, pancreas fibrosis, heart fibrosis, and bladder fibrosis

***Infectious Disease***

The term "infectious diseases" as used herein refers to conditions in which at least one cell of an organism (i.e., a subject) is infected by an infectious agent, such as a pathogen, that induces increased CD47 expression in at least one cell of the infected organism. For example, infectious agents include, but are not limited to bacteria, viruses, protozoans, and fungi. Therefore, it is understood that infectious diseases are disorders caused by infectious agents. Non-limiting examples of infectious diseases include diseases that are caused by a pathogen selected from a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella

pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania. In the context of the present invention, the term "infectious diseases" refers to diseased cells infected by a pathogen (e.g. virus), which express or overexpress CD47 gene and/or CD47 protein (preferably the CD47 protein is expressed at the cell surface, i.e. plasma membrane, and can convey or is associated with a "do not eat me signal" or "anti-phagocytic signal"). It is understood that the diseased cell will vary depending on the specific infectious disease and specific pathogen. The skilled person knows how to detect or identify said diseased cells using known methods in the art such as detecting the presence or absence of disease-specific, cell type-specific (molecular) makers, morphological characteristics, and the like.

In some embodiments, the condition is infectious disease. In some embodiments, the infection disease is caused by a virus, bacterium or protozoan. In some embodiments, the infectious disease is caused by a pathogen selected from the group consisting of a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

## Methods of Reducing Expression or Enzymatic Activity of QPCT and/or QPCTL

In one aspect, disclosed herein is a pharmaceutical composition comprising an active agent for use in a method of reducing binding between CD47 on the surface of a first cell and SIRP$\alpha$ on the surface of a second cell in a subject, wherein the active agent reduces expression or enzymatic activity of QPCTL, QPCT, or combinations thereof in said first cell with CD47 on the surface.

In some embodiments, the subject has a condition that would benefit from reducing binding between CD47 on the surface of a first cell and SIRP$\alpha$ on the surface of a second cell in the subject.

In some embodiments, disclosed herein is a pharmaceutical composition comprising an active agent for use in a method of treating a condition in a subject that would benefit from reducing binding between CD47 and SIRP$\alpha$ in the subject, wherein the active agent reduces expression or enzymatic activity of QPCTL, QPCT, or combinations thereof in a cell with CD47 on the surface.

In some embodiments, reducing the expression of QPCTL, QPCT, or combinations thereof comprises reducing the transcription, the translation, or combinations thereof of the gene encoding QPCTL, the gene encoding QPCT, or combinations thereof. In some embodiments, the active agent comprises a double-stranded RNA molecule, a small inhibitory RNA (siRNA) molecule, or an inhibitory RNA molecule (RNAi).

In some embodiments, the transcription and/or translation of the gene(s) encoding QPCTL, QPCT, or combinations thereof is reduced by the use of an active agent comprising a double-stranded RNA molecule, a small inhibitory RNA (siRNA) molecule, or an inhibitory RNA molecule (RNAi) designed to reduce the expression of QPCTL and/or QPCT, or a guideRNA (gRNA) designed to disrupt the QPCTL and/or QPCT gene, or using a CRISPR-Cas system such as CRISPRi system wherein in the CRISPRi system, a catalytically dead Cas 9 (dCas9), lacking endonuclease activity, is co-expressed with the gRNA. The gRNA is complementary to the region of the gene of interest one wishes to repress or activate. The skilled person is well-acquainted with methods for altering (e.g. reducing) transcription and/or translation of genes, e.g. gene (s) encoding QPCTL and/or QPCT.

In some embodiments, enzymatic activity of QPCTL, QPCT, or combinations thereof is reduced by the use of an active agent, which is an inhibitor of QPCTL, QPCT, or combinations thereof. Any active agents capable of reducing the enzymatic activity of QPCTL, QPCT, or combinations thereof may be used in the methods of the invention, such as for instance the active agents and pharmaceutical compositions thereof, as taught herein. In some embodiments, the inhibitor is selected from the group consisting of compounds of Formula (I), (II), (III), (IV), (V), (VI), (VII), or (VIII), or a compound disclosed in Table A, B, C, D, or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066, as taught herein. In some embodiments, the active agent is selected from the group consisting of PBD150, PQ912, and PQ1565.

The enzymatic activity of the QPCTL and/or QPCT protein or the expression of the QPCTL and/or QPCT protein and/or gene in a cell may be measured or assessed by any suitable methods. In some embodiments, enzymatic activity of QPCTL and QPCT is measured by contacting cells or cell lysates with a substrate for which the formation of pGlu can be detected. In some embodiments, the (level of) expression of QPCTL protein and/or QPCT protein is measured or determined by detecting the presence of QPCTL protein and/or QPCT protein in a cell using an antibody directed

against the QPCTL protein or QPCT protein. In some embodiments, the (level of) expression of QPCTL protein and/or QPCT protein is quantified by measuring the amount of QPCTL protein and/or QPCT protein using western blot methods, and the like. In some embodiments, the expression of QPCTL gene and/or QPCT gene is measured or determined by detecting the presence of QPCTL gene and/or QPCT gene in a cell (e.g. mRNA) using *in situ* hybridization using probes directed against the QPCTL gene or QPCT gene. In some embodiments, the expression of QPCTL gene and/or QPCT gene is quantified by measuring the amount of QPCTL gene and/or QPCT gene (DNA or mRNA) using PCR techniques and the like.

In a further aspect, the present invention relates to a pharmaceutical composition comprising a CD47 inhibitor (e.g. anti-CD47 IgA antibody) for use in a method of treating a condition in a subject, wherein the subject would benefit from reducing binding between CD47 on the surface of a first cell and SIRPα on the surface of a second cell, and wherein the CD47 inhibitor is an inhibitor that binds said CD47 on the surface of said first cell and thereby reduces the binding of said CD47 to said SIRPα on the surface of said second cell, and wherein the method of treating comprises reducing expression or enzymatic activity of QPCTL, QPCT, or combinations thereof in said first cell.

In a further aspect, the present invention relates to a pharmaceutical composition comprising a SIRPα inhibitor for use in a method of treating a condition in a subject, wherein the subject would benefit from reducing binding between CD47 on the surface of a first cell and SIRPα on the surface of a second cell, and wherein the SIRPα inhibitor is an inhibitor that binds said SIRPα on the surface of said second cell and thereby reduces the binding of said SIRPα to said CD47 on the surface of said first cell, and wherein the method of treating further comprises reducing expression or enzymatic activity of QPCTL, QPCT, or combinations thereof in said first cell.

In some embodiments, reducing the expression or enzymatic activity of QPCTL, QPCT, or combinations thereof in the cell with CD47 expressed on its surface while using a pharmaceutical composition comprising a CD47 inhibitor or a SIRP inhibitor as taught herein in a method of treating a condition in a subject that would benefit from reducing signaling or binding between SIRPα and CD47 in the subject, can be done as taught above, using the active agents (QPCT and QPCTL inhibitors) as taught herein. In some embodiments, reducing the expression or the enzymatic activity of QPCTL, QPCT, or combinations thereof in said first cell further reduces binding of said CD47 on the surface of said first cell to said SIRPα on the surface of said second cell. In some embodiments, the treatment comprises monitoring the said binding between CD47 on the surface of said first cell and SIRPα on the surface of said second cell in the subject, and wherein increased binding is indicative of a condition that would benefit from reducing said binding between CD47 on the surface of said first cell and SIRPα on the surface of said second cell in said subject. In some embodiments, the step of reducing the expression or enzymatic activity of QPCTL, QPCT, or combinations thereof in the cell with CD47 expressed on its surface while using a pharmaceutical composition comprising a CD47 inhibitor or a SIRPα inhibitor as taught herein in a method of treating a condition in a subject that would benefit from reducing signaling or binding between SIRPα and CD47 in the subject, is performed either simultaneously or sequentially with the step of using a pharmaceutical composition comprising a CD47 inhibitor or a SIRP an inhibitor as taught herein. In some embodiments, the CD47 inhibitor is an antibody.

In some embodiments, reducing expression or enzymatic activity of QPCTL, QPCT, or combinations thereof in the cell expressing CD47 at its surface further reduces binding of CD47 located on the surface of said cell (e.g. cancer cell) to SIRPα expressed on the surface of another cell (e.g. a phagocyte such as a macrophage). In some embodiments, this leads to a reduced number of cells (e.g. cancer cells) having a functional level of CD47 at their surface, i.e. functional in terms of being capable of binding to SIRPα expressed at the surface of another cell and thereby evading phagocytosis by a phagocyte (e.g. macrophage, neutrophil).

In some embodiments, the treatment comprises monitoring binding between SIRPα expressed at the surface of one cell (e.g. macrophage, neutrophil) and CD47 expressed at the surface of another cell (e.g. cancer cell) in the subject wherein increased binding is indicative of a condition that would benefit from reducing binding between SIRPα and CD47 in the subject. In some embodiments, binding between SIRPα expressed at the surface of one cell (e.g. macrophage, neutrophil) and CD47 expressed at the surface of another cell is assessed by using any suitable method in the art, e.g. by using a labelled recombinant SIRPα protein.

In some embodiments, the subject is a mammal. In some embodiments, the subject is human.

In some embodiments, the said first cell with CD47 on the surface is a diseased cell that is expressing or overexpressing CD47. In some embodiments, expression of CD47 in the diseased cell is 1.5-fold higher, 2.0-fold higher, 2.5-fold higher, 3.0-fold higher or more than in non-diseased cells.

In some embodiments, the condition that would benefit from reducing signaling or binding between SIRPα and CD47 in the subject is selected from the group consisting of cancer, atherosclerosis, fibrotic disease and infectious disease. In some embodiments, the diseased cell is selected from the group consisting of a cancer cell, vascular smooth muscle cell, endothelial cell, a cell infected by a pathogen, and a cell in a tissue undergoing fibrosis.

In some embodiments, the diseased cell is a cancer cell. In some embodiments, the diseased cell is a cancer cell selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon

cancer, breast cancer, leiomyosarcoma, pancreatic neuroendocrine tumors, small cell lung cancer, and bladder cancer, HNSCC, Gastric cancer, esophageal cancer, T-ALL, glioma, mesothelioma, glioblastoma, melanoma and NSCLC, and others). In some embodiments, the diseased cell is a cancer cell selected from the group consisting of a leukemia cell or acute myeloid leukemia (AML) cell.

In some embodiments, the condition that would benefit from reducing binding between CD47 on the surface of a first cell and SIRPα on the surface of a second cell in the subject is selected from the group consisting of cancer, atherosclerosis, fibrotic disease, and infectious disease.

In some embodiments, the condition is cancer. In some embodiments, the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, Gastric cancer, esophageal cancer, T-ALL, glioma, mesothelioma, glioblastoma, melanoma and NSCLC, and others). In some embodiments, the cancer is leukemia or AML.

In some embodiments, the condition is atherosclerosis.

In some embodiments, the condition is fibrotic disease. In some embodiments, the fibrotic disease is selected from the group consisting of idiopathic pulmonary fibrosis (IPF), scleroderma, myelofibrosis, kidney fibrosis, liver fibrosis, lung fibrosis, pancreas fibrosis, heart fibrosis, and bladder fibrosis.

In some embodiments, the condition is infectious disease. In some embodiments, the infectious disease is caused by a pathogen selected from virus, bacterium and protozoan. In some embodiments, the infectious disease is caused by a pathogen selected from the group consisting of a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

In some embodiments, the cell with CD47 on the surface is a cell selected from the group consisting of a diseased cell, a cancer cell expressing or overexpressing CD47, a vascular smooth muscle cells expressing or overexpressing CD47, a diseased endothelial cell expressing or overexpressing CD47, a diseased cell infected by a pathogen (e.g. virus) expressing or overexpressing CD47, and a diseased cell undergoing fibrosis expressing or overexpressing CD47 on its cell surface.

In some embodiments, the cell expressing the SIRPα on its surface is a myeloid cell. In some embodiments, the myeloid cell is selected from the group consisting of a macrophage, monocyte, neutrophil, basophil, eosinophil, and dendritic cell.

In some embodiments, reducing binding between said CD47 on the surface of said first cell and said SIRPα on the surface of said second cell targets said first cell with CD47 on the surface for phagocytosis. In some embodiments, phagocytosis of said first cell is increased.

In some embodiments, reducing binding between said CD47 on the surface of said first cell and said SIRPα on the surface of said second cell targets said first cell with CD47 on the surface for ADCC. In some embodiments, ADCC of said first cell is increased. In some embodiments, the killing of a diseased cell (e.g. cancer cells) via ADCC involves or uses IgA antibodies (e.g. anti- Her2-IgA1 antibody or anti-CD47-IgA antibody, and others).

In some embodiments, reducing binding between said CD47 on the surface of said first cell and said SIRPα on the surface of said second cell targets said first cell with CD47 on the surface for ADCP. In some embodiments, ADCP of said first cell is increased. In some embodiments, the killing of a diseased cell (e.g. cancer cells) via ADCP (using compounds as taught herein) involves or uses IgA antibodies (e.g. anti- Her2-IgA1 antibody or anti-CD47-IgA antibody, and others).

In some embodiments, the pharmaceutical compositions as taught herein are for use in a treatment for increasing phagocytosis (e.g. by a phagocyte cell such as a macrophage) or for use in a treatment for increasing killing or death of diseased cells (e.g. cancer cells) via ADCP or ADCC. In some embodiments, it is a use in a treatment for increasing phagocytosis of a diseased cell or it is a use in a treatment for increasing killing or death of a diseased cell (e.g. cancer cell) in a subject (e.g. human), such as a cancer cell expressing or overexpressing CD47, a vascular smooth muscle cells expressing or overexpressing CD47, a diseased endothelial cell expressing or overexpressing CD47, a diseased cell infected by a pathogen (e.g. virus) expressing or overexpressing CD47, or a diseased cell undergoing fibrosis expressing or overexpressing CD47 on its cell surface. In some embodiments, the killing of a diseased cell (e.g. cancer cells) via ADCC or ADCP (using compounds as taught herein) involves or uses IgA antibodies (e.g. anti- Her2-IgA1 antibody or anti-CD47-IgA antibody, and others).

In some embodiments, the pharmaceutical compositions disclosed herein further comprise a second active agent selected from the group consisting of anti-PD-L1 antibody, anti-CD20 antibody, anti-Her2 antibody, anti-EGFR

antibody, anti-CD20-CD47 bispecific antibody, anti-CD56 antibody, anti-TRP-1-PD-L1 bispecific antibody, and anti-CD271-sporin antibody. In some embodiments, the second active agent is an IgA antibody.

In some embodiments, the anti-PD-L1 antibody is Atezolizumab, Durvalumab, or Avelumab. In some embodiments, the anti-Her2 antibody is Trastuzumab. In some embodiments, the anti-CD20 antibody is Rituximab. In some embodiments, the anti-EGFR antibody is Cetuximab. In some embodiments, the anti-CD20-CD47 bispecific antibody is the antibody as described in Piccione et al (2015), mAbs, Vol. 7, pages 946-956. In some embodiments the anti-CD56 antibody is the antibody as described in Weiskoft et al (2016), Journal of Clinical Investigation, Vol. 126, pages 2610-2620. In some embodiments, the anti-CD271-sporin antibody is the antibody described in Ngo et al (2016) Cell Reports, Vol. 16, pages 1701-1716. In some embodiments, the anti-PD-1 antibody is Pembrolizumab.

In some embodiments, reducing the expression or the enzymatic activity of QPCTL, QPCT, or combinations thereof in the cell with CD47 on the surface is performed as taught above, using any of the compounds, active agents, and pharmacological compositions as taught herein.

**Screening Methods**

In another aspect, the present invention relates to an in vitro method for selecting or screening for active agents that reduce signaling or binding between CD47 on the surface of a first cell and SIRPα on the surface of a second cell, the method comprising screening for active agents that reduce expression or enzymatic activity of QPCTL, QPCT, or combinations thereof. The skilled person is well-acquainted with methods for assessing the effect of active agent on signaling or binding between SIRPα and CD47 in a subject. For instance, flow cytometric analysis of CD47 on cells using a CD47 antibody (e.g. CC2C6) that specifically binds or targets the pyroglutamyl residue present at the N-terminus of CD47 and a SIRPα, which is fused to human IgG1 so as to form a SIRPα-Fc, which is then subjected to an immune-staining procedure using a secondary antibody against human IgG1, followed by analysis of the immune-staining signal using flow cytometry technology. Likewise, assessing the effects of an active agent on the expression or enzymatic activity of QPCTL and/or QPCT in cells may be done by any suitable methods in the art such as those described herein.

In some embodiments, the method comprises screening for active agents that reduce expression or enzymatic activity of QPCTL, QPCT, or combinations thereof in said first cell expressing CD47 on its surface. Non-limiting examples of such cells include diseased cells such as cancer cells, diseased vascular smooth muscle cells, diseased endothelial cells, diseased cells infected by a pathogen (e.g. virus), and diseased cells undergoing fibrosis.

In some embodiments, the method for screening active agents as taught herein further comprises the steps of:

a. providing a cell with CD47 on the surface, wherein said cell is expressing QPCTL, QPCT, or combinations thereof;
b. contacting said cell with a test compound;
c. contacting said cell with a ligand capable of binding to CD47 (CD47 ligand), wherein the ligand is a SIRPα protein;
d. measuring the level of binding of the CD47 ligand to CD47; and
e. determining whether the test compound is an active agent that reduces binding between CD47 on the surface of a first cell and the SIRPα protein,

wherein the test compound is an active agent that reduces binding between CD47 on the surface of a first cell and the SIRPα protein if the binding of the CD47 ligand to the CD47 protein is reduced in said cells.

In some embodiments, the CD47 ligand is a SIRPα protein expressed on the surface of a second cell or is a SIRPα recombinant protein or parts thereof.

In some embodiments, the method for screening active agents as taught herein may (alternatively) comprise the steps of:

a. providing a cell with CD47 on the surface, wherein said cell is expressing QPCTL, QPCT, or combinations thereof;
b. contacting said cell with a test compound;
c. contacting said cell with a ligand capable of binding to CD47 (CD47 ligand), wherein the ligand is an antibody directed against the pyroglutamyl residue at the N-terminus of CD47 (pGlu residue);
d. measuring the level of binding of the CD47 ligand to CD47; and
e. determining whether the test compound is an active agent that reduces binding between CD47 on the surface of a first cell and the CD47 ligand,

wherein the test compound is an active agent that reduces binding between CD47 on the surface of a first cell and the CD47 ligand, if the binding of the CD47 ligand to the CD47 protein is reduced in said cells.

In some embodiments of step (a) relating to the methods for screening active agents as taught herein, cells expressing CD47 at their surface as well as the QPCTL protein and/or QPCT protein are any suitable cells such as mammalian cell or cell lines (e.g. human, mouse, rat, etc.). In some embodiments, cells with CD47 on the surface and

expressing QPCTL and/or QPCT include diseased cells such as cancer cells, diseased vascular smooth muscle cells, diseased endothelial cells, diseased cells infected by a pathogen (e.g. virus), and diseased cells undergoing fibrosis, and others.

In some embodiments of step (b) relating to the methods for screening active agents as taught herein, contacting the cells with a test compound are performed by adding the test compound directly in the culture media in a suitable (first) concentration or (first) dosage. The skilled person is acquainted with methods and techniques for determining a suitable (first) concentration or (first) dosage, and is aware that the test compound may need to be tested at more than one concentration or dosage to obtain the desired effects.

In some embodiments of step (c) relating to the methods for screening active agents as taught herein, the ligand capable of binding to CD47 (i.e. CD47 ligand) may be:

1) a SIRPα protein. In some embodiments, the CD47 ligand is SIRPα expressed on the surface of a second cell (e.g. a myeloid cell such as a macrophage, monocyte, neutrophil, basophil, eosinophil, or dendritic cell). In some embodiments, the CD47 ligand is SIRPα recombinant protein (SIRPα-FC). It is understood that observing decreased binding between the CD47 ligand (i.e. SIRPα protein) and the CD47 protein on the surface of the first cell (e.g. a diseased cell, such as a cancer cell) serves as an indication (readout) that the test compound is a compound capable of decreasing the expression or enzymatic activity of QPCTL and/or QPCT, which in turn blocks or reduces the formation of pGlu residue on the CD47 protein present on the first cells, which in turn blocks or reduces binding between CD47 on the surface of the first cell and the SIRPα protein.
Or

2) a CD47 ligand capable of binding to the CD47 protein located at the extracellular surface of the cell, particularly at the location or place on the CD47 protein which contains the pGlu residue. In some embodiments, such ligand includes the anti-CD47 antibody clone CC2C6 (Biolegend, catalogue number 323106), which specifically recognize the region on the CD47 protein containing the pGlu residue. It is understood that observing decreased binding between the CD47 ligand (i.e. CD47 antibody targeting pGlu residue on the CD47 protein, e.g. antibody clone CC2C6) and the CD47 protein on the surface of the first cell (e.g. a diseased cells, such as a cancer cell) serves as an indication (readout) that the test compound is a compound capable of decreasing the expression or enzymatic activity of QPCTL and/or QPCT, which in turn blocks or reduces the formation of pGlu residue on the CD47 protein present on the first cells, which in turn blocks or reduces binding between CD47 on the surface of the first cell and the CD47 antibody targeting pGlu residue on the CD47 protein (e.g. antibody clone CC2C6).

In some embodiments of step (d) relating to the methods for screening active agents as taught herein, measuring the level of binding of the CD47 ligand is performed using any suitable method in the art. For instance, flow cytometric analysis of CD47 on cells using SIRPα may be used, where SIRPα is fused to human IgG1 so as to form a SIRPα-Fc, which is then subjected to an immune-staining procedure using a secondary antibody against human IgG1, followed by analysis of the immune-staining signal using flow cytometry technology. Alternatively, flow cytometric analysis of CD47 on cells may be performed by using a CD47 antibody targeting pGlu residue on the CD47 protein (e.g. antibody clone CC2C6), which is then subjected to an immune-staining procedure using a secondary antibody, followed by analysis of the immune-staining signal using flow cytometry technology.

In a further aspect, described herein is an in vitro method for selecting or screening for active agents that reduce signaling or binding between CD47 on the surface of a first cell and SIRPα on the surface of a second cell, or active agents that reduce expression or enzymatic activity of QPCTL, QPCT, or combinations thereof, the method comprising:

a. providing a cell with CD47 on the surface, wherein said cell is expressing QPCTL, QPCT, or combinations thereof;
b. contacting said cell with a test compound;
c. detecting the presence of a pyroglutamyl residue at the N-terminus of CD47; and
d. determining whether the test compound is an active agent that reduces binding between CD47 on the surface of a first cell and SIRPα on the surface of a second cell, or an active agent that reduces expression or enzymatic activity of QPCTL, QPCT, or combinations thereof,

wherein the test compound is an active agent that reduces binding between CD47 on the surface of a first cell and SIRPα on the surface of a second cell, or an active agent that reduces expression or enzymatic activity of QPCTL, QPCT, or combinations thereof if the presence of a pyroglutamyl residue at the N-terminus of CD47 is reduced or absent.

In some embodiments, steps (a) and (b) are performed as taught above.

In some embodiments, step (c) is performed using any suitable methods in the art. In some embodiments, detection of the presence of a pyroglutamyl residue at the N-terminus of CD47 is performed using an antibody that recognize the pyroglutamyl residue present on the N-terminus of CD47 such as CD47 antibody clone CC2C6 (Biolegend,

catalogue number 323106).

In a further aspect, disclosed herein is a method of reducing or inhibiting the binding between CD47 on the surface of a first cell and SIRPα on the surface of a second cell in a cell with CD47 on the surface in a subject, wherein the method comprises providing to the subject an active agent that reduces expression or enzymatic activity of QPCTL, QPCT, or combinations thereof in said first cell with CD47 on the surface.

In some embodiments, the active agent is any suitable active agent capable of reducing the expression or enzymatic activity of QPCTL, QPCT, or combinations thereof in a cell expressing CD47 on its surface (e.g. cancer cell). In some embodiments, the active agent and pharmaceutical compositions are taught herein. In some embodiments, the active agent is selected from the group consisting of compounds of Formula (I), (II), (III), (IV), (V), (VI), (VII), or (VIII), or a compound disclosed in Table A, B, C, D, or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066, as taught herein. In some embodiments, the active agent is selected from the group consisting of PBD150, PQ912, and PQ1565.

In a further aspect, disclosed herein is a method of treatment of a condition in a subject that would benefit from reducing signaling or binding between CD47 on the surface of a first cell and SIRPα on the surface of a second cell in the subject, wherein the method comprises providing to the subject an active agent that reduces expression or enzymatic activity of QPCTL, QPCT, or combinations thereof in said first cell with CD47 on the surface.

In some embodiments, the active agent is any suitable active agent capable of reducing the expression or enzymatic activity of QPCTL, QPCT, or combinations thereof in a cell expressing CD47 on its surface (e.g. cancer cell). In some embodiments, the active agent is selected from the group consisting of compounds of Formula (I), (II), (III), (IV), (V), (VI), (VII), or (VIII), or a compound disclosed in Table A, B, C, D, or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066, as taught herein. In some embodiments, the active agent is selected from the group consisting of PBD150, PQ912, and PQ1565.

In some embodiments, the condition in a subject that would benefitfrom reducing signaling or binding between SIRPα and CD47 is selected from cancer, atherosclerosis, fibrotic diseases as well as infectious diseases caused by pathogens (e.g. virus), and others. Specific examples of cancer, atherosclerosis, fibrotic diseases as well as infectious diseases caused by pathogens (e.g. virus) are as described herein.

In some embodiments, the condition in a subject that would benefitfrom reducing signaling or binding between SIRPα and CD47 is cancer. In some embodiments, the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma, NSCLC, and others. In some embodiments, the cancer is leukemia or AML.

In another aspect, described herein is the use of an inhibitor of QPCTL, QPCT, or combinations thereof for reducing binding between CD47 on the surface of a first cell and SIRPα on the surface of a second cell in a subject. In some embodiments, the inhibitor is selected from the group consisting of compounds of Formula (I), (II), (III), (IV), (V), (VI), (VII), or (VIII), or a compound disclosed in Table A, B, C, D or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066, as taught herein. In some embodiments, the inhibitor is selected from the group consisting of PBD150, PQ912, and PQ1565.

In some embodiments, the subject has a condition that would benefit from reducing binding between CD47 on the surface of said first cell and SIRPα on the surface of said second cell in the subject. In some embodiments, the condition comprises overexpression of CD47 on the surface of said first cell. In some embodiments, the expression of CD47 is 1.5-fold higher, 2.0-fold higher, 2.5-fold higher, 3.0-fold higher or more in diseased cells than in non-diseased cells

In some embodiments, the condition is selected from the group consisting of cancer, atherosclerosis, fibrotic disease, and infectious disease.

In some embodiments, the condition is cancer and the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma, NSCLC, and others). In some embodiments, the cancer is leukemia or acute myeloid leukemia (AML).

In some embodiments, the condition is atherosclerosis.

In some embodiments, the condition is fibrotic disease and the fibrotic disease is selected from the group consisting of idiopathic pulmonary fibrosis (IPF), scleroderma, myelofibrosis, kidney fibrosis, liver fibrosis, lung fibrosis, pancreas fibrosis, heart fibrosis, and bladder fibrosis.

In some embodiments, the condition is infectious disease and the infectious disease is selected from the group consisting of diseases that are caused by a pathogen selected from a virus, bacterium or protozoan. In some embodiments, the infectious disease is caused by a pathogen selected from the group consisting of a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

## EXAMPLES

### EXAMPLE 1

Haploid genetic flow cytometry-based screen

In order to identify genetic regulators of CD47 cell surface expression, a batch of mutagenized HAP1 cells was prepared using gene-trap retrovirus expressing blue fluorescent protein (BFP) as described previously (Blomen et al (2015), Science, Vol. 350, pages 1092-1096).

Briefly, 50 million HAP1 cells were seeded and transduced with virus from two combined harvests on three consecutive days in the presence of 8 μg/mL protamine sulfate (Sigma).

The mutagenized library was then expanded to 30 T175 flasks at a confluence of about 80%. Subsequently, cells were dissociated with trypsin, washed once with PBS (Lonza) and stained with a FITC-labeled antibody directed against CD47 (Biolegend, clone CC2C6, catalogue number 323106) at 1:80 dilution for 30 minutes at 4 °C in 20 ml PBS containing 0.5% w/v bovine serum albumin (BSA; Sigma) and 0.2% w/v sodium azide (Sigma).

Next, the cells were washed three times with PBS containing 10% FCS and subsequently stained with a FITC-labelled polyclonal goat anti-mouse IgG (Biolegend, clone Poly4053, catalogue number 405319) at 1:100 dilution for 30 minutes at 4 °C in PBS containing 0.5% w/v BSA and 0.2% w/v sodium azide. Following two washes with PBS containing 10% FCS and one wash with PBS, cells were fixed with BD Fix Buffer I (BD biosciences) for 10 min at 37 °C.

After washing twice with PBS containing 10% FCS, the cells were filtered through a 40 μm strainer (BD Falcon) before sorting two populations of cells (i.e. '$CD47^{LOW}$' and '$CD47^{HIGH}$'). Specifically, the first cell population referred to as '$CD47^{LOW}$' constitutes about 1-2% of the lowest CD47-expressing cells from the total population. The second cell population referred to as '$CD47^{HIGH}$' constitutes about 1-2% of the highest CD47-expressing cells from the total population. In addition, in order to reduce potential confounding effects of diploid cells which are heterozygous for alleles carrying gene-trap integrations, the cells were sorted in parallel for DNA content (1n) by staining with propidium iodide (Life Technologies).

Cell sorting was carried out on a Biorad S3 Cell sorter until approximately 10 million cells per population were collected. Sorted cells were pelleted and genomic DNA was isolated using a DNA mini kit (Qiagen). To assist de-crosslinking of genomic DNA, the cell pellets were resuspended in PBS supplemented with Proteinase K (Qiagen) followed by overnight incubation at 56°C with lysis buffer AL (Qiagen) with agitation. Insertion sites of both sorted cell populations were amplified and mapped to the human genome as described previously (Blomen et al., 2015) using a Linear Amplification polymerase chain reaction (LAM-PCR) on the total yield of isolated genomic DNA.

In brief, samples were submitted for deep sequencing and gene-trap insertion sites were mapped and analyzed as follows: Insertion sites were retrieved from trimmed reads (50b) that aligned unambiguously to Hg19 using Bowtie (Langmead B. et al (2009), Genome Biology, Vol. 10, R25) allowing one mismatch. Using intersectBed, aligned reads were mapped to non-overlapping Refseq gene-coordinates. Intragenic gene-trap insertions in sense orientation with its gene were considered disruptive and kept for further analysis. For each gene, the number of unique disruptive insertions was compared between the $CD47^{LOW}$ and $CD47^{HIGH}$ population. Genes that were significantly enriched for insertions in either of the two populations (two-sided Fisher's Exact test with Benjamini-Hochberg multiple testing correction, $p < 0.05$) were considered as regulators of CD47 cell-surface levels. To reflect the directionality of the effect on CD47 abundance, a mutational index (MI)-score was calculated as follows:

$$\frac{Sum\ unique\ ins.\ in\ gene\ X\ in\ high\ pop.}{(Sum\ unique\ ins.\ in\ high\,pop) - (Sum\ unique\ ins.\ in\ gene\ X\ in\ high\,pop.)} \Big/ \frac{Sum\ unique\ ins. in\ gene\ X\ in\ low\ pop.}{(Total\ unique\ ins.\ in\ low\ pop) - (Sum\ unique\ ins.\ in\ gene\ X\ in\ low\ pop.)}$$

For those genes where in only one of the two populations disruptive insertions were identified, 1 insertion was assigned to the other population to prevent these genes to be omitted from the visualization plots.

Cell lines

HAP1 cells have been described previously (Carette et al (2011), Nature, Vol. 477, pages 340-343). HAP1 cells were cultured in IMDM (ThermoFisher Scientific) supplemented with 10% fetal calf serum (FCS, Sigma), 100U/ml penicillin-streptomycin (ThermoFisher Scientific) and L-glutamine.

A375, A549, DLD1 and RKO cells were purchased from American Type Culture Collection (ATCC). A375 and A549 cells were cultured in DMEM supplemented with FCS (8%) and penicillin/streptomycin (100U/ml). RKO and DLD1 cells were cultured in RPMI supplemented with FCS (8%) and penicillin/streptomycin (100U/ml).

Antibody and SIRPα-Fc staining

The antibodies and fusion protein used are listed in Table 1 below. Surface levels of CD47 were assessed by performing immunohistochemical staining cells with fluorochrome labelled antibodies directed against CD47 (clones CC2C6, 2D3, B6H12., see Table 1) at a dilution of 1:50 in PBS containing 0.5% w/v BSA (Sigma) and 0.2% w/v sodium azide (Sigma) ("FACS buffer") for 30 minutes, at 4 °C, protected from light. SIRPα binding to CD47 was assessed by incubating cells with recombinant extracellular domain of human SIRPα fused to human IgG ((SIRPα-Fc); Recombinant Human SIRP alpha/CD172a Fc Chimera Protein see Table 1) at a dilution of 1:50 in FACS buffer for 30 minutes, at 4 °C, protected from light. After 1 wash with FACS buffer to remove unbound SIRPα-Fc, cells were immunostained with a fluorochrome labelled antibody against human IgG (HP6017, see Table 1) for 30 minutes, at 4 °C, protected from light. After 2 washes with FACS buffer to remove unbound antibody, immunohistochemical staining intensity or binding intensity was analyzed on an LSRII (BD Bioscience).

Table 1. Antibody list and supplier information.

| **Product** | **Company** | **Cat#** | **Dilution used** |
|---|---|---|---|
| Anti-Human CD47 FITC (CC2C6) | BioLegend | 323106 | 1:50 |
| Anti-Human CD47 FITC (2D3) | BioLegend | 11-0478-41 | 1:50 |
| Anti-Human CD47 APC (B6H12) | BioLegend | 17-0479-41 | 1:50 |
| Alexa Fluor® 488 Goat anti-mouse IgG (minimal x-reactivity) Antibody (Poly4053) | BioLegend | 405319 | 1:100 |
| Recombinant Human SIRP alpha/CD172a Fc Chimera Protein, CF | R&D Systems | 4546-SA-050 | 1:50 |
| APC anti-human IgG Fc Antibody (HP6017) | BioLegend | 409305 | 1:100 |

Vector generation

The cDNA of the two transcript variants of QPCTL, glutaminyl-peptide cyclotransferase-like, transcript 1 (RefSeq: NM_017659.3) ("QPCTL(1)") or glutaminyl-peptide cyclotransferase-like, transcript 2 (RefSeq: NM_001163377.1) ("QPCTL(2)"), were ordered as a codon optimized gBlock Gene Fragment (IDT DNA). QPCTL(1) has 7 exonic and 6 intronic regions, whereas QPCTL(2) has 6 exonic and 5 intronic regions. QPCTL(1). Effectively, QPCTL(1) has an exonic region (exon 3/7) that is missing in QPCTL(2), containing the amino acid sequence FLEATLRSLTAGWHVELDPFTASTPLGPVDFGNVVATLDPRAARHLTLACHYDSKLFPP GSTPFVGATDSAVPCALLLELAQALDLELSRAKKQ (SEQ ID NO: 10). A codon optimized variant of the cDNA of CD47 transcript variant 2 (RefSeq: NM_198793.2), the most principal transcript variant of CD47 was generated to obtain "CD47 WT". In addition, a mutant was generated in which the glutamine (Q) at position 19 in the codon-optimized CD47 WT cDNA was replaced with an

aspargine (N) to obtain "CD47 MUT". All constructs were ordered as gBlock Gene Fragments (IDT DNA) and cloned into the pCDH-CMV-MCS-EF1-Puro (CD510-B1) vector containing a Puromycin selection cassette, using the restriction sites EcoRI and NotI. Constructs were verified by Sanger sequencing.

CRISPR/Cas9-mediated generation of QPCTL and CD47 knockout cells

Guide RNAs targeting *QPCTL* (5'-CGGGGAGGCTTCCGATCAAT-3' (SEQ ID NO:1) and 5'-CCTGCTGGTTGTGCGAACCC-3' (SEQ ID NO:2)) and CD47 (5'-CTACTGAAGTATACGTAAAG-3' (SEQ ID NO:3) and 5'-CTTGTTTAGAGCTCCATCAA-3' (SEQ ID NO:4)) were designed and cloned into the pX330 expression vector (Cong et al (2013) Science, Vol. 339, pages 819-823).

HAP1 cells were co-transfected with the gene-specific gRNA vectors and a plasmid containing an expression cassette for a guide RNA targeting the zebrafish *TIA* gene (5'-GGTATGTCGGGAACCTCTCC-3' (SEQ ID NO:5)) followed by a CMV promotor sequence driving expression of a blasticidin resistance gene flanked by two *TIA* target sites (Lackner et al., (2015), Nature Comm., Vol. 6, page 10237). Co-transfection of these plasmids occasionally results in the incorporation of the blasticidin resistance cassette at the site of the targeted genomic locus by non-homologous end joining, rendering cells resistant to blasticidin while also providing a genomic tag at the site of mutation. Four days after DNA transfection, culture medium was supplemented with 20 $\mu$g/mL blasticidin (Invivogen). Surviving colonies were clonally expanded and their mutations and/or genomic incorporation of the blasticidin resistance gene were verified by PCR and Sanger sequencing. The QPCTL locus was amplified using primers 5'-GTTTGAGGTAGGCTGGACCGGATGGTCTTG-3' (SEQ ID NO:6) and 5'-GGTACCCACCTTATAGGGCTGTCTGTTGCC-3' (SEQ ID NO:7). The CD47 locus was amplified using primers 5'-CAAAGCTTCCAAAGCCAGATACTACACCTGCATGTTCC-3' (SEQ ID NO: 8) and 5'-GGCCTCCTCTCGAAAGAGGATCAGGTTGCACC-3' (SEQ ID NO:9).

In parallel, a polyclonal population of CD47 knockout cells (referred to herein as 'CD47 poly') was obtained by flow cytometric cell sorting of HAP1 cells transfected with a plasmid expressing Cas9 and a guide RNA that targets CD47. The following cell populations were used:

- HAP1 QPCTL KO clone 10 ("HAP1 QPCTL KO cl10")
- HAP1 QPCTL KO clone 21 ("HAP1 QPCTL KO cl21")
- HAP1 CD47 KO clone 4 ("HAP1 CD47 KO cl4")
- HAP1 CD47 KO clone 17 ("HAP1 CD47 KO cl17")
- HAP1 CD47 KO clone 23 ("HAP1 CD47 KO cl23")

Flow cytometric analysis of cells

HAP1 wild-type (WT) or the respective CD47 or QPCTL clonal knock-out (KO) mutants were immunostained for CD47 using the anti-CD47 antibody clones CC2C6, 2D3 and B6H12.2, or with the extracellular domain of SIRP$\alpha$ fused to human IgG1 (SIRP$\alpha$-Fc) (followed by immunohistochemical staining with a secondary antibody against human IgG1) and analyzed by flow cytometry (See Table 1 for antibody information including dilution used and supplier information).

SIRP$\alpha$-Fc blocking assay

HAP1 WT, HAP1 CD47 KO or HAP1 QPCTL KO cells were left unstained (i.e. not exposed to CD47 antibodies) or stained (i.e. exposed to anti-CD47 antibodies for the purpose of masking or blocking pyroglutamyl residue at the N-terminus of CD47) with anti-CD47 antibody clones CC2C6 or 2D3 for 30 minutes at 4 °C (protected from light). Cells were then washed with FACS buffer, after which they underwent SIRP$\alpha$-Fc binding. Subsequently, the cells were washed and immunostained with AF488-labeled goat anti-mouse IgG secondary antibody and analyzed by flow cytometry to reveal levels of SIRP$\alpha$-Fc binding.

Generation and analysis of CD47 and QPCTL overexpressing cells

HAP1 QPCTL KO cells ("clone 10" or "clone 21") were transduced with a lentiviral vector containing the cDNA of QPCTL(1) or QPCTL(2) as described above. After 48 hours, transduced cells were selected with 2ug/mL Puromycin for 72 hours. Next, the cells were harvested and stained with anti-CD47 antibody clones CC2C6, 2D3 or with SIRP$\alpha$-Fc (followed by staining with a secondary antibody against human IgG) and analyzed by flow cytometry. See Table 1 for antibody information including dilution used and supplier information).

## Results

Flow-based genetic screen to identify regulators of CD47

In order to identify novel regulators of CD47, we made use of a forward genetic screening approach in haploid human HAP1 cells as we observed that HAP1 cells express CD47.

We created a library of loss-of-function mutants in HAP1 cells using a modified version of a retroviral gene trap (Jae et al (2013), Science, Vol. 340, pages 479-483), expanded these cells and subjected them to a staining for CD47 at the cell surface using an antibody against human CD47 (clone CC2C6). This resulted in distribution of signal intensity when analyzed by flow cytometry. For the genetic screen, we selected those mutants that displayed the strongest and the weakest CD47 staining and sorted approximately 10 million cells for each population, and then analyzed their gene-trap integration sites, similar as described before (Blomen et al (2015), Science, Vol. 350, pages 1092-1096).

The screen yielded a total of 667 hits where loss of expression in this gene results in altered CD47 levels. Of those, 406 outliers occurred in the CD47 high ("$CD47^{HIGH}$") population and 261 outliers in the CD47 low ("$CD47^{LOW}$") population. Besides the gene coding for CD47 itself, the CD47 low population included ELAVL1 (HuR), a RNA-binding protein that is part of a protein complex that is known to facilitate translocation of CD47 to the plasma membrane (Berkovits and Mayr (2015) Nature, Vol. 522, pages 363-367) and Glutaminyl-Peptide Cyclotransferase Like (QPCTL) (see Figure 1).

Anti-CD47 antibody CC2C6 and SIRPα-Fc show reduced binding to QPCTL KO HAP1 cells

Next, we sought to validate the involvement of QPCTL in CC2C6 antibody binding to CD47, and to assess its involvement in SIRPα binding to CD47 by Cas9 mediated-disruption of QPCTL or - as a control - CD47.

To evaluate the impact of QPCTL on CD47 expression, we immunostained HAP1 WT, CD47 KO and QPCTL KO cells with different anti-CD47 antibodies (see Table 1). We observed that, whereas binding of anti-CD47 antibodies 2D3 and B6H12.2 to CD47 in HAP1 QPCTL KO cells was unaffected, the binding of CC2C6 to CD47 on HAP1 QPCTL KO cells was decreased compared to HAP1 WT cells (see Figure 2A).

Importantly, SIRPα-Fc binding to CD47 on HAP1 QPCTL KO cells as compared to HAP1 WT cells was likewise decreased (Figure 2B). Thus, QPCTL modifies the binding of CD47 to its physiological ligand SIRPα and to anti-CD47 antibody CC26, while overall cell surface CD47 levels, as determined by immunostaining with the anti-CD47 antibodies 2D3 and B6H12.2 remain unaltered. To investigate whether QPCTL KO has the same effect on antibody and SIRPα binding in other cell lines, QPCTL and CD47 KO cells were generated in human melanoma cell line A375 and the human rectal carcinoma cell line RKO and stained with anti-CD47 antibody CD47 CC2C6, 2D3 or with SIRPα-Fc. As seen in HAP1 QPCTL KO cells, CC2C6 and SIRPα-Fc binding to CD47 was reduced in QPCTL KO cells as compared to the parental WT cell line, whereas 2D3 binding remains unaltered (Figures 3 and 4). Thus, the role of QPCTL in the regulation of binding of CD47 to its physiological ligand SIRPα and to anti-CD47 antibody CC26 extends beyond the HAP1 cell line, and has been observed in all cell lines for which this has been tested.

Anti-CD47 antibody CC2C6 blocks SIRPα-Fc binding to HAP1 cells

To investigate whether anti-CD47 antibody CC2C6 binds to a site on CD47 that overlaps with the binding site of SIRPα, HAP1 WT cells were left unstained (i.e. no subjected to immunohistochemical staining) or stained (i.e. subjected to immunohistochemical staining) with anti-CD47 antibodies CC2C6 or 2D3. After washing, the cells were immunostained with SIRPα-Fc. Flow cytometry analysis showed that when cells were first stained with anti-CD47 clone 2D3, subsequent SIRPα-Fc binding to CD47 was unaffected (Figure 5). In contrast, when the cells were first stained with anti-CD47 antibody CC2C6, subsequent SIRPα-Fc binding to HAP1 cells was decreased, demonstrating that anti-CD47 clone CC2C6 and SIRPα-Fc interact with the same surface are of CD47.

Restoration of anti-CD47 antibody CC2C6 and SIRPα-Fc binding to QPCTL KO cells by transduction with QPCTL transcript variant 1

To investigate whether the decreased binding of CD47 clone CC2C6 to CD47 on HAP1 QPCTL KO cells could be rescued by genetic reconstitution of QPCTL, HAP1 QPCTL KO cells were transduced with a vector expressing the cDNA of QPCTL transcript variant 1 ("QPCTL(1)") or QPCTL transcript variant 2 ("QPCTL(2)") as described above. After selection, the cells were immunostained with anti-CD47 antibodies CC2C6 or 2D3, or with SIRPα-Fc. Binding of anti-CD47 antibody 2D3 was not affected by introduction of either QPCLT(1) or QPCLT(2). In contrast, CC2C6 and SIRPα-Fc binding to CD47 HAP1 QPCTL KO cells was increased in cells that overexpressed QPCTL(1), to the level observed for HAP1 WT cells (Figure 6). QPCTL transcript variant 1 is the dominant transcript, whereas QPCTL transcript 2 is

considered to be the minor transcript.

Mutagenesis of the N-terminus of CD47 prevents binding of anti-CD47 antibody CC2C6 and of SIRPα-Fc

To investigate whether the glutamine (Q) amino acid that is present at the N-terminus of CD47 after removal of the signal peptide is involved in the binding to anti-CD47 clone CC2C6 and SIRPα-Fc, we generated a mutant CD47 protein in which the N-terminal Q is mutated to an asparagine (N) ("CD47 MUT"). Next, HAP1 CD47 KO cells ("clone 4", "clone 17" and "clone 21") were transduced with a lentiviral vector encoding either CD47 wild-type (WT) or CD47 MUT, immunostained with anti-CD47 antibodies CC2C6 and clone 2D3, or with SIRPα-Fc. Both antibodies and SIRPα-Fc could bind to HAP1 CD47 KO cells that were engineered to express the wild-type variant of CD47. In contrast, only clone 2D3 could bind to HAP1 CD47 KO cells that were engineered to express the mutant form of CD47 (Figure 7).

Small molecule inhibition of Glutaminyl cyclase

Here we set out to investigate whether inhibiting pyroglutaminyl cyclase activity by means of a small molecule inhibitor results in decreased anti-CD47 clone CC2C6 binding. For this experiment we choose PBD150 as the pyroglutamyl inhibitor (QPCT, QPCTL) (Schilling et al (2008), Nature medicine, Vol. 14, pages 1106-1111). HAP1 cells were cultured for 72 hours with PBD150 or vehicle and CD47 clone CC2C6 levels were assessed. Flow cytometry analysis showed that when cells were incubated with PBD150 (1000 microM), decreased levels of CC2C6 were bound to the surface, in a dose dependent manner (Figure 8A). To determine whether CC2C6 binding was transiently decreased we subsequently cultured the cells 48 hrs without PBD150. Flow cytometric analysis showed CC2C6 binding to cells was restored to normal levels, clearly indicating that it is a reversible process (Figure 8B). Thus both genetic and pharmacological inhibition of pyroglutaminyl cyclases (QPCTL) resulted in decreased CC2C6 binding.

Next, HAP1, RKO, A375, A549 and DLD1 cells were treated for 72 hours with PBD150 (1000 microM) or vehicle and medium was refreshed every 24 hours. Flow cytometric analysis showed that CC2C6 and SIRPα-Fc binding was decreased relative to cells that were incubated with PBD150, whereas 2D3 binding remained unchanged (Figure 9). Thus both genetic and pharmacological inhibition of pyroglutaminyl cyclases (QPCTL) resulted in decreased CC2C6 and SIRPα-Fc binding in 5 different cell lines.

## EXAMPLE 2

### Materials and Methods

Haploid genetic flow cytometry-based screen

Mutagenized HAP1 cells were prepared using gene-trap retrovirus expressing blue fluorescent protein (BFP). Briefly, 50 million HAP1 cells were seeded and transduced with virus from two combined harvests on three consecutive days in the presence of 8 μg/mL protamine sulfate (Sigma). The mutagenized cell library was then expanded to 30 T175 flasks at a confluence of about 80%. Subsequently, cells were dissociated with trypsin, washed once with PBS (Lonza) and stained with FITC-labelled αCD47-CC2C6 (Biolegend, clone CC2C6, catalogue number 323106) at a 1:80 dilution for 30 min at 4 °C in 20 mL PBS containing 0.5% w/v bovine serum albumin (BSA; Sigma) and 0.2% w/v sodium azide (Sigma). Subsequently, the cells were washed three times with PBS containing 10% FCS and stained with FITC-labelled polyclonal goat anti-mouse IgG (Biolegend, clone Poly4053, catalogue number 405319) at a 1:100 dilution for 30 minutes at 4 °C in PBS containing 0.5% w/v BSA and 0.2% w/v sodium azide. Following two washes with PBS containing 10% FCS and one wash with PBS, cells were fixed with BD Fix Buffer I (BD Biosciences) for 10 min at 37 °C. After washing twice with PBS containing 10% FCS, the cells were filtered through a 40 μm strainer (BD Falcon) before isolation of the two cell populations of interest (i.e. 'CD47-CC2C6$^{LOW}$' and 'CD47-CC2C6$^{HIGH}$') by fluorescence-activated cell sorting. Specifically, the first cell population, referred to as 'CD47-CC2C6$^{LOW}$', constitutes the approximately 1-2% of cells with the lowest level of αCD47-CC2C6 binding. The second cell population, referred to as 'CD47-CC2C6$^{HIGH}$', constitutes the approximately 1-2% of cells with the highest level of αCD47-CC2C6 binding.

To reduce potential confounding effects of diploid cells that are heterozygous for alleles carrying gene-trap integrations, cell sorting was restricted to cells with a 1n DNA content, as determined by staining with propidium iodide (Life Technologies). Cell sorting was carried out on a Biorad S3 Cell sorter until approximately 10 million cells were collected for each population.

Sorted cells were pelleted and genomic DNA was isolated using a DNA mini kit (Qiagen). To assist de-crosslinking of genomic DNA, cell pellets were resuspended in PBS supplemented with Proteinase K (Qiagen) followed by overnight incubation at 56 °C in lysis buffer AL (Qiagen) with agitation. Insertion sites present in both sorted cell populations were amplified and mapped to the human genome using a Linear AMplification polymerase chain reaction (LAM-PCR)

on the total yield of isolated genomic DNA.

Samples were submitted for deep sequencing and gene-trap insertion sites were mapped and analyzed as follows: insertion sites were retrieved from trimmed reads (50b) that aligned unambiguously to Hg19 using Bowtie allowing one mismatch. Using intersectBed, aligned reads were mapped to non-overlapping Refseq gene-coordinates. Intragenic gene-trap insertions in sense orientation within its gene were considered disruptive and kept for further analysis. For each gene, the number of unique disruptive insertions was compared between the CD47-CC2C6$^{LOW}$ and CD47-CC2C6$^{HIGH}$ population. Genes that were significantly enriched for insertions in either of the two populations (two-sided Fisher's Exact test with Benjamini-Hochberg multiple testing correction, P< 0.05) were considered as regulators of CD47-CC2C6 binding. To reflect the directionality of the effect on CD47 abundance, a mutational index (MI)-score was calculated as follows:

$$\frac{Sum\ unique\ ins.\ in\ gene\ X\ in\ high\ pop.}{(Sum\ unique\ ins.\ in\ high\ pop) - (Sum\ unique\ ins.\ in\ gene\ X\ in\ high\ pop.)} \Big/ \frac{Sum\ unique\ ins. in\ gene\ X\ in\ low\ pop.}{(Total\ unique\ ins.\ in\ low\ pop) - (Sum\ unique\ ins.\ in\ gene\ X\ in\ low\ pop.)}$$

For those genes for which disruptive insertions were identified in only one of the two populations, one insertion was assigned to the other population to allow inclusion of these genes in visualization plots.

Cell lines

HAP1 cells have been described previously (Carette, J.E. et al. Nature, 2011: 477, 340-343). A375, A431, A549, Ba/F3, DLD1, RKO, Raji and SKBR3 cells were purchased from American Type Culture Collection (ATCC). B16F10 cells were kindly provided by D. Peeper, B16-GM-CSF cells were kindly provided by N. Haining.

HAP1 cells were cultured in IMDM (ThermoFisher Scientific) supplemented with 10% Fetal Calf Serum (FCS, Sigma), 100 U/mL penicillin-streptomycin (ThermoFisher Scientific) and L-glutamine. A375, A549, B16F10 and B16-GM-CSF cells were cultured in DMEM supplemented with 10% FCS and 100 U/mL penicillin/streptomycin. A431, DLD1 and Raji cells were cultured in RPMI supplemented with 10% FCS and 100 U/mL penicillin/streptomycin. Ba/F3-Her2 cells were cultured in RPMI supplemented with 10% FCS, 100 U/mL penicillin/streptomycin, 0.2 ng/mL mouse IL-3 (Immunotools) and 5.0 μg/mL puromycin. SKBR3 cells were cultured in IMDM supplemented with 20% FCS and 100 U/mL penicillin-streptomycin.

Flow cytometry

The following antibodies and recombinant extracellular domains of SIRPα were used: anti-human CD47: CC2C6 (BioLegend), anti-human CD47: 2D3 (BioLegend), anti-human CD47: B6H12 (BioLegend), anti-mouse CD47: MIAP301 (BioLegend), recombinant human SIRP alpha/CD172a Fc Chimera Protein (R&D Systems), recombinant mouse SIRP alpha/CD172a Fc Chimera Protein (R&D Systems), goat anti-mouse IgG: Poly4053 (BioLegend), anti-human IgG Fc: HP6017 (BioLegend).

Binding to cell surface CD47 was assessed by staining of cells with fluorochrome labelled antibodies directed against human CD47 (clones CC2C6, 2D3, B6H12) or mouse CD47 (clone MIAP301) at a dilution of 1:50 (or 1:80 in case of αCD47-CC2C6/αCD47-B6H12 double stainings) in PBS containing 0.5% w/v BSA (Sigma) and 0.2% w/v sodium azide (Sigma) ("FACS buffer") for 30 min, at 4 °C, protected from light. SIRPα binding to CD47 was assessed by incubating cells with recombinant human SIRPα (hSIRPα-Fc) or recombinant mouse SIRPα (mSIRPα-Fc) at a dilution of 4 μg/mL or 2 μg/mL, respectively, in FACS buffer for 30 min, at room temperature, protected from light. After one wash with FACS buffer to remove unbound SIRPα-Fc, cells were immunostained with a fluorochrome labelled mouse antibody against human IgG (HP6017) or a goat polyclonal antibody against mouse IgG, at a dilution of 1:100 for 30 minutes, at 4 °C, protected from light. After indicated antibody stainings, cells were washed with FACS buffer to remove unbound antibody and DAPI was added to allow dead cell exclusion and samples were analyzed on an LSRII or LSRFortessa (BD Bioscience).

Ba/F3-Her2 and effector cells retrieved from the peritoneum of FcαRI transgenic BALB/c mice were analyzed after incubation with 5% normal mouse serum for 45 min at 4-7 °C. Subsequently, cells were stained for 45-60 min at 4-7 °C with the following fluorescently labelled antibodies to determine the composition of immune infiltrates: anti-mouse B220 (RA3-6B2) anti-mouse CD3ε (145-2C11), anti-mouse MHC class II (M5/114.15.2), anti-human CD89 (A59), anti-mouse CD8α (53-6.7), anti-mouse Ly-6G (1A8), anti-mouse CD45 (30-F11), anti-mouse CD4 (RM4-5), anti-mouse FcγRIV (CD16.2, 9E9), anti-mouse F4/80 (BM8) (Biolegend), anti-mouse CD11b (m1/70), and anti-mouse Siglec-F

(E50-2440) (BD biosciences). Measurements were performed on a FACSCantoII (BD Biosciences), data were analyzed using FACS Diva software (BD Biosciences).

Vector generation

The cDNA of the two human transcript variants of QPCTL, glutaminyl-peptide cyclotransferase-like, transcript 1 (RefSeq: NM_017659.3) and glutaminyl-peptide cyclotransferase-like, transcript 2 (RefSeq: NM_001163377.1), were ordered as codon optimized gBlock Gene Fragments (IDT DNA) encoding an N-terminal FLAG tag. QPCTL(1) consists of 7 exons, whereas QPCTL(2) consists of 6 exons, lacking an exonic region (exon 3) that encodes the amino acid sequence: FLEATLRSLTAGWHVELDPFTASTPLGPVDFGNVVATLDPRAARHLTLACHYDSKLFPP GSTPFVGATDSAVPCALLLELAQALDLELSRAKKQ (SEQ ID NO: 11). The cDNA of the mus musculus transcript variant of QPCTL, (RefSeq: NM_026111.3) ("mQPCTL"), was ordered as a codon optimized gBlock Gene Fragment. A codon optimized variant of the cDNA of CD47 transcript variant 2 (RefSeq: NM_198793.2), the most abundant transcript variant of CD47 containing the long 3' untranslated region was generated as a gBlock Gene Fragment that encodes a C-terminal HA-tag. The lentiviral pCDH-CMV-MCS-EF1-Puro vector encoding C-terminal His-tagged human QPCTL-WT (ENST00000012049.9) and the QPCTL (D326E) mutant were generated by cloning gBlock Gene Fragments digested with SpeI and EcoR1 into pCDH-CMV-MCS-EF1-Puro digested with NheI and EcoRI. All other constructs were cloned into the pCDH-CMV-MCS-EF1-Puro (CD510-B1) vector containing a puromycin selection cassette, or in the pCDH-CMV-MCS-mPGK-BSR vector (kindly provided by R. Agami) containing a blasticidin selection cassette using the restriction sites EcoRI and NotI. Constructs were verified by Sanger sequencing.

CRISPR/Cas9-mediated generation of CD47, QPCTL knockout cells

To generate CD47- and QPCTL-knockout HAP1 cells, cells were co-transfected with PX330 vector containing gene-specific gRNA against *QPCTL* or *CD47* and a plasmid containing an expression cassette for a guide RNA targeting the zebrafish *TIA* gene (5'-GGTATGTCGGGAACCTCTCC-3' (SEQ ID NO:5)) followed by a CMV promotor sequence driving expression of a blasticidin resistance gene flanked by two *TIA* target sites. Co-transfection of these plasmids occasionally results in the incorporation of the blasticidin resistance cassette at the site of the targeted genomic locus by non-homologous end joining, rendering cells resistant to blasticidin while also providing a genomic tag at the site of mutation. Four days after DNA transfection, culture medium was supplemented with 20 μg/mL blasticidin (Invivogen). Surviving colonies were clonally expanded and their mutations and/or genomic incorporation of the blasticidin resistance gene were verified by PCR and Sanger sequencing.

To generate CD47- and QPCTL-knockout A431, A375, A549, DLD1, RKO and SKBR3 cell lines, cells were transfected with pLentiCRISPR v.2 vector (Addgene 52961) encoding sgRNA targeting the *QPCTL* or *CD47* gene. One day after transfection, culture medium was supplemented with 2 μg/mL puromycin for two days. Single-cell clones were expanded and gene disruption was validated by sequencing the gene locus, TIDE analysis and, in case of CD47, flow cytometry.

To generate bulk CD47- and QPCTL-knockout B16F10 cells, cells were transfected with pLentiCRISPR v.2. vector encoding sgRNA targeting the murine *QPCTL* or *CD47* gene. One day after transfection, culture medium was supplemented with 2 μg/mL puromycin for two days. Selected cells were expanded and sorted on the basis of αmCD47-MIAP301$^{LOW}$mSIRPα-Fc$^{LOW}$ (in the case of CD47 knockout) and αmCD47-MIAP301$^{HIGH}$ mSIRPα-Fc$^{LOW}$ (in case of the QPCTL knockout) to obtain bulk knockout populations.

Her2-expressing Ba/F3 cells were generated by retroviral transduction with human HER2 (pMX-puro-Her2), and positive clones were selected using puromycin. To generate Ba/F3-Her2 CD47 and QPCTL-knockout cells, nucleofection was used to deliver pLentiCRISPR v.2. vector encoding sgRNA targeting the murine *QPCTL* or *CD47* gene, together with a plasmid containing Cas9 and a blasticidin resistance cassette and GFP. One day after nucleofection, culture medium was supplemented with 2 μg/mL blasticidin for two days. Selected cells were expanded and sorted to obtain bulk knockout populations. Next, single cells were isolated and expanded to obtain clonal knockout populations.

Generation and analysis of CD47 and QPCTL overexpressing cells

A375 wild-type and QPCTL-knockout cells (clone 4.1) were transduced with a lentiviral pCDH-Puro vector containing cDNA encoding CD47 plus a C-terminal HA-tag (CD47-HA) and that includes the 3' long untranslated region of CD47. Two days after transduction, cells were selected with 2 μg/mL puromycin for two to three days.

A375 QPCTL-knockout cells (clone 4.1 and clone 4.6), A431 QPCTL-knockout cells (clone 6 and clone 11) and A549 QPCTL-knockout cells (clone 3 and clone 9) were transduced with a lentiviral pCDH-Puro vector containing cDNA encoding human QPCTL(1)-FLAG (and also QPCTL(2)-FLAG in case of A375), as described above. B16F10

QPCTL-knockout cells (bulk KO#1 and KO#2) and Ba/F3-Her2 QPCTL-knockout cells (clone 8 and clone 30) were transduced with a lentiviral vector containing cDNA encoding mouse QPCTL-FLAG, as described above. Two days after transduction, cells were selected with 2 μg/mL puromycin for two to three days.

SEN177 and PQ912 treatment

For flow cytometry analysis, cells were plated in triplicate in the appropriate medium containing 0.03% (v/v) DMSO (vehicle control), 10 μM SEN177 (Sigma Aldrich), or 10 μM PQ912 (Syncom). DMSO or inhibitor was refreshed every day and after four days, cells were analyzed by flow cytometry.

Immunoprecipitation, SDS-PAGE, western blot analysis and isoelectric focusing

For SDS-PAGE and western blot analysis, cells were plated to obtain 70-90% confluency the next day. At the day of analysis, cells were washed with PBS and lysed with RIPA buffer (1% Triton, 0.1% SOC, 0.1% SDS,1 mM EDTA, 10 mM Tris pH 8.0, 140 mM NaCl) supplemented with protease inhibitor cocktail (Roche) and 1mM PMSF (Sigma). After 30 minutes incubation on ice, cell lysates were centrifuged at 20,000 g for 20 minutes at 4 °C. Supernatants were subsequently processed and protein concentrations were measured using the Pierce BCA Protein Assay Kit, according to manufacturer's instruction (ThermoFisher Scientific). Equal amounts of protein supernatants were subsequently processed using a Novex NuPAGE Electrophoresis System (ThermoFisher Scientific) and Trans-Blot Turbo Transfer System (Bio-Rad), according to the manufacturers' instructions. QPCTL(1)-FLAG or QPCTL(2)-FLAG expression was detected using anti-FLAG® M2 (Sigma) (1:1000) and anti-mouse HRP (1:10,000 dilution).

For immunoprecipitation and pulse-chase analysis, A375 WT and QPCTL KO cells overexpressing CD47-HA or CD47 KO cells were plated to obtain 70-90% confluency the next day and when indicated, treated with 10 μM SEN177 inhibitor for 16h. At the day of analysis, cells were starved in methionine- and cysteine-free medium for 1h at 37 °C.

Subsequently, cells were pulse-labelled with 0.75 mCi/600 μL [$^{35}$S]Cys/[$^{35}$S]Met (PerkinElmer) for the indicated time period. Cells were washed with PBS to remove residual [$^{35}$S]Cys/[$^{35}$S]Met and then cultured in regular medium with 1 mM 'cold' methionine and cysteine for the indicated time period. Cells were lysed and [$^{35}$S]Cys/[$^{35}$S] incorporation was measured via TCA precipitation of aliquots of lysates on 3 MM Whatman paper and counting in a Perkin Elmer LSC 2800 ultima gold scintillation counter. Next, for pre-clearing and IP purposes, purified mouse IgG1 kappa isotype control (Biolegend, 400102), anti-human CD47 antibody B6H12.2 (Novus, NBP2-31106), and purified anti-human CD47 antibody CC2C6 (Biolegend, 323102) was bound to protein-G-coated Dynabeads (ThermoFisher Scientific) according to manufacturer's instructions. Protein lysates were incubated with mouse IgG1 kappa isotype control/bead mixtures for 1 h at 4 °C to reduce unspecific binding. Next, pre-cleared protein supernatants were incubated with anti-CD47 B6H12.2-bead or anti-CD47 CC2C6-bead mixtures at 4 °C overnight. Immunoprecipitates were either left untreated treated with Endoglycosidase H (EndoH, New England Biolabs) or N-glycosidase F (PNGase F, New England Biolabs), according to the manufacturer's instructions. Next, immunoprecipitates were heated at 50 °C for 10 min with 2x Laemmli buffer and analyzed using a Novex NuPAGE Gel Electrophoresis System (Thermo Fisher Scientific). Gels were treated with 1 M Na salicylate pH 5.6 before drying and then analyzed on Fujifilm BAS-MP phosphor imager screens 4 °C. Screens were analyzed on a lasser scanner Typhoon FLA 9500.

1D-IEF was performed essentially as described previously (Neefjes, J. J., et al., Hum. Immunol., 1986: 16, 169-181. Immunoprecipitates from indicated cell lines were prepared as described above and were eluted with 30 μL IEF buffer (9.0M ureum, 2% Triton-X100, 2(v/v)% Ampholite pH 3-10, 5% beta-mercapto-ethanol), and samples were analyzed on freshly prepared IEF gels (9.5M ureum , 2% Triton-X100, 4.5% Acrylamide / 0.24% bisAcrylamide, 4% Ampholite pH 5-7, 1% Ampholite pH 3-10 , 0.4% Ampholite pH 6.5-9), which was run for 16 hours. Next, the gel was fixed with 10% acetic acid, 45% methanol and dried. Gels were exposed on a Fuji imaging plate and read out on a Typhoon FLA 9500 laser scanner.

ADCC of $^{51}$Cr-labeled Ba/F3-Her2 target cells by human effector cells

Briefly, $1\times10^6$target cells were labelled with 100 μCi (3.7MBq) $^{51}$Cr for 2 h. After extensive washing, cell numbers were adjusted to $1\times10^5$/mL. The polymorphonuclear leukocyte (PMN) fraction from peripheral blood of healthy donors (UMC Utrecht, Utrecht) was isolated by Ficoll/Histopaque separation (GE Healthcare; Sigma-Aldrich). Effector cells and target cells were added to round-bottom microtiter plates (Corning Incorporated) (E:T ratio = 40:1), in the presence of the indicated concentration of a Her2 antibody. After 4 h incubation at 37 °C, $^{51}$Cr release was measured. Percentage specific lysis was calculated using the following formula: ((experimental cpm - basal cpm)/(maximal cpm - basal cpm)) $\times$ 100, with maximal lysis determined in the presence of 3% triton and basal lysis determined in the absence of antibody and effector cells. For experiments with SEN177, 10 μM SEN177 or DMSO was added three days before the assay, added freshly on the day of the assay, and kept present during the assay. All experiments were performed

in triplicate.

ADCC of $^{51}$Cr-labeled Ba/F3-Her2 target cells by mouse effector cells.

In brief, to obtain mouse effector cells, blood was collected from pegylated granulocyte colony-stimulating factor (G-CSF)-stimulated human FcαRI transgenic Balb/c mice from the retro-orbital plexus into Li-heparin tubes. Erythrocytes were lysed by incubation in water for 30 min, and total leukocytes were resuspended in medium (half the volume of the original blood volume). 50 μL of total leukocytes, containing ~70% PMNs, were added per well.

In vivo killing assays

The peritoneal Ba/F3 tumor model in human FcaR transgenic mice has been described previously (P. Boross et al., EMBO Mol Med. 5, 1213-1226 (2013)). Briefly, Ba/F3-Her2 and Ba/F3-Her2 CD47 KO or Ba/F3-Her2 QPCTL KO cells were labelled with 10 μM or 2 μM CT violet (Invitrogen, Thermofisher) respectively, for 15 min at room temperature. Subsequently, 1:1 mixtures of Ba/F3-Her2 and Ba/F3-Her2 CD47 KO cells, or Ba/F3-Her2 and Ba/F3-Her2 QPCTL KO cells were generated. Subsequently, mice were injected intraperitoneally with $1\times10^7$ cells, in 200 μL PBS. Directly after injection of tumor cells, mice received PBS or 100 μg 1-Her2 by intraperitoneal injection. Sixteen hours after injection, mice were euthanized, the peritoneal cavity was washed with PBS containing 5 mM EDTA, the absolute number of Ba/F3-Her2, Ba/F3-Her2 CD47 KO, and Ba/F3-Her2 QPCTL KO cells was determined by flow cytometry using TruCount tubes (BD Biosciences), and the ratio of Ba/F3-Her2 and Ba/F3-Her2 CD47 KO or Ba/F3 QPCTL KO cells was calculated. Indicated effector cell types were measured in the peritoneum by staining with the indicated antibodies and quantification relative to a constant amount of flow cytometry beads (Invitrogen).

The use of human blood samples and mice in described experiments was approved by the ethical committee of Amsterdam, and the IVD committee, Utrecht.

Results

To reveal novel genetic determinants of CD47-SIRPα binding, a fluorescence-activated cell sorting (FACS)-based haploid genetic screen was performed using an antibody against human CD47 (αCD47-CC2C6) that binds to the SIRPα recognition site. Analysis of gene-trap integration sites in cells with impaired αCD47-CC2C6 binding revealed two strong hits, the *CD47* gene itself, and the enzyme glutaminyl-peptide cyclotransferase-like (*QPCTL, isoQC*) (Fig. 10A).

To determine how QPCTL influences the CD47 protein we generated CD47-deficient and QPCTL-deficient HAP1 cells. CD47 deficiency led to impaired binding of both recombinant SIRPα (SIRPα-Fc) and all anti-CD47 antibodies tested (αCD47-CC2C6, αCD47-2D3, αCD47-B6H12). In contrast, QPCTL-knockout selectively affected binding of recombinant SIRPα and αCD47-CC2C6, while overall cell surface CD47 levels, as determined by binding of other anti-CD47 antibodies (αCD47-2D3 and αCD47-B6H12), remained unaltered (Fig. 10B, C and D). The role of QPCTL as a modifier of CD47 was not restricted to HAP1 but was likewise observed in malignant melanoma (A375), epidermoid carcinoma (A431), lung cancer (A549), colorectal cancer (DLD1) and rectal carcinoma (RKO) cancer cells (Fig. 10E and Fig. 14A, B and C). Reconstitution experiments demonstrated that this activity is encoded by QPCTL transcript variant 1 (Fig. 15A, B, C and D), and introduction of a catalytically dead QPCTL variant (D326E, based on homology with QPCT) demonstrated that the enzymatic activity of QPCTL is essential for its role as a CD47 modifier (Fig. 15E, F).

To assess where in the protein-life cycle CD47 is modified by QPCTL, the fate of CD47 molecules in wild-type and QPCTL-knockout melanoma cells transduced with an HA-tagged CD47 gene product was analyzed by pulse-chase analysis. Comparison of immunoprecipitates obtained with αCD47-CC2C6 and αCD47-B6H12 revealed a selective loss of the CD47 conformation recognized by αCD47-CC2C6 in QPCTL deficient cells (Fig. 11A). At all time-points analyzed no discernible levels of CD47 protein could be isolated by αCD47-CC2C6, indicating that pyroglutamate formation on CD47 is strictly dependent on QPCTL, and does not involve an appreciable level of spontaneous conversion. QPCTL-mediated CD47 modification occurs very early in the protein life-cycle, as demonstrated by the presence of αCD47-CC2C6-reactive CD47 molecules that are sensitive to deglycosylation by endoglycosidase H, indicating endoplasmic reticulum/early Golgi residence (Fig. 11A), and as demonstrated by the fact that a maximal level of pyroglutamate-modified CD47 is already reached after a 10 minute labelling (Fig. 11B).

Treatment with the glutaminyl cyclase inhibitor SEN177 ($IC_{50}$ of 0.013 μM for QPCTL) reduced SIRPα-Fc staining for 8 out of 8 cell lines tested (Fig. 12A and B, Fig. 16A), and to the same extent as seen in QPCTL deficient cells (Fig. 10C, D and E, Fig. 14), while CD47 surface levels remained unaffected. Treatment with the glutaminyl cyclase inhibitor PQ912 yielded similar results (Fig. 16B and C).

Upon cyclisation of an N-terminal residue to form a pyroglutamate, a leaving amino group is replaced by a hydroxyl group, thereby altering the isoelectric point (pI) of the molecule. One-dimensional isoelectric focusing (1D-IEF)

was used to visualize this alteration of pI of HA-tagged CD47 in wild-type and QPCTL-knockout A375 melanoma cells. CD47 from QPCTL-knockout cells was characterized by an increased pI compared to CD47 from wild-type cells (Fig. 12C). Furthermore, treatment with SEN177 increased the pI of CD47 present in wild-type cells to that of QPCTL-knockout cells, but did not affect the pI of CD47 molecules isolated from QPCTL-knockout cells. As a second approach to assess to what extent CD47 modification can be influenced by small molecule inhibition, Western blot analysis was performed on immunoprecipitates of HA-tagged CD47 from untreated or SEN177-treated cells. SEN177 resulted in a near-complete inhibition of pyroglutamate-modified CD47 (Fig. 3D). SEN177-treatment of QPCTL-deficient lung cancer (A549) and epidermoid carcinoma (A431) cells did not further reduce SIRPα binding, as assessed by flow cytometry (Fig. 12D). Together these data demonstrate that glutaminyl cyclase inhibitors alter the CD47 protein by inhibiting QPCTL function, and that the resulting block in pE-modified CD47 is near complete.

The role of QPCTL as a modifier of the CD47 protein was conserved in mice. Specifically, deletion of QPCTL in either B16F10 melanoma cells or Ba/F3 pro-B cells reduced binding of murine SIRPα (mSIRPα-Fc), and this could be restored by lentiviral overexpression (Fig. 17A and B). Likewise, treatment with SEN177 led to reduced binding of SIRPα without altering total CD47 surface levels for both cell lines (Fig. 17C). Ba/F3 cells that express human Her2 were used to evaluate whether inhibition of pyroglutamate formation could increase killing of tumor cells by human neutrophils in the presence of anti-Her2 antibody. Both QPCTL-knockout and SEN177 treatment synergized with anti-Her2 treatment to induce neutrophil-mediated lysis of tumor cells (Fig. 13A and B). Killing efficiency of CD47- and QPCTL-deficient tumor cells by neutrophils was not further enhanced by SEN 177-treatment, demonstrating that the functional effects of SEN 177 are dependent on the CD47 pathway (Fig. 17D). The same effects of QPCTL deletion and small molecule inhibition were observed when using murine immune cells isolated from whole blood as effector cells (Fig. 17E and F).

Her2-expressing Ba/F3 cells were used in a short-term syngeneic peritoneal tumor model to assess the role of QPCTL in tumor cell killing in vivo (de Haij, S., et al., Cancer Res., 2010: 70, 3209-3217; Boross, P., et a/., Haematologica.2011: 96, 1822-1830; Boross, P. et al., EMBO Mol Med., 2013: 5, 1213-1226). Human FcαRI transgenic (Tg) BALB/c mice were injected with a 1:1 mixture of wild-type and QPCTL-deficient cells or, as comparison, wild-type and CD47-deficient cells. Subsequently, mice were treated with anti-Her2 antibody or PBS, and after 16 hours the ratio of QPCTL-deficient versus wild-type cells was analyzed (Fig. 18A). In PBS-treated mice, the ratio of QPCTL deficient cells versus wild-type cells remained unaffected, indicating that QPCTL does not influence short-term tumor cell engraftment. In contrast, in mice treated with anti-Her2 a profound killing of QPCTL-deficient tumor cells over wild-type cells was observed (ratio WT: QPCTL deficient of PBS/anti-Her2 is 1.01/0.10) (Fig. 13C and D, Fig. 18B). This selective killing of QPCTL-deficient cells in anti-Her2-treated mice was accompanied by a large influx of polymorphonuclear leukocytes (PMNs), demonstrating a positive feedback loop that enhances anti-tumor immunity (Fig. 13E and Fig. 18C). An independent experiment confirmed enhanced tumor cell killing achieved by blockade of pyroglutamate formation and indicated that this was similar to that achieved by full genetic deficiency of the CD47 checkpoint (Fig. 18D-F).

## EXAMPLE 3

Cell lines

HAP1 cells have been described previously (Carette et al (2011), Nature, Vol. 477, pages 340-343). HAP1 cells were cultured in IMDM (ThermoFisher Scientific) supplemented with 10% fetal calf serum (FCS, VWR), 100U/ml penicillin, 100μg/ml streptomycin and 2.92μg/ml L-glutamine (ThermoFisher Scientific).

A375 and RKO cells were purchased from American Type Culture Collection (ATCC). A375 and RKO cells were cultured in DMEM supplemented with 10% FCS (BioWest), 100U/ml penicillin, 100μg/ml streptomycin and 2.92μg/ml L-glutamine (ThermoFisher Scientific).

Vector generation

LentiCRISPRv2 vector (Genscript) was cut with PacI and EcoRI restriction enzymes (New England Biolabs) and the backbone was isolated from gel using a Qiaquick Gel Extraction Kit (Qiagen) according to the manufacturer's protocol. A synthetic DNA fragment (CAGGGACAGCAGAGATCCAGTTTGGTTAATTAAGGTACCGAGGGCCTATTTCCCATGATTCCTTCATATTTGCAT ATACGATACAAGGCTGTTAGAGAGATAATTAGAATTAATTTGACTGTAAACACAAAGATATTAGTACAAAATACGT GACGTAGAAAGTAATAATTTCTTGGGTAGTTTGCAGTTTTAAAATTATGTTTTAAAATGGACTATCATATGCTTACC GTAACTTGAAAGTATTTCGATTTCTTGGCTTTATATATCTTGTGGAAAGGACGAAACACCGGAGACGGATTAATTA AACCGTCTCAGTTTAAGAGCTAGAAATAGCAAGTTTAAATAAGGCTAGTCCGTTATCAACTTGAAAAAGTGGCAC CGAGTCGGTGCTTTTTTGAATTC GCTAGCTAGGTCTTGAAAGGAGTGG (SEQ ID NO 12), IDTDNA) was inserted using NEBuilder HiFi DNA Assembly Master Mix (New England Biolabs) to obtain pSCNC-LentiCRISPR. Subsequently,

BsmBI (New England Biolabs) digested pSCNC-LentiCRISPR was isolated from gel using a Qiaquick Gel Extraction Kit (Qiagen) and Oligonucleotides encoding gRNAs targeting QPCTL (TATCTTGTGGAAAGGACGAAACACCGCGGGGAGGCTTCCGATCAATGTTTAAGAG CTAGAAATAGCAAGTTTAAA) (SEQ ID NO: 13) or CD47 (TATCTTGTGGAAAGGACGAAACACCGCCTGCTGGTTGTGCGAACCCGTTTAAGAGCTAGAAATAGCAAGTTTAAA) (SEQ ID NO: 14) were cloned in using NEBuilder HiFi DNA Assembly Master Mix (New England Biolabs) to obtain pSCNC-LentiCRISPR-QPCTL and pSCNC-LentiCRISPR-CD47 respectively.

Creation of QPCTL and CD47 knockout cells

HAP1, A375 or RKO cells were transfected with pSCNC-LentiCRISPR-QPCTL or pSCNC-LentiCRISPR-CD47 using Lipofectamin 2000 (ThermoFisher Scientific) according to the manufacturer's instructions. After 24 hours incubation, cells were selected with puromycin (Invivogen, 1μg/ml) for 48 hours. Cells were harvested using TrypLE dissociation reagent (ThermoFisher Scientific), spun down for 3' at 3000rpm and resuspended in FACS buffer (10% FCS in PBS) before counting using a TC10 cell counter (BioRad). 750,000 Cells were transferred to a new vial, spun down 3' at 3000rpm and incubated in 100μl FACS buffer containing 1:100 FITC-conjugated anti-human CD47 clone CC2C6 in case of cells in which QPCTL was targeted and 1:100 FITC-conjugated anti-human CD47 clone 2D3 for cells in which CD47 was targeted, for 30 minutes at 4 degrees. Cells were spun down and washed once with FACS buffer to remove unbound antibody. Cells were again spun down and taken up in 500μl FACS buffer. Negative cells were sorted out directly in cell culture medium using a FACSAria III and FACS Diva software (BD Biosciences).

Compounds

Compounds 000044, 000060 and 000066 were synthesized as racemates. Separation of the enantiomers was performed on a Waters 80Q Preparative SFC system with a Chiralpak AS column, 250×30mm I.D., 10um particle size at room temperature using isocratic elution with 50% Phase A (Supercritical $CO_2$), 50% Phase B (MeOH, 0.1%$NH_3H_2O$ for 000044 and 000066; EtOH, 0.1%$NH_3H_2O$ for 000060). Approximately 30ml MeOH was added into the sample which was injected at 4ml/injection. Flow rate was 70g/min, back pressure 100 bar and UV at 220nm. For compound 000044 the peak with a retention time of 2.69 minute was isolated, for compound 000060 the peak with a retention time of 3.28 min and for compound 000066 the peak with a retention time of 2.93 min. In all three cases this refers to the faster of the two peaks.

QPCTL Inhibitor assays

In order to test compounds for their effect on pyroglutamylation and SIRPα binding, 10-fold dilutions of compounds were made in 24-well plates (Corning Life Science) with the highest concentration either approaching the maximum medium solubility with a maximum of 500μM. An equal volume of HAP1 (200,000/well), A375 (50,000/well) or RKO (100,000/well) cells was added and QPCTL and CD47 knockout cells were taken along in control wells.

After 48 hours incubation at 37 degrees, 5% $CO_2$, cells were washed once with PBS before releasing with 100μl TrypIE dissociation reagent per well and transfer to V-bottom 96-well plates (Greiner Bio-One) pre-filled with 100μl FACS buffer (10% FCS/PBS). Cells were spun 3' at 3000rpm, washed once with FACS buffer and again spun 3' at 3000rpm. Next, cells were incubated for 2 hours at 4 degrees with a mix of 1:500 FITC-conjugated anti-human CD47 clone 2D3 and 1:500 Alexa647-conjugated anti-human CD47 clone CC2C6 for the pyroglutamylation assays. Alternatively, cells were incubated with 50μl 1:50 recombinant SIRPα-human Fc fusion protein in FACS buffer for the SIRPα assays. For the SIRPα assays, two wash steps followed by spinning 3' at 3000rpm and resuspending the cells in FACS buffer before spinning a final time and resuspending and incubating the pellet for 1 hour at 4 degrees in 100μl 1:100 APC-conjugated rabbit-anti-human antibody. Cells of both assay were spun down and washed twice with FACS buffer to remove unbound antibody. Cells were again spun down and taken up in 300μl FACS buffer before analyzed on a FACSAria III using FACS Diva software (BS Biosciences). The median fluorescence intensity (MFI) of each sample was linearly transformed to scale between the MFIs of wildtype and CD47 knockout cells in case of the 2D3 antibody and the wildtype and QPCTL knockout cells in case of the CC2C6 antibody.

Results

Since QC and isoQC have highly similar enzymatic and structural characteristics (Stephan et al., FEBS journal, 2009), compounds that inhibit QC are highly likely to also inhibit isoQC. Therefore, we tested 38 compounds with reported QC inhibitory activity pertaining to five structural clusters for their effect on CD47 pyroglutamylation in HAP1 cells, along with known isoQC inhibitors PQ912 (Ki=5nM, Lues et al., Alzheimer's & Dementia, 2015) and SEN-177 (Ki=13nM, Jimenez-Sanchez, Nat. Chem. Biol., 2015) (Figures 19-25). For 34 of the tested compounds, a dose dependent reduction

of pE-CD47 signal was observed, as measured using the pE-CD47 specific antibody clone CC2C6. Control stainings using antibody clone 2D3, which binds to CD47 independent of its pyroglutamylation state, show that overall levels of CD47 are not negatively affected by the compounds (Figures 19-24). Since knocking out *QPCT* (encoding QC) in HAP1 cells did not influence the levels of pE-CD47, the observed reductions cannot be explained by inhibition of QC enzyme and are thus attributed to the isoQC inhibitory activity of the compounds used. Four compounds did not show inhibition of CD47 pyroglutamylation despite being QC inhibitors, showing that not necessarily all QC inhibitors have activity in this isoQC assay (Figure 25, and Table 2).

**Table** 2. Compounds having no activity in the isoQC-mediated assay (see Fig. 25).

| | | |
|---|---|---|
| **000015** | **1-(1 H-Benzoimidazol-5-yl)-4-cyclohexanecarbonyl-5-(3,4-dichlorophenyl )-3-hydroxy-1,5-di hydro-pyrrol-2-one** | 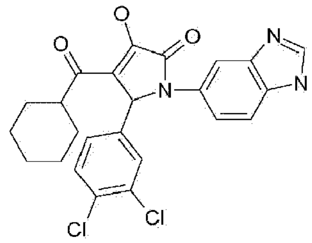 |
| 000033 | 1-(1 H-benzo[d]imidazol-5-yl)-4-benzoyl-5-(2,3-difluorophenyl)-3-hydroxy-1 H-pyrrol-2(5H)-one | 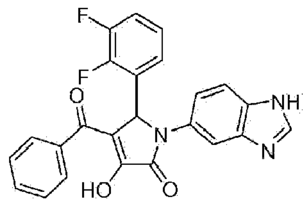 |
| 000046 | 3-Hydroxy-4-(4-hydroxy-phenyl)-1-(3-imidazol-1-yl-propyl)-5-p-tolyl-1,5-dihydropyrrol-2-one | 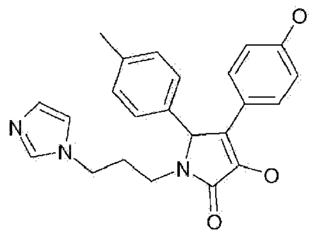 |
| 000040 | 1-(1H-benzo[d]imidazol-5-yl)-5-(2,3-difluoro-4-methylphenyl)-4-thioxoimidazolidin-2-one | 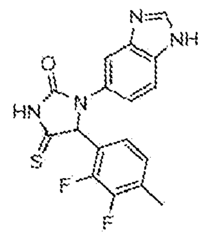 |

We repeated the experiments for at least one compound in every cluster on A375 and RKO cells and obtained very comparable results, showing that the observed reduction in CD47 pyroglutamylation is likely cell-line independent.

Finally, as a functional readout of CD47 pyroglutamylation, we assessed the extent of SIRPα binding to A375 cells that were incubated for 48 hours with at least 1 compound of every cluster. This demonstrated a dose-dependent reduction of SIRPα binding that correlates well with the observed reductions in pE-CD47, confirming a functional consequence of the isoQC inhibitors tested here.

## EXAMPLE 4

Recombinant isoQC

The Golgi luminal, enzymatically active region of human isoQC (S53-L382, Huang et al., JBC, 286, 12439-12449, 2011) was obtained by expression in *E. coli* of an N-terminally GST-Enterokinase, C-terminally 6xHis tagged construct in the pET41(+) vector and subsequent purification and enterokinase digestion. Absence of pyroglutamylating activity in an enzymatic dead variant that was cloned, expressed and purified in parallel ruled out that the purification strategy

isolates any endogenously present pyroglutamylating activity.

IsoQC Assay

Inhibition of isoQC activity was assessed by incubating 30μl 50mM Tris pH8.0 solutions containing 10μM H-Gln-AMC (Bachem cat.no. 4003647.0100, dissolved in 25mM HEPES), 1% DMSO and 750pg/μl recombinant isoQC in the presence or absence of inhibitor for 1 hour at 37°C. A 5-minute incubation at 98°C followed to stop the reaction and inactivate the enzyme. Next, 25μl of the reaction was transferred to a 384-well plate containing 25μl pGAPase enzyme (1:200 diluted in 50mM Tris pH8.0, 10mM Dithiothreitol (DTT), enzyme obtained from Qiagen, cat. no. 34342) and incubated for 10 minutes at 37°C before reading the fluorescence using a SpectraMax Id3 platereader with excitation wavenlength set at 380nm and emission at 450nm. Readings were corrected by subtracting background signal from a control well containing no isoQC enzyme and subsequently normalized by dividing through the signal of a well containing enzyme but no inhibitor.

pGAPase Assay

Inhibition of pGAPase activity was tested similarly to the isoQC assay, except that instead of glutamine-aminocoumaric acid (H-Gln-AMC), 2μM of already fully pyroglutamylated AMC (Pyr-AMC, Bachem cat.no. 4004069.0050, dissolved in DMSO) was used. Background subtraction was performed on a control well containing no pGAPase enzyme and subsequently normalized by dividing through the signal of a well containing enzyme but no inhibitor.

Results

Inhibitors representative of each of the five structural classes of inhibitors were tested for inhibition of isoQC pyroglutamylating activity in a coupled assay. In this assay, isoQC first converts H-Gln-AMC into Pyr-AMC and in a subsequent step, the amount of formed Pyr-AMC is determined after saturating conversion by the enzyme pyroglutamylaminopeptidase (pGAPase) into AMC which can be fluorescently detected in a plate reader. As can be seen in Figure 26, all inhibitors tested showed inhibitory activity across a range of concentrations. Full conversion of Pyr-AMC to AMC in a concomitant pGAPase assay in the presence of the same concentrations of inhibitors further showed that the reduction in isoQC mediated signal was not due to the inhibition of the pGAPase enzyme (Figure 27). Together, these data confirm that these compounds are inhibitors of isoQC enzymatic activity.
Some inhibitors did not show convincing reactivity in the cell-based assay (SC-000015, SC-000033, SC-000040, SC-000045 and SC-000046 (SC-00045 is the enantiomer of SC-000046)), but did show some activity in the enzymatic assay shown in Fig. 26. The compounds without convincing activity in the cell-based assay are the ones with the lowest efficacy in the enzymatic assay. In addition to the diminished inhibitory capacity, the lack of cellular activity may also be attributed to different cell penetrance, intracellular compound degradation, higher concentrations needed for intracellular inhibition, etc..

**EXAMPLE 5**

**Material and methods**

Cell lines: Short-term cell lines from primary melanoma patient-derived xenografts (PDX) were generated as described (Kemper et al., EMBO Mol. Med. 7, 1104-1118, 2015) and were a gift from D. Peeper and K. Kemper. PDX cell lines were treated with 10 μM SEN177 for 4 days, with a refreshment of medium containing SEN177 every 24 hrs. 24 hrs after treatment, binding of CD47 antibodies 2D3 and CC2C6 and recombinant SIRPα-Fc was determined by FACS (as described under examples 1 and 2).

**Results**

To determine whether inhibition of QPCTL in several melanoma lines derived from patients affected the binding of CD47 antibodies 2D3 (a measure for total CD47 expression) and CC2C6 (a measure for pyroglutamylated CD47 expression) and recombinant SIRPα-Fc (which can only bind to CD47 if it is pyroglutamylated, six short-term cultures of melanoma xenografts were treated with SEN177 (Fig. 28). Binding of SIRPα to cells treated with the QPCTL inhibitor was significantly decreased as compared to untreated cells, whereas CD47 protein levels remained unaltered.
The following numbered paragraphs set out preferred features and preferred combinations of features of the present invention

1. A pharmaceutical composition comprising an active agent for use in a method of reducing binding between CD47 on the surface of a first cell and SIRPα on the surface of a second cell in a subject, wherein the active agent reduces expression or enzymatic activity of QPCTL, QPCT, or combinations thereof in said first cell with CD47 on the surface.

2. The pharmaceutical composition according to para 1, wherein the subject has a condition that would benefit from reducing binding between CD47 on the surface of a first cell and SIRPα on the surface of a second cell in the subject.

3. A pharmaceutical composition comprising an active agent for use in a method of treating a condition in a subject that would benefit from reducing binding between CD47 on the surface of a first cell and SIRPα on the surface of a second cell in the subject, and wherein the active agent reduces expression or enzymatic activity of QPCTL, QPCT, or combinations thereof in said first cell with CD47 on the surface.

4. The pharmaceutical composition according to any one of paras 1-3, wherein reducing the expression of QPCTL, QPCT, or combinations thereof comprises reducing the transcription, the translation, or combinations thereof of the gene encoding QPCTL, the gene encoding QPCT, or combinations thereof.

5. The pharmaceutical composition according to para 4, wherein the active agent comprises a double-stranded RNA molecule, a small inhibitory RNA (siRNA) molecule, or an inhibitory RNA molecule (RNAi).

6. The pharmaceutical composition according to any one of paras 1-5, wherein enzymatic activity of QPCTL, QPCT, or combinations thereof is reduced by the use of an active agent which is an inhibitor of QPCTL, QPCT, or combinations thereof.

7. The pharmaceutical composition according to para 6, wherein the inhibitor is selected from the group consisting of compounds of Formula (I), (II), (III), (IV), (V), (VI), (VII), or (VIII), or a compound disclosed in Table A, B, C, D, or E.

8. The pharmaceutical composition according to paras 6-7, wherein the inhibitor is selected from the group consisting of compounds PBD150, PQ912, PQ1565, 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, and 000066.

9. A pharmaceutical composition comprising a CD47 inhibitor for use in a method of treating a condition in a subject, wherein the subject would benefit from reducing binding between CD47 on the surface of a first cell and SIRPα on the surface of a second cell, and wherein the CD47 inhibitor is an inhibitor that binds said CD47 on the surface of said first cell and thereby reduces the binding of said CD47 to said SIRPα on the surface of said second cell, and wherein the method of treating comprises reducing expression or enzymatic activity of QPCTL, QPCT, or combinations thereof in said first cell.

10. A pharmaceutical composition comprising a SIRPα inhibitor for use in a method of treating a condition in a subject, wherein the subject would benefit from reducing binding between CD47 on the surface of a first cell and SIRPα on the surface of a second cell, and wherein the SIRPα inhibitor is an inhibitor that binds said SIRPα on the surface of said second cell and thereby reduces the binding of said SIRPα to said CD47 on the surface of said first cell, and wherein the method of treating comprises reducing expression or enzymatic activity of QPCTL, QPCT, or combinations thereof in said first cell.

11. The pharmaceutical composition of paras 9-10, further comprising a second active agent wherein said second active agent reduces expression or enzymatic activity of QPCTL, QPCT, or combinations thereof in said first cell with CD47 on the surface.

12. The pharmaceutical composition according to paras 9-11, whereby reducing the expression or the enzymatic activity of QPCTL, QPCT, or combinations thereof in said first cell further reduces binding of said CD47 on the surface of said first cell to said SIRPα on the surface of said second cell.

13. The pharmaceutical composition according to paras 9-12, wherein the treatment comprises monitoring the said binding between CD47 on the surface of said first cell and SIRPα on the surface of said second cell in the subject, and wherein increased binding is indicative of a condition that would benefit from reducing said binding between CD47 on the surface of said first cell and SIRPα on the surface of said second cell in said subject.

14. The pharmaceutical composition according to any one of paras 9-13 wherein the CD47 inhibitor is an antibody or an IgA antibody.

15. The pharmaceutical composition according to any one of paras 9-13, wherein the SIRPα inhibitor is an antibody.

16. The pharmaceutical composition according to any one of paras 1-15, wherein the subject is a mammal.

17. The pharmaceutical composition according to any one of paras 1-16, wherein the subject is a human.

18. The pharmaceutical composition according to any one of paras 1-17, wherein the said first cell with CD47 on the surface is a diseased cell that is expressing or overexpressing CD47.

19. The pharmaceutical composition according to para 18, wherein the diseased cell is overexpressing CD47, wherein expression of CD47 is 1.5-fold higher, 2.0-fold higher, 2.5-fold higher, 3.0-fold higher or more than in non-diseased cells.

20. The pharmaceutical composition according to paras 18-19, wherein the diseased cell is selected from the group consisting of a cancer cell, vascular smooth muscle cell, endothelial cell, a cell infected by a pathogen, and a cell in a tissue undergoing fibrosis.

21. The pharmaceutical composition according to para 20, wherein the diseased cell is a cancer cell selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma and non-small cell lung cancer (NSCLC).

22. The pharmaceutical composition according to para 21, wherein the cancer cell is selected from the group consisting of a leukemia cell or acute myeloid leukemia (AML) cell.

23. The pharmaceutical composition according to any one of paras 2-22, wherein the condition that would benefit from reducing binding between CD47 on the surface of a first cell and SIRPα on the surface of a second cell in the subject is selected from the group consisting of cancer, atherosclerosis, fibrotic disease, and infectious disease.

24. The pharmaceutical composition according to para 23, wherein the condition is cancer and the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma and NSCLC.

25. The pharmaceutical composition according to para 24, wherein the cancer is leukemia or acute myeloid leukemia (AML).

26. The pharmaceutical composition according to para 23, wherein the condition is atherosclerosis.

27. The pharmaceutical composition according to para 23, wherein the condition is fibrotic disease and the fibrotic disease is selected from the group consisting of idiopathic pulmonary fibrosis (IPF), scleroderma, myelofibrosis, kidney fibrosis, liver fibrosis, lung fibrosis, pancreas fibrosis, heart fibrosis, and bladder fibrosis.

28. The pharmaceutical composition according to para 23, wherein the condition is infectious disease and the infectious disease is selected from the group consisting of diseases that are caused by a pathogen selected from a virus, bacterium or protozoan.

29. The pharmaceutical composition according to para 28, wherein the infectious disease is caused by a pathogen selected from the group consisting of a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

30. The pharmaceutical composition according to any one paras 1-29 wherein said second cell with SIRPα on the surface is a myeloid cell.

31. The pharmaceutical composition according to para 30, wherein the myeloid cell is selected from the group consisting of a macrophage, monocyte, neutrophil, basophil, eosinophil, or dendritic cell.

32. The pharmaceutical composition according to any one of paras 1-31, wherein reducing binding between said CD47 on the surface of said first cell and said SIRPα on the surface of said second cell targets said first cell with CD47 on the surface for phagocytosis or antibody-dependent cellular cytotoxicity (ADCC) or antibody-dependent cellular phagocytosis" (ADCP)

33. The pharmaceutical composition according to para 32, wherein phagocytosis or ADCC or ADCP of said first cell is increased.

34. The pharmaceutical composition according to any one of paras 1-33, further comprising a second active agent selected from the group consisting of anti-PD-L1 antibody, anti-CD20 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody.

35. A pharmaceutical composition comprising a first active agent selected from the group of anti-PD-L1 antibody, anti-CD20 antibody, anti-Her2 antibody, anti-EGFR antibody, anti-CD20-CD47 bispecific antibody, anti-CD56 antibody, anti-TRP-1-PD-L1 bispecific antibody, and anti-CD271-sporin antibody, for use in a method of treating a condition in a subject that would benefit from reducing binding between CD47 on the surface of a first cell and SIRPα on the surface of a second cell in a subject, wherein the method of treating comprises reducing expression or enzymatic activity of QPCTL, QPCT, or combinations thereof in said first cell with CD47 on the surface.

36. The pharmaceutical composition of para 35, further comprising a second active agent wherein said second active agent reduces expression or enzymatic activity of QPCTL, QPCT, or combinations thereof in said first cell with CD47 on the surface

37. An in vitro method for selecting or screening for active agents that reduce binding between CD47 on the surface of a first cell and SIRPα on the surface of a second cell, the method comprising screening for active agents that reduce expression or enzymatic activity of QPCTL, QPCT, or combinations thereof.

38. The method of para 37, the method comprising screening for active agents that reduce the expression or the enzymatic activity of QPCTL, QPCT, or combinations thereof in said first cell with CD47 on the surface.

39. The method of paras 37-38, further comprising the steps of:

a. providing a cell with CD47 on the surface, wherein said cell is expressing QPCTL, QPCT, or combinations thereof;
b. contacting said cell with a test compound;
c. contacting said cell with a ligand capable of binding to CD47 (CD47 ligand), wherein the ligand is a SIRPα protein;
d. measuring the level of binding of the CD47 ligand to CD47; and
e. determining whether the test compound is an active agent that reduces binding between CD47 on the surface of a first cell and the SIRPα protein,

wherein the test compound is an active agent that reduces binding between CD47 on the surface of a first cell and the SIRPα protein if the binding of the CD47 ligand to the CD47 protein is reduced in said cells.

40. The method of para 39, wherein the ligand of CD47 is a SIRPα protein expressed on the surface of a second cell or a SIRPα recombinant protein.

41. The method of paras 37-38, further comprising the steps of:

a. providing a cell with CD47 on the surface, wherein said cell is expressing QPCTL, QPCT, or combinations thereof;
b. contacting said cell with a test compound;
c. contacting said cell with a ligand capable of binding to CD47 (CD47 ligand), wherein the ligand is an antibody directed against the pyroglutamyl residue at the N-terminus of CD47;
d. measuring the level of binding of the CD47 ligand to CD47; and
e. determining whether the test compound is an active agent that reduces binding between CD47 on the surface of a first cell and the CD47 ligand,

wherein the test compound is an active agent that reduces binding between CD47 on the surface of a first cell and the CD47 ligand, if the binding of the CD47 ligand to the CD47 protein is reduced in said cells.

42. An in vitro method for selecting or screening for active agents that reduce binding between CD47 on the surface of a first cell and SIRPα on the surface of a second cell, or active agents that reduce expression or enzymatic activity of QPCTL, QPCT, or combinations thereof, the method comprising:

a. providing a cell with CD47 on the surface, wherein said cell is expressing QPCTL, QPCT, or combinations thereof;
b. contacting said cell with a test compound;
c. detecting the presence of a pyroglutamyl residue at the N-terminus of CD47; and
d. determining whether the test compound is an active agent that reduces binding between CD47 on the surface of a first cell and SIRPα on the surface of a second cell, or an active agent that reduces expression or enzymatic activity of QPCTL, QPCT, or combinations thereof,

wherein the test compound is an active agent that reduces binding between CD47 on the surface of a first cell and SIRPα on the surface of a second cell, or an active agent that reduces expression or enzymatic activity of QPCTL, QPCT, or combinations thereof if the presence of a pyroglutamyl residue at the N-terminus of CD47 is reduced or absent.

43. A method of reducing or inhibiting binding between CD47 on the surface of a first cell and SIRPα on the surface of a second cell in a subject, wherein the method comprises providing to the subject an active agent that reduces expression or enzymatic activity of QPCTL, QPCT, or combinations thereof in said first cell with CD47 on the surface.

44. A method of treatment of a condition in a subject that would benefit from reducing binding between CD47 on the surface of a first cell and SIRPα on the surface of a second cell in the subject, wherein the method comprises providing to the subject an active agent that reduces expression or enzymatic activity of QPCTL, QPCT, or combinations thereof in said first cell with CD47 on the surface.

45. The method of paras 43-44, wherein the method further comprises providing to the subject a CD47 inhibitor, wherein the CD47 inhibitor is an inhibitor that binds CD47 on the surface of said first cell and thereby reduces the binding of said CD47 to said SIRPα on the surface of said second cell.

46. The method of paras 43-44, wherein the method further comprises providing to the subject a SIRPα inhibitor, wherein the SIRPα inhibitor is an inhibitor that binds SIRPα on the surface of said second cell and thereby reduces the binding of said SIRPα to said CD47 on the surface of said first cell.

47. Use of an inhibitor of QPCTL, QPCT, or combinations thereof for reducing binding between CD47 on the surface of a first cell and SIRPα on the surface of a second cell in a subject.

48. The use according to para 47, wherein the inhibitor is selected from the group consisting of compounds of Formula (I), (II), (III), (IV), (V), (VI), (VII), or (VIII), or a compound disclosed in Table A, B, C, D or E.

49. The use according to paras 47-48, wherein the inhibitor is selected from the group consisting of PBD150, PQ912, PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066.

50. The use according to paras 47-49, wherein the subject has a condition that would benefit from reducing binding between CD47 on the surface of said first cell and SIRPα on the surface of said second cell in the subject.

51. The use according to para 50, wherein the condition comprises overexpression of CD47 on the surface of said first cell.

52. The use according to para 51, wherein the expression of CD47 is 1.5-fold higher, 2.0-fold higher, 2.5-fold higher, 3.0-fold higher or more in diseased cells than in non-diseased cells.

53. The use according to any one of paras 47-52, wherein the condition is selected from the group consisting of cancer, atherosclerosis, fibrotic disease, and infectious disease.

54. The use according to para 53, wherein the condition is cancer and the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma and NSCLC.

55. The use according to para 54, wherein the cancer is leukemia or acute myeloid leukemia (AML).

56. The use according to para 53, wherein the condition is atherosclerosis.

57. The use according to para 53, wherein the condition is fibrotic disease and the fibrotic disease is selected from the group consisting of idiopathic pulmonary fibrosis (IPF), scleroderma, myelofibrosis, kidney fibrosis, liver fibrosis, lung fibrosis, pancreas fibrosis, heart fibrosis, and bladder fibrosis.

58. The use according to para 53, wherein the condition is infectious disease and the infectious disease is selected from the group consisting of diseases that are caused by a pathogen selected from a virus, bacterium or protozoan.

59. The use according to para 58, wherein the infectious disease is caused by a pathogen selected from the group consisting of a lentivirus, human T-lymphotropic virus (HTLV), an hepadna virus, hepatitis B virus, a herpes virus, human papilloma virus, la crosse virus, Yersinia sp., Yersinia pestis, Yersinia pseudotuberculosis, Yersinia enterocolitica, Franciscella sp., Helicobacter sp., Helicobacter pylori, Pasturella sp., Vibrio sp., Vibrio cholerae, Vibrio parahemolyticus, Legionella sp., Legionella pneumophila, Listeria sp., Listeria monocytogenes, Mycoplasma sp., Mycoplasma hominis, Mycoplasma pneumoniae, Mycobacterium sp., Mycobacterium tuberculosis, Mycobacterium leprae, Rickettsia sp., Rickettsia rickettsii, Rickettsia typhi, a Plasmodium, a Trypanosoma, a Giardia, a Toxoplasma, and a Leishmania.

## Claims

**1.** A compound capable of inhibiting the enzymatic activity of glutaminyl-peptide cyclotransferase-like protein (QPCTL) protein, in combination with
a second active agent, which is a therapeutic antibody,
for use the treatment of cancer in a subject.
***

**2.** The combination for use of claim 1, wherein the therapeutic antibody is an IgA antibody.

**3.** The combination for use of claim 1 or claim 2, wherein the therapeutic antibody is selected from the list consisting of: an anti-CD47 antibody; an anti-CD20 antibody; an anti-PD-L1 antibody; an anti-Her2 antibody; an anti-EGFR antibody; an anti-CD20-CD47 bispecific antibody; an anti-CD56 antibody; an anti-CD271-sporin antibody; an anti-SIRPα antibody.

**4.** The combination for use of claim 3, wherein the compound is used to complement or enhance the effects of a monotherapy therapeutic antibody treatment.

**5.** The combination for use of any one of claims 1-4, wherein the therapeutic antibody is an anti-CD47 antibody.

**6.** The combination for use of any one of claims 1-4, wherein the therapeutic antibody is an anti-SIRPα antibody.
***

**7.** The combination for use of any one of claims 1-6, wherein the compound and second active agent are administered by the same route.

**8.** The combination for use of any one of claims 1-6, wherein the compound and second active agent are administered by different routes.
***

**9.** The combination for use of any one of claims 1-8, wherein the compound and second active agent are administered simultaneously in a pharmaceutical composition including both or administered separately in separate pharmaceutical compositions in a sequential manner.

**10.** The combination for use of any one of claims 1-9, wherein the compound and second active agent are administered:

(a) close in time, optionally simultaneously; or
(b) within a specified period, which is optionally within 1, 2, 6, 8, 12, 24 hours, two, three, four, five, six, seven days, one, two, three, four weeks, one, two, three, four, five, six or more months.

**11.** The combination for use of claim 10, wherein one of the compound and second active agent is provided daily whereas the other is provided weekly or wherein one of the compound and second active agent is provided for a certain period after which the other is administered.
***

**12.** The combination for use of any one of claims 1-11, wherein the subject is a mammal, which is optionally a human.
***

**13.** The combination for use of any one of claims 1-12, wherein the compound is a small molecule which has a molecular-weight limit of approximately 900 daltons, and optionally has a molecular-weight of less than 500 daltons.

**14.** The combination for use of any one of claims 1-13, wherein the compound is selected from the group consisting of compounds of Formula (I), (II), (III), (IV), (V), (VI), (VII), or (VIII), or a compound disclosed in Table A, B, C, D, or E, e.g. PBD150, PQ912 and PQ1565, and compounds 000051, 000054, 00016, 000034, 000035, 000037, 000055, 000024, 000027, 000050, 000020, 000021, 000022, 000023, 000025, 000010, 000026, 000011, 000036, 000029, 000048, 000049, 000012, 000030, 000031, 000013, 000014, 000032, 000052, 000053, 000064, 000044, or 000066.
***

**15.** The combination for use of any one of claims 1-14, wherein the cancer is selected from the group consisting of leukemia, acute myeloid leukemia (AML), chronic myeloid leukemia, acute lymphoblastic leukemia (ALL), non-Hodgkin's lymphoma (NHL), multiple myeloma (MM), ovarian cancer, gliomas, colon cancer, breast cancer, leiomyosarcoma, pancreatic neuroendocrine tumors, small cell lung cancer, and bladder cancer, HNSCC, gastric cancer, esophageal cancer, T-ALL, glioma, mesothelioma, glioblastoma, melanoma, NSCLC.

Fig. 1

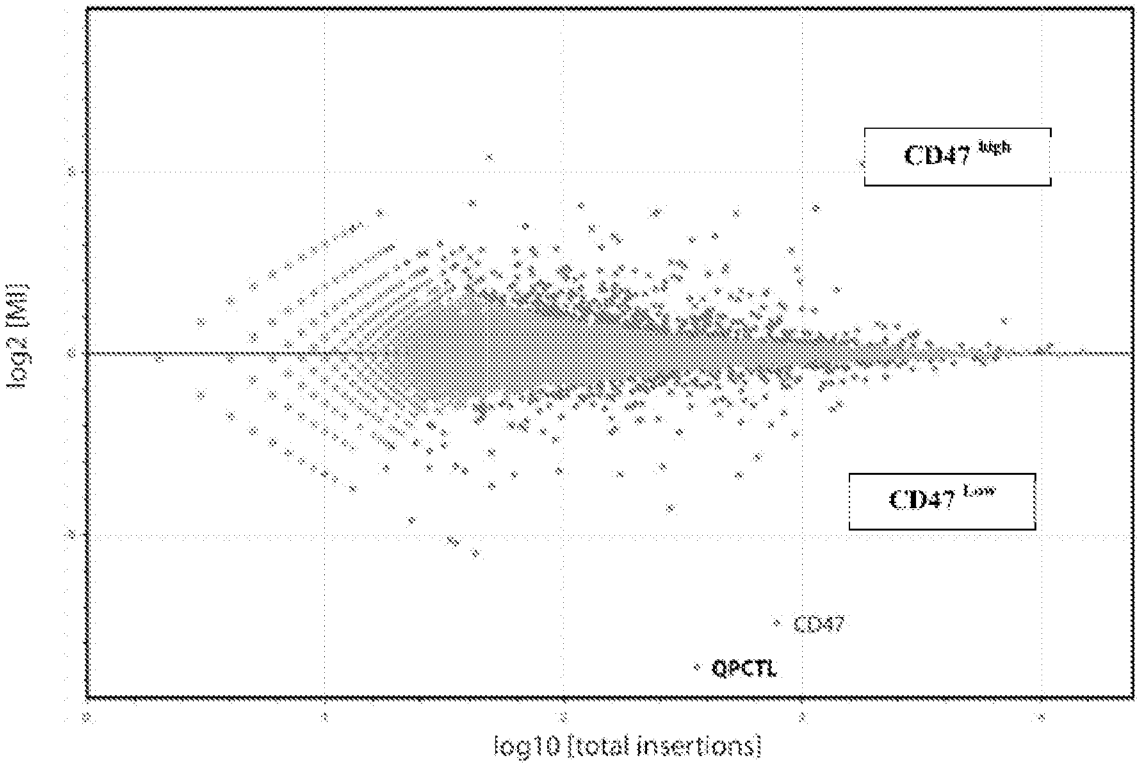
CD47 high
CD47 Low
CD47
QPCTL
log2 [MI]
log10 [total insertions]

Fig. 2A

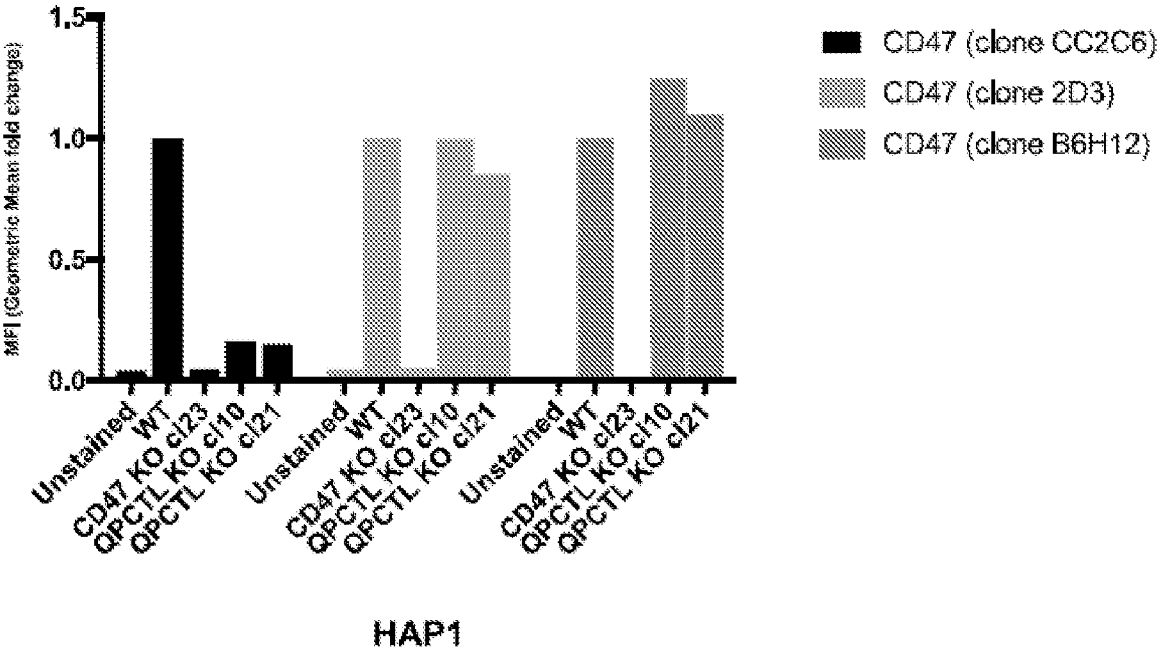
MFI (Geometric Mean fold change)
1.5
1.0
0.5
0.0
Unstained
WT
CD47 KO cl23
QPCTL KO cl10
QPCTL KO cl21
CD47 (clone CC2C6)
CD47 (clone 2D3)
CD47 (clone B6H12)
HAP1

Fig. 2B

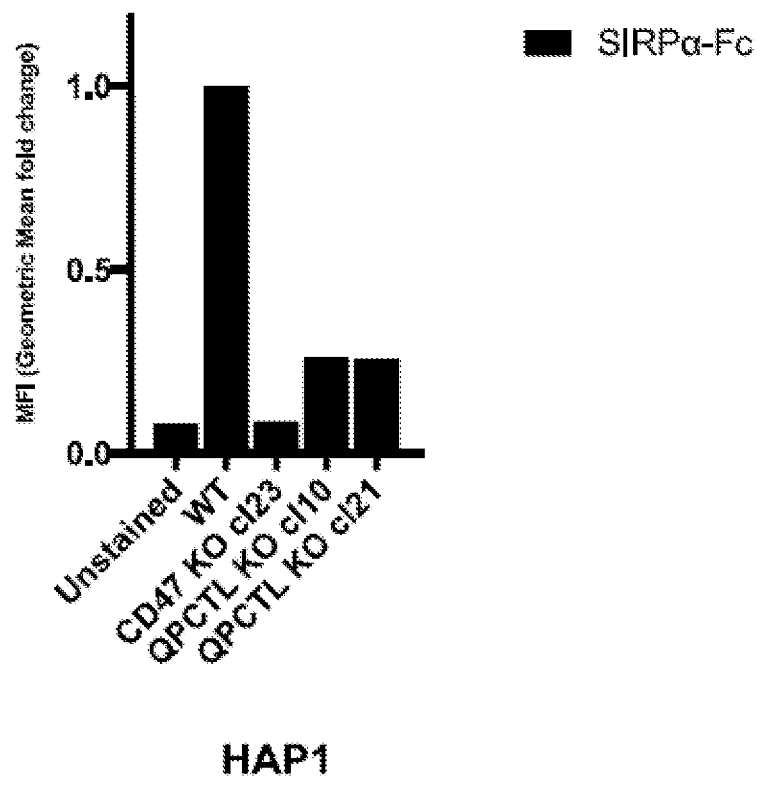
SIRPα-Fc
MFI (Geometric Mean fold change)
1.0
0.5
0.0
Unstained
WT
CD47 KO cl23
QPCTL KO cl10
QPCTL KO cl21
HAP1

Fig. 3

A

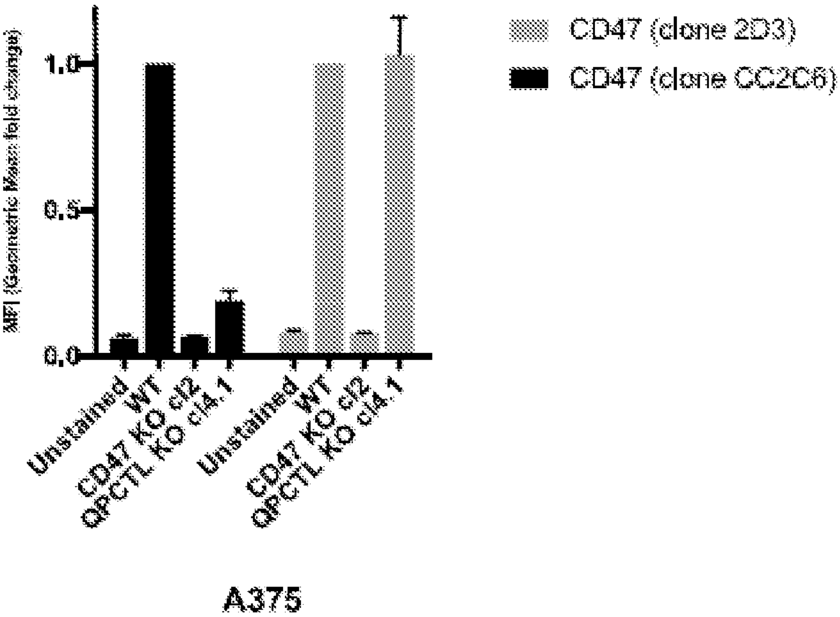
MFI (Geometric Mean fold change)
1.0
0.5
0.0
CD47 (clone 2D3)
CD47 (clone CC2C6)
Unstained
WT
CD47 KO cl2
QPCTL KO cl4.1
Unstained
WT
CD47 KO cl2
QPCTL KO cl4.1
A375

B

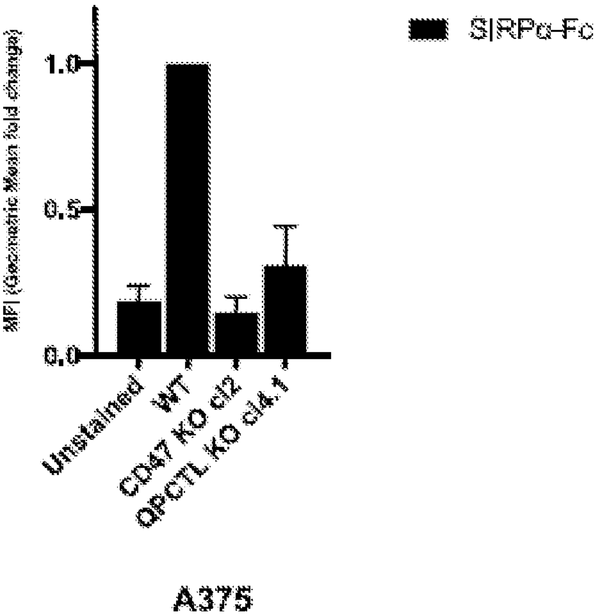
MFI (Geometric Mean fold change)
1.0
0.5
0.0
SIRPα-Fc
Unstained
WT
CD47 KO cl2
QPCTL KO cl4.1
A375

Fig. 4

A

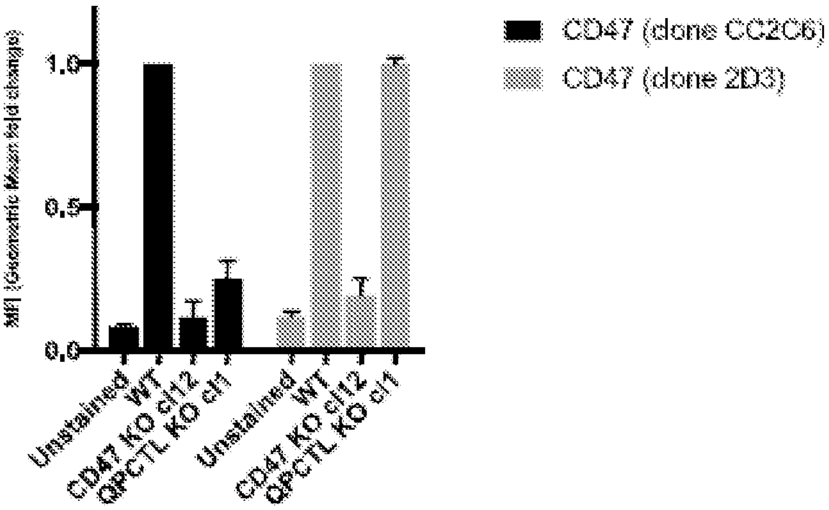


B RKO

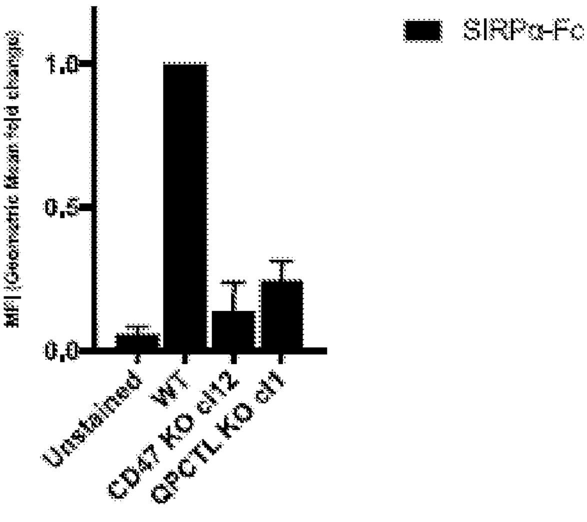


RKO

Fig. 5

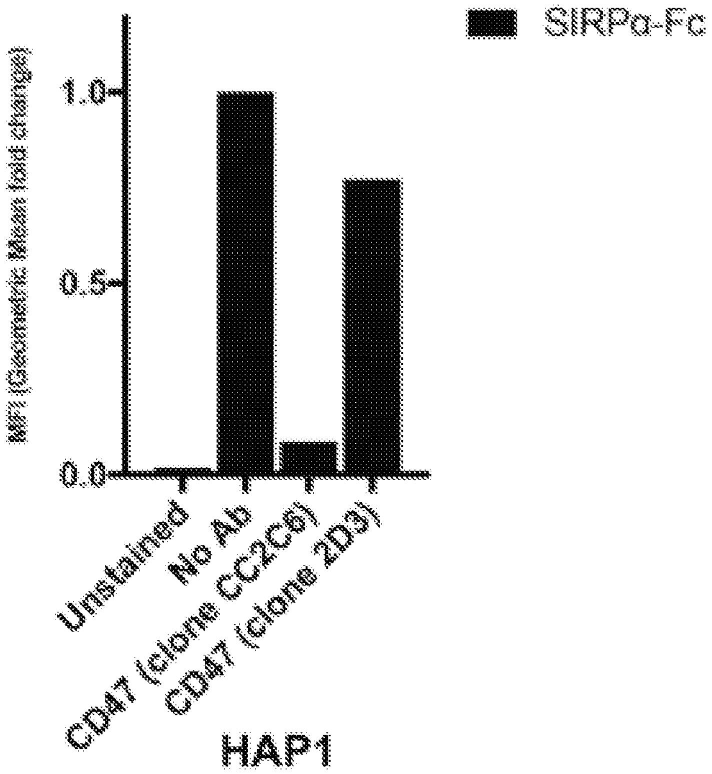
SIRPα-Fc
MFI (Geometric Mean fold change)
1.0
0.5
0.0
Unstained
No Ab
CD47 (clone CC2C6)
CD47 (clone 2D3)
HAP1

Fig. 6

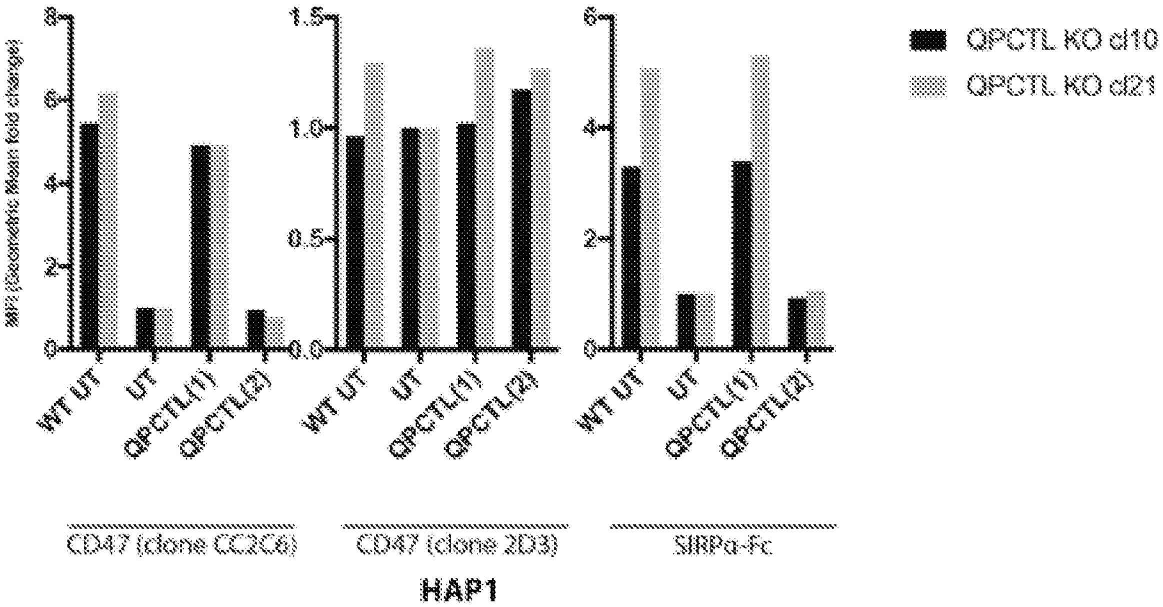
MFI (Geometric Mean fold change)
8
6
4
2
0
1.5
1.0
0.5
0.0
WT UT
UT
QPCTL(1)
QPCTL(2)
QPCTL KO cl10
QPCTL KO cl21
CD47 (clone CC2C6)
CD47 (clone 2D3)
SIRPα-Fc
HAP1

Fig. 7

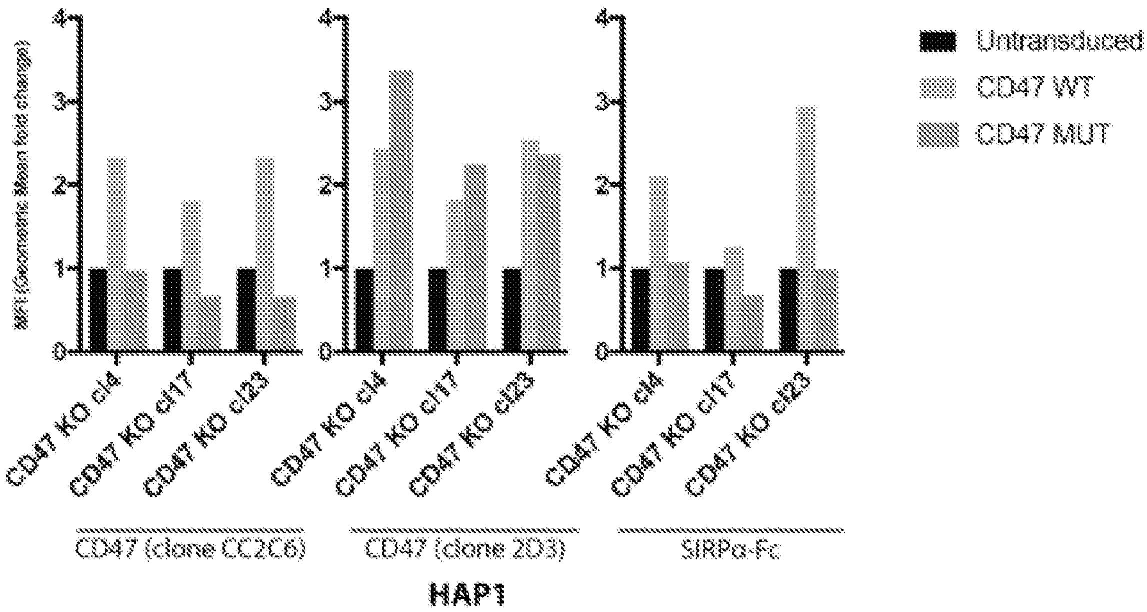
MFI (Geometric Mean fold change)
0
1
2
3
4
CD47 KO cl4
CD47 KO cl17
CD47 KO cl23
CD47 (clone CC2C6)
CD47 (clone 2D3)
SIRPα-Fc
HAP1
Untransduced
CD47 WT
CD47 MUT

Fig. 8

A

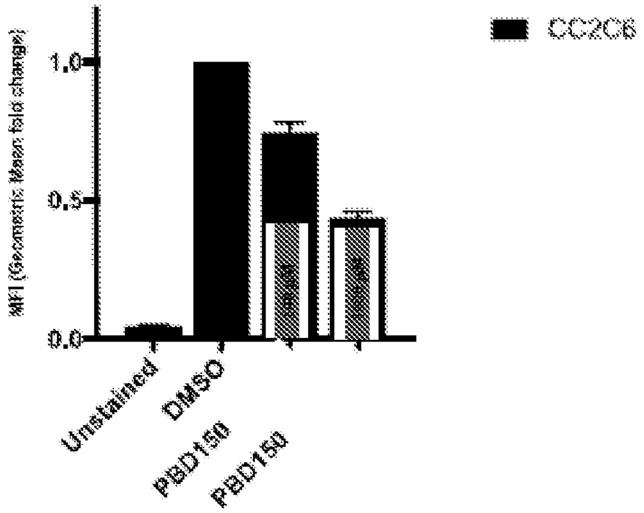


HAP1

B

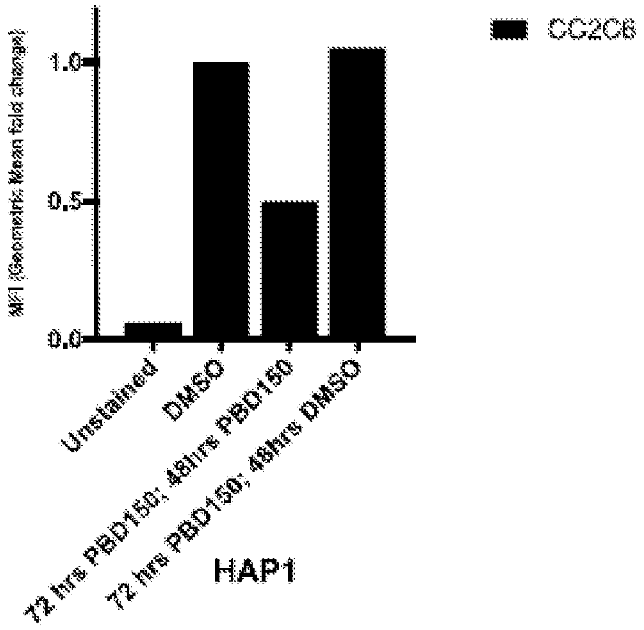


HAP1

**Fig. 9**

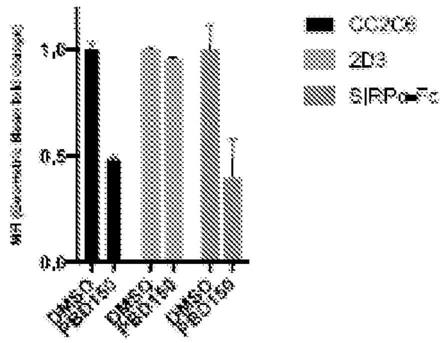


A375

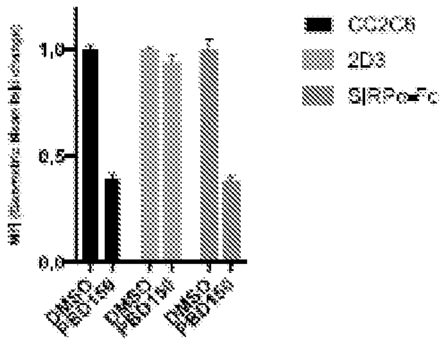


A549

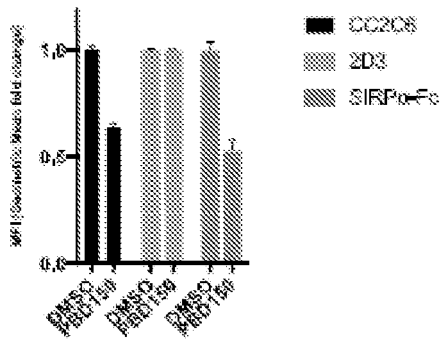


DLD1

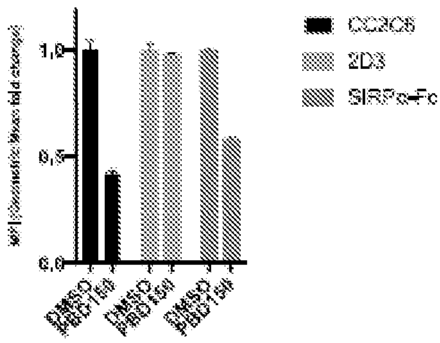


HAP1

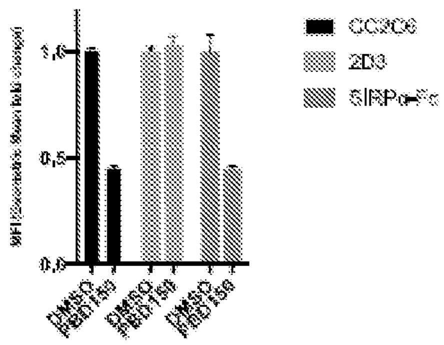


RKO

Fig. 10 A and B

A

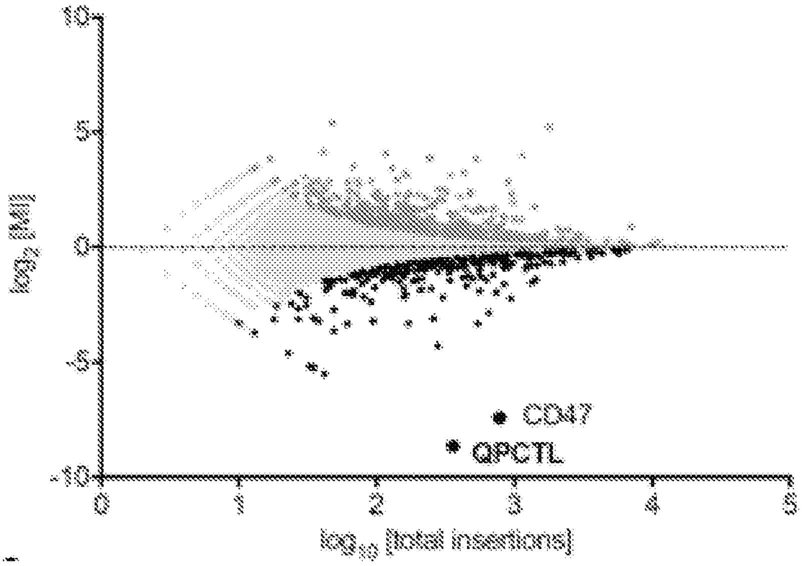


B

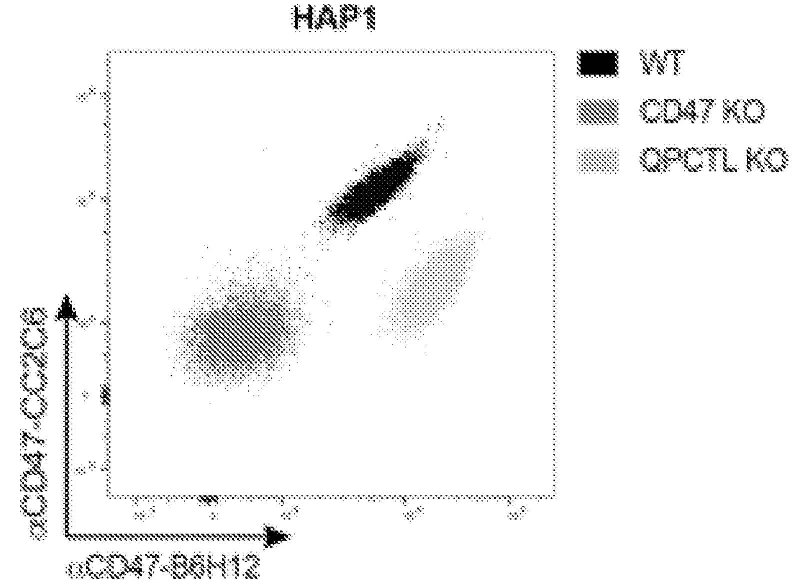

Fig. 10 C and D (Con't)

C

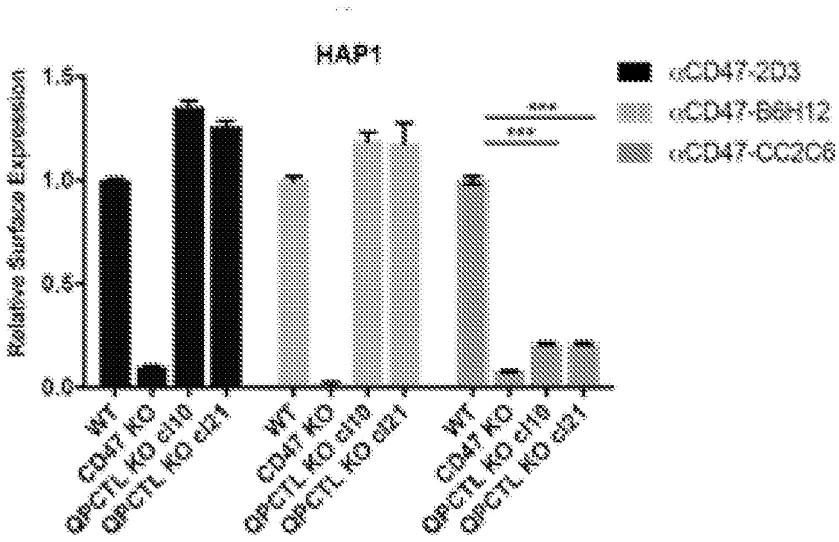


D

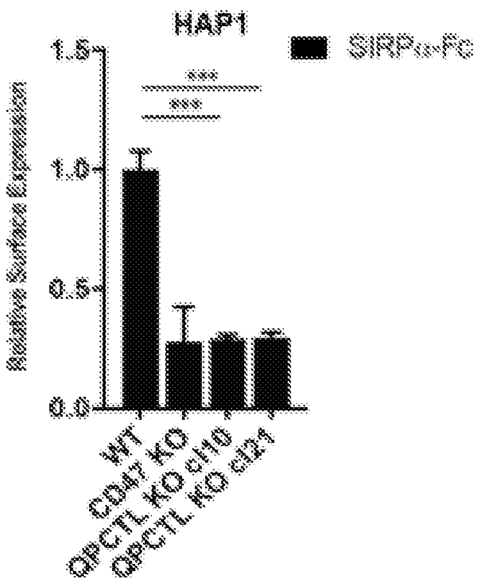

Fig. 10 E (con't)

E

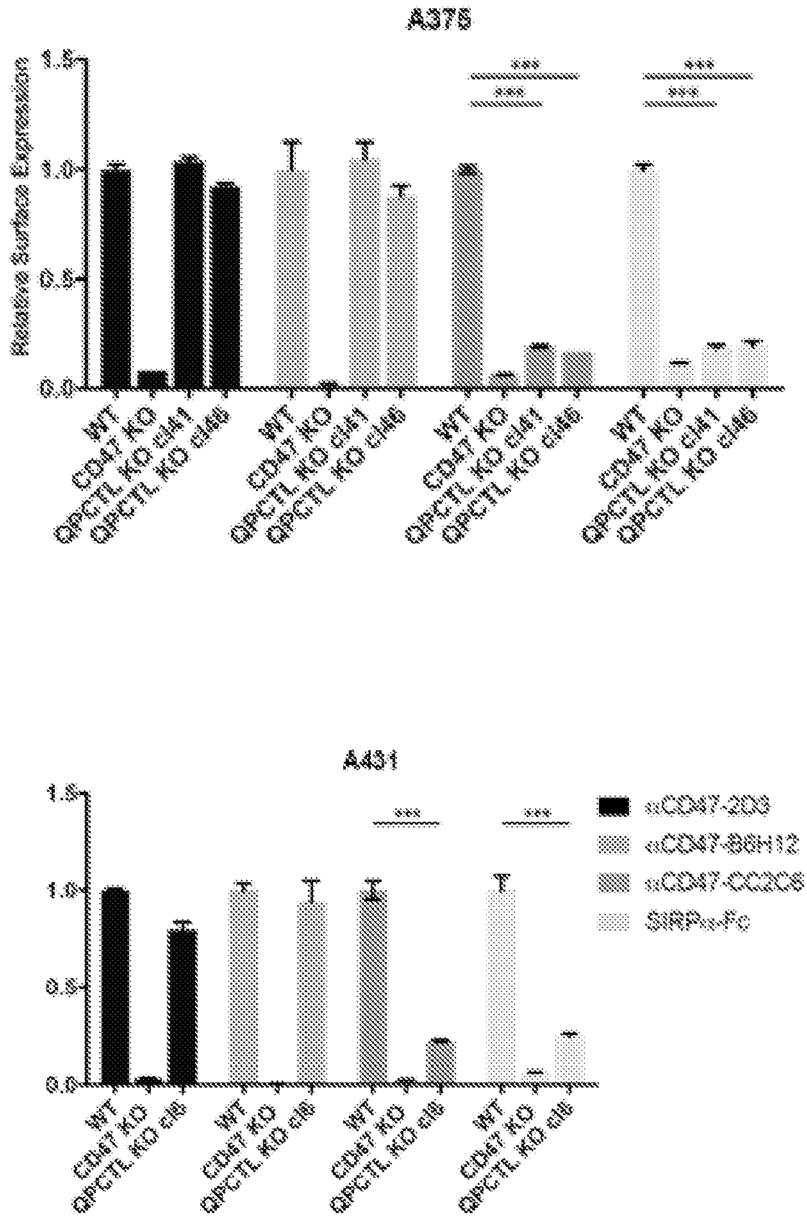

Fig. 11

A

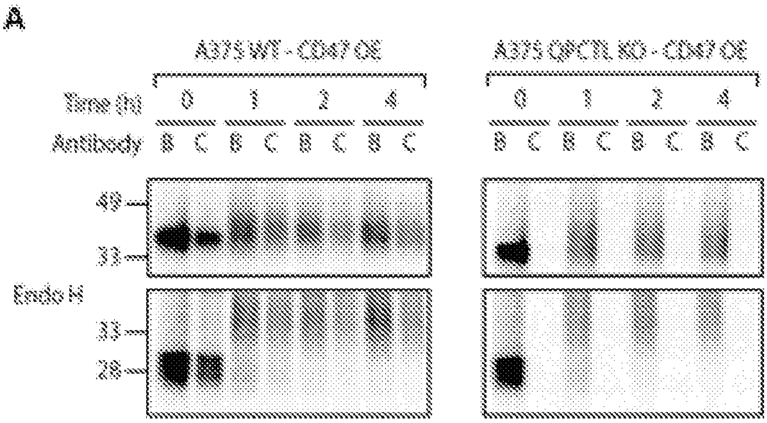
A375 WT - CD47 OE
A375 QPCTL KO - CD47 OE
Time (h)
0
1
2
4
Antibody
B C B C B C B C
49
33
Endo H
33
28

B

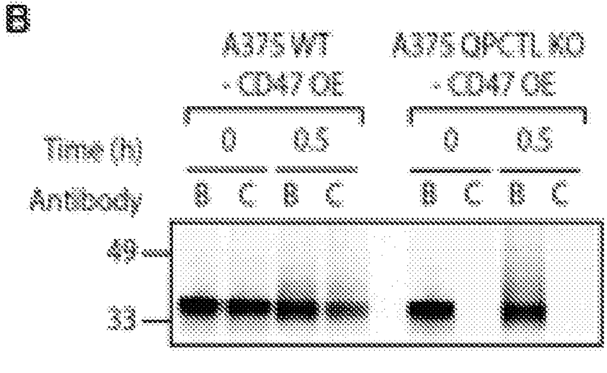
A375 WT - CD47 OE
A375 QPCTL KO - CD47 OE
Time (h)
0
0.5
Antibody
B C B C
49
33

Fig. 12 A

A

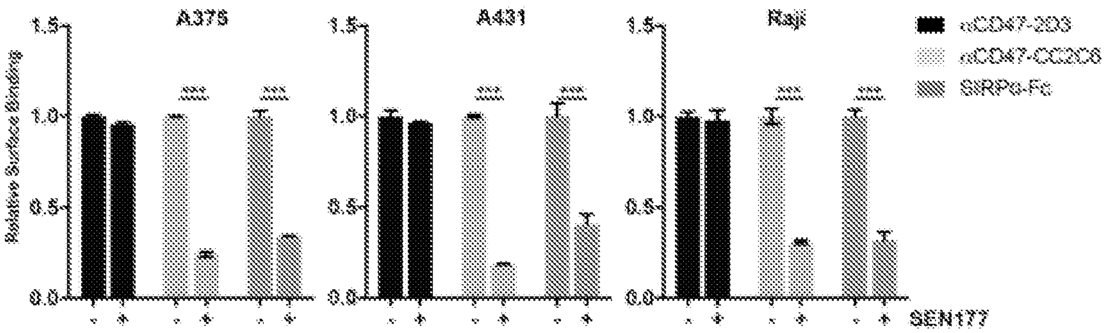
A375
A431
Raji
αCD47-2D3
αCD47-CC2C6
SIRPα-Fc
Relative Surface Binding
1.5
1.0
0.5
0.0
***
-
+
SEN177

Fig. 12 B, C and D (Con't)

B

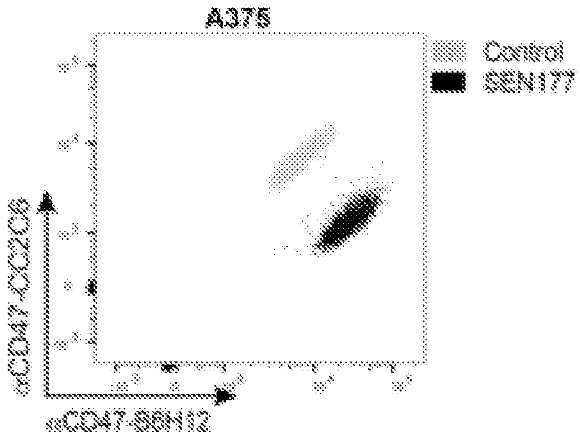


C

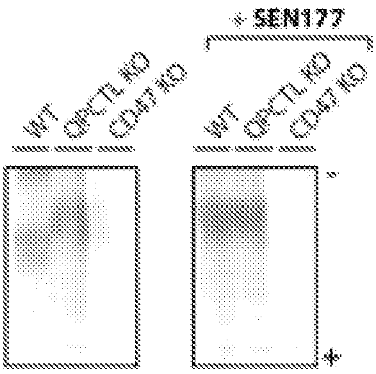


D

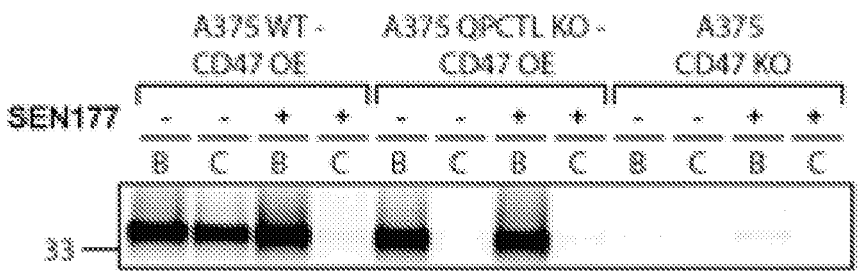

Fig. 13 A and B

A

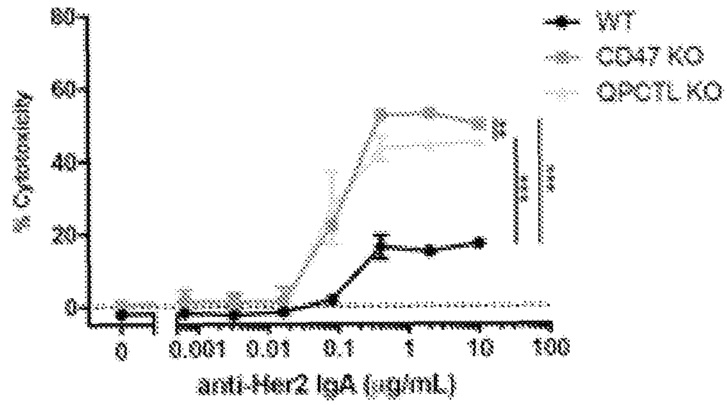


B

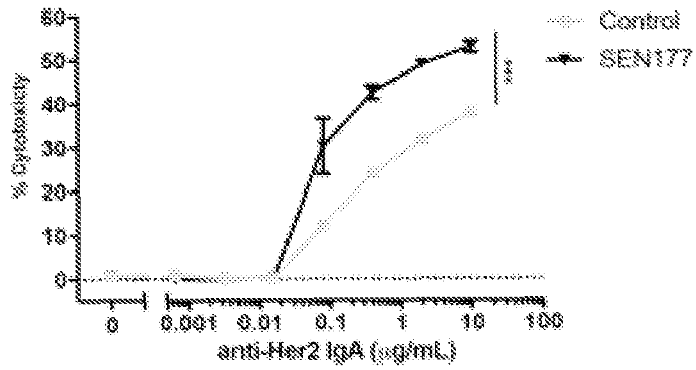

Fig. 13 C and D (Con't)

C

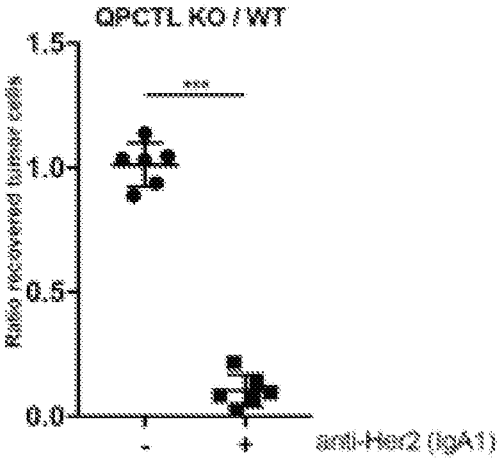


D

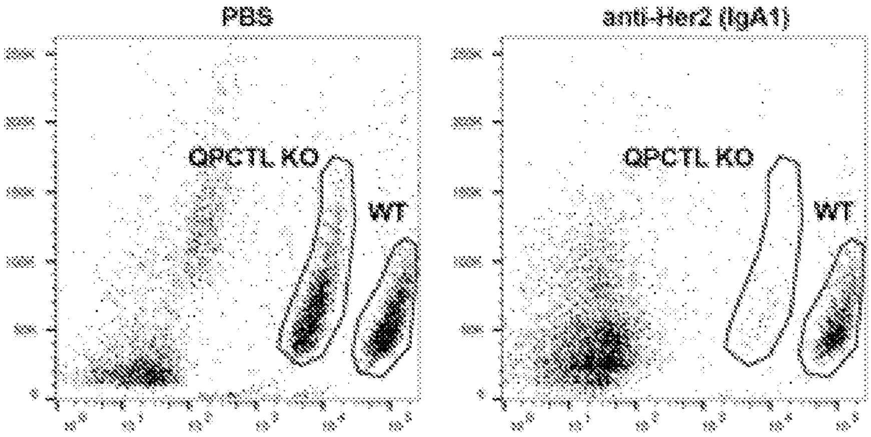

Fig. 13 E (Con't)

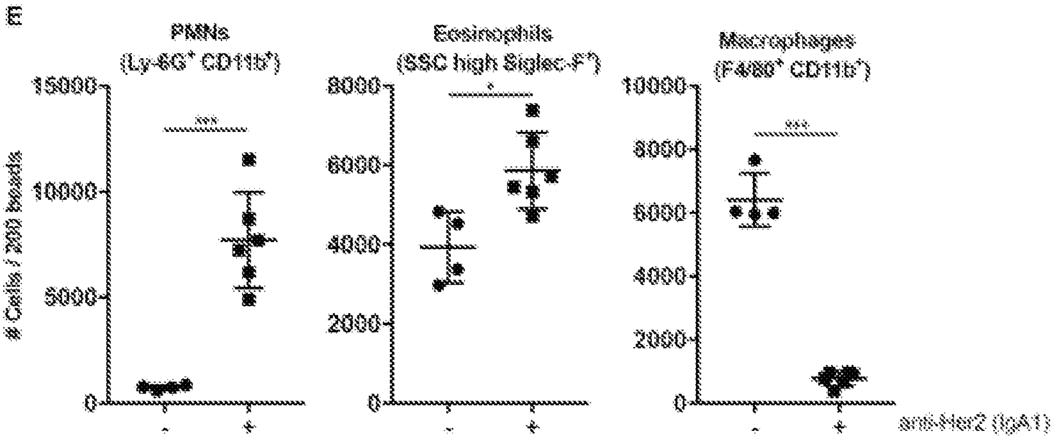
E
PMNs
(Ly-6G+ CD11b+)
Eosinophils
(SSC high Siglec-F+)
Macrophages
(F4/80+ CD11b+)
# Cells / 200 beads
15000
10000
5000
0
8000
6000
4000
2000
10000
***
*
-
+
anti-Her2 (IgA1)

Fig. 14 A

A

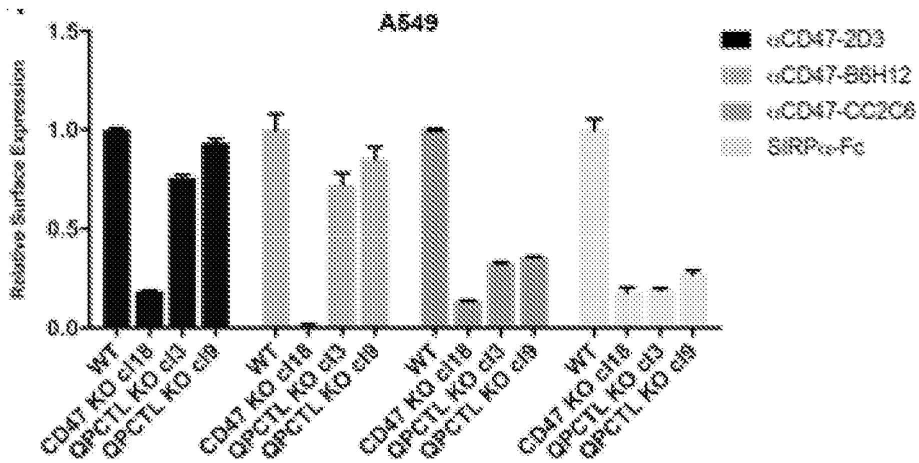
A549
Relative Surface Expression
1.5
1.0
0.5
0.0
αCD47-2D3
αCD47-B6H12
αCD47-CC2C6
SIRPα-Fc
WT
CD47 KO cl18
QPCTL KO cl3
QPCTL KO cl9

Fig 14 B (Con't)

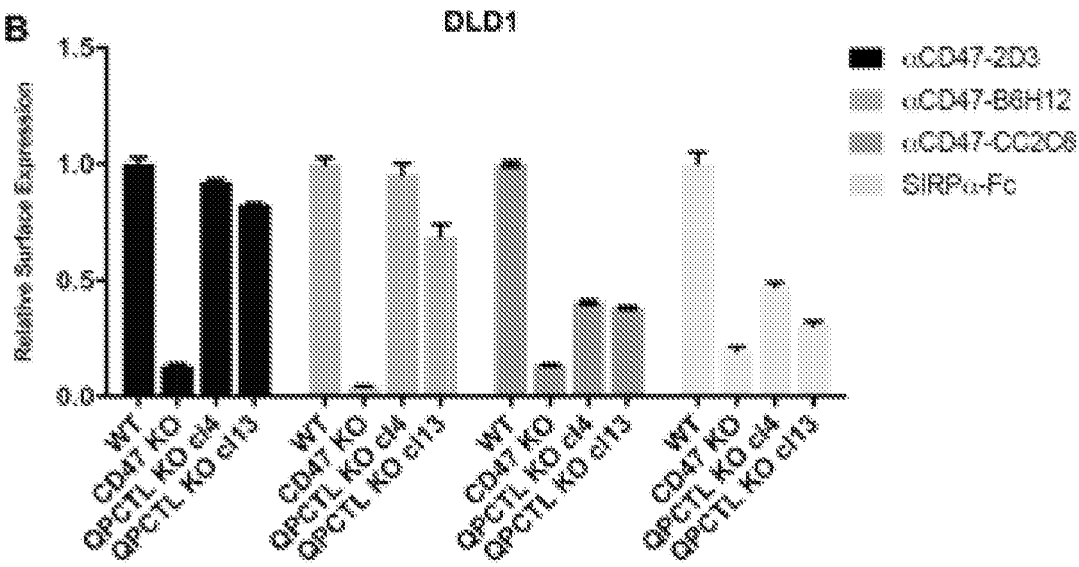
B
DLD1
Relative Surface Expression
1.5
1.0
0.5
0.0
αCD47-2D3
αCD47-B6H12
αCD47-CC2C6
SIRPα-Fc
WT
CD47 KO
QPCTL KO cl4
QPCTL KO cl13

Fig. 14 C (Con't)

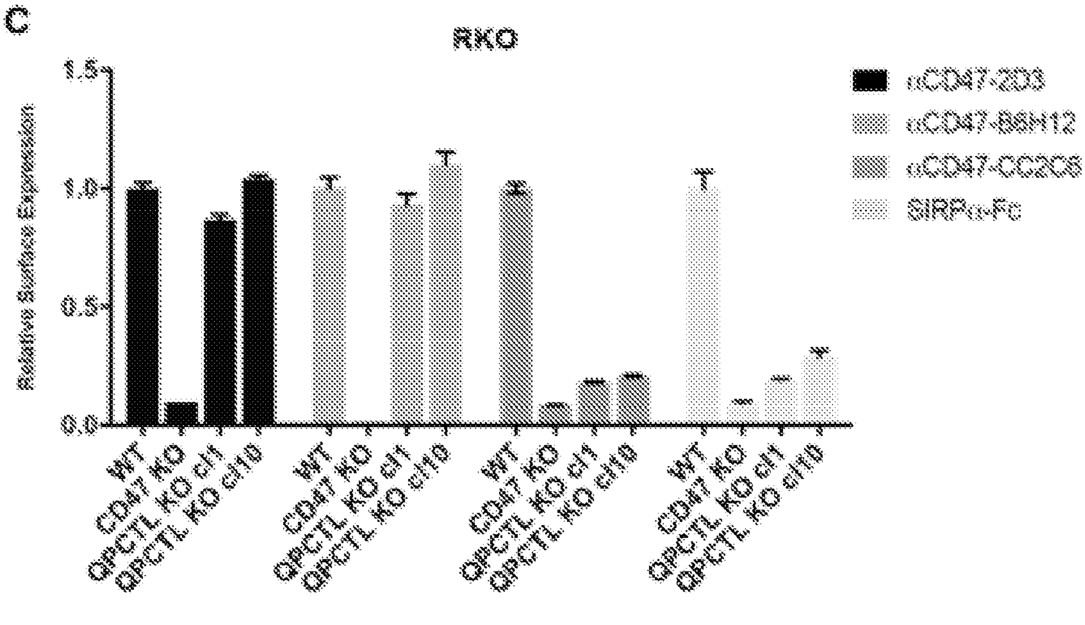
C
RKO
Relative Surface Expression
1.5
1.0
0.5
0.0
αCD47-2D3
αCD47-B6H12
αCD47-CC2C6
SIRPα-Fc
WT
CD47 KO
QPCTL KO cl1
QPCTL KO cl10

Fig. 15 A and B

A

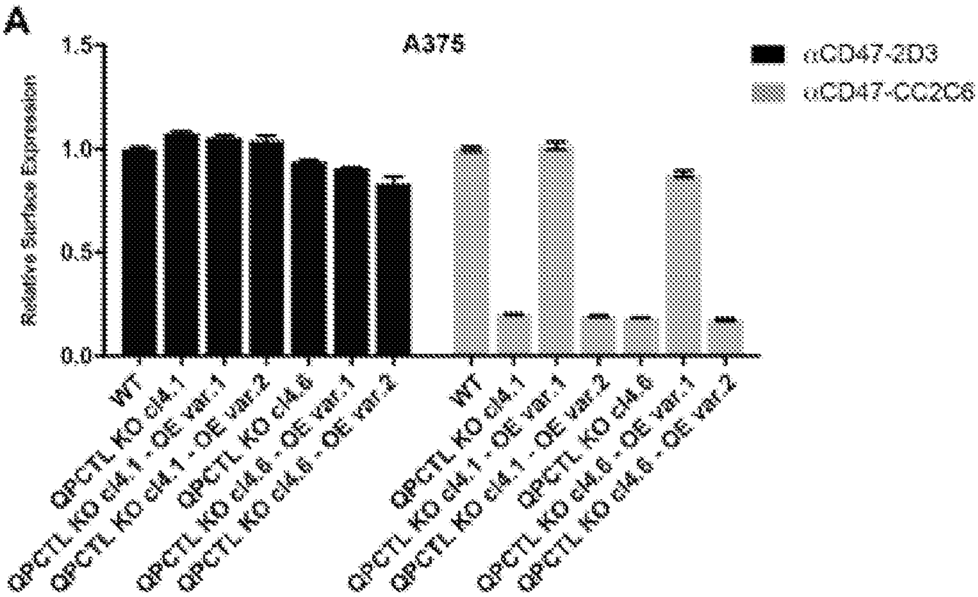


B

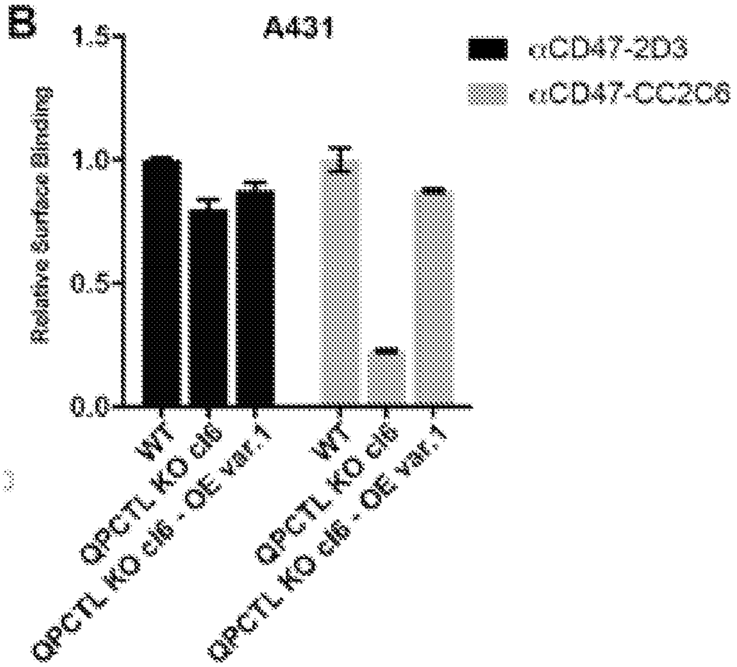

Fig. 15 C and D (Con't)

C

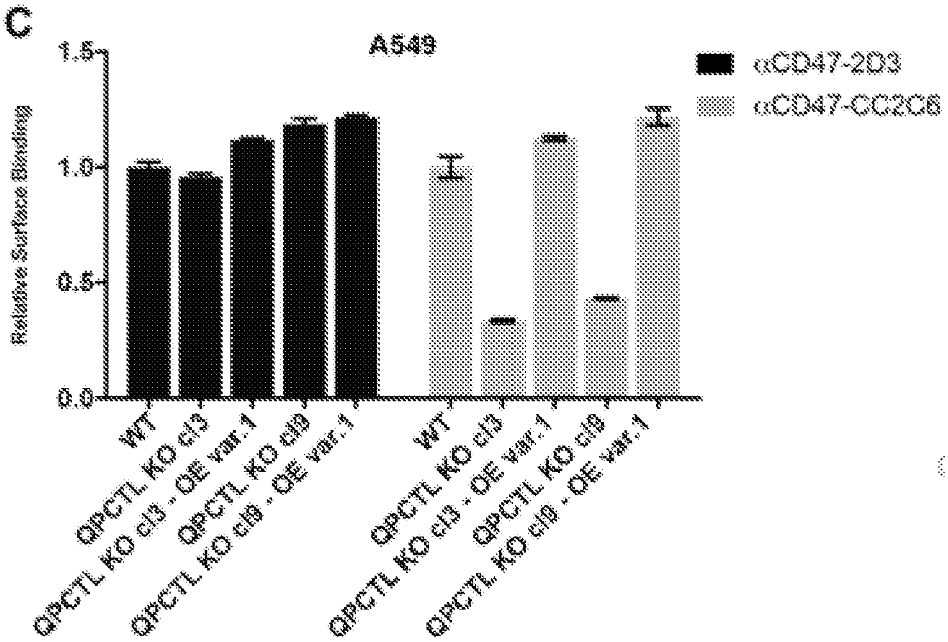


D

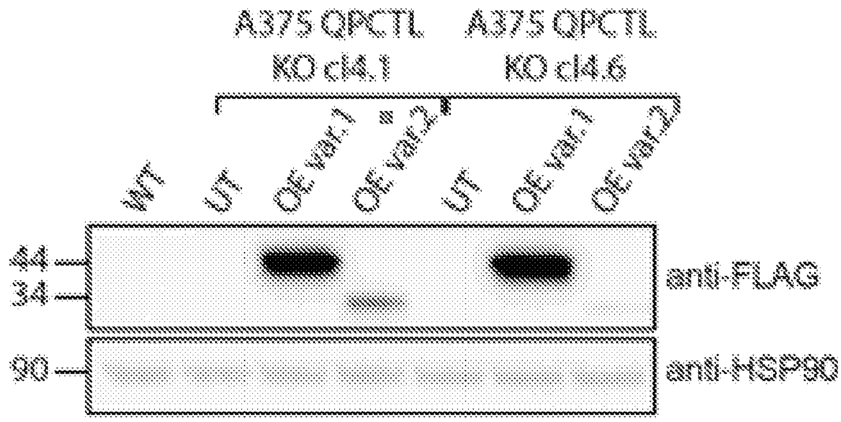

Fig. 15 E and F (Con't)

E

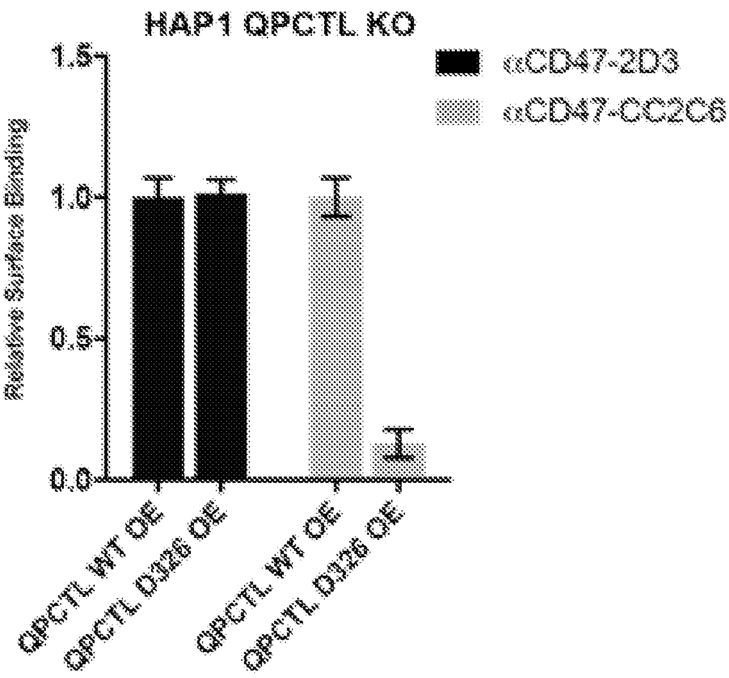


F

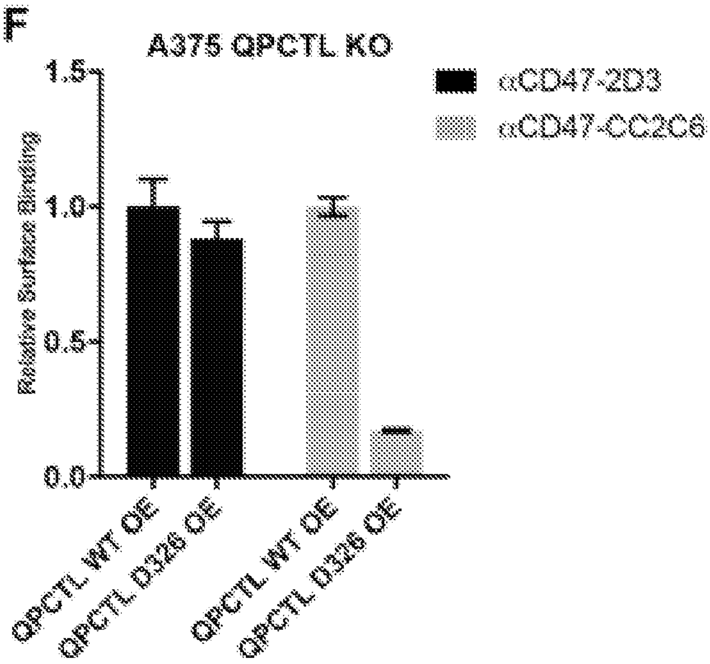

Fig. 16 A and B

A

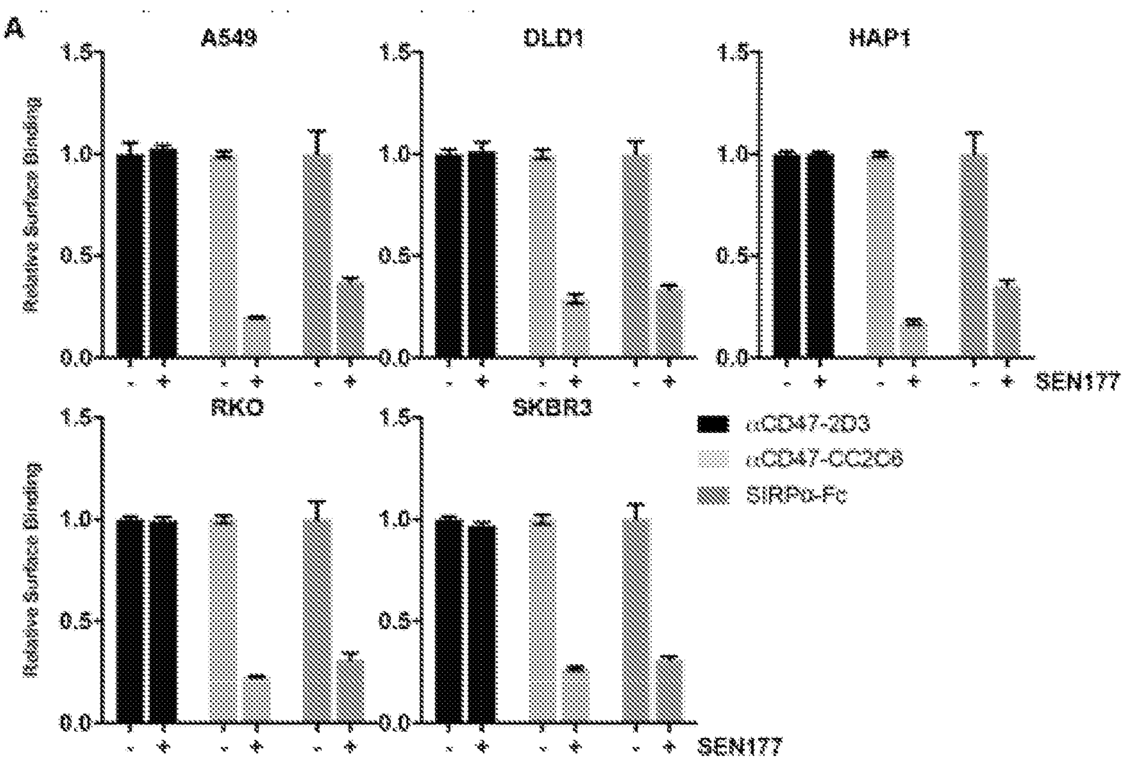


B

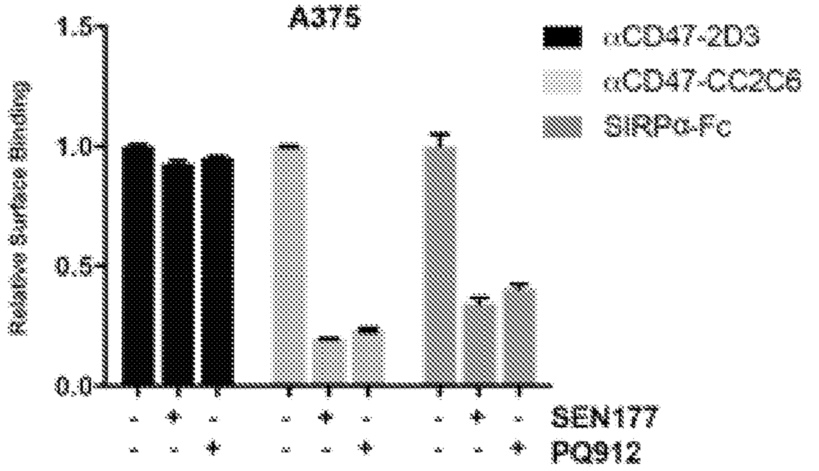

Fig 16 C and D (Con't)

C

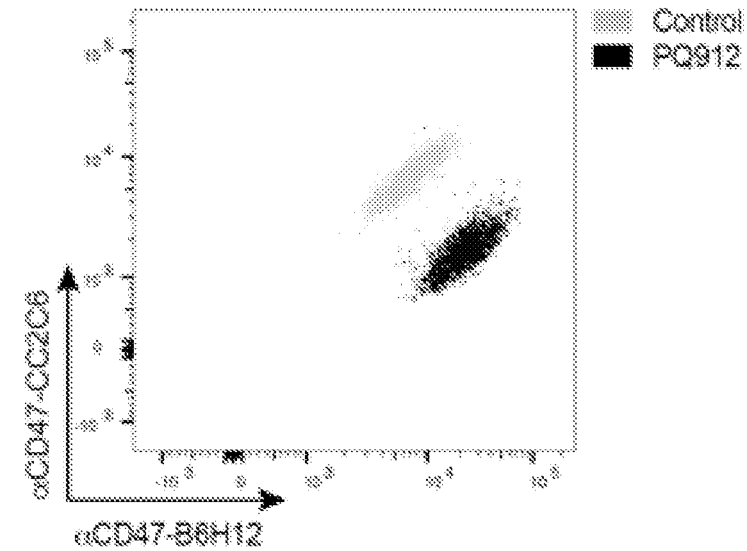


D

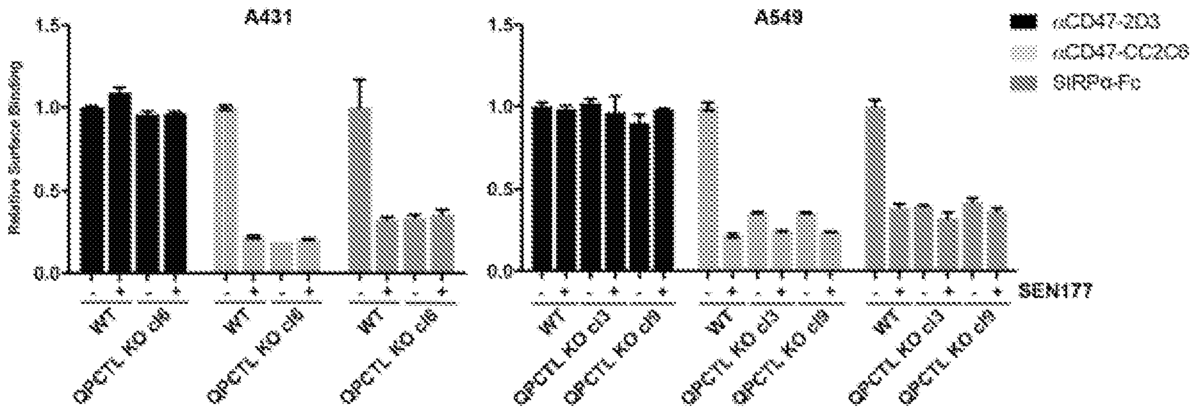

Fig. 17 A and B

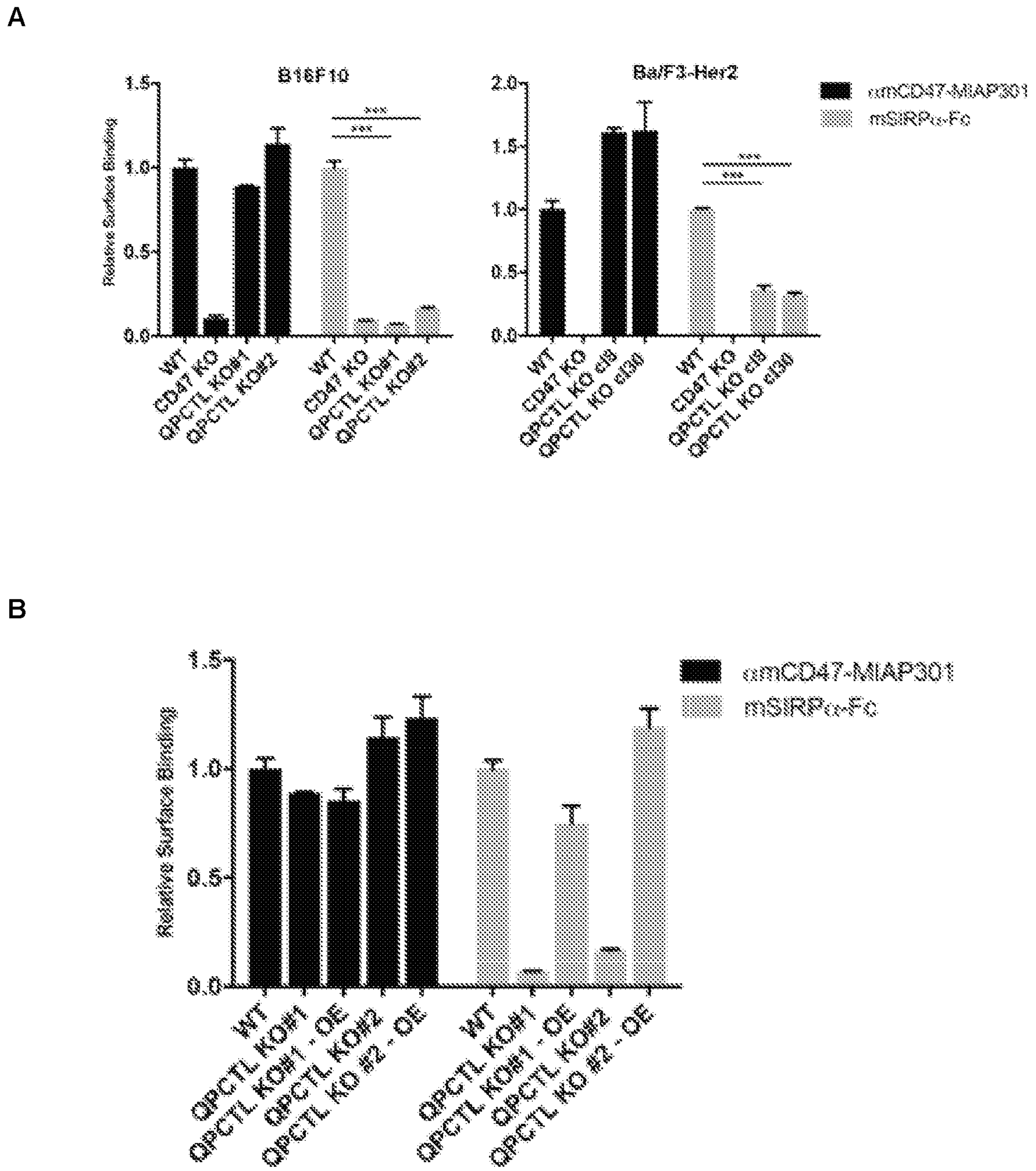
A
B16F10
Ba/F3-Her2
Relative Surface Binding
αmCD47-MIAP301
mSIRPα-Fc
WT
CD47 KO
QPCTL KO#1
QPCTL KO#2
QPCTL KO cl8
QPCTL KO cl36
B
QPCTL KO#1 - OE
QPCTL KO #2 - OE

Fig. 17 C and D (Con't)

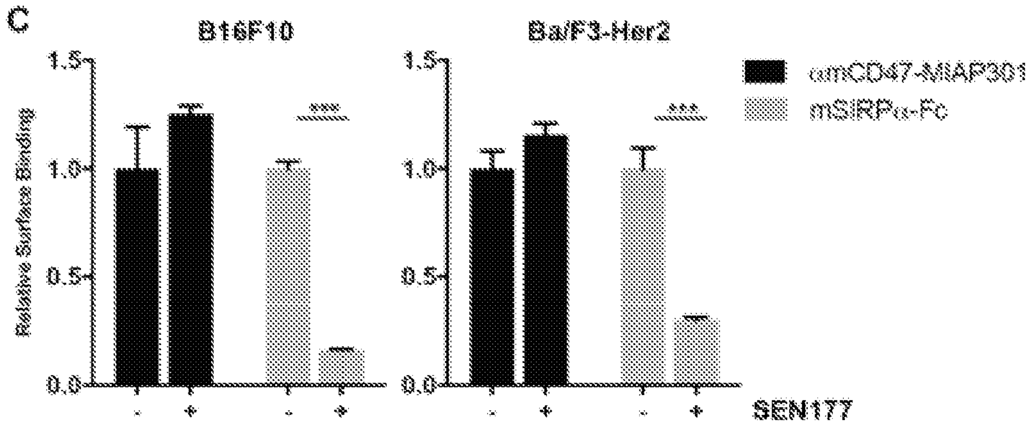


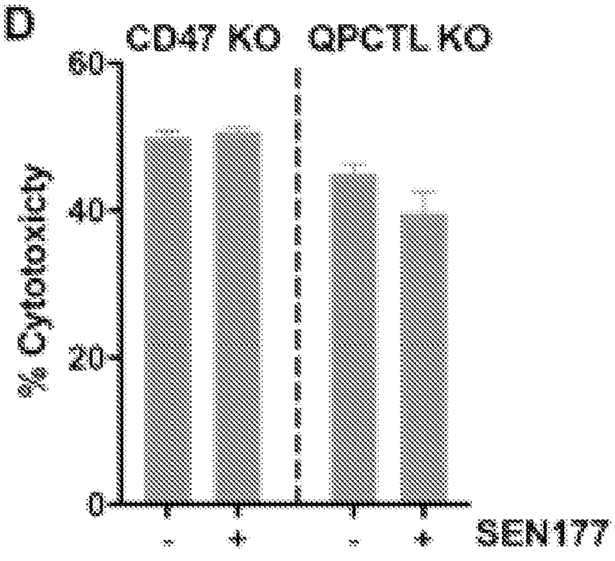

Fig. 17 E and F (Con't)

E

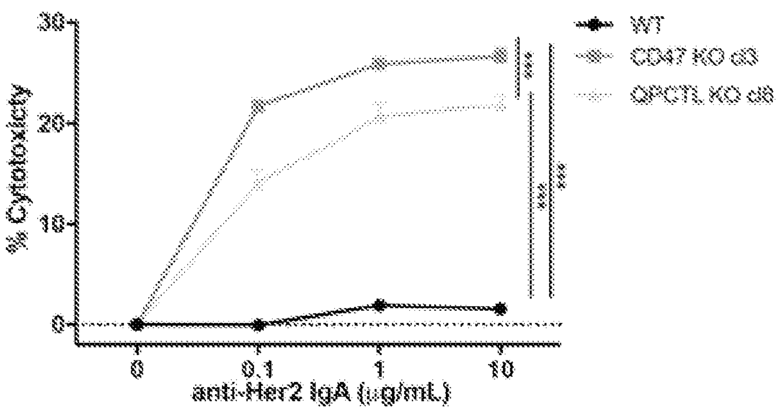


F

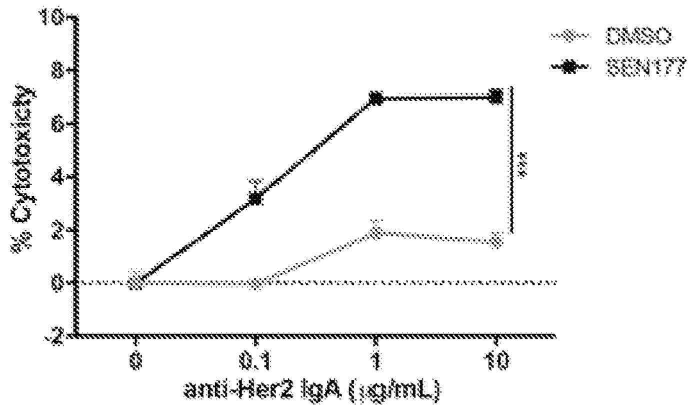

Fig. 18 A and B

A

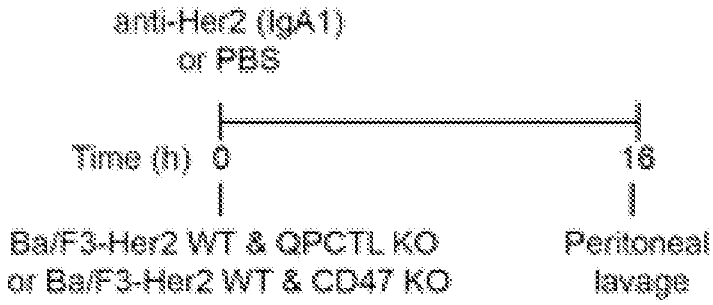


B

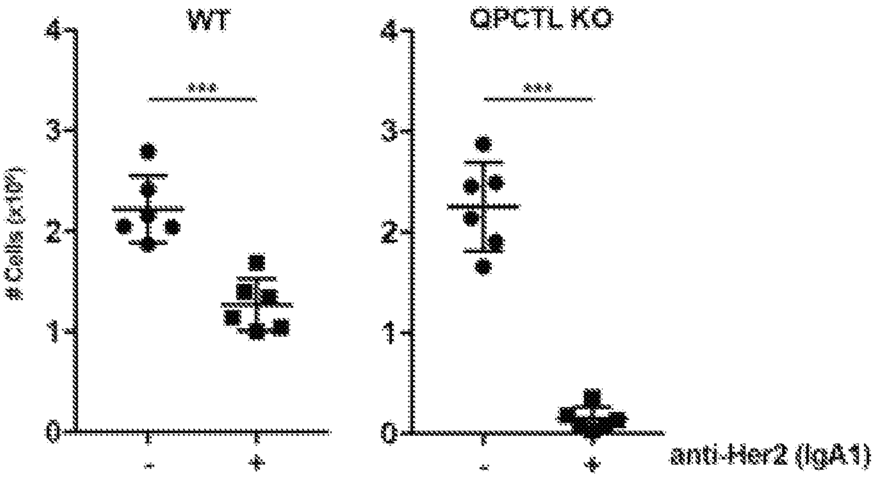

Fig. 18 C and D (Con't)

C

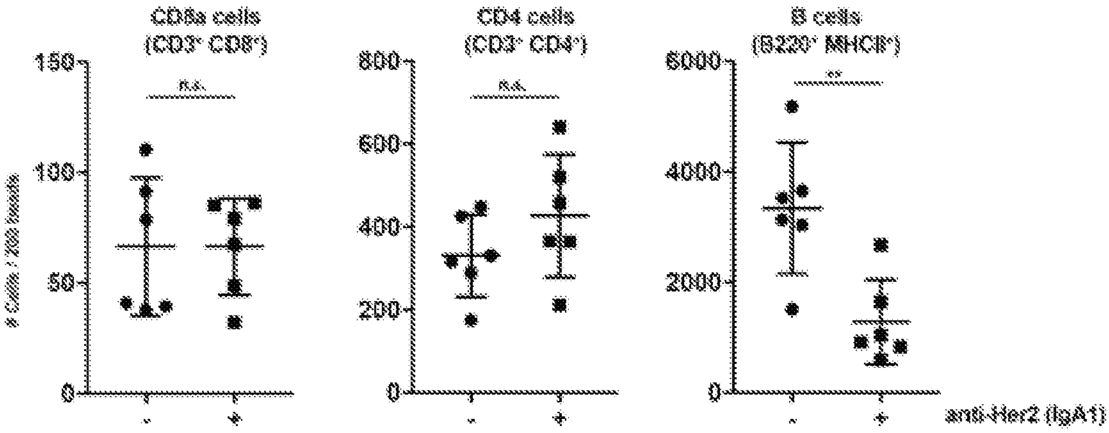


D

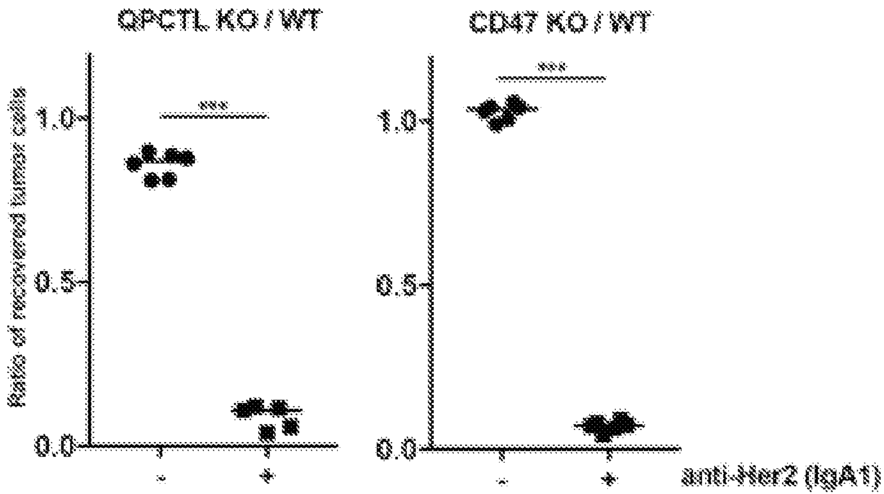

## Fig. 18 E and F (con't)

E

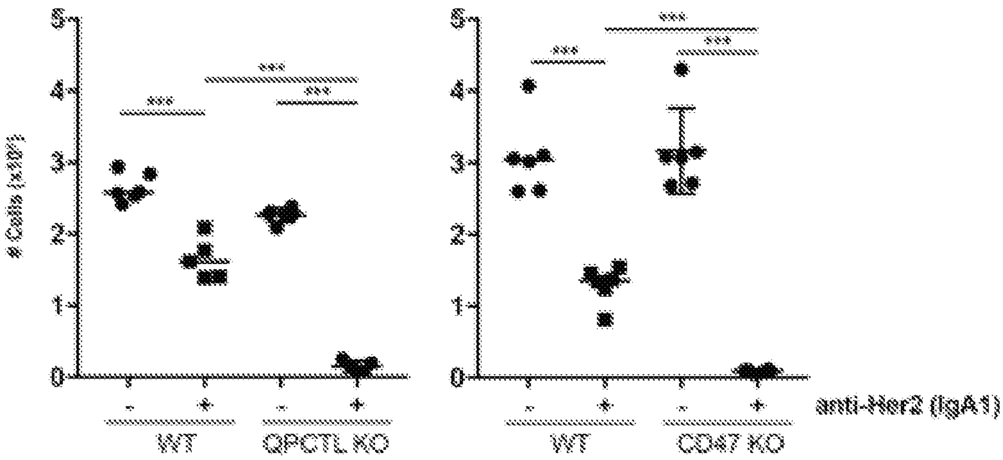


F

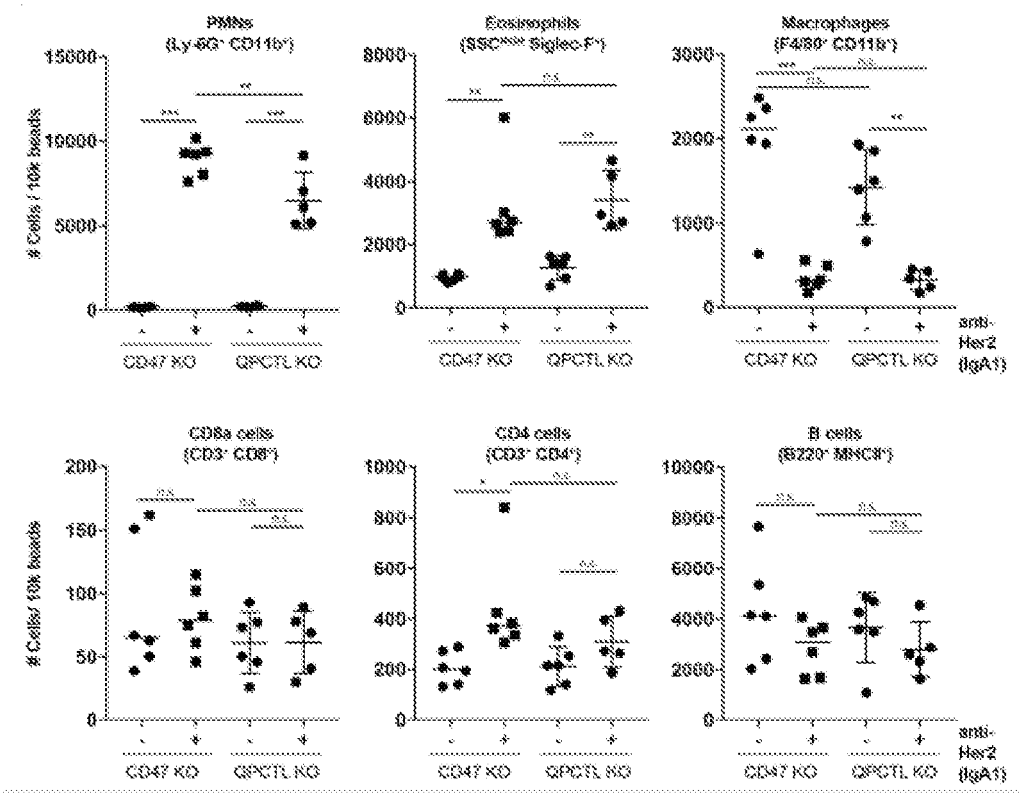

Fig. 19

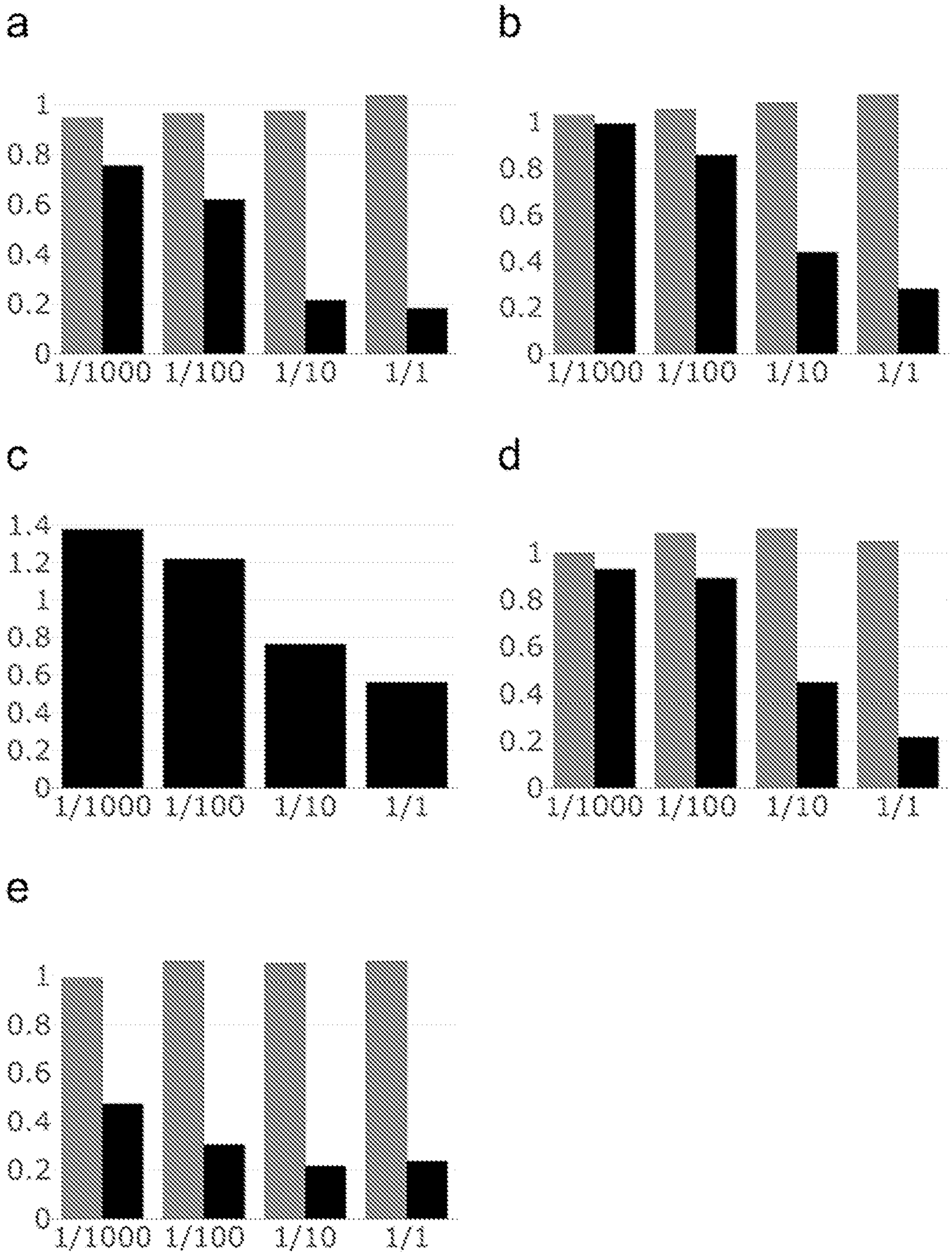
a
1
0.8
0.6
0.4
0.2
0
1/1000 1/100 1/10 1/1
b
1
0.8
0.6
0.4
0.2
0
1/1000 1/100 1/10 1/1
c
1.4
1.2
1
0.8
0.6
0.4
0.2
0
1/1000 1/100 1/10 1/1
d
1
0.8
0.6
0.4
0.2
0
1/1000 1/100 1/10 1/1
e
1
0.8
0.6
0.4
0.2
0
1/1000 1/100 1/10 1/1

Fig. 20 a-h

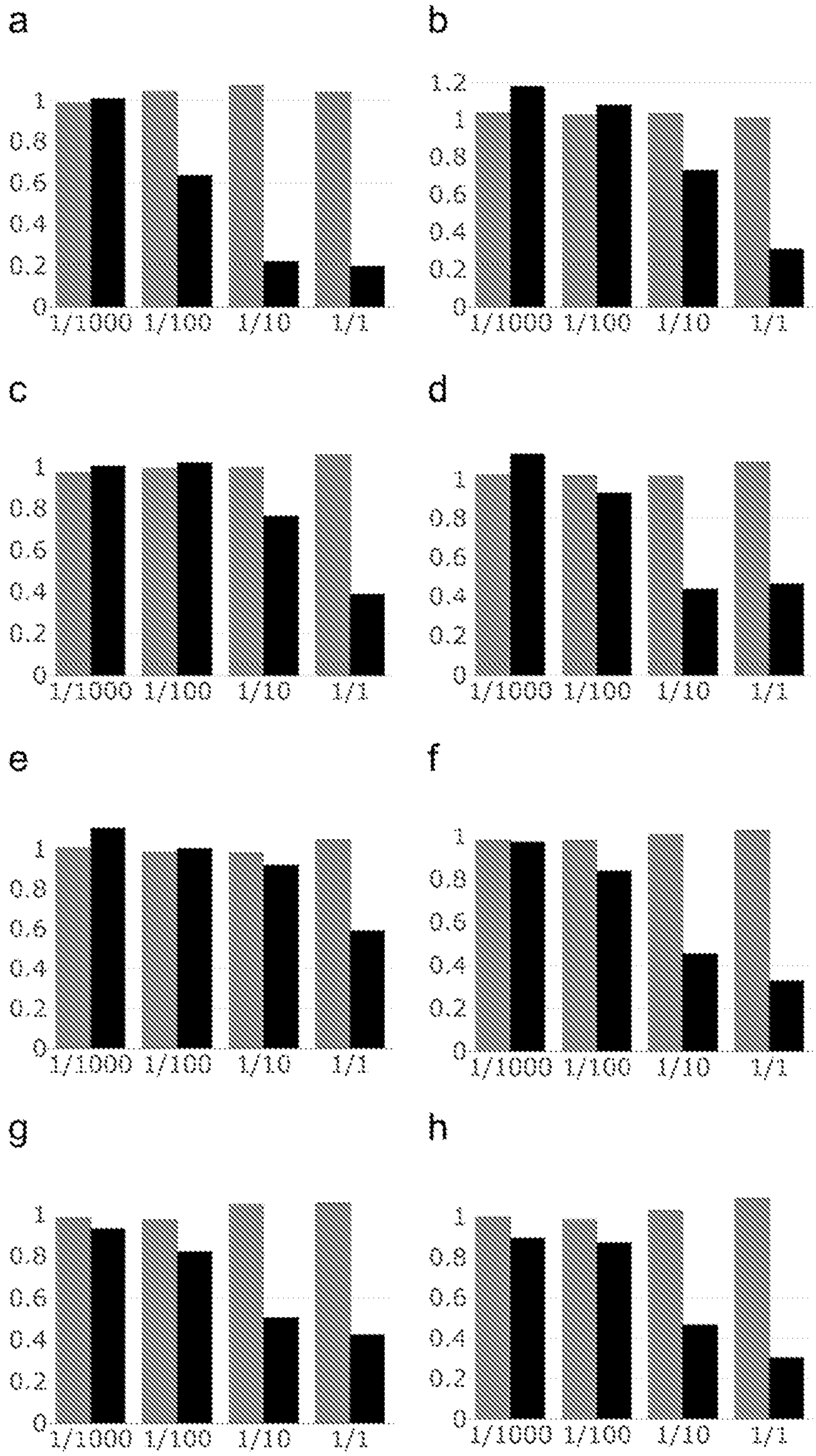

Fig. 20 i-n (Con't)

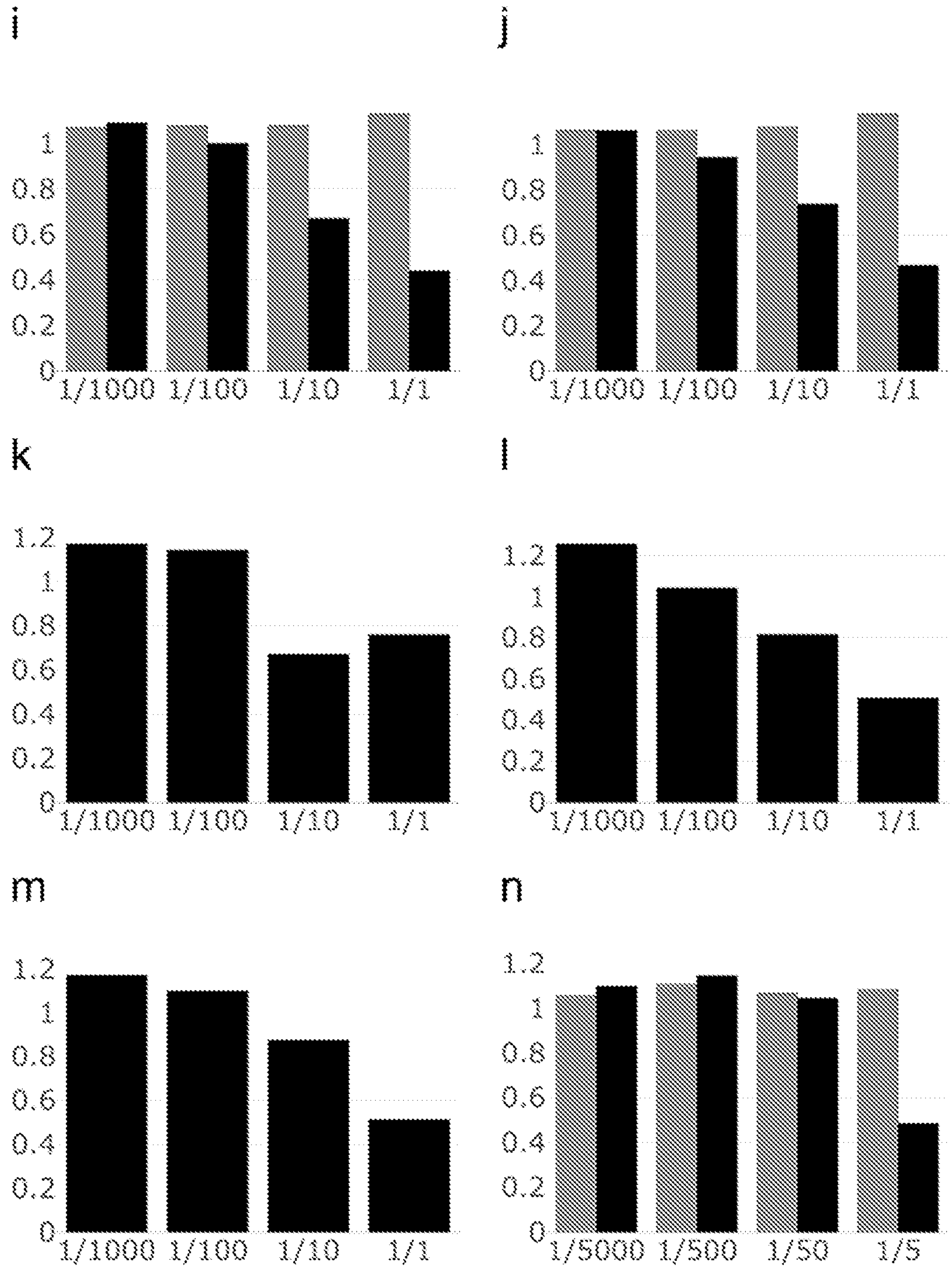

Fig. 21

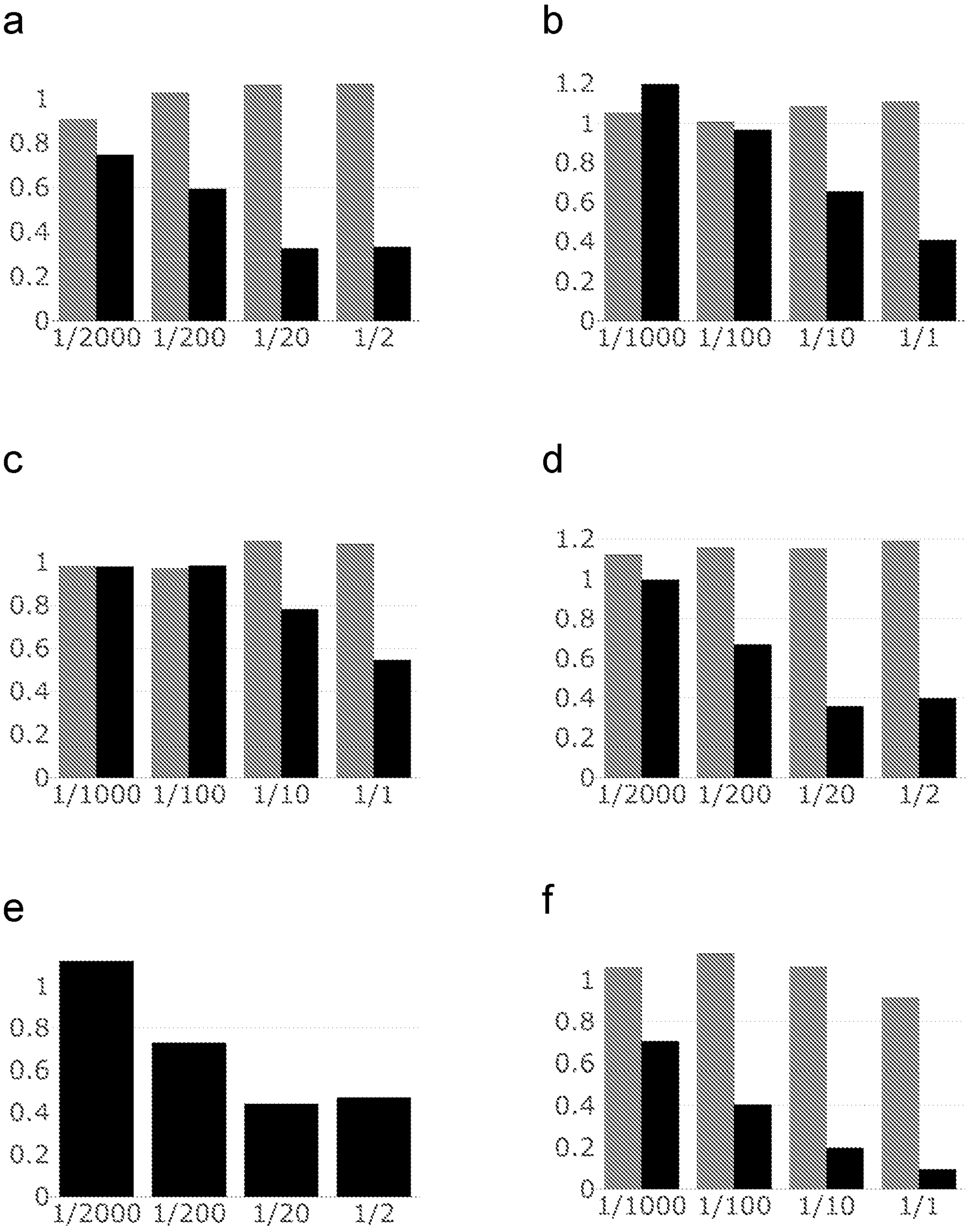
a
1
0.8
0.6
0.4
0.2
0
1/2000 1/200 1/20 1/2
b
1.2
1
0.8
0.6
0.4
0.2
0
1/1000 1/100 1/10 1/1
c
1
0.8
0.6
0.4
0.2
0
1/1000 1/100 1/10 1/1
d
1.2
1
0.8
0.6
0.4
0.2
0
1/2000 1/200 1/20 1/2
e
1
0.8
0.6
0.4
0.2
0
1/2000 1/200 1/20 1/2
f
1
0.8
0.6
0.4
0.2
0
1/1000 1/100 1/10 1/1

Fig. 22 a-h

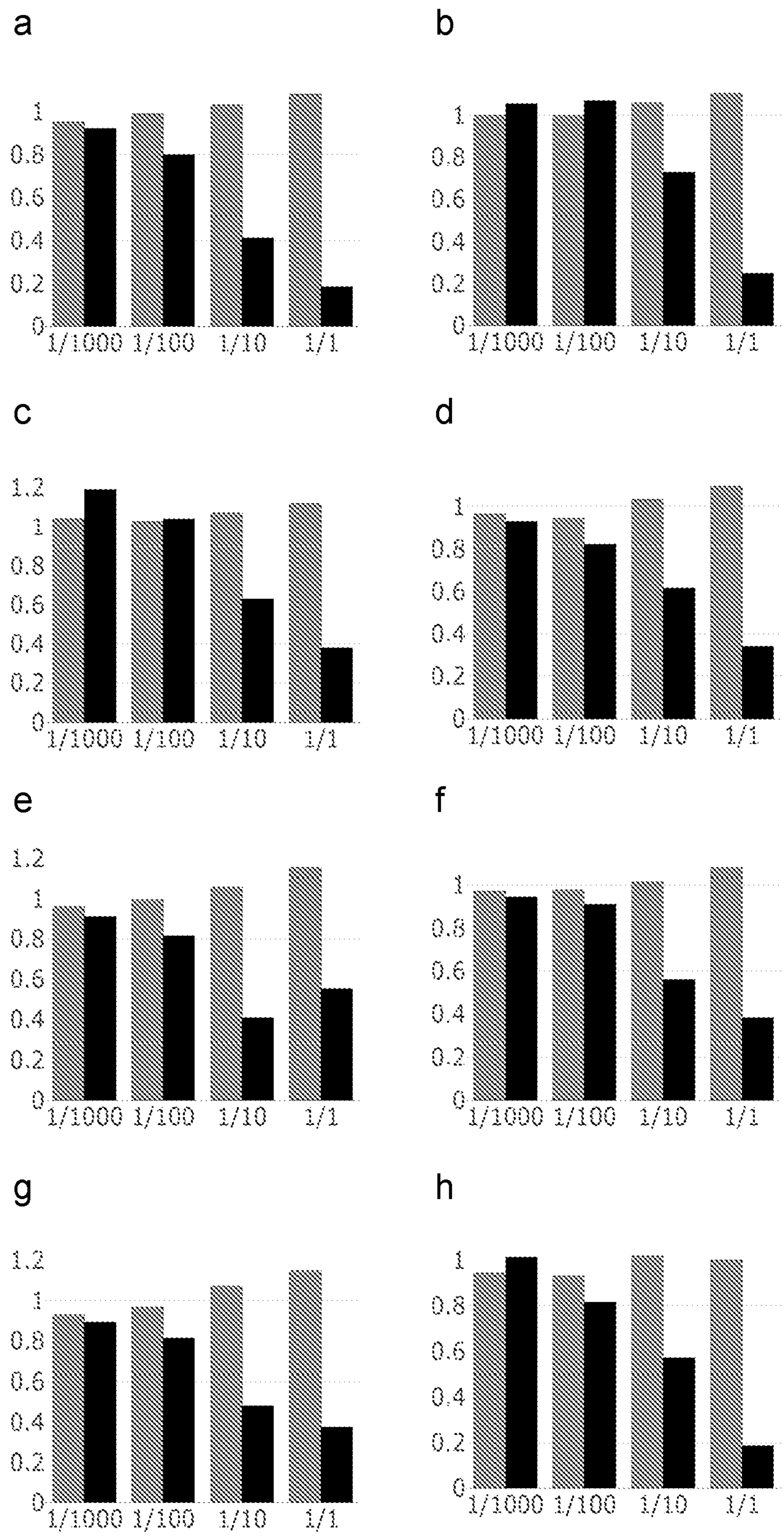

Fig. 22 i-p (con't)

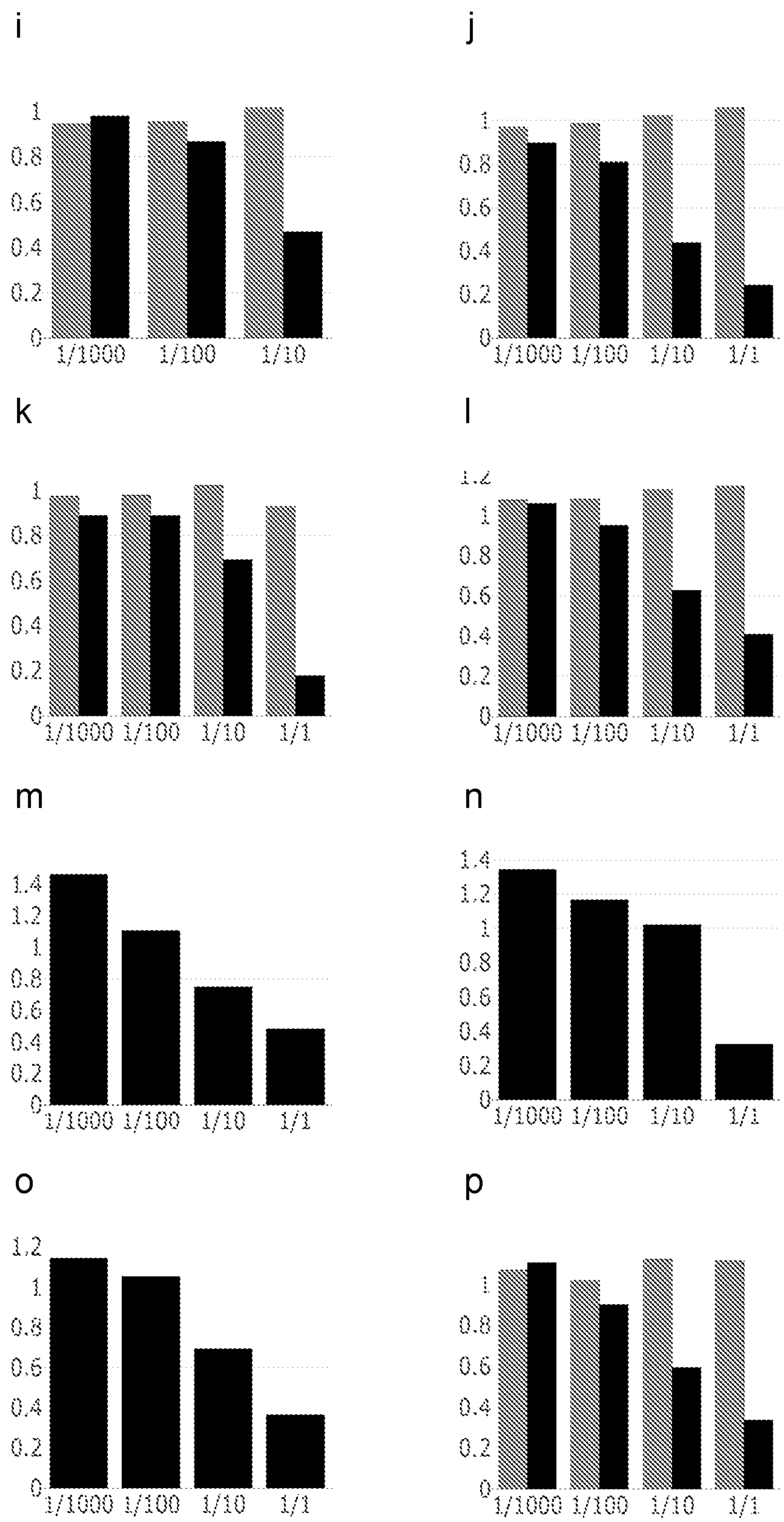

Fig. 23 a-h

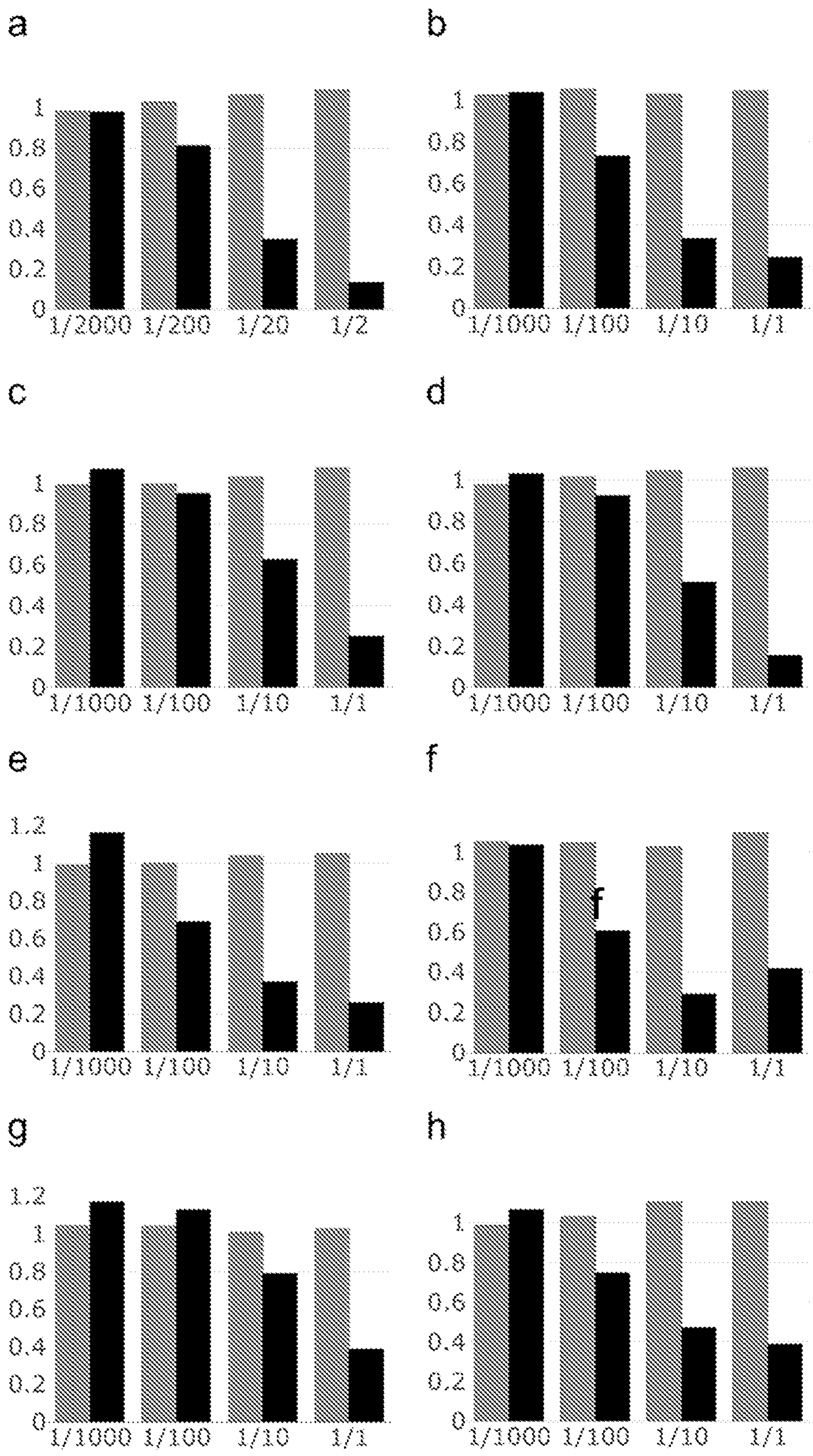
a
1
0.8
0.6
0.4
0.2
0
1/2000 1/200 1/20 1/2
b
1
0.8
0.6
0.4
0.2
0
1/1000 1/100 1/10 1/1
c
1
0.8
0.6
0.4
0.2
0
1/1000 1/100 1/10 1/1
d
1
0.8
0.6
0.4
0.2
0
1/1000 1/100 1/10 1/1
e
1.2
1
0.8
0.6
0.4
0.2
0
1/1000 1/100 1/10 1/1
f
1
0.8
f
0.6
0.4
0.2
0
1/1000 1/100 1/10 1/1
g
1.2
1
0.8
0.6
0.4
0.2
0
1/1000 1/100 1/10 1/1
h
1
0.8
0.6
0.4
0.2
0
1/1000 1/100 1/10 1/1

Fig. 23 i-p (con't)

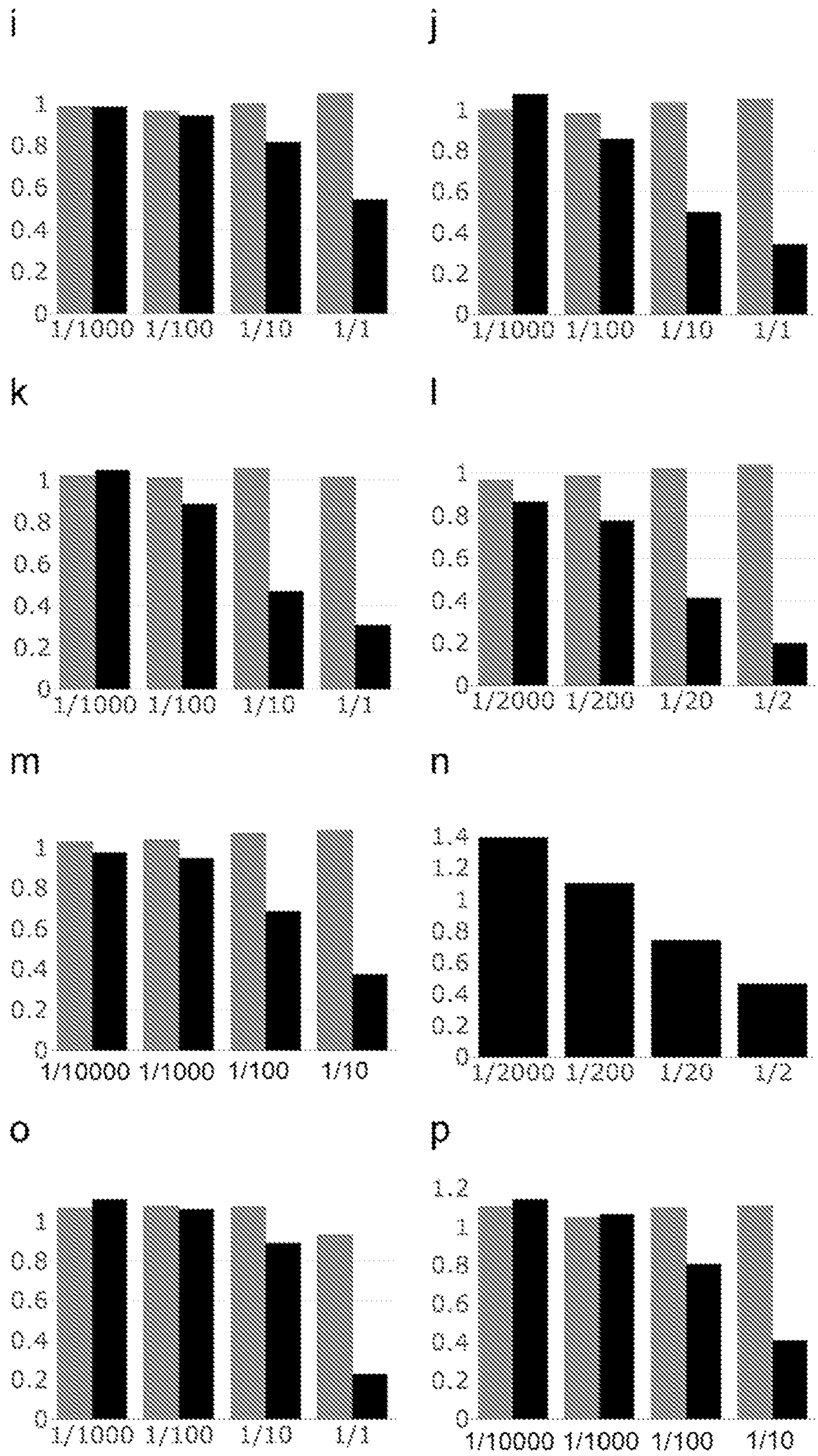

Fig. 24

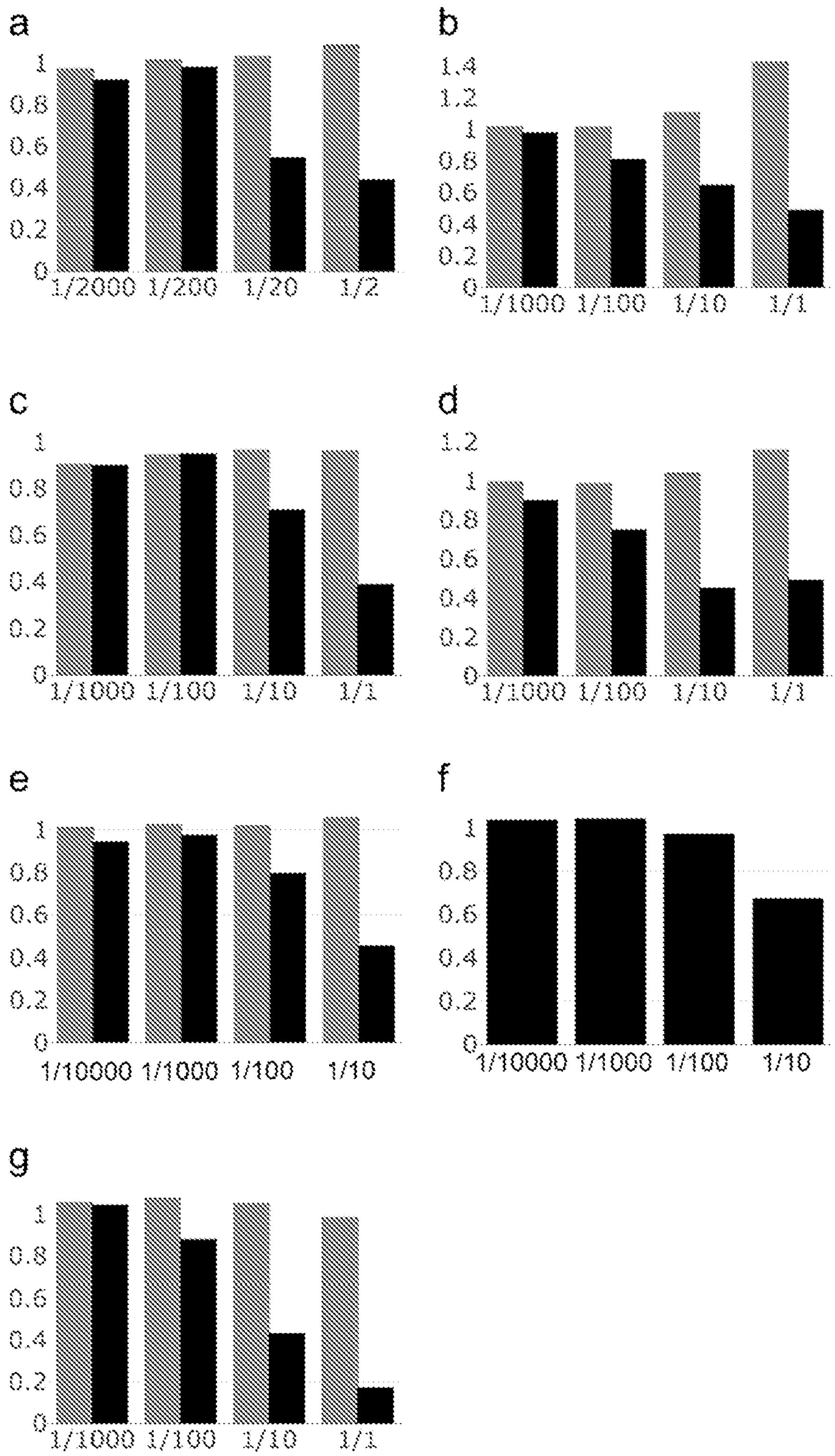
a
1
0.8
0.6
0.4
0.2
0
1/2000
1/200
1/20
1/2
b
1.4
1.2
1
0.8
0.6
0.4
0.2
0
1/1000
1/100
1/10
1/1
c
1
0.8
0.6
0.4
0.2
0
1/1000
1/100
1/10
1/1
d
1.2
1
0.8
0.6
0.4
0.2
0
1/1000
1/100
1/10
1/1
e
1
0.8
0.6
0.4
0.2
0
1/10000
1/1000
1/100
1/10
f
1
0.8
0.6
0.4
0.2
0
1/10000
1/1000
1/100
1/10
g
1
0.8
0.6
0.4
0.2
0
1/1000
1/100
1/10
1/1

Fig. 25

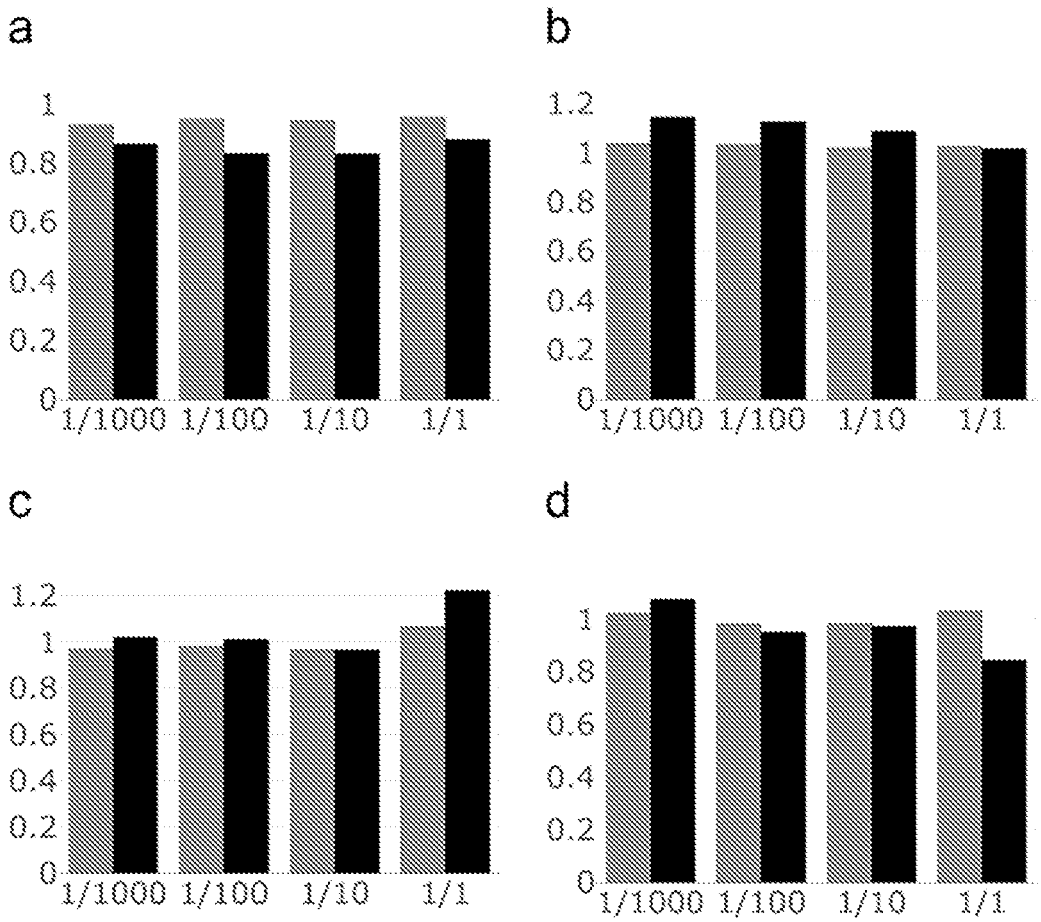
a
1
0.8
0.6
0.4
0.2
0
1/1000 1/100 1/10 1/1
b
1.2
1
0.8
0.6
0.4
0.2
0
1/1000 1/100 1/10 1/1
c
1.2
1
0.8
0.6
0.4
0.2
0
1/1000 1/100 1/10 1/1
d
1
0.8
0.6
0.4
0.2
0
1/1000 1/100 1/10 1/1

Fig. 26

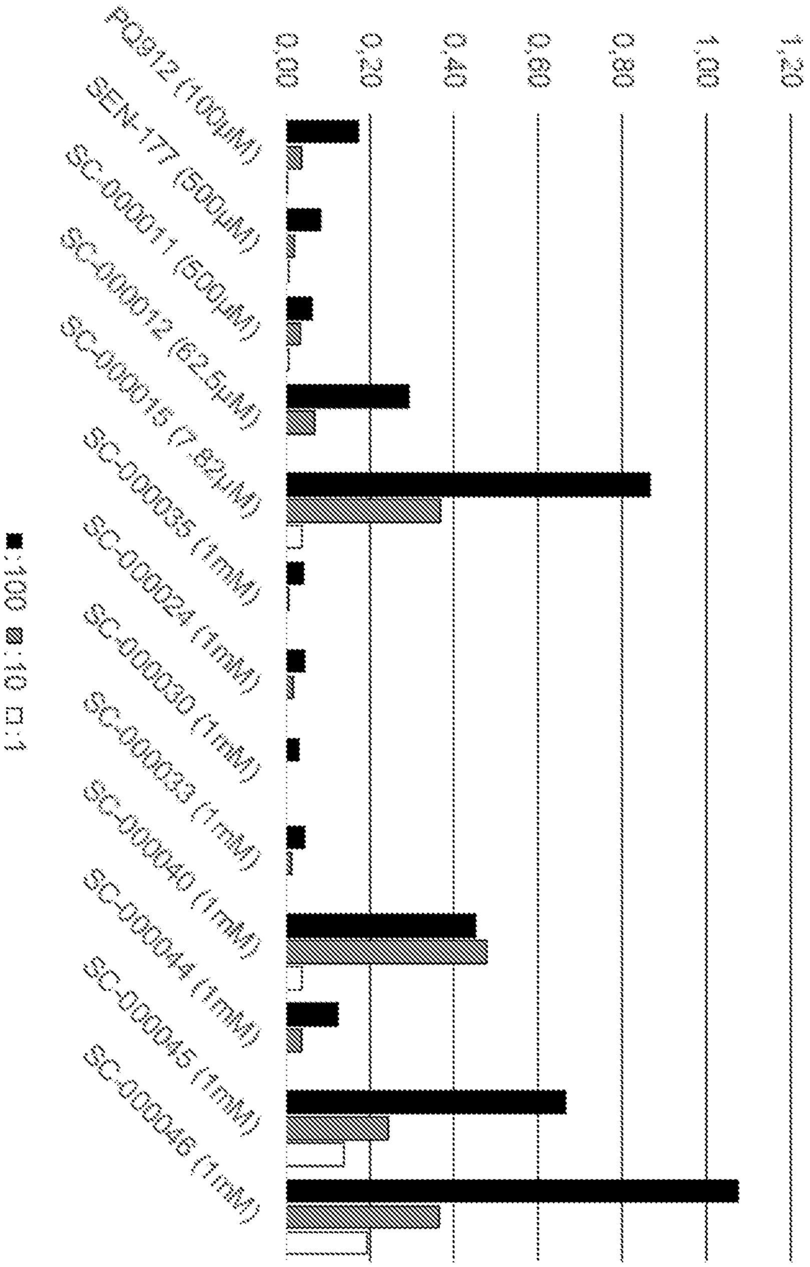
0,00
0,20
0,40
0,60
0,80
1,00
1,20
PQ912 (100µM)
SEN-177 (500µM)
SC-000011 (500µM)
SC-000012 (62,5µM)
SC-000015 (7,82µM)
SC-000035 (1mM)
SC-000024 (1mM)
SC-000030 (1mM)
SC-000033 (1mM)
SC-000040 (1mM)
SC-000044 (1mM)
SC-000045 (1mM)
SC-000046 (1mM)
:100
:10
:1

Fig. 27

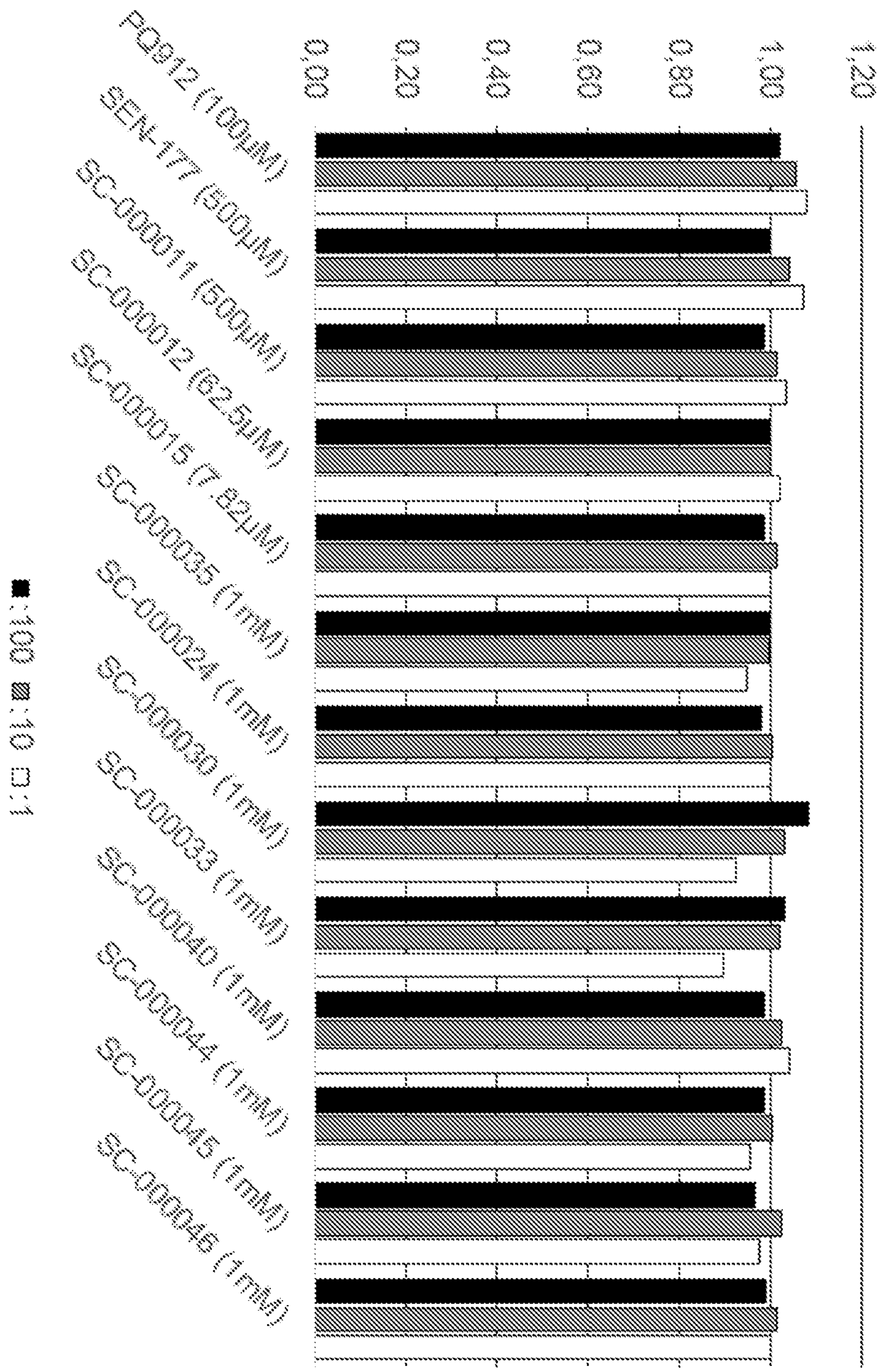

0,00
0,20
0,40
0,60
0,80
1,00
1,20
PQ912 (100μM)
SEN-177 (500μM)
SC-000011 (500μM)
SC-000012 (62,5μM)
SC-000015 (7,82μM)
SC-000035 (1mM)
SC-000024 (1mM)
SC-000030 (1mM)
SC-000033 (1mM)
SC-000040 (1mM)
SC-000044 (1mM)
SC-000045 (1mM)
SC-000046 (1mM)
■ :100 ▨ :10 □ :1

Fig. 28

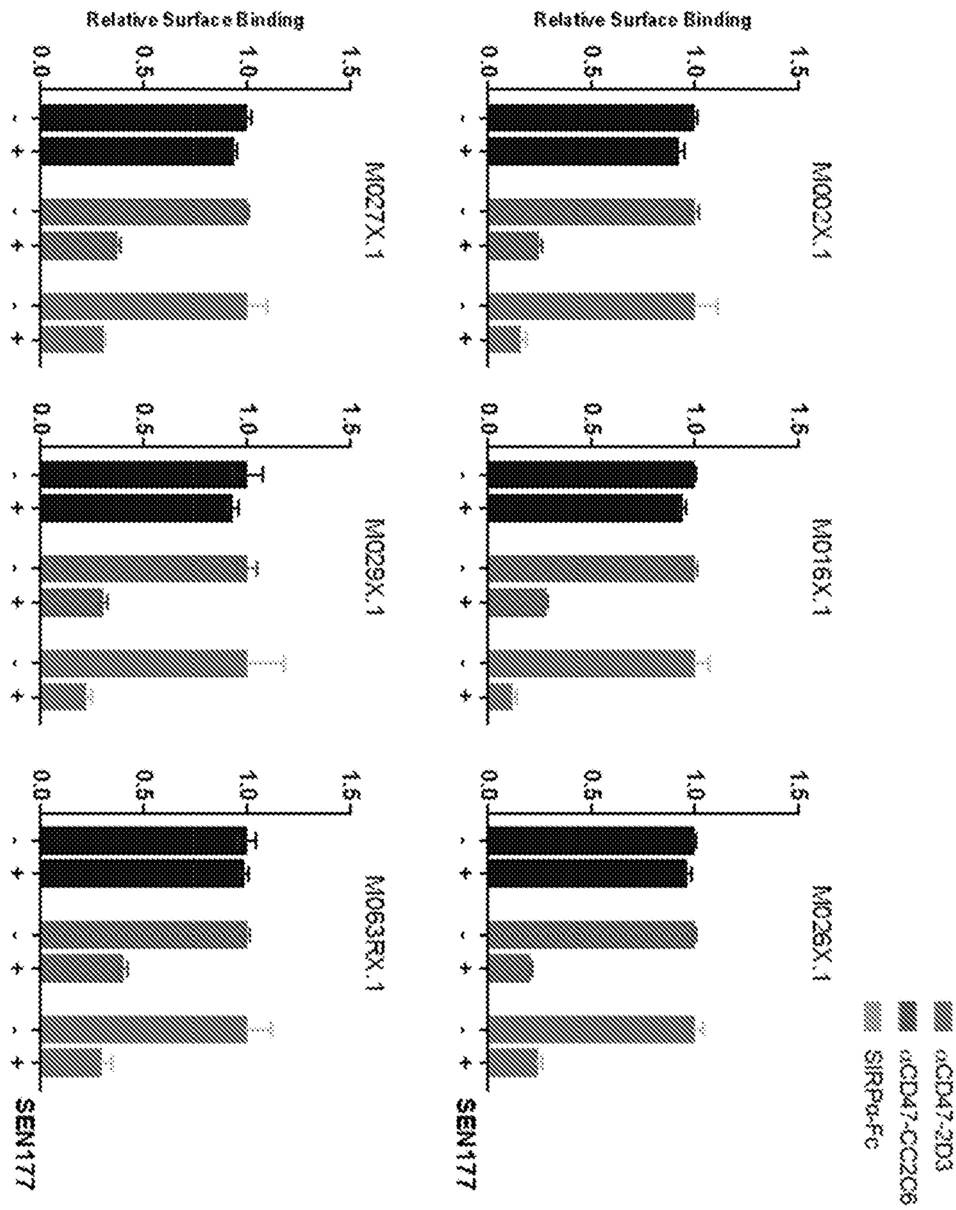

Relative Surface Binding
0.0
0.5
1.0
1.5
MO2X.1
MO16X.1
MO26X.1
MO27X.1
MO29X.1
MO63RX.1
SEN177
αCD47-2D3
αCD47-CC2C6
SIRPα-Fc

Europäisches Patentamt
European Patent Office
Office européen des brevets

# EUROPEAN SEARCH REPORT

Application Number
EP 20 18 3625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HOLGER CYNIS ET AL: "The isoenzyme of glutaminyl cyclase is an important regulator of monocyte infiltration under inflammatory conditions : IsoQC as drug target in inflammatory disorders", EMBO MOLECULAR MEDICINE, vol. 3, no. 9, 1 September 2011 (2011-09-01), pages 545-558, XP055459940, Weinheim ISSN: 1757-4676, DOI: 10.1002/emmm.201100158 * abstract * * page 549, column 2, paragraph 2 * ----- | 1-15 | INV. A61K31/417 A61K39/00 A61K45/00 A61P31/00 A61P9/10 A61P25/28 |
| Y | WO 2008/104580 A1 (PROBIODRUG AG [DE]; SCHILLING STEPHAN [DE]; CYNIS HOLGER [DE]; HOFFMAN) 4 September 2008 (2008-09-04) * abstract; figures; examples * * page 61, last line - page 62, line 5 * ----- | 1-15 | |
| A | WO 2017/079547 A2 (SCRIPPS RESEARCH INST [US]) 11 May 2017 (2017-05-11) * claims * ----- | 1-15 | TECHNICAL FIELDS SEARCHED (IPC) A61P A61K |
| Y | US 2017/081407 A1 (GROSVELD FRANK [NL] ET AL) 23 March 2017 (2017-03-23) * abstract; claims * ----- -/-- | 1-15 | |

The present search report has been drawn up for all claims

| Place of search | Date of completion of the search | Examiner |
|---|---|---|
| Munich | 25 September 2020 | Vandenbogaerde, Ann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

1

Europäisches Patentamt
European Patent Office
Office européen des brevets

# EUROPEAN SEARCH REPORT

Application Number
EP 20 18 3625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YOKO KOJIMA ET AL: "CD47-blocking antibodies restore phagocytosis and prevent atherosclerosis", NATURE, vol. 536, no. 7614, 20 July 2016 (2016-07-20), pages 86-90, XP055459908, GB ISSN: 0028-0836, DOI: 10.1038/nature18935 * abstract * ----- | 1-15 | |
| A | WO 2014/124028 A1 (WEISKOPF KIPP [US]; HASENKRUG KIM J [US]; STODDART CHERYL A [US]; MCCU) 14 August 2014 (2014-08-14) * claims * ----- | 1-15 | |
| A | GERLINDE WERNIG ET AL: "Unifying mechanism for different fibrotic diseases", PROCEEDINGS NATIONAL ACADEMY OF SCIENCES PNAS, vol. 114, no. 18, 19 April 2017 (2017-04-19), pages 4757-4762, XP055459914, US ISSN: 0027-8424, DOI: 10.1073/pnas.1621375114 * abstract; figures 4E,4F * * page 4761, column 2, paragraph 2 * ----- | 1-15 | TECHNICAL FIELDS SEARCHED (IPC) |
| Y | S. M. CHIAVENNA ET AL: "State of the art in anti-cancer mAbs", JOURNAL OF BIOMEDICAL SCIENCE, vol. 24, no. 1, 20 February 2017 (2017-02-20), XP055459924, DOI: 10.1186/s12929-016-0311-y * the whole document * ----- -/-- | 1-15 | |

The present search report has been drawn up for all claims

1

| Place of search | Date of completion of the search | Examiner |
|---|---|---|
| Munich | 25 September 2020 | Vandenbogaerde, Ann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 18 3625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | A. P. A. THEOCHARIDES ET AL: "Disruption of SIRP? signaling in macrophages eliminates human acute myeloid leukemia stem cells in xenografts", BLOOD, vol. 119, no. 18, 3 September 2012 (2012-09-03), pages 4333-1899, XP055126663, ISSN: 0006-4971, DOI: 10.1182/blood-2011-11-391367 * abstract * ----- | 1-15 | |
| Y | WO 2015/138600 A2 (UNIV LELAND STANFORD JUNIOR [US]) 17 September 2015 (2015-09-17) * paragraph [00107] - paragraph [00111] * ----- | 1-15 | |

TECHNICAL FIELDS SEARCHED (IPC)

The present search report has been drawn up for all claims

| Place of search | Date of completion of the search | Examiner |
|---|---|---|
| Munich | 25 September 2020 | Vandenbogaerde, Ann |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 18 3625

This annex lists the patent family members relating to the patent documents cited in the above-mentioned European search report. The members are as contained in the European Patent Office EDP file on
The European Patent Office is in no way liable for these particulars which are merely given for the purpose of information.

25-09-2020

| Patent document cited in search report | Publication date | Patent family member(s) | Publication date |
|---|---|---|---|
| WO 2008104580 A1 | 04-09-2008 | AU 2008220785 A1 | 04-09-2008 |
| | | CA 2679446 A1 | 04-09-2008 |
| | | CN 101668525 A | 10-03-2010 |
| | | EA 200901140 A1 | 30-04-2010 |
| | | EP 2117540 A1 | 18-11-2009 |
| | | EP 2481408 A2 | 01-08-2012 |
| | | IL 200315 A | 31-08-2017 |
| | | JP 5930573 B2 | 15-06-2016 |
| | | JP 2010520168 A | 10-06-2010 |
| | | KR 20090115951 A | 10-11-2009 |
| | | NZ 579310 A | 30-03-2012 |
| | | WO 2008104580 A1 | 04-09-2008 |
| | | ZA 200905537 B | 27-10-2010 |
| WO 2017079547 A2 | 11-05-2017 | US 2018318260 A1 | 08-11-2018 |
| | | WO 2017079547 A2 | 11-05-2017 |
| US 2017081407 A1 | 23-03-2017 | AU 2016326423 A1 | 26-04-2018 |
| | | CA 2999277 A1 | 30-03-2017 |
| | | CN 108290948 A | 17-07-2018 |
| | | EP 3353209 A1 | 01-08-2018 |
| | | GB 2558131 A | 04-07-2018 |
| | | HK 1257309 A1 | 18-10-2019 |
| | | HK 1257310 A1 | 18-10-2019 |
| | | JP 2018535692 A | 06-12-2018 |
| | | US 2017081407 A1 | 23-03-2017 |
| | | US 2017204181 A1 | 20-07-2017 |
| | | US 2018105591 A1 | 19-04-2018 |
| | | US 2018201677 A1 | 19-07-2018 |
| | | US 2020291114 A1 | 17-09-2020 |
| | | WO 2017053423 A1 | 30-03-2017 |
| WO 2014124028 A1 | 14-08-2014 | CA 2900256 A1 | 14-08-2014 |
| | | DK 3311824 T3 | 14-04-2020 |
| | | EP 2953633 A1 | 16-12-2015 |
| | | EP 3311824 A1 | 25-04-2018 |
| | | EP 3721888 A1 | 14-10-2020 |
| | | ES 2649165 T3 | 10-01-2018 |
| | | HR P20200565 T1 | 07-08-2020 |
| | | US 2015376288 A1 | 31-12-2015 |
| | | US 2017362332 A1 | 21-12-2017 |
| | | US 2019092873 A1 | 28-03-2019 |
| | | WO 2014124028 A1 | 14-08-2014 |
| WO 2015138600 A2 | 17-09-2015 | AU 2015229448 A1 | 01-09-2016 |
| | | CA 2939293 A1 | 17-09-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 18 3625

This annex lists the patent family members relating to the patent documents cited in the above-mentioned European search report. The members are as contained in the European Patent Office EDP file on
The European Patent Office is in no way liable for these particulars which are merely given for the purpose of information.

25-09-2020

| Patent document cited in search report | Publication date | Patent family member(s) | Publication date |
| --- | --- | --- | --- |
| | | CN 106456749 A | 22-02-2017 |
| | | EP 3116544 A2 | 18-01-2017 |
| | | JP 6606505 B2 | 13-11-2019 |
| | | JP 2017510251 A | 13-04-2017 |
| | | SG 11201607143U A | 29-09-2016 |
| | | US 2017073414 A1 | 16-03-2017 |
| | | US 2018319883 A1 | 08-11-2018 |
| | | WO 2015138600 A2 | 17-09-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014124028 A
- WO 2004098625 A
- EP 1620082 A
- WO 2004098591 A
- EP 1620091 A
- WO 2005039548 A
- EP 1675578 A
- WO 2005075436 A
- EP 1713780 A
- WO 2011029920 A
- EP 2475428 A
- WO 2014140279 A
- EP 2970235 A
- US 8889709 B2
- WO 2008128983 A
- EP 2160380 A
- WO 2015120350 A


### Non-patent literature cited in the description


- **RING et al.** *Proceedings of the National Academy of Sciences of the United States of America,* 2017, vol. 114 (49), E10578-E10585, http://doi.org/10.1073/pnas.1710877114
- **HO et al.** *The Journal of Biological Chemistry,* 2015, vol. 290 (20), 12650-12663, http://doi.org/10.1074/jbc.M115.648220
- **SOCKOLOSKY et al.** *Proceedings of the National Academy of Sciences of the United States of America,* 2016, vol. 113 (19), E2646-54, http://doi.org/10.1073/pnas.1604268113
- **TREFFERS ; 2017 et al.** *European Journal of Immunology,* vol. 48
- **TREFFERS et al.** *European Journal of Immunology,* 2017, vol. 48
- **TREFFERS et al.** *Eur. J. Immunol.,* 2017, vol. 48 (2), 1-11
- **KEHLEN et al.** *Endocrine-Related Cancer,* 2013, vol. 20, 79-90
- **GILLIS J. S.** *Journal of Translational Medicine,* 2006, vol. 4 (27), 1-7
- **CYNIS et al.** *J, Mol Biol,* 2008, vol. 379, 966-89
- **STEPHAN et al.** *FEBS Journal,* 2009, vol. 276, 6522-36
- **SUYA DAI et al.** *Cellular Immunology,* 2014, vol. 290, 72-79
- **GIANCHECCHI et al.** *Autoimmun. Rev.,* 2013, vol. 12, 1091-1100
- **WEI F et al.** *PNAS,* 2013, vol. 110, E2480-2489
- **GORDON et al.** *Nature,* 2017, vol. 545, 495-499
- **MANGUSO et al.** *Nature,* 2017, vol. 547, 413-418
- **WIERSMA et al.** *Atlas of Genetics and Cytogenetics in Oncology and Haematology,* 2015, vol. 19, 417-431
- **LINDBERG et al.** *Journal of Cell Biology,* 1993, vol. 123, 485-496
- **CHAO et al.** *Current Opinion Immunol,* 2012, vol. 24, 225-3
- **MATLUNG et al.** *Immunol Rev.,* 2017, vol. 276, 145-164
- **MAJETI et al.** *Cell,* 2009, vol. 138, 286-99
- **CHAO et al.** *Curr Opin Immunol,* 2012, vol. 24, 225-32
- **POELS et al.** *J. Natl. Cancer Inst.,* 1986, vol. 76, 781-91
- **BARCLAY ; VAN DEN BERG.** *Annu Rev Immunol,* 2014, vol. 32, 25-50
- **HATHERLEY et al.** *Molecular cell,* 2008, vol. 31, 266-77
- **MATOZAKI et al.** *Trends Cell Biol.,* 2009, vol. 19 (2), 72-80
- **DUBOIS et al.** *Nature Biotechnology,* 2011, vol. 29, 1011-1018
- **OLDENBORG et al.** *Science,* 2000, vol. 288, 2051-2054
- **JAISWAL et al.** *Cell,* 2009, vol. 138, 271-285
- **JAISWAL et al.** *Cell,* vol. 138, 271-285
- **KOJIMA et al.** *Nature,* 2016, vol. 536, 86-90
- **WERNIG et al.** *PNAS,* 2017, vol. 114, 4757-4762
- **CHAO et al.** *Cancer Res.,* 2011, vol. 71, 1374-84
- **CHAO et al.** *Cancer Res.,* 2011, vol. 71, 1374-1384
- **EDRIS et al.** *PNAS,* 2012, vol. 109, 6656-6661

- **WILLINGHAM et al.** *PNAS,* 2012, vol. 109, 6662-6667
- **SARFATI et al.** *Curr Drug Targets,* 2008, vol. 9, 842-50
- **ZAO et al.** *PNAS,* 2011, vol. 108, 18342-18347
- **HO et al.** *J. Biol. Chem,* 2015, vol. 290, 12650-12663
- **SIKIC et al.** *J Clin. Oncol.,* 2016, vol. 34
- **CHHABRA et al.** *Science Translational Medicine,* 2016, vol. 8 (351), 351ra105
- **BARKAL et al.** *Nat Immunol.,* 2018, vol. 19 (1), 76-84
- **BUCHHOLZ M et al.** *J. Med. Chem.,* 2009, vol. 52, 7069-7080
- **BUCHHOLZ M et al.** *J. of Medicinal Chemistry,* 2006, vol. 49, 664-677
- **SCHILLING et al.** *Nature Medicine,* 2008, vol. 14, 1106-1111
- **LUES et al.** *Alzheimer's & Dementia: Translational Research & Clinical Interventions,* 2015, vol. 1, 182-195
- **HOANG et al.** *J. Med. Chem,* 2017, vol. 60, 2573-2590
- **BUCHHOLZ et al.** *J. Med. Chem,* 2006, vol. 49, 664-677
- **BUCHHOLZ et al.** *J. Med. Chem,* 2009, vol. 52, 7069-7080
- **RAMSBECK et al.** *J. Med. Chem,* 2013, vol. 56, 6613-6625
- **HOFFMANN et al.** *The Journal of Pharmacology and Experimental Therapeutics,* 2017, vol. 362, 119-130
- **PICCIONE et al.** *mAbs,* 2015, vol. 7, 946-956
- **WEISKOFT et al.** *Journal of Clinical Investigation,* 2016, vol. 126, 2610-2620
- **NGO et al.** *Cell Reports,* 2016, vol. 16, 1701-1716
- **YOKO KOJIMA et al.** *Nature,* 2016, vol. 536 (7614), 86-90
- **ROSS et al.** *Am Heart J.,* 1999, vol. 138, 419-420
- **WANG et al.** *Circ Res,* 2012, vol. 11 (1), 245-259
- **QUINN et al.** *PNAS,* 1987, vol. 84, 2995-2998
- **ROSENBLOOM et al.** *Ann. Intern. Med.,* 2010, vol. 152, 159
- **WYNN et al.** *Nat. Rev. Immunol.,* 2004, vol. 4, 583
- **BLOMEN et al.** *Science,* 2015, vol. 350, 1092-1096
- **LANGMEAD B. et al.** *Genome Biology,* 2009, vol. 10, R25
- **CARETTE et al.** *Nature,* 2011, vol. 477, 340-343
- **CONG et al.** *Science,* 2013, vol. 339, 819-823
- **LACKNER et al.** *Nature Comm.,* 2015, vol. 6, 10237
- **JAE et al.** *Science,* 2013, vol. 340, 479-483
- **BERKOVITS ; MAYR.** *Nature,* 2015, vol. 522, 363-367
- **SCHILLING et al.** *Nature medicine,* 2008, vol. 14, 1106-1111
- **CARETTE, J.E. et al.** *Nature,* 2011, vol. 477, 340-343
- **NEEFJES, J. J. et al.** *Hum. Immunol,* 1986, vol. 16, 169-181
- **P. BOROSS et al.** *EMBO Mol Med.,* 2013, vol. 5, 1213-1226
- **HAIJ, S. et al.** *Cancer Res.,* 2010, vol. 70, 3209-3217
- **BOROSS, P.** *Haematologica,* 2011, vol. 96, 1822-1830
- **BOROSS, P. et al.** *EMBO Mol Med.,* 2013, vol. 5, 1213-1226
- **STEPHAN et al.** *FEBS journal,* 2009
- **LUES et al.** *Alzheimer's & Dementia,* 2015
- **JIMENEZ-SANCHEZ.** *Nat. Chem. Biol.,* 2015
- **HUANG et al.** *JBC,* 2011, vol. 286, 12439-12449
- **KEMPER et al.** *EMBO Mol. Med.,* 2015, vol. 7, 1104-1118